(19) Europäisches Patentamt
European Patent Office
Office européen des brevets

(11) EP 0 567 026 B1

(12) EUROPEAN PATENT SPECIFICATION

(45) Date of publication and mention of the grant of the patent:
**26.03.2003 Bulletin 2003/13**

(51) Int Cl.[7]: **C07D 267/14**, C07D 413/04, C07D 413/06, C07D 413/12, A61K 31/55, A01N 43/72

(21) Application number: **93106276.4**

(22) Date of filing: **17.04.1993**

(54) **4,1-Benzoxazepin derivatives as squalene synthase inhibitors and their use in the treatment of hypercholesteremia and as fungicides**

4,1-Benzoxazepinderivate als Squalen-Synthetase Inhibitoren und ihre Verwendung zur Behandlung von Hypercholesterämie und als Fungizide

Dérivés de 4,1-benzoxazepine comme inhibiteurs de la synthétase du squalène et leur application dans le traitement d'hypercholesteremie et comme fongicides

(84) Designated Contracting States:
**AT BE CH DE DK ES FR GB GR IE IT LI LU NL PT SE**

(30) Priority: **20.04.1992 JP 9954192**
**21.12.1992 JP 33994792**

(43) Date of publication of application:
**27.10.1993 Bulletin 1993/43**

(73) Proprietor: **TAKEDA CHEMICAL INDUSTRIES, LTD.**
**Chuo-ku, Osaka 541 (JP)**

(72) Inventors:
- **Yukimasa, Hidefumi**
  **Nara 630 (JP)**
- **Tozawa, Ryuichi**
  **Osaka 567 (JP)**
- **Kori, Masakuni**
  **Hyogo 673-05 (JP)**
- **Kitano, Kazuaki**
  **Osaka 590-01 (JP)**

(74) Representative:
**von Kreisler, Alek, Dipl.-Chem. et al**
**Patentanwälte**
**von Kreisler-Selting-Werner**
**Postfach 10 22 41**
**50462 Köln (DE)**

(56) References cited:
**EP-A- 0 142 361**
**EP-A- 0 475 706**
**WO-A-92/15579**
**GB-A- 2 075 012**

- **CHEMICAL AND PHARMACEUTICAL BULLETIN. vol. 34, no. 1, 1986, TOKYO JP pages 140 - 149 MASUOKA Y; ASAKO T; GOTO G; NOGUCHI S 'Syntheses of Medium-Sized Heterocycles using an Intramolecular Michael-Reaction.'**
- **JOURNAL OF MEDICINAL CHEMISTRY. vol. 31, no. 10, October 1988, WASHINGTON US pages 1869 - 1871 S. A. BILLER. ET. AL 'Isoprenoid (Phosphinylmethyl)phosphonates as Inhibitors of Squalene Synthetase'**
- **DATABASE WPI Week 1482, Derwent Publications Ltd., London, GB; AN 82-27355E**

Remarks:
The file contains technical information submitted after the application was filed and not included in this specification

EP 0 567 026 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

FIELD OF THE INVENTION

This invention relates to 4,1-benzoxazepin-2-one derivatives or salts which are useful for inhibiting squalene synthetase and fungal growth, and their use.

BACKGROUND OF THE INVENTION

Hypercholesteremia, high blood pressure and smoking are known as three major dangerous factors of causing ischemic diseases. Adequate control of cholesterol concentration in blood is remarkably important for the prophylaxis or therapy of, besides ischemic diseases, coronary sclerosis.

As pharmaceutical compositions for lowering chlolesterol in blood, attention has been drawn to those for controlling the biosynthesis of cholesterol, besides those of inhibiting its absorption by binding bile acid including, among others, cholestyramine, colestipol (disclosed in, for example, US-A-4027009), and those of suppressing the intestinal absorption of cholesterol by inhibiting acy coenzyme A cholesterol acyl transferase (ACAT) including melinamide (disclosed in FR-A-1476569). As pharmaceutical preparations for controlling the biosynthesis of cholesterol, lovastatin (disclosed in US-A-4231938), simvastatin (disclosed in US-A-4444784), pravastatin (US-A-4346227), which are capable of inhibiting especially 3-hydroxy-3-methyl glutaryl coenzyme (HMG-CoA) reductase, are provided for medicinal use. However, when HMG-CoA reductase is inhibited, not only the biosynthesis of cholesterol but the biosynthesis of some other components such ubiquinone, dolichol and heme A, which are necessary for the living body, is also inhibited, so that occurrences of undesirable side effects to be caused thereby are feared.

Squalene synthetase is the enzyme involved in the committeed step of the de novo cholesterol biosynthetic pathway. This enzyme catalyzes the reductive dimerization of two molecules of famesyl pyrophosphate to form squalene.

On the other hand, the compounds expected as inhibitors of cholesterol biosynthesis by inhibiting squalene synthetase are disclosed in JPA H1(1989)-213288, JPA H2(1990)-101088, JPA H2(1990)-235820, JPA H2(1990)-235821, JPA H3(1991)-20226, JPA H3(1991)-68591, JPA H3(1991)-48288, US-A-5,019,390, Journal of Medicinal Chemistry, 31 (10), p.1869-1871 (1988), US-A-5,135,935 and WO-A-9215579.

Also, the compounds expected as inhibitors of fungal growth by inhibiting squalene synthetase are disclosed in JPA H4 (1992)-279589, EP-A-475706, EP-A-494622 and EP-A-503520.

Among 4,1-benzoxazepine derivatives, in 4,1-benzoxazepin-2-one derivatives in which 2-position is substituted with ketone group, those in which one of the hydrogen atoms at 3-position is replaced with a different substituent, are disclosed in JPA S57(1982)-345765, GB-A-2075012A, and Chem. Pharm. Bull., 34, 140 (1986).

Ubiquinone, dolichol and heme A have been known as being synthesized from farnesyl pyrophophspate along the cholesterol biosynthesis pathwasy. Therefore, for avoiding occurrence of side effects due to loss of these substances, it is desirable to inhibit enzymes subsequent to farnesyl pyrophosphate, especially squalene synthetase, in the cholesterol biosynthetic pathway.

EP-A-0 142 361 discloses N-substituted-5-phenyl-4,1-benzoxazepin-2(3H)ones having therapeutic utility.

SUMMARY OF THE INVENTION

The present inventors found that 4,1-benzoxazepin-2-one derivatives exhibit excellent squalene synthetase inhibitory action and antifungal action, and developed the present invention.

Accordingly, the present invention provides:

(1) a 4,1-Benzoxazepin-2-one derivative represented by the formula (I):

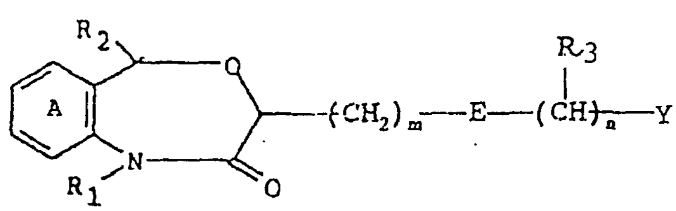

(I)

wherein

R$_1$ is a $C_{1-7}$ alkyl group, which is unsubstituted or substituted with phenyl, an amino group, a thiol group, a hydroxyl group or halogen;

R$_2$ is

(i) hydrogen;

(ii) a phenyl group unsubstituted or substituted by 1 to 3 substituent(s) selected from among

(1) halogen atoms,

(2) $C_{1-4}$ alkyl groups unsubstituted or substituted with 1 to 3 halogen atoms,

(3) $C_{1-4}$ alkoxy groups unsubstituted or substituted with 1 to 3 halogen atoms,

(4) hydroxyl groups unsubstituted or substituted with benzyl, in which the two adjoining substituents on the phenyl group may cooperate therewith to form a ring of methylenedioxy;

(iii) thienyl, and pyridyl;

m is 0, 1 or 2;

n is 0, 1, 2 or 3;

E stands for a bond or an oxygen atom, sulfur atom, sulfone,

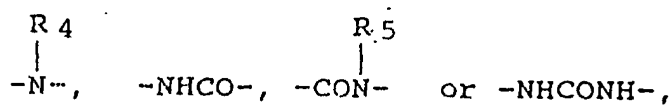

wherein

R$_3$ and R$_5$ independently stand for

(i) a hydrogen atom;

(ii) a $C_{1-6}$ alkyl group unsubstituted or substituted with indolyl;

(iii) benzyl;

(iv) a phenyl group;

R$_4$ stands for a hydrogen atom, a $C_{1-4}$ alkyl, a $C_{1-6}$ alkanoyl group, a $C_{1-3}$ alkanesulfonyl group, or p-toluenesulfonyl;

Y is

(i) an optionally esterified carboxyl group selected from

(a) a carboxyl group, or

(b) $C_{1-5}$ alkoxycarbonyl groups,

wherein the group (b) may have a carboxyl group as substituent(s);

(ii) a hydroxyl group unsubstituted or substituted with a $C_{1-4}$ alkyl group, or benzyl;

(iii) an amino group, unsubstituted or substituted with

(1) $C_{1-4}$ alkyl group(s) or

(2) $C_{3-6}$ cycloalkyl(s),

in which the two substituents on the nitrogen atom may form, taken together with the nitrogen atom, a cyclic amino group of 1-pyrrolidinyl or 1-piperazinyl optionally substituted by 4-halophenyl;

(iv) a phenyl group unsubstituted or substituted with substituents selected from among

(1) $C_{1-4}$ alkyl groups unsubstituted or substituted with a carboxyl group,

(2) a carboxyl group,

(3) phenyl groups unsubstituted or substituted with substituent(s) selected from a carboxyl

group, and
(4) tetrazol-5-yl;

(v) a carbamoyl group unsubstituted or substituted with 1 to 2 substituent(s) selected from among (1) $C_{1-4}$ alkyl groups, benzyl or tetrazol-5-yl, or wherein two substituents on the nitrogen atom cooperate therewith to form the cyclic amino group 1-pyrrolidinyl, 1-piperazinyl or 1- piperazinyl having a $C_{1-3}$ alkyl substituent
(vi) tetrazol-5-yl or 2,5-dihydro-5-oxo-1,2,4-oxadiazol-3-yl;

provided that, when m is 1, E stands for a bond, n is 0, and Y is a carboxyl group, or an alkoxycarbonyl group, then $R_1$ is a $C_{5-7}$ alkyl group; and

ring A being optionally substituted by one to three substituents selected from halogen atoms, or

a pharmaceutically acceptable salt thereof;

(2) A pharmaceutical composition for inhibiting a squalene synthetase which comprises a therapeutically effective amount of a 4,1-Benzoxazepin-2-one derivative according to claims 1-6 or a salt thereof, and a pharmaceutical acceptable carrier;

(3) A pharmaceutical composition for inhibiting a fungal growth which comprises a therapeutically effective amount of a 4,1-Benzoxazepin-2-one derivative according to claims 1-6 or a salt thereof, and a pharmaceutical acceptable carrier.

Further, the present invention discloses methods of producing the novel compounds represented by the formula (I) or salts thereof.
In the above-mentioned formula (I), the $C_{1-7}$ alkyl group shown by $R_1$ is straight-chain or branched alkyl group, exemplified by methyl, ethyl, propyl, isopropyl, butyl, isobutyl, sec-butyl, tert-butyl, pentyl, isopentyl, neopentyl, tert-pentyl, 1-ethylpropyl, hexyl, isohexyl, 1,1-dimethylbutyl, 2,2-dimethylbutyl, 3,3-dimethylpropyl, 3,3-dimethylbutyl, 2-ethylbutyl and 1-ethylpropyl, with preference given to $C_{2-5}$ alkyl groups, more preferably $C_{4-5}$ alkyl groups.
As the substituent of the $C_{1-7}$ alkyl group shown by $R_1$, mention is made of phenyl, an amino groups, a hydroxyl group a thiol group or halogen (e.g. fluorine, chlorine, bromine and iodine). Number of these optional substituents ranges from 1 to 5 (preferably 1 to 3).
In the above mentioned formula (I), the substituents of "optionally substituted phenyl group" shown by $R_2$ are 1-3 halogen atoms (e.g. fluorine, chlorine, bromine or iodine), optionally by 1-3 halogen atoms substituted $C_{1-4}$ alkyl groups (e.g. methyl, ethyl, propyl, isopropyl, butyl and t-butyl), optionally by 1-3 halogen atoms substituted $C_{1-4}$ alkoxy groups (e.g. methoxy, ethoxy, propoxy, isopropoxy, butoxy and t-butoxy), optionally substituted hydroxyl group. Halogen atom means fluorine, chlorine, bromine or iodine. The optionally substitutent of the hydroxyl group, is benzyl, in which the two adjoining substituents on the phenyl group may form a ring of methylene dioxy of the following formula:

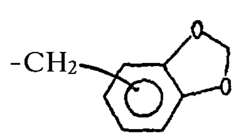

$R_2$ is furthermore thienyl and pyridyl, m is 0, 1 or 2 and n is 0, 1, 2 or 3; E stands for a bond or oxygen atom, sulfur atom, sulfone,

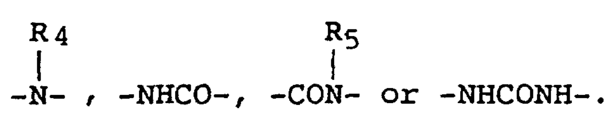

Herein, $R_3$ and $R_5$ independently stand for a hydrogen atom, a $C_{1-6}$ alkyl group (optionally substituted with indolyl), a benzyl group or a phenyl group.
$R_4$ stands for a hydrogen atom, a $C_{1-4}$ alkyl group, a $C_{1-6}$ alkanoyl group, a $C_{1-3}$ alkane-sulfonyl group, or p-toluenesulfonyl group.
The alkyl groups shown by $R_3$ and $R_5$, are $C_{1-6}$ straight-chain or branched alkyl groups (e.g. methyl, ethyl, n-propyl, isopropyl, n-butyl, isobutyl, t-butyl, n-pentyl, isopentyl, neopentyl.

Said alkyl group, may be substituted by indolyl.

In the definition of $R^4$ $C_{1-4}$ alkyl group in e.g. methyl, ethyl, propyl, butyl, t-butyl, and $C_{1-6}$ alkanoyl group is e.g. formyl, acetyl, propionyl, butyryl, isobutyryl, valeryl, isovaleryl, pivaloyl and hexanoyl. $C_{1-3}$ alkanesulfonyl group (mesyl, ethanesulfonyl and propanesulfonyl).

In the defintion of Y $C_{1-5}$ alkoxycarbonyl groups include methoxycarbonyl, ethoxycarboxyl, propoxycarbonyl, isopropoxycarbonyl, butoxycarbonyl, tert-butoxycarbonyl, sec-butoxycarbonyl, pentyloxycarbonyl, isopentyloxycarbonyl, neopentyloxycarbonyl and tert-pentyloxycarbonyl.

Said $C_{1-5}$ alkoxycarbonyl groups may have 1 or more carboxyl substituents at any possible positions.

In the definition of Y as optionally by $C_{1-4}$ alkyl substituted hydroxyl group $C_{1-4}$ alkyl means methyl, ethyl, propyl, isopropyl, butyl and t-butyl.

If Y is a substituted amino group the substituents are e.g. $C_{1-4}$ alkyl group(s) (e.g. methyl, ethyl, propyl, isopropyl, butyl and t-butyl), $C_{3-6}$ cycloalkyl group(s) (cyclopropyl, cyclobutyl, cyclopentyl and cyclohexyl), and a phenyl.

Also, two substituents on a nitrogen atom may cooperate therewith to form a cyclic amino group forming cyclic amino groups which are 1-pyrrolidinyl, or 1-piperazinyl, and 1-piperazinyl having a 4-halophenyl substituent.

If Y is a phenyl optionally substituted by $C_{1-4}$-alkyl groups (e.g. methyl, ethyl, propyl, isopropyl, butyl and t-butyl), $C_{1-4}$ alkyl can optionally be substituted with carboxyl group.

If Y is an optionally substituted carbamoyl group these substituent are selected from $C_{1-4}$ alkyl groups (e.g. methyl, ethyl, propyl, isopropyl, butyl and t-butyl), benzyl and tetrazol-5-yl.

Also, two substituents on a nitrogen atom may cooperate therewith to form a cyclic amino group which is 1-pyrrolidinyl, 1-piperazinyl, or 1-piperazinyl having a $C_{1-3}$ alkyl substituent (e.g. methyl, ethyl, propyl).

Ring A, may be substituted by 1-3 halogen atoms (e.g. fluorine, chlorine, bromine, iodine) preferably one or two, of these substituents.

In the side chain

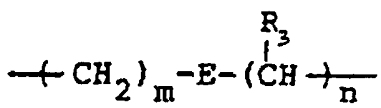

E is preferably a bond or -CONH-. When E is -CONH-, preferably m=1, n=1 or m=2, n=1, especially m=1, n=1. In this case, $R_3$ is preferably hydrogen benzyl and 3-indolyl methyl, especially hydrogen or and 3-indolyl methyl. When E is -CONH- Y is preferably a carboxyl group or an $C_{1-5}$ alkoxycarboxyl group, especially carboxyl group.

Preferably ring A is unsubstituent, or preferably substituted by a chlorine atom.

As salts of the compound (I), mention is made of pharmacologically acceptable salts including inorganic salts such as hydrochloride, hydrobromide, sulfate, nitrate, phosphate, organic acid salts such as acetate, tartrate, citrate, fumarate, maleate, toluensulfonate, methanesulfonate, metal salts such as sodium salt, potassium salt, calcium salt, aluminium salt, and basic salts such as the triethylamine salt, guanidine salt, ammonium salt, hydrazine salt, quinine salt, or cinchonine salt.

The compounds represented by the formula (I) can be produced as follows.

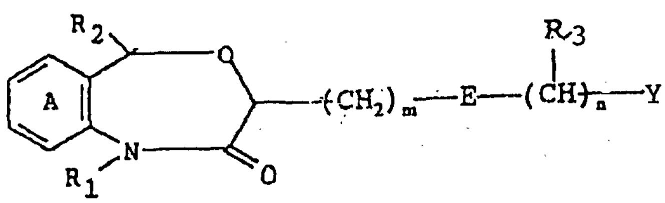

(I)

wherein each of the symbols is of the same meaning as defined above or a pharmaceutically acceptable salt thereof, by subjecting a compound represented by the following formula :

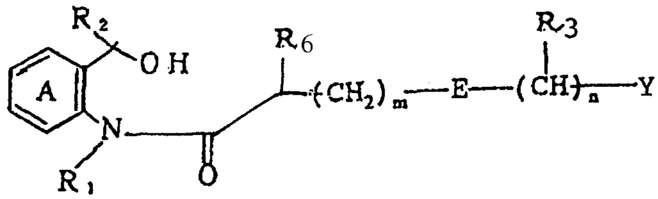

wherein $R_6$ is halogen (e.g. fluorine, chlorine, bromine and iodine) or $-OSO_8CH_9$ and other symbols are of the same meaning as defined above, to a cyclization reaction.

Methods of producing compounds of this invention are described below:

Among the compounds of formula (I) compounds represented by the formula (Ia)

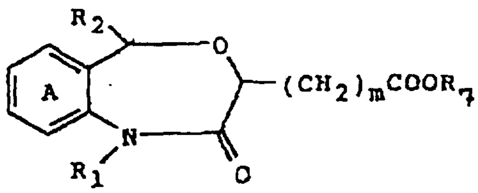

(Ia)

, wherein $R_7$ stands for a $C_{1-4}$ alkyl group, and other symbols are of the same meaning as defined above, can be produced by the following methods.

2-aminobenzophenones as the starting material can be synthesized by, or in accordance with, the method described in D.A. Walsh, Synthesis, 677 (1980) or the method cited in said reference.

[Method A]

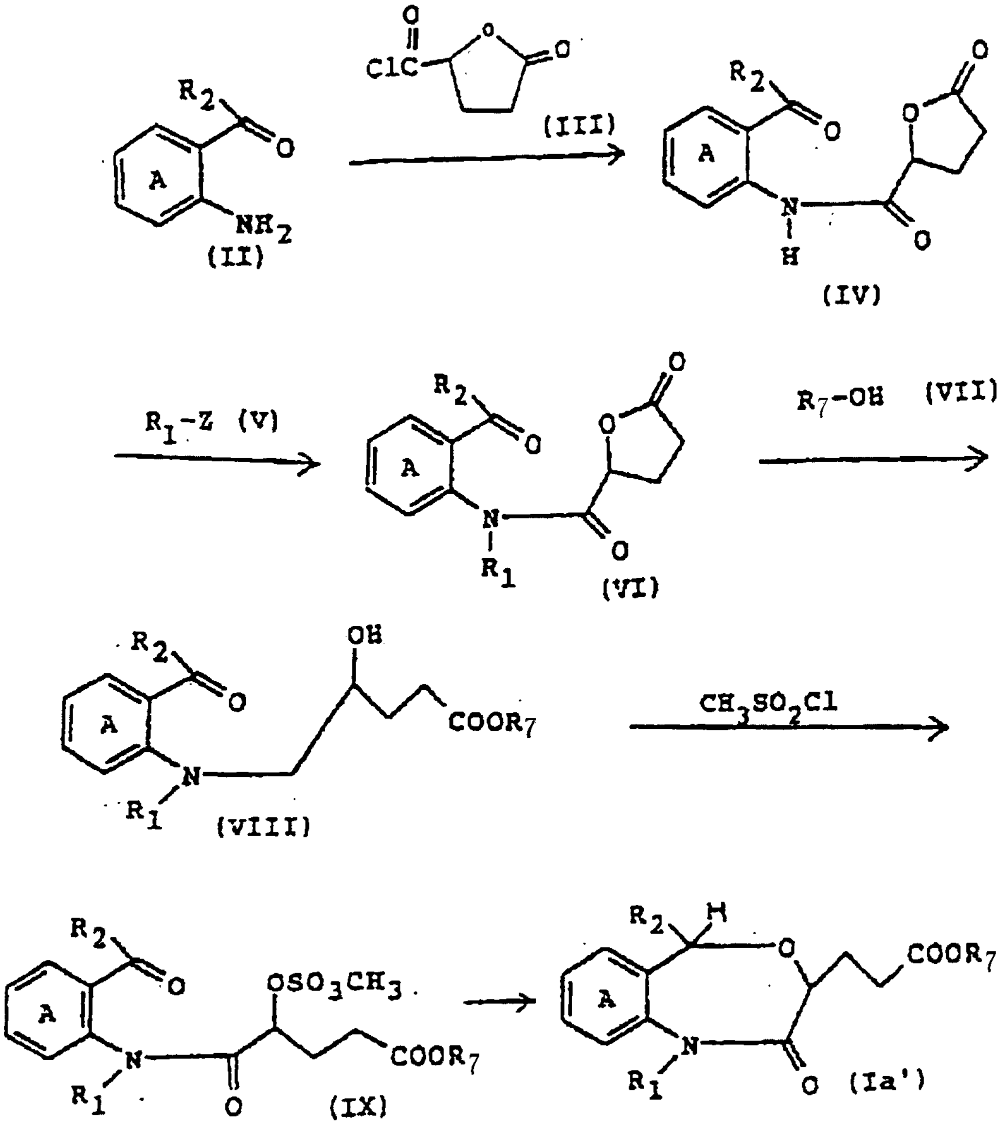


(II) (IV) (VI) (VIII) (IX) (Ia')

[Method B]

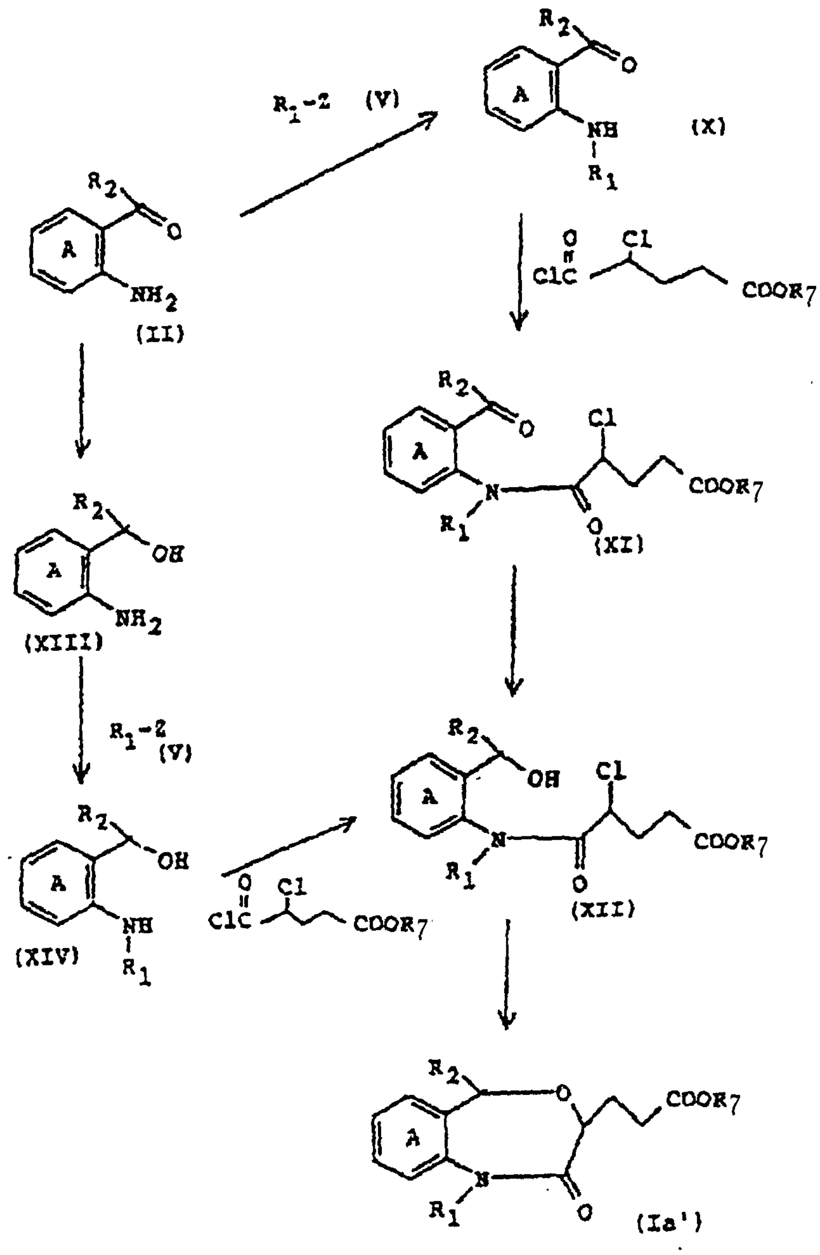

[Method C]

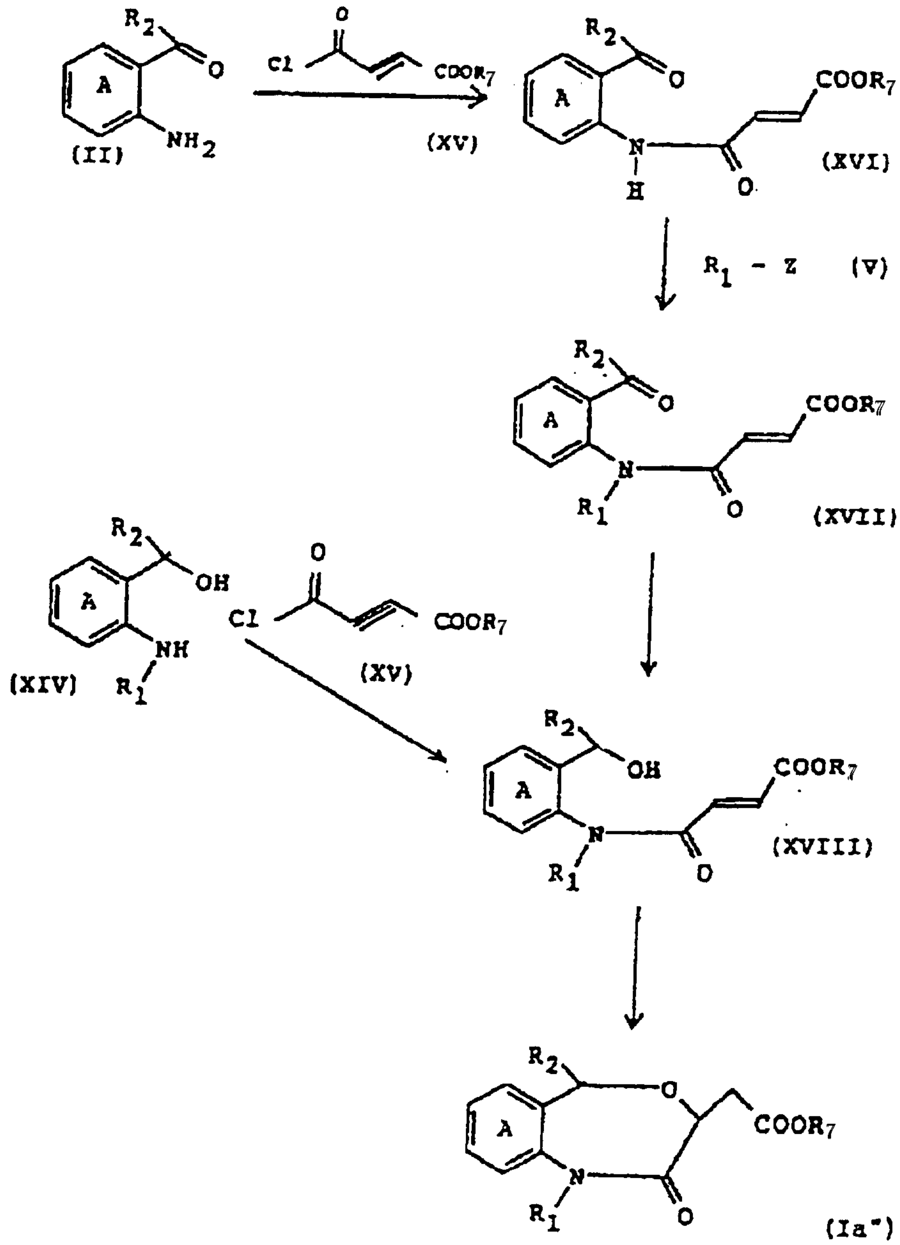

, wherein Z stands for a halogen atom, and other symbols are of the same meaning as defined above.

The reactions from the formula (II) to the formula (IV) and from the formula (VIII) to the formula (IX) in the above-mentioned [Method A] or those from the formula (X) to the formula (XI) and from the formula (XIV) to the formula (XII) in the above-mentioned [Method B] can be carried out by utilizing a per se known acylation reaction. For example, the acylation reaction of this invention can be conducted in a solvent exemplified by an ether-type solvent such as diethyl ether, tetrahydrofuran, dioxane, a halogen-type solvent such as dichloromethane, dichloroethane, chloroform, carbon tetrachloride, a hydrocarbon-type solvent such as benzene, toluene, hexane, heptane, dimethylformamide, dimethyl sulfoxide, and, depending on necessity, in the presence of water and a base (e.g. an organic base such as 4-dimethylaminopyridine, triethylamine, triethylenediamine, tetramethylethylenediamine, an inorganic base such as sodium hydrogencarbonate, potassium hydrogencarbonate, sodium carbonate, potassium carbonate, sodium hydroxide, potassium hydroxide, sodium hydride, potassium hydride) . Relative to one mole of a compound represented by the formulae (II), (VIII), (X) and (XIV), acid chloride and methanesulfonyl chloride by the formula (III) and ester of

4-chloroformyl butyric acid are usually employed in an amount of 1 to 10 mol, preferably 1 to 3 mol. And, the reaction time usually ranges from 1 to 48 hours, preferably from 5 to 10 hours. The reaciton temperature ranges from -50 to 100°C, preferably from 0 to 50°C.

And, the reaction from the formula (IV) to the formula (VI) in [Method A] and the reactions from the formula (II) to the formula (X) and from the formula (XIII) to the formula (XIV) in [Method B] can be carried out in an ether type solvent such as diethyl ether, tetrahydrofuran, dioxane a hydrocarbon type solvent such as benzene, toluene, hexane, heptane, an alcohol type solvent such as methanol, ethanol, propanol, butanol, acetone or dimethylformamide, and, depending on necessity, in the presence of a base (e.g. sodium hydrogencarbonate, potassium hydrogencarbonate, sodium carbonate, potassium carbonate, sodium hydride, potassium hydride). Relative to one mole of a compound represented by the formula (II), the formula (IV) or the formula (XIII), a compound represented by the formula (V) is employed usually in an amount ranging from 1 to 10 mol, preferably 1 to 2 mol. The reaction temperature ranges from 0 to 100°C, preferably from 20 to 50°C. The reaction time ranges usually from 1 to 24 hours, preferably 3 to 10 hours.

And, the reduction reaction of carbonyl group from the formula (IX) to the formula (Ia') in [Method A], from the formula (II) to the formula (XIII) and from the formula (XI) to the formula (XII) in [Method B] can be conducted in a protonic solvent (e.g. methanol, ethanol, propanol, butanol, etc.) or a non-protonic solvent (e.g. ethyl ether, tetrahydrofuran, dioxane, etc.) in the presence of a metal hydrogen complex (e.g. lithium aluminum hydride, sodium aluminum hydride, sodium triethoxyaluminium hydride, sodium borohydride). Relative to one each mol of compounds shown by the formula (IX), the formula (II) and the formula (XI), such a metal hydrogen complex as mentioned above is employed in an amount, usually ranging from 0.3 to 5 mol. equivalents, preferable from 0.5 to 2 mol equivalents. The reaction temperature ranges from -20 to 100°C, preferably from 20 to 50°C.

The cyclization reaction to the formula (Ia') from the formula (XII) in [Method B] can be carried out in a solvent, for example, an ether type solvent such as diethyl ether, tetrahydrofuran, dioxane, a hydrocarbon type solvent such as benzene, toluene, hexane, heptane, an alcohol type solvent such as methanol, ethanol, propanol, butanol, acetone, dimethylformamide in the presence of, depending on necessity, a base (e.g. sodium hydrogencarbonate, potassium hydrogencarbonate, sodium carbonate, potassium carbonate, sodium hydride, potassium hydride). Relative to one mol of a compound shown by the formula (XII), a base as exemplified above is employed in an amount usually ranging from 1 to 5 mol, preferable from 1 to 2 mol. The reaction temperature ranges usually from -20 to 200°C, preferably from 20 to 100°C. The reaction time ranges usually from 1 to 20 hours, preferably from 2 to 5 hours.

The reaction from the formula (VI) to (VIII) in [Method A] can be conducted in an alcohol shown by the formula (VII), and, depending on necessity, in the presence of an inorganic acid such as nitric acid, hydrochloric acid, hydrobromic acid or sulfuric acid, or an organic acid such as toluenesulfonic acid or methanesulfonic acid, at temperatures ranging from -20 to 100°C, preferably from 20 to 50°C. The reaction time ranges from 10 to 100 hours, preferably from 10 to 48 hours.

The compound shown by the formula (Ia") can be produced in accordance with a conventional method.

Among the compounds shown by the formula (I) and (I'), a compound represented by the formula (Ib)

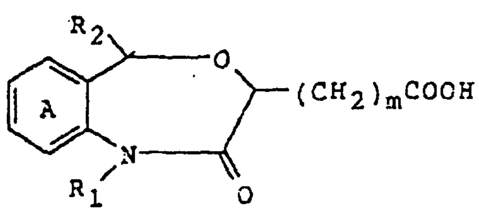

(Ib)

, wherein the symbols are of the same meaning as defined above, can be produced by subjecting a compound shown by the formula (Ia) to hydrolysis, for example, by processing a compound shown by the formula (Ia) with an acid or a base. More specifically, a compound shown by the formula (Ia) is processed in an aqueous solution of a mineral acid (e.g. nitric acid, hydrochloric acid, hydrobromic acid, iodic acid, sulfuric acid ) or an alkali metal hydroxide (sodium hydroxide, potassium hydroxide, barium hydroxide, lithium hydroxide) at temperatures ranging from 0 to 150°C, preferably from 20 to 50°C. The strength of the acid or the base ranges usually from 1 to 10 N, preferably from 4 to 10N. The reaction time varies with the reaction temperature, but usually ranges from 1 to 24 hours, preferably from about 2 to 10 hours.

A compound shown by the formula (Ib')

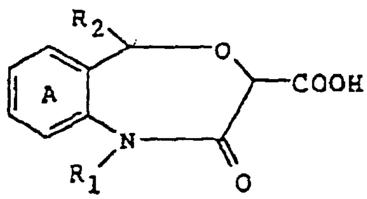

(Ib')

, wherein the symbols are of the same meaning as defined above, can be produced by subjecting a compound shown by the formula (Ic')

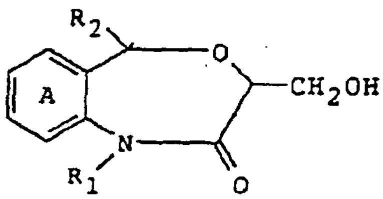

(Ic')

, wherein the symbols are of the same meaning as defined above, to oxidation. The solvent then to be used may be any one so long as it does not hamper the reaction, which is exemplified by acetone, dioxane, tetrahydrofuran, dichloromethane, dichloroethane or chloroform. As the oxidizing agent, permanganate, chromic acid or nickel peroxide can be used, for example. In this case, the oxidizing agent is used in an amount ranging from 0.5 to 20 mol. equivalents, preferably from 1 to 3 mol. equivalents, relative to one mol of the compound shown by the formula (Ic'), the reaction temperature ranges from 0 to 100°C, preferably from 15 to 50°C and the reaction time ranges from 0.5 to 5 hours, preferably from about 1 to 2 hours.

Among the compounds to be used as intermediates for synthesizing a compound of formula (I) or (I'), a compound shown by the formula (XIX)

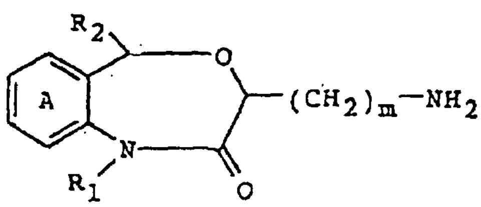

(XIX)

, wherein the symbols are of the same meaning as defined above, can be produced by allowing a compound shown by the formula (Ib) to react with diphenylphosphoryl azide in a solvent in the presence of a base, then by processing the resultant with an acid in a solvent. The solvent to be used in the reaction between a compound shown by the formula (Ib) and diphenylphosphoryl azide may be any one, so long as it does not hamper the reaction, which is exemplified by dimethylformamide, a halogen-type solvent such as dichloromethane, chloroform or dichloroethane and an ether-type solvent such as ether, tetrahydrofuran or dioxane. Typical examples of the base to be employed include triethylamine, 4-dimethylaminopyridine, triethylenediamine and tetramethylethylenediamine. Relative to 1 mol of a compound shown by the formula (Ib), 1 to 10 mol equivalents, preferably 1.5 to 3 mol equivalents, of diphenylphosphoryl azide is employed. The reaction temperature ranges from -20 to 50°C, preferably from 0 to 20°C, and the reaction time ranges from 0.5 to 5 hours, preferably from about 1 to 2 hours.

In the case of processing the product obtained by the above-described reactions, the solvent to be used is exemplified by water, dioxane, dimethylformamide and, as the acid to be employed, a mineral acid such as sulfuric acid, hydrochloric acid, nitric acid or hydrobromic acid can be exemplified. The reaction temperature ranges from 20 to 200°C, preferably from about 50 to 100°C, and the reaction time ranges fro 0.5 to 5 hours, preferably from 1 to 2 hours.

Among the compounds represented by the formula (I) and (I'), a compound shown by the formula (Ic)

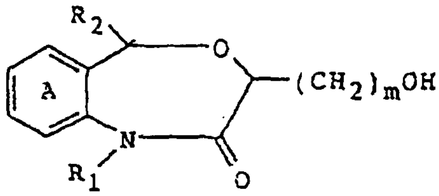 (Ic)

, wherein the symbols are of the same meaning as defined above, can be produced by subjecting a compound shown by the formula (Ia) to reduction. More specifically, the compound shown by the formula (Ic) can be produced by processing the compound shown by the formula (Ia) with a metal hydride complex (e.g. lithium aluminum hydride, sodium aluminum hydride, sodium borohydride) in a protonic solvent (e.g. methanol, ethanol,propanol, butanol) or a non-protonic solvent (e.g. ethyl, ether, tetrahydrofuran, dioxane) Relative to one mol of the compound shown by the formula (Ia), such a metal hydride complex compound is used in an amount ranging usually from 0.3 to 5 mol equivalents, preferably from 0.5 to 2 mol equivalents. The reaction temperature ranges from -20°C to 100°C, preferably from 0 to 20°C. The reaction time ranges from 0.5 to 10 hours, preferably from 1 to 3 hours.

And, a compound shown by the formula (Ic) can be produced also by converting the amine portion of a compound shown by the formula (XIX) into hydroxyl group. For example, a compound of the formula (Ic) can be produced by adding sodium nitrite to a compound of the formula (XIX) in a solvent in the presence of an acid then by processing the resultant azide compound in a solvent in the presence of a base. In the method of azidation, for example, 0.5 to 3, preferably 1 to 1.5, mol equivalents of sodium nitrite, is employed relative to 1 mol of a compound of (XIX). As the acid, any one can be employed so long as it does not hamper the reaction, typically exemplified by acetic acid and sulfuric acid. The reaction temperature ranges form -20 to 20°C, preferably 0 to 5°C, and the reaction time ranges from 5 to 60 minutes, preferably 10 to 30 minutes. The method of converting the azide of thus-obtained azide compound into hydroxyl group comprises, for example, use of, as the base, for example, sodium hydrogencarbonate, potassium hydrogencarbonate, sodium carbonate, potassium carbonate, sodium hydroxide, potassium hydroxide in water or an aqueous organic solvent (e.g. an alcohol-type solvent such as methanol, ethanol, propanol, butanol, an ether-type solvent such as tetrahydrofuran, dioxane, dimethylformamide). The reaction temperature ranges from 20 to 200°C, preferably from 50 to 100°C, and the reaction time ranges from 5 minutes to 2 hours, preferably from 15 to 30 minutes.

Among the compounds represented by the formula (I) and (I'), a compound shown by the formula (Id)

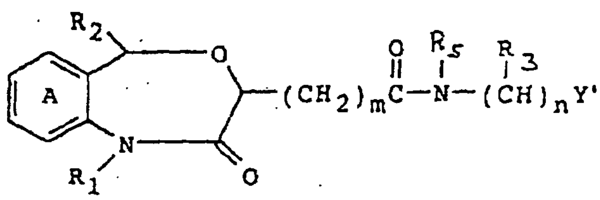 (Id)

, wherein Y' stands for, among the definitions given to Y as above, esterified carboxyl group and hydroxyl group, and other the symbols are of the same meaning as defined above, can be produced by subjecting a compound shown by the formula (Ib) to condensation with a compound represented by the formula (XXI)

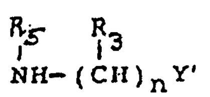 (XXI)

, wherein the symbols are of the same meaning as defined above, more specifically, the compound (Ib) and the compound (XXI) are subjected to condensation in a solvent by using a condensing agent, and, upon necessity, in the presence of a base. The solvents to be employed are exemplified by hydrocarbon-type solvents such as benzene, toluene, hexane, heptane halogen-type solvents such as dichloromethane, dichloroethane, chloroform, carbon tetrachloride, ether-type solvents such as ethyl ether, tetrahydrofuran, dioxane, acetonitrile, dimethylformamide. As the base, use is made of e.g. triethylamine, 4-dimethyl aminopyridine, triethylenediamine and tetramethyl ethylenediamine. As condensing agents, mention is made of those to be employed for peptide synthesis, which are exemplified by cyclohexyl carbodiimide, diethyl cyanophosphate and 1-ethyl-3-(3-dimethylaminopropyl)carbodiimide. Relative to one mol of the compound shown by the formula (Ib), a compound represented by the formula (XXI) is used in an amount ranging from 0.5 to 2 mol equivalents, preferably from 1 to 1.2 mol equivalent, and a condensing agent is used in an amount ranging from 0.5 to 5 mol equivalents, preferably 1 to 2 mol equivalents. The reaction temperature ranges from

0 to 100°C, preferably from 20 to 50°C, and the reaction time ranges from 0.5 to 24 hours, preferably from 1 to 5 hours.

Among the compounds represented by the formula (I) and (I'), a compound shown by the formula (Ie)

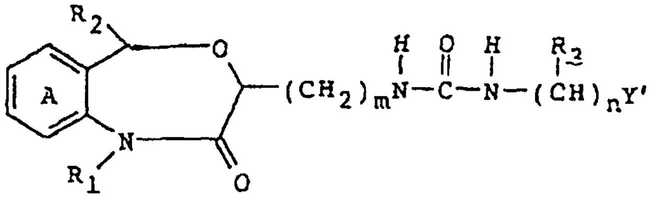

(Ie)

, wherein the symbols are of the same meaning as defined above, can be produced by allowing a compound (Ib) to react with diphenyl phosphoryl azide in a solvent in the presence of a base, then by allowing the resultant compound to react with a compound (XXI). As the solvent to be employed in the reaction between the compound (Ib) and diphenyl phosphoryl azide, any one can be used so long as it does not hamper the reaction, which is exemplified by dimethylformamide or a halogen-type solvent such as dichloromethane, chloroform, dichloroethane, and an ether-type solvent such as ether, tetrahydrofuran, dioxane. The base to be employed is typically exemplified by triethylamine, 4-dimethyl aminopyridine, triethylene diamine or tetramethyl ethylenediamine. Relative to one mol of the compound shown by the formula (Ib), 1-10, preferably about 1.5 to 3 mol equivalents of diphenyl phosphoryl azide is employed. The reaction temperature ranges from -20 to 50°C, preferably from 0 to 20°C, and the reaction time ranges from 0.5 to 5 hours, preferably from 1 to 2 hours.

The solvent to be employed in the reaction between the compound obtained thus above and a compound shown by the formula (XXI) is exemplified by a halogen-type solvent such as dichloromethane, dichloroethane, chloroform, an ether-type solvent such as ether, tetrahydrofuran, dioxane, acetonitrile, dimethylformamide. And, depending on necessity, a base is employed. As the base, an organic base including triethylamine, 4-dimethyl aminopyridine, triethylenediamine and tetramethyl ethylenediamine can be used. Relative to one mol of the compound (Ib), 0.5 to 3, preferably 1 to 1.5, mol equivalents of the compound (XXI). The reaction temperature ranges from 0 to 150°C, preferably from 30 to 100°C, and the reaction time ranges from 0.5 to 24, preferably 1 to 3, hours.

Among the compounds represented by the formula (I) and (I'), a compound shown by the formula (If)

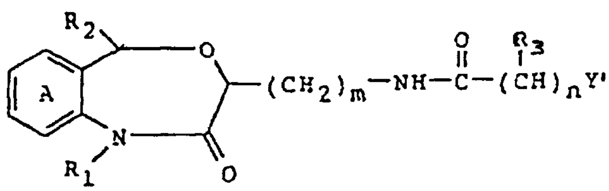

(If)

, wherein the symbols are of the same meaning as defined above, can be produced by subjecting a compound of (XIX) to condensation with a compound represented by the formula (XXII)

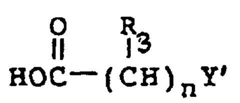

(XXII)

, wherein the symbols are of the same meaning as defined above. This reaction can be conducted in completely the same manner as in the production of the compound (Id).

Among the compounds represented by the formula (I) and (I'), a compound shown by the formula (Ig)

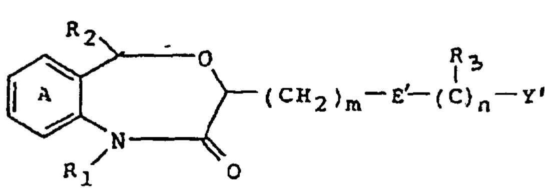

(Ig)

, wherein E' stands for, among the definitions given above,oxygen atom or -NH-, and other the symbols are of the same meaning as defined above, can be produced by allowing a compound shown by the formula (Ic) or a compound shown by the formula (XIX) to react with a compound represented by the formula (XXIII)

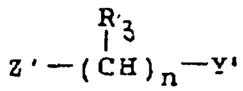

(XXIII)

, wherein Z' is a halogen atom (Chloro, bromo, iodo) and the other symbols are of the same meaning as defined above. More specifically, the compound (Ic) or the compound (XIX) is allowed to react with the compound (XXIII) in an alcohol-type solvent such as methanol, ethanol, propanol, butanol, an ether-type solvent such as diethyl ether, tetrahydrofuran, dioxane or dimethylformamide , in the presence of a base including an inorganic base such as sodium hydrogencarbonate, potassium hydrogencarbonate, sodium carbonate, potassium carbonate, an organic base such as triethylamine, 4-dimethyl aminopyridine, triethylenediamine, tetramethyl ethylenediamine or sodium hydride. Relative to one mol of the compound (Ic) or (XIX), 0.5 to 1.5 mol equivalent of the compound (XXIII) is used, and, relative to one mol of the compound (Ic) or (XIX), 1 to 5, preferably 1 to 2, mol equivalents of the base is used. The reaction temperature ranges from 0 to 200°C, preferably from 20 to 100°C, and the reaction time ranges from 0.5 to 24 hours, preferably from 1 to 3 hours.

Among the compounds represented by the formula (I) and (I'), a compound shown by the formula (Ih)

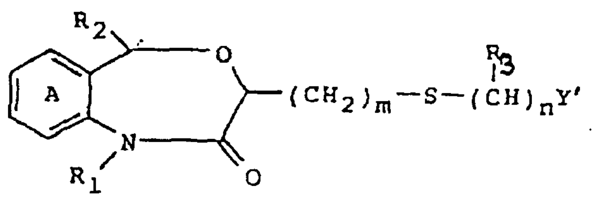

(Ih)

, wherein the symbols are of the same meaning as defined above, can be produced by allowing a compound (XX) to react with a compound represented by the formula (XXV)

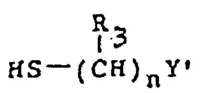

(XXV)

, wherein the symbols are of the same meaning as defined above. The solvent to be employed is a non-protonic solvent exemplified by ethyl ether, tetrahydrofuran, dioxane, acetonitrile or dimethylformamide. Depending on necessity, an inorganic base such as sodium hydrogencarbonate, potassium hydrogencarbonate, sodium carbonate or potassium carbonate, an organic base such as triethylamine, 4-dimethyl aminopyridine, triethylenediamine or tetramethyl ethylenediamine, sodium hydride or, cesium fluoride may be used. Relative to one mol of the compound (XX), 0.5 to 5, preferably 1 to 2, mol equivalents of the compound (XXV) is employed. The reaction temperature ranges from 0 to 200°C, preferably 20 to 100°C, and the reaction time ranges from 10 minutes to 5 hours, preferably from 30 minutes to 2 hours.

Among the compounds represented by the formula (I) and (I'), a compound shown by the formula (Ii)

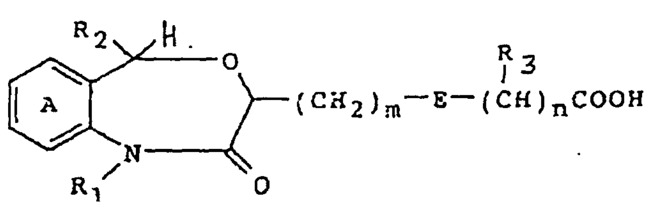

(Ii)

, wherein the symbols are of the same meaning as defined above, can be produced by subjecting a compound represented by the formula (Ij)

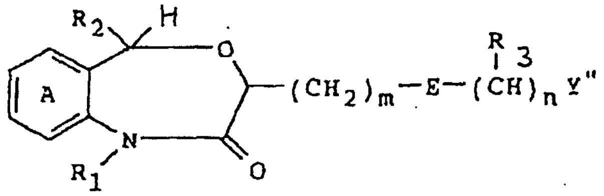

(Ij)

, wherein Y" stands for, among the groups defined by Y as above, esterified carboxyl group, and other the symbols are of the same meaning as defined above, to hydrolysis. More specifically, the hydrolysis is conducted in a solvent such as water, methanol, ethanol, propanol or butanol in the presence of an alkali metal hydroxide (e.g. sodium hydroxide, potassium hydroxide, barium hydroxide or lithium hydroxide), sodium hydrogencarbonate, potassium hydrogencarbonate, sodium carbonate or potassium carbonate, or, in the presence of a mineral acid (e.g. nitric acid, hydrochloric acid, hydrobromic acid, iodic acid or sulfuric acid) or trifluoroacetic acid, at temperatures ranging from 10 to 150°C, preferably from 10 to 50°C. While the reaction time varies with the reaction temperature, it ranges usually from 1 to 24 hours, preferably from 2 to 10 hours.

While the compound represented by the formula (I ) of this invention has a squalene synthetase inhibiting action and an antifungal action, among the compounds used in the present invention, there are compounds capable of inhibiting other enzymes in the pathway of cholesterol biosynthesis. Be the matter as it may, the compound represented by the formula (I") of this invention inhibits biosynthesis of cholesterol, which is useful for the prophylaxis or therapy of hypercholesteremia or coronary sclerosis of mammals (e.g. mouse, rat, rabbit, dog, cat, cow, pig and human being) and, further, for the prophylaxis or therapy of fungus infections.

Said compound (I ) can be administered to man orally or non-orally. The orally administrable compositions may be in a solid or liquid form, more specifically tablets (including sugar-coated tablets and film-coated tablets), syrups, emulsions or suspensions. These compositions can be prepared by a per se known method and contain carriers or excipients conventionally used in the field of pharmaceutical preparation, for example, carriers or excipients such as lactose, starch, sucrose or magnesium stearate for preparing tablets.

The non-orally administrable compositions are exemplified by injections and suppositories, and the injections include hypodermic injections, intradermal injections and intramuscular injections. These injections can be prepared by a per se known method, more specifically, by suspending or emulsifying the compound of this invention in a sterile water or oil conventionally used for preparing injectable compositions. The aqueous liquid to be used for preparation of injections include physiological saline solution and isotonic solution, and, depending on necessity, a suitable suspending agent such as sodium carboxymethyl cellulose, a non-ionic surfactant or the like may be jointly used. As the oil, mention is made of sesame oil, soybean oil, and benzyl benzoate, benzyl alcohol as a solubilizer may be jointly used. Injections thus prepared are, in general, filled in appropriate ampoules.

The compound represented by the formula (I ) or a salt thereof can be used safely with low toxicity. Although the daily dose varies depending on the condition and weight of the patient, kind of the compound, route of administration and other factors, for example, in the case of administering the compound of the present invention for the therapy of hypercholesteremia, a daily oral dosage per adult human is 1 to 500 mg, preferably 10 to 200 mg. Within this range, no toxicity is observed at all.

The compound of the formula (I ) for squalene synthetase inhibition is effectively administered to a mammal, e.g. human, in a therapeutically effective amount which is generally provided with an oral daily dosage per adult of from 1 to 500mg, preferably 10 to 200mg. In terms of a non-oral daily dosage range such as for an injection or suppository, a therapeutically effective amount is generally in the range of from 0.1 to 100mg, preferably 1 to 20mg.

The present invention compounds also demonstrate broad spectrum antifungal activity as determined by broth or agar dilution methods.

In case of administering the compound of the present invention for the therapy of fungus infections, generally from 2 to 5 mg/kg should be employed as a unit dosage in an antifungal treatment.

The compound of the formula (I ) for fungus treatment is effectively administered to a mammal, e.g. human, in a therapeutically effective amount which is generally provided with an oral daily dosage per adult of from 0.1 to 100mg, preferably 1 to 50mg. In terms of a non-oral daily dosage range such as for an injection or suppository, a therapeutically effective amount is generally in the range of from 0.1 to about 100mg, preferably 1 to 50mg.

Abbreviations for amino acids, and other used in the present specification are based on abbreviations specified by the IUPAC-IUB Commission on Biochemical Nomenclature or abbreviations in common use in relevant fields. Some examples are given below. When an optical isomer may be present in amino acid, it is of the L-configuration, unless

otherwise stated.

| | |
|---|---|
| Trp or Y | Tryptophan |
| Ser or S | Serine |
| Asp or D | Aspartic acid |
| Glu or E | Glutamic acid |
| Me | Methyl |
| Et | Ethyl |
| Ph | Phenyl |

[Examples]

The following examples, reference examples, formulation examples and test examples are intended to illustrate the present invention in further detail.

In the following description, two types of racemic diastereomers are obtained depending on the kinds of compounds, which is due to the presence of asymmetric carbon atoms at 3- and 5-positions. Isomers in which the substituents at 3- and 5-positions are oriented in the same direction relative to the face of 7-membered ring are named cis-isomers, while those in which the substituents at 3- and 5-positions are oriented in the adverse directions to each other are named trans-isomers.

Reference Example 1

Trans-7-chloro-5-(2-chlorophenyl)-1,2,3,5-tetrahydro-1-methyl-2-oxo-4,1-benzoxazepine-3-acetic acid ethyl ester

(1) Ethyl 3-[N-[2-(2-chlorobenzoyl)-4-chlorphenyl] carbamoyl]acrylate

To a suspension of 5.0 g of 2-amino-5-chloro-2'-chlorobenzophenone and 2.5 g of sodium hydrogencarbonate in 100 ml of methylene chloride was added dropwise 3.3 g of monoethyl ester of fumaric acid semi-chloride in the course of 30 minutes. The mixture was stirred for 2 hours at room temperature, to which was then added water, followed by shaking. The organic layer was separated, which was dried over anhydrous sodium sulfate, followed by distilling off the solvent under reduced pressure to leave 4.8 g of 3-[N-[2-(2-chlorobenzoyl)-4-chlorophenyl] carbamoyl] acrylic acid ethyl ester as crystals, m.p. 113-114°C.

| Elemental Analysis for $C_{19}H_{15}Cl_2NO_4$: | | | |
|---|---|---|---|
| Calcd. | C 58.18; | H 3.85; | N 3.57 |
| Found | C 58.27; | H 3.88; | N 3.48 |

(2) Ethyl ester of 3-[N-[2-(2-chlorobenzoyl)-4-chloro phenyl]-N-methylcarbamoyl]acrylic acid

To a suspension of 0.15 g of sodium hydride in 20 ml of N,N-dimethylformamide was added, while stirring under ice-cooling, 3.0 g of the crystals obtained above. The mixture was stirred for 10 minutes at 0°C, to which was added 2.0 g of methyl iodide, then the mixture was stirred for 2 hours at room temperature. The reaction mixture was poured into ice-water, which was subjected to extraction with ethyl acetate. The ethyl acetate layer was washed with water and dried, from which the solvent was distilled off under reduced pressure to leave 2.8 g of ethyl ester of 3-[N-[2-(2-chlorobenzoyl)-4-chlorophenyl]-N-methyl carbamoyl] acrylic acid as an oily product.
$IR\nu_{max}^{Neat} cm^{-1}$ : 1725(C=O), 1670(C=O), 1490, 1370, 1300, 1180, 1115, 920, 740
$^1$H-NMR spectrum (200MHz, $CDCl_3$) δ : 1.27(3H,t,$CH_2$ $\underline{CH_3}$), 3.18(3H,s,-N $\underline{CH_3}$), 4.18(2H,q, $\underline{CH_2}$ $CH_3$), 6.78(2H,d, -CH=CH-), 7.1-7.7(7H,m)

(3) Ethyl ester of 3-[N-[4-chloro-2-(a-hydroxy-2-chlorobenzyl)phenyl]-N-methylcarbamoyl] acrylic acid

In 50 ml of methanol was dissolved 2.8 g of the oily product obtained above, to which was added 0.2 g of sodium borohydride, and the mixture was stirred for 30 minutes at room temperature. The reaction mixture was concentrated under reduced pressure, which was subjected to extraction with ethyl acetate. The organic layer was washed with water, dried and subjected to distillation under reduced pressure. Crystallization of the residue afforded 2.4 g of ethyl ester of 3-[N-[4-chloro-2-(a-hydroxy-2-chlorobenzyl)phenyl]-N-methylcarbamoyl] acrylic acid, m.p. 128-130°C.

| Elemental Analysis for $C_{20}H_{19}Cl_2NO_4.1/4H_2O$: | | | |
|---|---|---|---|
| Calcd. | C 58.19; | H, 4.76; | N 3.39 |
| Found | C 58.38; | H, 4.79; | N 3.31 |

(4) Ethyl trans-7-chloro-5-(2-chlorophenyl)-1,2,3,5-tetrahydro-1-methyl-2-oxo-4,1-benzoxazepine-3-acetate

In 30 ml of ethanol was dissolved 2.2 g of the crystals obtained above, to which was added 1.5 g of potassium carbonate, and the mixture was stirred for 3 hours at room temperature. The reaction mixture was concentrated under reduced pressure. To the concentrate was added water, which was subjected to extraction with ethyl acetate. The organic layer was washed with water and dried, followed by distilling off the solvent under reduced pressure. The residue was purified by means of a silica gel chromatography (eluent, hexane:ethyl acetate = 3:1) to afford 1.85 g of ethyl ester of trans-7-chloro-5-(2-chlorophenyl)-1,2,3,5-tetrahydro-1-methyl-2-oxo-4,1-benzoxazepine-3-acetic acid as crystals, m.p. 147-148°C.

| Elemental Analysis for $C_{20}H_{19}Cl_2NO_4$: | | | |
|---|---|---|---|
| Calcd. | C 58.84; | H 4.69; | N 3.43 |
| Found | C 58.76; | H 4.79; | N 3.28 |

Reference Example 2

Ethyl trans-7-chloro-5-(2-chlorophenyl)-1-cyclohexylmethyl-1,2,3,5-tetrahydro-2-oxo-4,1-benzoxazepine-3-acetate

(1) N-cyclohexylmethyl-4-chloro-2-[α-hydroxy-(2-chlorophenyl)methyl]aniline

In 50 ml of glacial acetic acid was dissolved 5.0 g of 4-chloro-2-[α-hydroxy-(2-chlorophenyl)methyl]aniline. To the solution was added 2.5 g of cyclohexane carboxaldehdye. To the mixture was added, while stirring under ice-cooling, 1.06 g of sodium borohydride in the course of 40 minutes. The reaction mixture was poured into ice-water, which was subjected to extraction with ethyl acetate. The organic layer was washed with a dilute aqueous solution of sodium hydroxide, then with water, followed by drying. The solvent was distilled off under reduced pressure to leave 6.2 g of N-cyclohexylmethyl-4-chloro-2-[α-hydroxy-(2-chlorophenyl)methyl]aniline as crystals, m.p. 91-92°C.

| Elemental Analysis for $C_{20}H_{23}Cl_2NO$: | | | |
|---|---|---|---|
| Calcd. | C 65.94; | H 6.36; | N 3.84 |
| Found | C 65.79; | H 6.32; | N 3.71 |

(2) Ethyl ester of 3-[N-[4-chloro-2-(α-hydroxy-2-chloro phenylmethyl)phenyl]-N-cyclohexylmethyl-carbamoyl]acrylic acid

In 80 ml of methylene chloride was dissolved 6.0 g the crystals obtained above. To the solution was added dropwise, while stirring under ice-cooling, 2.8 g of monoethyl ester of fumaric acid chloride (a solution in 20 1 of methylene chloride) in the course of 30 minutes. The reaction mixture was stirred for 20 minutes at room temperature, which was washed with water, dried and subjected to concentration under reduced pressure. The concentrate was purified by means of a silica gel chromatography (eluent, hexane:ethyl acetate=3:1) to afford 6.5 g of ethyl ester of 3-[N-[4-chloro-2-(α-hydroxy-2-chlorophenylmethyl)phenyl-N-cyclohexylmethylcarbamoyl] acrylic acid as an oily product.
$IR\nu^{Neat}_{max} cm^{-1}$ : 3400(OH), 2940, 1730(C=O), 1660(C=O), 1630, 1300, 1180, 1040
$^1H$-NMR spectrum (200MHz, $CDCl_3$) δ : 0.8-2.0(14H, multiplet, $CH_2CH_3$, -cyclohexane), 2.6-3.5(2H, multiplet, -N $\underline{CH_2}$-cyclohexane), 4.0-4.5(2H, multiplet, $\underline{CH_2}$ $CH_3$), 6.05-7.7(10H, multiplet)

(3) Ethyl ester of trans-7-chloro-5-(2-chlorophenyl)-1-cyclohexylmethyl-1,2,3,5-tetrahydro-2-oxo-4,1-benzoxazepine-3-acetic acid

In 100 ml of ethanol was dissolved 6.5 g of the oily product obtained above, to which was added 5 g of potassium carbonate, and the mixture was stirred for 15 hours at room temperature. The reaction mixture was concentrated under reduced pressure. The concentrate was subjected to extraction with ethyl acetate. The organic layer

was washed with water and dried, from which was distilled off the solvent under reduced pressure. The residue was purified by means of a silica gel chromatography (eluent, hexane:ethyl acetate = 8:1) to afford 5.2 g of ethyl ester of 3,5-trans-7-chloro-5-(2-chlorophenyl)-1-cyclohexylmethyl-1,2,3,5-tetrahydro-2-oxo-4,1-benzoxazepine-3-acetic acid as a powdery product.
$IR\nu_{max}^{Neat} cm^{-1}$ : 2940, 1740(C=O), 1680(C=O), 1600, 1490, 1380, 1270

| Elemental Analysis for $C_{26}H_{29}Cl_2NO_4$: | | | |
|---|---|---|---|
| Calcd. | C 63.68; | H 5.96; | N 2.86 |
| Found | C 63.48; | H 5.98; | N 2.71 |

Reference Example 3

By substantially the same procedure as in Reference Example 2, compounds shown in Table 1 through Table 4 were obtained.

Table 1

Reference Example 3

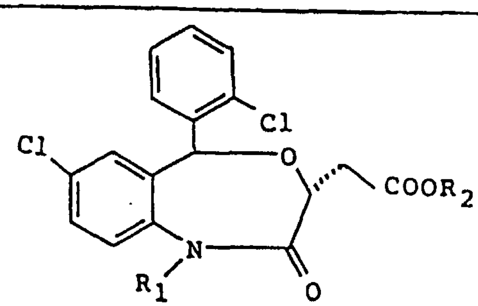

| Compd. No. | $R_1$ | $R_2$ | m.p. (℃) | Formula | Elemental Analysis (Found) C | H | N |
|---|---|---|---|---|---|---|---|
| 1 | H | $C_2H_5$ | 148-150 | $C_{19}H_{17}Cl_2NO_4$ | 57.88 (57.92) | 4.35 (4.60) | 3.55 (3.33) |
| 2 | 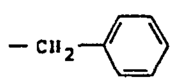 | $C_2H_5$ | 126-127 | $C_{26}H_{23}Cl_2NO_4$ | 64.47 (64.67) | 4.79 (4.65) | 2.89 (2.91) |

Table 2

Reference Example 3

| Compd. No. | $R_1$ | $R_2$ | m.p. (°C) | Formula | Elemental Analysis (Found) C H N |
|---|---|---|---|---|---|
| 3 | 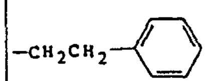 | $C_2H_5$ | oil | $C_{27}H_{25}Cl_2NO_4$ | IR$\nu_{max}^{neat}$ $cm^{-1}$: 3000, 1740 (CO), 1680 (CO), 1490, 1170 |
| | | $^1$H-NMR ($CDCl_3$) $\delta$ : 1.26 (3H, t, $CH_2\underline{CH_3}$), 3.8 (1H, dd, $C_3$-H), 4.15 (2H, q, $\underline{CH_2}CH_3$), 6.04 (1H, s, $C_5$-H) | | | |
| 4 | $-CH(CH_3)_2$ | $C_2H_5$ | oil | $C_{22}H_{23}Cl_2NO_4$ | IR$\nu_{max}^{neat}$ $cm^{-1}$: 2980, 1740 (CO), 1670 (CO) |
| | | $^1$H-NMR ($CDCl_3$) $\delta$ : 1.1-1.6 (9H, m, $CH_2\underline{CH_3}$, $CH(\underline{CH_3})_2$), 2.77 (1H, dd), 3.07 (1H, dd), 4.13 (2H, q,), 4.33 (1H, t, $C_3$-H), 6.01 (1H, s, $C_5$-H) | | | |

Table 3

Reference Example 3

| Compd. No. | $R_1$ | $R_2$ | m.p. (℃) | Formula | Elemental Analysis (Found) C H N |
|---|---|---|---|---|---|
| 5 | $-C_2H_5$ | $C_2H_5$ | 119-120 | $C_{21}H_{21}Cl_2NO_4$ | 59.73 5.01 3.32 (59.60) (4.95) (3.35) |
| 6 | 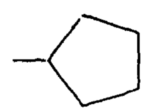 | $C_2H_5$ | oil | $C_{24}H_{25}Cl_2NO_4$ | $^1$H-NMR($CDCl_3$) δ : 1.25(3H, t, $CH_2\underline{CH_3}$), 1.5-2.4(8H,m), 2.78,3.05(2H,both dd), 4.15(2H,m), 4.31(1H,dd,$C_3$-H), 4.69(1H,t), 6.01(1H,s,$C_5$-H), 6.50(1H,d,$C_6$-H), 7.2-7.6(6H,m) |

Table 4

Refernece Example 3

| Compd. No. | $R_1$ | $R_2$ | m.p. (°C) | Formula | Elemental Analysis (Found) C H N |
|---|---|---|---|---|---|
| 7 | $-CH_2CH_2CH_3$ | $C_2H_5$ | oil | $C_{22}H_{23}Cl_2NO_4$ | $^1H$-NMR($CDCl_3$) δ : 0.97 (3H, t, $CH_3$), 1.25(3H, t), |
| | | | 1.5-1.9(2H, m), 2.78, 3.07(2H, both dd), 3.53(1H, m), 4.13(2H, dq), 4.4(2H, m), 6.03(1H, s), 6.50(1H, d), 7.2-7.8(6H, m) | | |

Example 1

By substantially the same procedure as in Reference Example 2, compounds shown in Table 5 and Table 6 corresponding to the following formula were obtained :

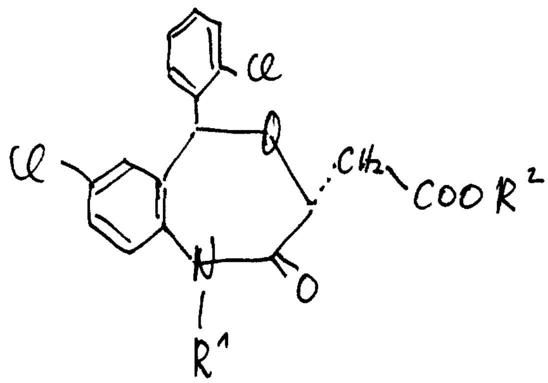

Table 5

Example 1

| Compd. No. | $R_1$ | $R_2$ | m.p. (℃) | Formula | Elemental Analysis (Found) C H N |
|---|---|---|---|---|---|
| 1 | $-(CH_2)_6CH_3$ | $C_2H_5$ | 80-81 | $C_{26}H_{31}Cl_2NO_4$ | 63.42 6.35 2.84 (63.58) (6.58) (2.79) |
| 2 | $-CH_2CH(CH_3)_2$ | $C_2H_5$ | oil | $C_{23}H_{25}Cl_2NO_4$ | $^1H$-NMR($CDCl_3$) δ : 0.92, 1.03(6H, both d, $CH_3$), 1.25(3H, t, $CH_3$), 1.98(1H, m), 2.8, 3.06(2H, both dd), 3.45(1H, dd), 4.13(2H, q), 4.2-4.5(2H, m), 6.14(1H, s, $C_6$-H), 6.51(1H, d), 7.2-7.8(6H, m) |
| 3 | $-CH_2CH(C_2H_5)_2$ | $C_2H_5$ | oil | $C_{25}H_{29}Cl_2NO_4$ | $^1H$-NMR($CDCl_3$) δ : 0.85, 0.89(6H, both t, $CH_3$), 1.25(3H, t, $CH_3$), 1.2-1.7(4H, m), 2.8, 3.05(2H, both dd), 4.14(2H, q, $\underline{CH_2}CH_3$), 3.45(1H, dd), 4.45(2H, m), 6.11(1H, s, $C_5$-H), 6.51(1H, d), 7.2-7.8(6H, m) |
| 4 | $-CH_2C(CH_3)_3$ | $C_2H_5$ | 101-102 | $C_{24}H_{27}Cl_2NO_4$ | 62.07 5.86 3.02 (62.36) (5.87) (2.99) |
| 5 | $-(CH_2)_2CH(CH_3)_2$ | $C_2H_5$ | oil | $C_{24}H_{27}Cl_2NO_4$ | $^1H$-NMR($CDCl_3$) δ : 0.91, 0.92(6H, both t. $CH_3$), 1.25(3H, t, $CH_3$), 1.4-1.8(3H, m), 2.79, 3.06(2H, both dd), 3.55(1H, m), 4.05-4.6(4H, m), 6.01(1H, s, $C_5$-H), 6.51(1H, d), 7.2-7.8(6H, m) |

Table 6

Example 1

| Compd. No. | $R_1$ | $R_2$ | m.p. (℃) | Formula | Elemental Analysis (Found) C H N |
|---|---|---|---|---|---|
| 6 | $-CH(C_2H_5)_2$ | $C_2H_5$ | oil | $C_{24}H_{27}Cl_2NO_4$ | $^1$H-NMR($CDCl_3$) δ : 0.98, 1.04(6H, both t, $CH_3$), 1.24(3H, t), 1.6-2.1(4H,m), 2.76, 3.1(2H, both dd), 4.13,(2H,q, $OCH_2CH_3$), 4.2-4.4(2H,m), 6.09(1H,s,$C_5$-H), 6.51(1H,d) 7.2-7.8(6H,m) |
| 7 | $-(CH_2)_3CH_3$ | $C_2H_5$ | oil | $C_{23}H_{25}Cl_2NO_4$ | 1H-NMR($CDCl_3$) δ : 0.91(3H, t), 1.25(3H, t), 1.3-1.8(4H, m), 2.78, 3.06(2H, both dd), 3.45-3.63(1H, m), 4.13(2H, dq), 4.35-4.55(2H, m), 6.03(1H, s), 6.51(1H, d), 7.2-7.75 (6H, m) |

In Table 7 through Table 12, physicochemical properties of intermediates obtained in Reference Example 3 and Example 1 are shown.

Table 7

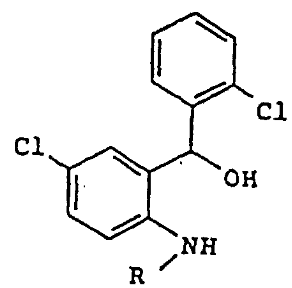

| R | m.p. (℃) | Formula | Elemental Analysis (Found) C | H | N |
|---|---|---|---|---|---|
| $-CH_2CH_2-$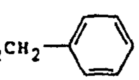 | 119-120 | $C_{21}H_{19}Cl_2NO$ | 67.75<br>(67.83) | 5.14<br>(5.12) | 3.76<br>(3.71) |
| $-CH(CH_3)_2$ | oil | $C_{16}H_{17}Cl_2NO$ | | | |
| | NMR ($CDCl_3$) δ : 1.12(3H,d), 1.18(3H,d), 3.6(1H,m), 6.07(1H,m), 6.5-7.5(7H,m) | | | | |

Table 8

| R | m.p. (℃) | Formula | Elemental Analysis (Found) C | H | N |
|---|---|---|---|---|---|
| $-(CH_2)_6CH_3$ | oil | $C_{20}H_{25}Cl_2NO$ | $^1$H-NMR($CDCl_3$) δ : 0.87(3H,t,$CH_3$), 1.0-1.7 (10H,m), 2.8-3.1(2H,m), 6.1-7.7(8H,m) | | |
| $-CH_2CH(CH_3)_2$ | 87-88 | $C_{17}H_{19}Cl_2NO$ | 62.97 (62.97) | 5.91 (6.05) | 4.32 (4.44) |
| 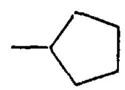 | oil | $C_{18}H_{19}Cl_2NO$ | $^1$H-NMR($CDCl_3$) δ : 1.2-2.2(8H,m), 3.75(1H,m), 6.05(1H,s), 6.5-7.5(7H,m) | | |
| $-CH_2CH(C_2H_5)_2$ | oil | $C_{19}H_{23}Cl_2NO$ | $^1$H-NMR($CDCl_3$) δ : 0.7-1.6(10H,m), 2.97(2H, d), 6.11(1H,s), 6.6(1H,d), 6.9-7.5(6H,m) | | |
| $-CH_2C(CH_3)_3$ | 110-111 | $C_{18}H_{21}Cl_2NO$ | 63.91 (64.12) | 6.26 (6.30) | 4.14 (4.31) |
| $-(CH_2)_2CH(CH_3)_2$ | oil | $C_{18}H_{21}Cl_2NO$ | $^1$H-NMR($CDCl_3$) δ : 0.90,0.93(6H, both d,$CH_3$), 1.4-1.8(3H,m), 3.09(2H,t), 6.12(1H,s), 6.60(1H,d), 6.90(1H,d), 7.1-7.5(5H,m) | | |

Table 9

| R | p.m. (℃) | Formula | Elemental Analysis(Found) C H N |
|---|---|---|---|
| $-CH(C_2H_5)_2$ | oil | $C_{18}H_{21}Cl_2NO$ | $^1H$-NMR($CDCl_3$) δ: 0.75(3H,t,$CH_3$), 0.88(3H,t,$CH_3$), 1.1-1.7(4H,m), 3.2(1H,m), 6.1(1H,s), 6.5-7.5(7H,m) |
| $-CH_2CH_2CH_3$ | oil | $C_{16}H_{17}Cl_2NO$ | $^1H$-NMR($CDCl_3$) δ : 0.95(3H, t), 1.65(2H, m), 3.06(2H, t), 6.12(1H, s), 6.55-7.5(7H, m) |
| $-(CH_2)_3CH_3$ | oil | $C_{17}H_{19}Cl_2NO$ | $^1H$-NMR($CDCl_3$) δ : 0.92(3H, t), 1.2-1.7(4H, m), 3.07(2H, t), 6.10(1H, s), 6.59(1H, d), 6.87(1H, d), 7.1-7.5(5H, m) |

Table 10

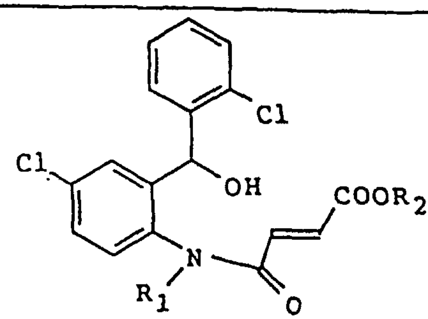

| R1 | R2 | m.p. (℃) | Formula | Elemental Analysis (Found) C | H | N |
|---|---|---|---|---|---|---|
| 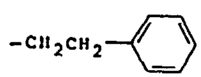 | $-C_2H_5$ | oil | $C_{27}H_{25}Cl_2NO_4$ | | | |
| | | $^1$H-NMR ($CDCl_3$) $\delta$ : 1.1-1.4 (3H, m), 2.7-4.7 (6H, m), 6.0-7.7 (15H, m) | | | | |

Table 11

| $R_1$ | $R_2$ | p.m. (℃) | Formula | Elemental Analysis(Found) C H N |
|---|---|---|---|---|
| $-C_2H_5$ | $-C_2H_5$ | oil | $C_{21}H_{21}Cl_2NO_4$ | $^1$H-NMR($CDCl_3$) δ: 0.9-1.5(6H,m), 3.5-4.5(4H,m), 6.0-7.9(10H,m) |
| $-(CH_2)_6CH_3$ | $-C_2H_5$ | oil | $C_{26}H_{31}Cl_2NO_4$ | $^1$H-NMR($CDCl_3$) δ: 0.85(3H,t,$CH_3$), 0.95-1.7(13H,m), 2.7-4.5(4H,m) 6.0-7.7(10H,m) |
| $-CH_2CH(CH_3)_2$ | $-C_2H_5$ | 136-138 | $C_{23}H_{25}Cl_2NO_4$ | 61.34 5.59 3.11<br>(61.26) (5.71) (3.06) |
| 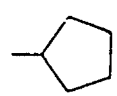 | $-C_2H_5$ | 192-194 | $C_{24}H_{25}Cl_2NO_4$ | 62.34 5.45 3.03<br>(62.05) (5.50) (3.07) |

Table 12

| $R_1$ | $R_2$ | p.m. (℃) | Formula | Elemental Analysis (Found) C H N |
|---|---|---|---|---|
| $-CH_2CH(C_2H_5)_2$ | $-C_2H_5$ | oil | $C_{25}H_{29}Cl_2NO_4$ | $^1$H-NMR($CDCl_3$) δ: 0.6-1.7(14H,m), 2.7-4.6(4H,m), 5.9-7.7(10H,m) |
| $-CH_2C(CH_3)_3$ | $-C_2H_5$ | oil | $C_{24}H_{27}Cl_2NO_4$ | $^1$H-NMR($CDCl_3$) δ: 0.7-1.4(12H,m), 2.8-4.6(4H,m), 6.1-7.7(10H,m) |
| $-(CH_2)_2CH(CH_3)_2$ | $-C_2H_5$ | oil | $C_{24}H_{27}Cl_2NO_4$ | $^1$H-NMR($CDCl_3$) δ: 0.6-1.7(12H,m), 2.6-4.6(4H,m), 5.9-7.7(10H,m) |
| $-CH(C_2H_5)_2$ | $-C_2H_5$ | oil | $C_{24}H_{27}Cl_2NO_4$ | $^1$H-NMR($CDCl_3$) δ: 0.7-2.1(13H,m), 4.0-4.4(3H,m), 6.0-7.5(10H,m) |
| $-CH_2CH_2CH_3$ | $-C_2H_5$ | oil | $C_{22}H_{23}Cl_2NO_4$ | $^1$H-NMR($CDCl_3$) δ: 0.77, 0.86(3H, both d), 1.22, 1.24 (3H, both t), 1.4-1.9(2H, m), 2.6-4.5(4H, m), 6.0-7.6 (10H, m) |
| $-(CH_2)_3CH_3$ | $-C_2H_5$ | oil | $C_{23}H_{25}Cl_2NO_4$ | $^1$H-NMR($CDCl_3$) δ: 0.7-1.9(10H, m), 2.6-4.5(4H, m), 5.9-7.7(10H, m) |

Reference Example 4

Trans-7-chloro-5-(2-chlorophenyl)-1-methyl-1,2,3,5-tetrahydro-2-oxo-4,1-benzoxazepine-3-acetic acid

In a mixture of 10 ml of methanol and 4 ml of water was suspended 0.5 g of ethyl ester of 3,5-trans-7-chloro-5-(2-chlorophenyl)-1-methyl-1,2,3,5-tetrahydro-2-oxo-4,1-benzoxazepine-3-acetic acid. To the suspension was added 0.8 g of potassium carbonate, and the mixture was stirred for 3 hours at 60°C. The reaction mixture was concentrated under reduced pressure, to which was added water, followed by extraction with ether. The aqueous layer was acidified with dilute hydrochloric acid, followed by extraction with ethyl acetate. The organic layer was washed with water and dried, followed by concentration under reduced pressure. The concentrate was purified by means of a silica gel chromatography to afford 0.21 g of 3,5-trans-7-chloro-5-(2-chlorophenyl)-1-methyl-1,2,3,5-tetrahydro-2-oxo-4,1-benzoxazepine-3-acetic acid as crystals, m.p. 211-213°C.

| Elemental Analysis for $C_{18}H_{15}Cl_2NO_4$: | | | |
|---|---|---|---|
| Calcd. | C 56.86; | H 3.98; | N 3.68 |
| Found | C 56.86; | H 4.24; | N 3.53 |

Reference Example 5

By substantially the same procedure as in Reference Example 4, compounds shown in Table 13 through Table 15 were obtained.

Table 13

Reference Example 5

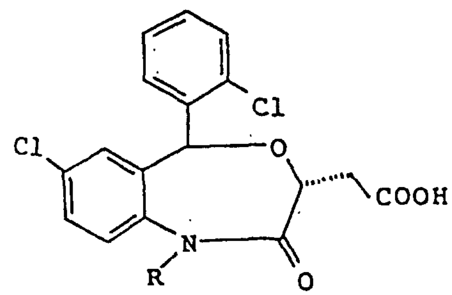

| Compd. No. | R | m.p. (℃) | Formula | Elemental Analysis (Found) C | H | N |
|---|---|---|---|---|---|---|
| 1 | 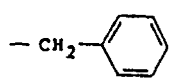 | 175-178 | $C_{24}H_{19}Cl_2NO_4$ | 63.17 (63.18) | 4.20 (4.24) | 3.07 (3.23) |

Table 14

Refernece Example 5

| Compd. No. | R | p. m. (℃ ) | Formula | Elemental Analysis (Found) C | H | N |
|---|---|---|---|---|---|---|
| 2 | 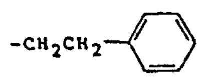 | 212-214 | $C_{25}H_{21}Cl_2NO_4$ | 63.84 (63.94) | 4.50 (4.71) | 2.98 (3.17) |
| 3 | 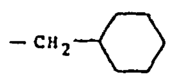 | 241-242 | $C_{24}H_{25}Cl_2NO_4$ | 62.34 (62.27) | 5.45 (5.45) | 3.03 (3.02) |
| 4 | $-CH(CH_3)_2$ | 208-209 | $C_{20}H_{19}Cl_2NO_4$ | 58.84 (58.78) | 4.69 (4.83) | 3.43 (3.38) |

Table 15
Reference Example 5

| Compd. No. | R | p. m. (℃) | Formula | Elemental Analysis (Found) C | H | N |
|---|---|---|---|---|---|---|
| 5 | $-C_2H_5$ | 215-216 | $C_{19}H_{17}Cl_2NO_4$ | 57.88 (57.67) | 4.35 (4.27) | 3.55 (3.54) |
| 6 | 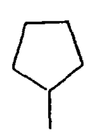 | 224-225 | $C_{22}H_{21}Cl_2NO_4$ | 60.84 (60.64) | 4.87 (4.94) | 3.22 (3.32) |
| 7 | $-CH_2CH_2CH_3$ | 189-190 | $C_{20}H_{19}Cl_2NO_4$ | 58.84 (58.78 | 4.69 4.95 | 3.43 3.60) |

Example 2

By substantially the same procedure as in Reference Example 4, compounds shown in Table 16, were obtained.

Table 16

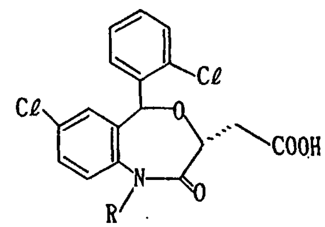

Example 2

| Compd. No. | R | p. m. (℃) | Formula | Elemental Analysis(Found) C | H | N |
|---|---|---|---|---|---|---|
| 1 | $-(CH_2)_6CH_3$ | 188-189 | $C_{24}H_{27}Cl_2NO_4$ | 62.07 (62.21) | 5.86 (5.83) | 3.02 (3.00) |
| 2 | $-CH_2CH(CH_3)_2$ | 220-221 | $C_{21}H_{21}Cl_2NO_4$ | 59.73 (59.98) | 5.01 (5.24) | 3.32 (3.14) |
| 3 | $-CH_2CH(C_2H_5)_2$ | 188-189 | $C_{23}H_{25}Cl_2NO_4$ | 61.34 (61.39) | 5.60 (5.74) | 3.11 (2.99) |
| 4 | $-CH_2-C(CH_3)_3$ | 247-248 | $C_{22}H_{23}Cl_2NO_4$ | 60.56 (60.55) | 5.31 (5.47) | 3.21 (3.11) |
| 5 | $-(CH_2)_2CH(CH_3)_2$ | 166-167 | $C_{22}H_{23}Cl_2NO_4$ | 60.56 (60.40) | 5.31 (5.49) | 3.21 (3.22) |
| 6 | $-CH(C_2H_5)_2$ | 220-221 | $C_{22}H_{23}Cl_2NO_4$ | 60.56 (60.45) | 5.31 (5.29) | 3.21 (3.20) |
| 7 | $-(CH_2)_3CH_3$ | 195-196 | $C_{21}H_{21}Cl_2NO_4$ | 59.73 (59.68 | 5.01 4.97 | 3.32 3.54) |

Reference Example 6

Trans-1-benzyl-7-chloro-5-phenyl-2-oxo-1,2,3,5-tetrahydro-4,1-benzoxazepine-3-acetic acid

0.3 g of the ethyl ester of trans-1-benzyl-7-chloro-5-phenyl-2-oxo-1,2,3,5-tetrahydro-4,1-benzoxazepine-3-acetic acid disclosed in JPA S57(1982)-35576 was subjected to hydrolysis in the manner to be described in the following Reference Example 4 to afford 0.12 g of trans-1-benzyl-7-chloro-5-phenyl-2-oxo-1,2,3,5-tetrahydro-4,1-benzoxazepine-3-acetic acid as a white powdery product.

Example 3

Ethyl ester of trans-1-isobutyl-2-oxo-5-(o-tolyl)-1,2,3,5-tetrahydro-4,1-benzoxazepine-3-acetic acid

(1) 2-Isobutylamino-α-(o-tolyl)benzyl alcohol

Using 2-amino-α-(o-tolyl)benzyl alcohol (2.5 g) and isobutyl aldehyde (1.17 ml), reaction is conducted in substantially the same manner as in Reference Example 2 (1) to give 2-isobutylamino-α-(o-tolyl)benzyl alcohol (3.1 g) as an oily product.
IR$\nu_{max}^{Neat}$ $cm^{-1}$ : 3400(OH); 1720, 1655, 1630(C=O,C=C)
$^1$H-NMR($CDCl_3$) δ : 0.7-1.1(6H,m), 1.21(3H,t,J=7.0Hz), 1.5-2.0(1H,m), 2.29(3H,s), 2.0-2.3 and 2.9-3.1(1H, each m), 3.9-4.5(3H,m), 5.9-6.4(2H,m), 6.6-6.9(1H,m), 6.95-7.9(8H,m)

(2) Ethyl ester of 3-[N-[2-(α-hydroxy-2-methylbenzyl)phenyl-N-isobutyl]carbamoyl]acrylic acid

In substantially the same manner as in Reference Example 2 (2), 2-isobutylamino-α-(o-tolyl)benzyl alcohol (1.3 g) obtained in (1) above was allowed to react with fumaric acid chloride monoethyl ester to give ethyl ester of 3-[N-[2-(α-hydroxy-2-methylbenzyl)phenyl-N- isobutyl]carbamoyl]acrylic acid (3.1 g) as an oily product.
IR$\nu_{max}^{Neat}$ $cm^{-1}$ : 3400(OH); 1720, 1655, 1630(C=C, C=O)
$^1$H-NMR spectrum (200MHz, $CDI_3$) δ : 0.7-1.1(6H,m), 1.21(3H,t,J=7.0Hz), 1.5-2.0(1H,m), 2.29(3H,s), 2.0-2.3 and 2.9-3.1(1H, each m), 3.9-4.5(3H,m), 5.9-6.4(2H,m), 6.6-6.9(1H,m), 6.95-7.9(8H,m)

(3) Ethyl ester of trans-1-isobutyl-2-oxo-5-(o-tolyl)-1,2,3,5-tetrahydro-4,1-benzoxazepine-3-acetic acid

In the same manner as in Reference Example 2 (3), ethyl ester of 3-[N-[2-(α-hydroxy-2-methylbenzyl) phenyl-N-isobutyl]carbamoyl] acrylic acid (1.8 g) was subjected to reaction. The reaction product was crystallized from water-ethanol to give ethyl ester of trans-1-isobutyl-2-oxo-5-(o-toluyl)-1,2,3,5-tetrahydro-4,1-benzoxazepine-3-acetic acid (2,41 g) as prisms, 88°C - 90°C.
IR$\nu_{max}^{KBr}$ $cm^{-1}$ : 1730, 1670(C=O)

| Elemental Analysis for $C_{24}H_{29}NO_4$: | | | |
|---|---|---|---|
| Calcd. | C 72.89; | H 7.39; | N 3.54 |
| Found | C 73.18; | H 7.25; | N 3.54 |

Example 4

By substantially the same synthetic procedure as in Example 3, compounds listed in Table 17 and Table 18 were obtained.

Table 17

Example 4

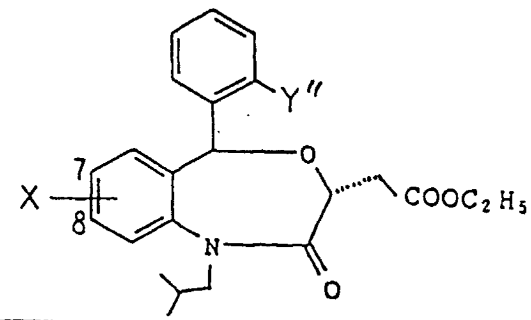

| Compd. No. | X | Y″ | p.m. (℃) | Formula | Elemental Analysis(Found) C H N |
|---|---|---|---|---|---|
| 1 | H | Cl | 99-100 | $C_{23}H_{28}ClNO_4$ | 66.42 6.30 3.37<br>(66.20) (6.57) (3.42) |
| 2 | (7)-Cl | F | oil | $C_{23}H_{25}ClFNO_4$ | $^1$H-NMR($CDCl_3$) δ: 0.89, 0.97(6H, both d, $CH_3$), 1.26(3H, t, $CH_2CH_3$), 2.04(1H,m), 2.78, 3.07(2H, both dd), 3.4 (1H,dd), 4.13(2H,q), 4.2-4.5(2H,m), 6.13(1H, s, $C_5$-H), 6.63(1H,d), 7.0-7.7 (6H,m) |

Table 18

Example 4

| Compd. No. | X | Y" | m.p. (℃) | Formula | Elemental Analysis(Found) C H N |
|---|---|---|---|---|---|
| 3 | H | F | oil | $C_{23}H_{26}FNO_4$ | $^1$H-NMR($CDCl_3$) δ : 0.89, (3H,d), 0.98(3H,d), 1.24(3H,t), 1.9-2.2(1H,m), 2.78(1H,dd), 3.06(1H,dd), 3.46 (1H,dd), 4.13(2H,q), 4.25-4.5(2H,m), 6.18(1H,s) 6.67(1H,d), 6.9-7.75(7H,m) |
| 4 | H | $OCH_3$ | 132-133 | $C_{24}H_{29}NO_5$ | 70.05 7.10 3.40<br>(70.20) (7.13) (3.56) |

In Table 19 through Table 22, physicochemical properties of intermediates are shown.

Table 19

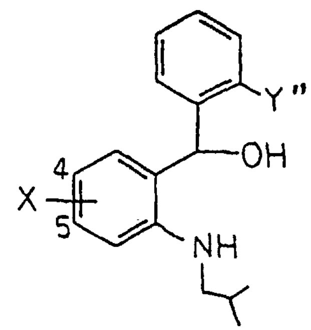

| X | Y" | m.p. (℃) | Formula | Elemental Analysis (Found) C H N |
|---|---|---|---|---|
| H | Cl | oil | $C_{17}H_{20}ClNO$ | $^1$H-NMR($CDCl_3$) δ : 0.8-1.1(6H,m), 1.9(1H,m), 2.95 (2H,d), 5.29(1H, s), 6.17(1H,s), 6.5-7.6(7H,m) |
| (4)-Cl | F | oil | $C_{17}H_{19}ClFNO$ | $^1$H-NMR($CDCl_3$) δ : 0.8-1.1(6H,m, $CH_3$), 1.85(1H,m), 2.89(2H,d,NH$CH_2$), 6.05(1H,s), 6.57(1H,d), 6.9-7.5 (6H,m) |

Table 20

| X | Y″ | m.p. (℃) | Formula | Elemental Analysis (Found) C H N |
|---|---|---|---|---|
| H | F | oil | $C_{17}H_{20}FNO$ | $^1$H-NMR($CDCl_3$) δ : 0.95(6H,d, J=6.8Hz), 1.8-2.1(1H,m), 2.93(2H, d,J=6.6Hz), 6.11(1H,s), 6.55-6.75 (2H,m), 6.9-7.5(6H,m) |
| H | $OCH_3$ | oil | $C_{18}H_{23}NO_2$ | $^1$H-NMR($CDCl_3$) δ : 0.94(6H,d, J=6.6Hz), 1.75-2.05(1H,m), 2.94 (2H,d), 3.86(3H,s), 6.02(1H,s), 6.5-6.75(2H,m), 6.85-7.0(3H,m), 7.1-7.4(3H,m) |

Table 21

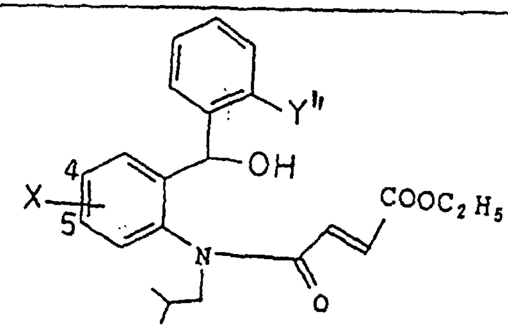

| X | Y" | m.p. (℃) | Formula | Elemental Analysis (Found) C H N |
|---|---|---|---|---|
| H | Cl | oil | $C_{23}H_{26}ClNO_4$ | $^1$H-NMR($CDCl_3$) δ : 0.7-1.4(9H,m), 1.9(1H,m), 2.6-4.5(4H,m), 6.1-7.7 (11H,m) |
| (4)-Cl | F | oil | $C_{23}H_{25}ClFNO_4$ | $^1$H-NMR($CDCl_3$) δ : 0.7-1.4(9H,m), 1.85(1H,m), 2.5-4.4(4H,m), 5.9-7.9(10H,m) |

Table 22

| X | Y" | m.p. (°C) | Formula | Elemental Analysis (Found) C H N |
|---|---|---|---|---|
| H | F | oil | $C_{23}H_{26}FNO_4$ | $^1$H-NMR($CDCl_3$) δ: 0.75-1.15(6H,m), 1.20(3H,t,J=7.0Hz), 1.5-2.1(1H,m), 2.6-2.8, 2.95-3.15(1H, both m), 4.0-4.5(3H,m), 6.0-6.5(2H,m), 6.7-7.8(9H,m) |
| H | $OCH_3$ | oil | $C_{24}H_{29}NO_5$ | $^1$H-NMR($CDCl_3$) δ: 0.7-1.1(6H,m), 1.20(3H,t.J=7.1Hz), 1.7-2.0(1H,m), 2.5-3.2(2H,m), 3.74, 3.78(3H, s), 4.0-4.45(3H,m), 6.0-6.5(2H,m), 6.7-7.85(9H,m) |

Example 5

Trans-1-isobutyl-2-oxo-5-(o-tolyl)-1,2,3,5-tetrahydro-4,1-benzoxazepine-3-acetic acid

In substantially the same manner as in Reference Example 4, ethyl ester of trans-1-isobutyl-2-oxo-5-(o-tolyl)-1,2,3,5- tetrahydro-4,1-benzoxazepine-3-acetic acid (2.0 g) obtained in Example 3 was subjected to hydrolysis to give trans-1-isobutyl-2-oxo-5-(o-tolyl)-1,2,3,5-tetrahydro-4,1-benzoxazepine-3-acetic acid (1.23 g) as prisms, m.p. 192°C - 195°C.

IR$\nu_{max}^{KBr}$ $cm^{-1}$ : 1735, 1650(C=O)

| Elemental Analysis for $C_{22}H_{25}NO_4$: | | | |
|---|---|---|---|
| Calcd. | C 71.91; | H 6.66; | N 3.81 |
| Found | C 71.86; | H 6.78; | N 3.80 |

Example 6

In substantially the same manner as in Reference Example 4, using compounds obtained in Example 4, compounds listed in Table 23 were obtained.

Table 23
Example 6

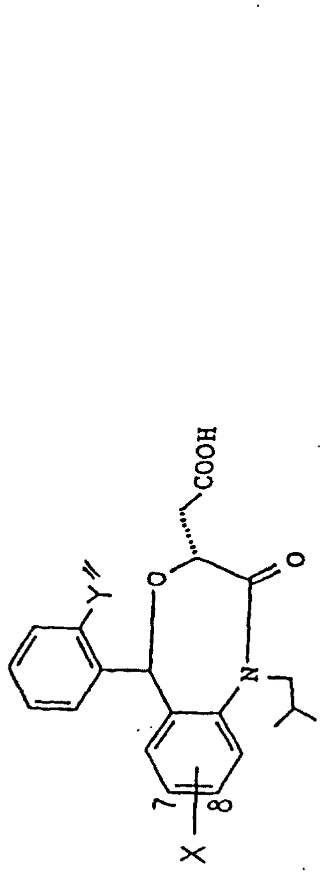

| X | Y'' | m.p. (°C) | Formula | Elemental Analysis (Found) C | H | N |
|---|---|---|---|---|---|---|
| H | Cl | 192-193 | $C_{21}H_{22}ClNO_4$ | 65.03 (64.73) | 5.72 (5.63) | 3.61 (3.76) |
| (7)-Cl | F | 153-154 | $C_{21}H_{21}ClFNO_4$ | 62.15 (62.17) | 5.22 (5.19) | 3.45 (3.50) |
| H | F | 185-187 | $C_{21}H_{22}FNO_4$ | 67.91 (67.93) | 5.97 (6.01) | 3.77 (3.65) |
| H | $OCH_3$ | 243-245 | $C_{22}H_{25}NO_5$ | 68.91 (68.89) | 6.57 (6.60) | 3.65 (3.74) |

Reference Example 7

Trans-7-chloro-5-(2-chlorophenyl)-1-(2,4-dimethoxybenzyl)-2-oxo-1,2,3,5-tetrahydro-4,1-benzoxazepine-3-acetic acid

To a solution of trans-7-chloro-5-(2-chlorophenyl)-1-(2,4-dimethoxybenzyl)-2-oxo-1,2,3,5-tetrahydro-4,1-benzoxazepine-3-acetic acid ethyl ester (1.0 g) obtained in Reference Example 7 in methanol (20 ml) was added an aqueous solution (10 ml) of potassium carbonate (0.51 g), followed by stirring for one hour at 60°C. The reaction mixture was acidified with 1N hydrochloric acid (50 ml), which was subjected to extraction with ethyl acetate (100 ml). The ethyl acetate layer was washed with water and dried over anhydrous magnesium sulfate, then the solvent was distilled off

under reduced pressure. The residue was purified by means of a silica gel column chromatography (hexane : ethyl acetate = 1:1 ~ hexane : methylene chloride : ethanol = 5:5:1) to afford trans-7-chloro-5-(2-chlorophenyl)-1-(2,4-dimethoxybenzyl)-2-oxo-1,2,3,5-tetrahydro-4,1-benzoxazepine-3-acetic acid (0.43 g) as a powdery product.
$IR\nu_{max}^{KBr}$ $cm^{-1}$ : 1715, 1670(C=O)

| Elemental Analysis for $C_{26}H_{23}Cl_2NO_6$: | | | |
|---|---|---|---|
| Calcd. | C 60.48; | H 4.49; | N 2.71 |
| Found | C 60.21; | H 4.73; | N 2.72 |

Example 7

Ethyl ester of 1-benzyl-2-oxo-5-phenyl-1,2,3,5-tetrahydro-4,1-benzoxazepine-3-propionic acid

(1) 2-(5-Oxotetrahydrofuran-2-carbonyl)aminobenzophenone

A mixture of 4.9 g of 5-oxotetrahydrofuran-2-carboxylic acid and 5.5 ml of thionyl chloride was subjected to reflux for 2 hours. Thionyl chloride was then distilled off under reduced pressure to leave 5-oxotetrahydrofuran-2-carbonyl chloride. This product was mixed with 5.0 g of 2-aminobenzophenone, 200 ml of ethyl acetate and 200 ml of a saturated aqueous solution of sodium hydrogencarbonate, which was stirred for one hour at room temperature. The ethyl acetate layer was washed with water and dried over anhydrous magnesium sulfate, then the solvent was distilled off under reduced pressure. The residue was purified by means of a silica gel column chromatography (eluent, hexane : ethyl acetate = 2:1) to give 2-(5-oxotetrahydrofuran-2-carbonyl)aminobenzophenone (6.5 g) as needles, m.p. 100°C - 102°C.
$IR\nu_{max}^{KBr}$ $cm^{-1}$ : 3300(NH), 1790, 1690, 1640(C=O)

| Elemental Analysis for $C_{18}H_{15}NO_4$: | | | |
|---|---|---|---|
| Calcd. | C 69.89; | H 4.88; | N 4.53 |
| Found | C 69.98; | H 5.01; | N 4.41 |

(2) 2-[N-benzyl-N-(5-oxotetrahydrofuran-2-carbonyl)] aminobenzophenone

In 20 ml of N,N-dimethylformamide was dissolved 3.0 g of 2-(5-oxotetrahydrofuran-2-carbonyl)aminobenzophenone obtained in (1). To the solution were added 1.73 ml of benzyl bromide, 2.67 g of potassium carbonate and 0.1 g of tetrabutyl ammonium iodide. The mixture was stirred overnight at room temperature. The reaction mixture was subjected to extraction with a mixture of 200 ml of ethyl acetate and 100 ml of water. The ethyl acetate layer was washed with 0.1N hydrochloric acid and an aqueous solution of sodium hydrogencarbonate, which was then dried over anhydrous magnesium sulfate. The solvent was distilled off under reduced pressure. The residue was purified by means of a silica gel column chromatography (eluent, hexane : ethyl acetate = 2:1) to give crystals. Recrystallization from hexane - ethyl acetate afforded 2-[N-benzyl-N-(5-oxotetrahydrofuran-2-carbonyl)] aminobenzophenone (3.71 g) as needles, m.p. 142-143°C.
$IR\nu_{max}^{KBr}$ $cm^{-1}$ : 1780, 1660, 1650(C=O)

| Elemental Analysis for $C_{25}H_{21}NO_4$: | | | |
|---|---|---|---|
| Calcd | C 75.17; | H 5.30; | N 3.51 |
| Found | C 75.05; | H 5.59; | N 3.49 |

(3) 2-[N-benzyl-N-(4-ethoxycarbonyl-2-hydroxy)butyryl] aminobenzophenone

In 100 ml of ethanol was dissolved 5.0 g of 2-[N-benzyl-N-(5-oxotetrahydrofuran-2-carbonyl)]aminobenzophenone obtained in (2). To the solution was added 0.2 ml of conc. sulfuric acid, and the mixture was left standing for 7 days. Ethanol was distilled off under reduced pressure. The residue was subjected to extraction with a mixture of 100 ml of water and 200 ml of ethyl acetate. The ethyl acetate layer was washed with an aqueous solution of sodium hydrogencarbonate, which was then dried over anhydrous magnesium sulfate. The solvent was then distilled off under reduced pressure. The residue was purified by means of a silica gel column chromatography (eluent, hexane : ethyl acetate = 2:1) to afford 2.8 g of 2-[N-benzyl-N-(4-ethoxycarbonyl-2-hydroxy) butyryl]aminobenzophenone as an oily

product.
IR$\nu_{max}^{Neat}$ $cm^{-1}$ : 3440(OH), 1735, 1670(C=O)
Mass spectrum (m/e) : 445 ($M^+$)
$^1$H-NMR spectrum (200MHz,$CDCl_3$) δ : 1.20(3H,t,J=7.2Hz), 1.65-2.0(2H,m), 2.2-2.6(2H,m), 3.65-3.7(1H,br), 4.05(2H, q,J=7.2Hz), 4.0-4.35(1H,m), 4.69(1H,d,J=14.4Hz), 4.87(1H,d,J=14.4Hz), 6.9-7.9(14H,m)

(4) 2-[N-benzyl-N-(4-ethoxycarbonyl-2-methanesulfonyloxy)butyryl]aminobenzophenone

2-[N-Benzyl-N-(4-ethoxycarbonyl-2-hydroxy)butyryl]aminobenzophenone (2.8 g) obtained in (3) was dissolved in 30 ml of ethyl acetate. To the solution were added, under ice-cooling, 0.73 ml of methanesulfonyloxy chloride and 1.31 ml of triethylamine. The mixture was stirred for 2 hours at room temperature. The reaction mixture was then washed with 1N HCl and an aqueous solution of sodium hydrogencarbonate, successively, which was dried over anhydrous magnesium sulfate, followed by distilling off the solvent under reduced pressure. The residue was purified by means of a silica gel column chromatography (eluent, hexane : ethyl acetate = 2:1) to afford 3.2 g of 2-[N-benzyl-N-(4-ethoxycarbonyl-2-methanesulfonyloxy) butyryl]aminobenzophenone as an oily product.
IR$\nu_{max}^{Neat}$ $cm^{-1}$ : 1730, 1670(C=O)
Mass spectrum (m/e): 523 ($M^+$)
$^1$H-NMR spectrum (200MHz,$CDCl_3$) δ : 1.1-1.35(3H,m), 2.0-2.6(4H,m), 3.14+3.38(3H,each s), 3.9-4.7(3H,m), 4.93(d, J=14.2Hz)+5.53(d,J=14.8Hz)(1H), 5.05-5.15(m)5.15-5.3(m)(1H), 7.0-7.85(14H,m)

(5) Ethyl-cis-l-benzyl-2-oxo-5-phenyl-1,2,3,5-tetrahydro-4,1-benzoxazepine-3-propionate and ethyl-trans-1-benzyl-2-oxo-5-phenyl-1,2,3,5-tetrahydro-4,1-benzoxazepine-3-propionate

In 30 ml of ethanol was dissolved 3.2 g of 2-[N-benzyl-N-(4-ethoxycarbonyl-2-methanesulfonyloxy) butyryl] aminobenzophenone obtained in (4). To the solution was added, under ice-cooling, 0.32 g of sodium borohydride. The reaction mixture was stirred for 45 minutes at 50°C, followed by cooling the reaction mixture to room temperature. The reaction mixture was acidified by the addition of 100 ml of 1N HCl, which was subjected to extraction with 200 ml of ethyl acetate. The extract solution was washed with an aqueous solution of sodium hydrogencarbonate, dried over magnesium sulfate, then the solvent was distilled off under reduced pressure. The residue was purified by means of a silica gel column chromatography (eluent, hexane : ethyl acetate = 10:1 to 5:1) to afford, as the first fraction, 0.73 g of ethyl ester of cis-1-benzyl-2-oxo-5-phenyl-1,2,3,5-tetrahydro-4,1-benzoxazepine-3-propionic acid as needles, m.p. 107°C - 108°C.
IR$\nu_{max}^{KBr}$ $cm^{-1}$ : 1735, 1670(C=O)

| Elemental Analysis for $C_{27}H_{27}NO_4$: | | | |
|---|---|---|---|
| Calcd. | C 75.50; | H 6.34; | N 3.26 |
| Found | C 75.21; | H 6.44; | N 3.27 |

$^1$H-NMR spectrum (200MHz,$CDCl_3$) δ : 1.21(3H,t,J=7.2Hz), 2.2-2.4(2H,m), 2.55(2H,t,J=7.0Hz), 3.72(1H,d,J=16.0Hz), 4.10(2H,q,J=7.2Hz), 4.23(1H,t,J=6.5Hz), 4.70(1H,d,J=16.0Hz), 5.95(1H,s), 7.0-7.5(14H,m)
As the second fraction, 0.3 g of ethyl ester of trans-1-benzyl-2-oxo-5-phenyl-1,2,3,5-tetrahydro-4,1-benzoxazepine-3-propionic acid as an oily product.
IR$\nu_{max}^{Neat}$ $cm^{-1}$ : 1730, 1670(C=O)
Mass spectrum (m/e) : 429 ($M^+$)
$^1$H-NMR spectrum (200MHz,$CDCl_3$) δ : 1.17(3H,t,J=7.1Hz), 2.05-2.4(2H,m), 2.49(2H,t,J=7.0Hz), 4.06(2H,q,J=7.1Hz), 3.95-4.05(1H,m), 4.86(1H,d,J=14.6Hz), 5.46(1H,s), 5.50(1H,d,J=14.6Hz), 6.53(1H,d,J=7.8Hz), 7.0-7.45(13H,m)

Example 8

Ethyl ester of cis-1-benzyl-7-chloro-5-(2-chlorophenyl)-2-oxo-1,2,3,5-tetrahydro-4,1-benzoxazepine-3-propionic acid

(1) 2',5-Dichloro-2-(5-oxotetrahydrofuran-2-carbonyl) aminobenzophenone

In substantially the same manner as in Example 7 (1), 15 g of 2-amino-2',5-dichlorobenzophenone was allowed to react with 5-oxotetrahydrofuran-2-carbonyl chloride to afford 18.1 g of 2',5-dichloro-2-(5-oxotetrahydrofuran-2-carbonyl) aminobenzophenone, m.p. 170°C-173°C.
IR$\nu_{max}^{KBr}$ $cm^{-1}$ : 3250(NH), 1775, 1695, 1640(C=O)

| Elemental Analysis for $C_{18}H_{13}Cl_2NO_4$: | | | |
|---|---|---|---|
| Calcd. | C 57.16; | H 3.46; | N 3.70 |
| Found | C 57.29; | H 3.55; | N 3.57 |

(2) 2-[N-benzyl-N-(5-oxotetrahydrofuran-2-carbonyl)]amino-2',5-dichlorobenzophenone

In substantially the same manner as in Example 7 (2), 20 g of 2',5-dichloro-2-(5-oxotetrahydrofuran-2-carbonyl) aminobenzophenone obtained in (1) was allowed to react with benzyl bromide to afford 24.5 g of 2-[N-benzyl-N-(5-oxotetrahydrofuran-2-carbonyl)]amino-2',5-dichlorobenzophenone as an oily product.
$IR\nu_{max}^{Neat}cm^{-1}$ : 1780, 1670(C=O)
$^1$H-NMR spectrum (200MHz,$CDCl_3$) δ :2.1-2.55(2H,m), 2.65-3.0(2H,m), 4.01(d,J=14.4Hz)+4.29(d,J=14.4Hz)(1H), 4.75-4.9(1H,m), 5.29(d=14.4Hz)+5.47(d,J=14.4Hz)(1H), 6.6-7.6(13H,m)

(3) 2-[N-benzyl-N-(4-ethoxycarbonyl-2-hydroxy)butyryl] amino-2',5-dichlorobenzophenone

23 g of 2-[N-benzyl-N-(5-oxotetrahydrofuran-2-carbonyl)]amino-2',5-dichlorobenzophenone obtained in (2) was subjected to substantially the same reaction as in Example 8 (3) to afford 17 g of 2-[N- benzyl-N-(4-ethoxycarbonyl-2-hydroxy)butyryl]amino-2',5-dichlorobenzophenone.
$IR\nu_{max}^{Neat}cm^{-1}$ : 1730, 1660(C=O)
$^1$H-NMR spectrum (200MHz,$CDCl_3$) δ : 1.1-1.35(3H,m), 1.6-2.0(2H,m); 2.3-2.8(2H,m), 3.9-4.8(4H,m), 5.34(d, J=14.4Hz)+5.69(d,J=14.4Hz)(1H), 6.8-7.6(12H,m)

(4) 2-[N-benzyl-N-(4-ethoxycarbonyl-2-methanesulfonyloxy)butyryl]amino-2',5-dichlorobenzophenone

In substantially the same manner as in Example 7 (4), 17 g of 2-[N-benzyl-N-(4-ethoxycarbonyl-2-hydroxy) butyryl] amino-2',5-dichlorobenzophenone was allowed to react with methanesulfonyl chloride to afford 19 g of 2-[N-benzyl-N-(4-ethoxycarbonyl-2-methanesulfonyloxy)butyryl]amino-2',5-dichlorobenzophenone as an oily product.
$IR\nu_{max}^{Neat}cm^{-1}$ : =1730, 1675(C=O)
$^1$H-NMR spectrum (200MHz,$CDCl_3$) δ : 1.1-1.35(3H,m), 1.9-2.6(4H,m), 3.17+3.32(3H,each s), 4.85-5.0(m)+5.05-5.15 (m)(lH), 5.45(d,J=14.4Hz)+5.72(d,J=14.4Hz)(1H), 6.9-7.6(12H,m)

(5) Ethyl ester of cis-1-benzyl-7-chloro-5-(2-chlorophenyl)-2-oxo-1,2,3,5-tetrahydro-4,1-benzoxazepine-3-propionic acid and ethyl ester of trans-1-benzyl-7-chloro-5-(2-chlorophenyl)-2-oxo-1,2,3,5-tetrahydro-4,1-benzoxazepine-3-propionic acid

19 g of 2-[N-benzyl-N-(4-ethoxycarbonyl-2-methane sulfonyloxy)butyryl]amino-2',5-dichlorobenzophenone was subjected to substantially the same reaction as in Example 8 (5). The reaction mixture was purified by means of a silica gel column chromatography. As the first fraction, 3.1 g of ethyl ester of cis-1-benzyl-7-chloro-5-(2-chlorophenyl)-2-oxo-1,2,3,5-tetrahydro-4,1-benzoxazepine-3-propionic acid was obtained in the form of an oily product.
$IR\nu_{max}^{Neat}cm^{-1}$ : 1730, 1670(C=0)

As the second fraction, 3.3 g of ethyl ester of trans-1-benzyl-7-chloro-5-(2-chlorophenyl)-2-oxo-1,2,3,5-tetrahydro-4,1-benzoxazepine-3-propionic acid as prisms, m.p. 122 C-123°C.
$IR\nu_{max}^{Neat}cm^{-1}$ : 1740,1670(C=O)

| Elemental Analysis for $C_{27}H_{25}Cl_2NO_4$: | | | |
|---|---|---|---|
| Calcd. | C 65.07; | H 5.06; | N 2.81 |
| Found | C 65.30; | H 5.09; | N 2.81 |

Example 9

Ethyl ester of 7-chloro-5-(2-chlorophenyl)-1-methyl-2-oxo-1,2,3,5-tetrahydro-4,1-benzoxazepine-3-propionic acid

(1) 2',5-Dichloro-2-[N-methyl-N-(5-oxotetrahydrofuran-2-carbonyl)]aminobenzophenone

In 100 ml of acetone was dissolved 15 g of 2',5-dichloro-2-(5-oxotetrahydrofuran-2-carbonyl)aminobenzophenone obtained in Example 8 (1). To the solution were added 4.96 ml of methyl iodide and 11 g of potassium carbonate, and the mixture was stirred for 3 days at room temperature. The reaction mixture was subjected to distillation under reduced pressure. The residue was subjected to extraction by the addition of 200 ml of water and 300 ml of ethyl acetate. The ethyl acetate layer was washed with water and dried over anhydrous magnesium sulfate, then the solvent was distilled off under reduced pressure. The residue was purified by means of a silica gel chromatography (eluent, hexane : ethyl acetate = 2:1 to 1:1) to afford 13.5 g of 2',5-dichloro-2-[N-methyl-N-(5-oxotetrahydrofuran-2-carbonyl)]aminobenzophenone as an oily product.
$IR\nu^{Neat}_{max}cm^{-1}$ : 1780,1675(C=O)
$^1$H-NMR spectrum (200MHz,$CDCl_3$) δ : 2.1-3.0(4H,m), 3.08+3.14(3H,each s), 4.75-4.9(1H,m), 7.1-7.7(7H,m)

(2) 2',5-Dichloro-2-[N-(4-ethoxycarbonyl-2-hydroxy) butyryl-N-methyl]aminobenzophenone

13.5 g of 2',5-dichloro-2-[N-methyl-N-(5-oxotetrahydro furan-2-carbonyl)]aminobenzophenone was subjected to substantially the same reaction as in Example 7 (3) to afford 9.5 g of 2',5-dichloro-2-[N-(4-ethoxycarbonyl-2-hydroxy) butyryl-N-methyl]aminobenzophenone as an oily product.
$IR\nu^{Neat}_{max}cm^{-1}$ : 1730, 1670, 1660(C=O)
$^1$H-NMR spectrum (200MHz, $CDCl_3$) δ : 1.1-1.4(3H,m), 1.5-1.9(2H,m), 2.0-2.75(2H,m), 3.0-3.5(3H,m), 3.95-4.5(4H, m), 7.2-7.7(7H.,)

(3) 2',5-Dichloro-2-[N-(4-ethoxycarbonyl-2-methanesulfonyloxy)butyryl-N-methyl]aminobenzophenone

In substantially the same manner as in Example 7 (4), 9.5 g of 2',5-dichloro-2-[N-(4-ethoxycarbonyl-2-hydroxy) butyryl-N-methyl]aminobenzophenone obtained in (2) was allowed to react with methanesulfonyl chloride to give 11.0 g of 2',5-dichloro-2-[N-(4-ethoxycarbonyl-2-methanesulfonyloxy)butyryl-N-methyl]aminobenzophenone as an oily product.
$IR\nu^{Neat}_{max}cm^{-1}$ : 1730, 1680(C=O)
$^1$H-NMR spectrum (200MHz, $CDCl_3$) δ : 1.1-1.35(3H,m), 1.9-2.7(4H,m), 3.0-3.7(6H,m), 3.9-4.25(2H,m), 4.9-5.7(1H, m), 7.3-7.75(7H,m)

(4) Ethyl ester of 7-chloro-5-(2-chlorophenyl)-1-methyl-2-oxo-1,2,3,5-tetrahydro-4,1-benzoxazepine-3-propionic acid

11.0 g of 2',5-dichloro-2-[N-(4-ethoxycarbonyl-2-methanesulfonyloxy)butyryl-N-methyl]aminobenzophenone obtained in (3) was subjected to substantially the same reaction in Example 7 (5), and the reaction product was purified by means of a silica gel column chromatography to give 4.17 g of the mixture of cis- and trans-isomers of ethyl ester of 7-chloro-5- (2-chlorophenyl)-1-methyl-2-oxo-1,2,3,5-tetrahydro-4,1-benzoxazepine-3-propionic acid as an oily product.
$IR\nu^{Neat}_{max}cm^{-1}$ : 1735, 1675(C=O)
$^1$H-NMR spectrum (200MHz,$CDCl_3$) δ : 1.19(3H,t,J=7.2Hz), 1.21(3H,t,J=7.2Hz), 2.0-2.4(4H,m), 2.4-2.6(4H,m), 3.15 (3H,s), 3.51(3H,s), 3.95-4.2(5H,m), 4.36(1H,t,J=6.6Hz), 5.99(1H,s), 6.16(1H,s), 6.50(1H,d,J=2.4Hz), 7.05-7.8(13H,m)

Example 10

Ethyl ester of 1-benzyl-5-(4-methoxyphenyl)-2-oxo-1,2,3,5-tetrahydro-4,1-benzoxazepine-3-propionic acid

(1) 4'-Methoxy-2-(5-oxotetrahydrofuran-2-carbonyl)amino benzophenone

In substantially the same manner as in Example 7 (1), 8 g of 2-amino-4'-methoxybenzophenone was allowed to react with 5-oxotetrahydrofuran-2-carbonyl chloride to give 12 g of 4'-methoxy-2-(5-oxotetrahydrofuran-2-carbonyl) aminobenzophenone as an oily product.
$IR\nu^{Neat}_{max}cm^{-1}$ : 1780, 1685, 1625(C=O)
$^1$H-NMR spectrum (200MHz, $CDCl_3$) δ : 2.3-2.8(4H,m), 3.89(3H,s), 4.9-5.05(1H,m), 6.9-7.85(7H,m), 8.5-8.6(1H,m), 11.2(1H,br)

(2) 2-[N-benzyl-N-(5-oxotetrahydrofuran-2-carbonyl)]amino-4'-methoxybenzophenone

In substantially the same manner as in Example 7 (2), 12 g of 4'-methoxy-2-(5-oxotetrahydrofuran-2-carbonyl) aminobenzophenone obtained in (1) was allowed to react with benzyl bromide to give 13.2 g of 2[N-benzyl-N-(5-oxotetrahydrofuran-2-carbonyl)]amino-4'-methoxybenzo-phenone as prisms, m.p. 137°C-138°C.

$IR\nu_{max}^{KBr}$ $cm^{-1}$ : 1775, 1660, 1645(C=O)

| Elemental Analysis for $C_{26}H_{23}NO_5$: | | | |
|---|---|---|---|
| Calcd. | C 72.71; | H 5.40; | N 3.26 |
| Found | C 72.98; | H 5.46; | N 3.22 |

(3) 2-[N-benzyl-N-(4-ethoxycarbonyl-2-hydroxy)butyryl] amino-4'-methoxybenzophenone

13.0 g of 2-[N-benzyl-N-(5-oxotetrahydrofuran-2-carbonyl)]amino-4'-methoxybenzophenone obtained in (2) was subjected to substantially the same reaction as in Example 7 (3) to give 10.5 g of 2-[N-benzyl-N-(4-ethoxycarbonyl-2-hydroxy)butyryl]amino-4'-methoxybenzophenone as an oily product.
$IR\nu_{max}^{Neat}$ $cm^{-1}$ : 1730, 1650(C=O)
$^1H$-NMR spectrum (200MHz,$CDCl_3$) $\delta$ : 1.1-1.35(3H,m), 1.7-2.1(2H,m), 2.2-2.55(2H,m), 3.88(3H,s), 3.9-4.3(3H,m), 4.52(1H,d,J=14.4Hz), 4.99(1H,d,J=14.4Hz), 6.8-7.9(13H,m)

(4) 2-[N-benzyl-N-(4-ethoxycarbonyl-2-methanesulfonyloxy)butyryl]amino-4'-methoxybenzophenone

In substantially the same manner as in Example 7 (4), 10.5 g of 2-[N-benzyl-N-(4-ethoxycarbonyl-2-hydroxy) butyryl]amino-4'-methoxybenzophenone was allowed to react with methanesulfonyl chloride to give 8.5 g of 2-[N-benzyl-N-(4-ethoxycarbonyl-2-methanesulfonyloxy)butyryl]amino-4'-methoxybenzophenone as an oily product.
$IR\nu_{max}^{Neat}$ $cm^{-1}$ : 1730, 1670, 1660(C=O)
$^1H$-NMR spectrum (200MHz,$CDCl_3$) $\delta$ : 1.1-1.3(3H,m), 2.0-2.6(4H,m), 3.14+3.38(3H,each s), 3,89+3.91(3H,each s), 3.9-4.6(3H,m), 4.9-5.65(2H,m), 6.8-7.9(13H,m)

(5) Ethyl ester of cis-1-benzyl-5-(4-methoxyphenyl-2-oxo-1,2,3,5-tetrahydro-4,1-benzoxazepine-3-propionic acid and ethyl ester of trans-1-benzyl-5-(4-methoxyphenyl)-2-oxo-1,2,3,5-tetrahydro-4,1-benzoxazepine-3-propionic acid

8.5 g of 2-[N-benzyl-N-(4-ethoxycarbonyl-2-methanesulfonyloxy)butyryl]amino-4'-methoxybenzophenone obtained in (4) was subjected to substantially the same reaction as in Example 7 (5) to give a mixture product, which was purified by means of a silica gel column chromatography to give, as the first fraction, 2.0 g of ethyl ester of cis-1-benzyl-5-(4-methoxyphenyl)-2-oxo-1,2,3,5-tetrahydro-4,1-benzoxazepine-3-propionic acid as needles, m.p. 95-96°C.
$IR\nu_{max}^{KBr}$ $cm^{-1}$ : 1730, 1670(C=O)

| Elemental Analysis for $C_{28}H_{29}NO_5$: | | | |
|---|---|---|---|
| Calcd. | C 73.18; | H 6.36; | N 3.05 |
| Found | C 73.09; | H 6.42; | N 3.19 |

As the second fraction, 0.88 g of ethyl ester of trans-l-benzyl-5-(4-methoxyphenyl)-2-oxo-1,2,3,5-tetra-hydro-4,1-benzoxazepine-3-propionic acid was obtained as an oily product.
$IR\nu_{max}^{Neat}$ $cm^{-1}$ : 1730, 1670(C=O)
$^1H$-NMR spectrum (200MHz,$CDCl_3$) $\delta$ : 1.17(3H,t,J=7.1Hz), 2.1-2.4(2H,m), 2.47(2H,t,J=7.1Hz), 3.82(1H,s), 3.95-4.2 (3H,m), 4.87(1H,d,J=14.6Hz), 5.42(1H,s), 6.58(1H,d,J=7.2Hz), 6.8-7.5(12H,m)

Example 11

Methyl ester of 1-benzyl-2-oxo-5-phenyl-1,2,3,5-tetrahydro-4,1-benzoxazepine-3-propionic acid

(1) 2-[N-benzyl-N-(2-chloro-4-methoxycarbonyl)butyryl]aminobenzophenone

A mixture of 7.6 g of 2-chloro-4-methoxycarbonyl valeric acid, 9.2 ml of thionyl chloride and 30 ml of toluene was stirred for 30 minutes at 80°C, followed by distilling off the solvent under reduced pressure to leave 2-chloro-4-methoxycarbonyl butyryl chloride. A mixture of this compound, 5.0 g of 2-benzylaminobenzophenone, 100 ml of ethyl acetate and 100 ml of a saturated aqueous solution of sodium hydrogencarbonate was stirred for 30 minutes at room temperature. The ethyl acetate layer was washed with water and dried over anhydrous magnesium sulfate, then the solvent was distilled off under reduced pressure. The residue was purified by means of a silica gel column chromatography (eluent, hexane : ethyl acetate = 5:1) to give 1.2 g of 2-[N-benzyl-N-(2-chloro-4-methoxy carbonyl)butyryl]aminobenzophenone as an oily product.
IR$\nu_{max}^{Neat}$cm$^{-1}$ : 1735, 1655(C=O)
Mass spectrum (m/e) : 449 ($M^+$)
$^1$H-NMR spectrum (200MHz,$CDCl_3$) δ : 2.3-2.8(4H,m), 3.62(3H,s), 4.2-4.3(1H,m), 4.53(1H,d,J=14.4Hz), 4.92(1H,d, J=14.4Hz), 6.9-7.75(14H,m)

(2) 2-[N-benzyl-N-(2-chloro-4-methoxycarbonyl)butyryl] aminodiphenyl methanol

[Method A]

In 30 ml of methanol was dissolved 1.2 g of 2-[N-benzyl-N-(2-chloro-4-methoxycarbonyl)butyryl]amino benzophenone. To the solution was added, under ice-cooling, 0.135 g of sodium borohydride. The mixture was stirred for 30 minutes at room temperature, then the solvent was distilled off under reduced pressure. To the residue was added 100 ml of 1N hydrochloric acid to make the solution acid, which was subjected to extraction with 100 ml of ethyl acetate. The ethyl acetate layer was washed with an aqueous solution of sodium hydrogencarbonate and dried over anhydrous magnesium sulfate, then the solvent was distilled off. The residue was purified by means of a silica gel column chromatography (eluent, hexane : ethyl acetate = 2:1) to give 1.1 g of 2-[N-benzyl-N-(2-chloro-4-methoxycarbonyl) butyryl] aminodiphenyl methanol.
IR$\nu_{max}^{Neat}$cm$^{-1}$ : 3430(OH), 1735, 1660(C=O)
$^1$H-NMR spectrum (200MHz,$CDCl_3$) δ : 1.95-2.65(4H,m), 3.2-4.4(6H,m), 5.1-5.5(1H,m), 5.75-6.15(1H,m), 6.5-7.85 (14H,m)

[Method B]

A mixture of 6.2 g of 2-chloro-4-methoxycarbonyl valeric acid and 12.6 ml of thionyl chloride was refluxed for 30 minutes, then the solvent was distilled off under reduced pressure to leave 2-chloro-4-methoxycarbonyl butyryl chloride. A mixture of this product, 5.0 g of 2-benzylaminodiphenyl methanol, 100 ml of ethyl acetate and 100 ml of a saturated aqueous solution of sodium hydrogencarbonate was stirred for one hour at room temperature. The ethyl acetate layer was washed with water and dried over anhydrous magnesium sulfate. then the solvent was distilled off under reduced pressure. The residue was purified by means of a silica gel column chromatography (eluent, hexane : ethyl acetate = 2:1) to give 5.3 g of 2-[N-benzyl-N-(2-chloro-4-methoxycarbonyl)butyryl]aminodiphenyl methanol as an oily product.

(3) Methyl ester of cis-1-benzyl-2-oxo-5-phenyl-1,2,3,5-tetrahydro-4,1-benzoxazepine-3-propionic acid and methyl ester of trans-l-benzyl-2-oxo-5-phenyl-1,2,3,5-tetrahydro-4,1-benzoxazepine-3-propionic acid

In 20 ml of tetrahydrofuran was dissolved 1.1 g of 2-[N-benzyl-N-(2-chloro-4-methoxycarbonyl)butyryl]aminodiphenyl methanol. To the solution was added 107 mg of sodium hydride (60% in oil), and the mixture was stirred for one hour at room temperature. To the reaction mixture was added 50 ml of 1N hydrochloric acid to make the solution acid, which was subjected to extraction with 100 ml of ethyl acetate. The ethyl acetate layer was washed with an aqueous solution of sodium hydrogencarbonate and dried over anhydrous magnesium sulfate, then the solvent was distilled off under reduced pressure. The residue was purified by means of a silica gel column chromatography (eluent, hexane : ethyl acetate = 5:1). From the first fraction, 0.4 g of methyl ester of cis-1-benzyl-2-oxo-5-phenyl-1,2,3,5-tetrahydro-4,1-benzoxazepine-3-propionic acid as plates, m.p. 134-136°C
IR$\nu_{max}^{KBr}$cm$^{-1}$ : 1740, 1670(C=O)

| Elemental Analysis for $C_{25}H_{25}NO_4$: | | | |
|---|---|---|---|
| Calcd. | C 75.16; | H 6.06; | N 3.37 |
| Found | C 74.74; | H 5.97; | N 3.38 |

From the second fraction, 0.18 g of of methyl ester of trans-1-benzyl-2-oxo-5-phenyl-1,2,3,5-tetrahydro-4,1-benzoxazepine- 3-propionic acid was obtained as needles, m.p. 116-118°C.
IR$\nu_{max}^{KBr}$cm$^{-1}$ : 1740, 1670(C=O)

| Elemental Analysis for $C_{26}H_{25}NO_4$: | | | |
|---|---|---|---|
| Calcd. | C 75.16; | H 6.06; | N 3.73 |
| Found | C 75.22; | H 5.94; | N 3.58 |

Example 12

Cis-1-benzyl-2-oxo-5-phenyl-1,2,3,5-tetrahydro-4,1-benzoxazepine-3-propionic acid

In 20 ml of methanol was dissolved 0.5 g of ethyl ester of cis-1-benzyl-2-oxo-5-phenyl-1,2,3,5-tetrahydro-4,1-benzoxazepine-3-propionic acid obtained in Example 8. To the solution was added 7 ml of 1N sodium hydroxide, and the mixture was stirred for 30 minutes at room temperature. The reaction mixture was acidified with 100 ml of 1N hydrochloric acid, which was subjected to extraction with 150 ml of ethyl acetate. The ethyl acetate layer was washed with water and dried over anhydrous magnesium sulfate. Then the solvent was distilled off under reduced pressure to leave 0.46 g of cis-1-benzyl-2-oxo-5-phenyl-1,2,3,5-tetrahydro-4,1-benzoxazepine-3-propionic acid as plates, m.p. 112-114°C.
IR$\nu_{max}^{KBr}$ $cm^{-1}$ : 1710, 1670(C=O)

| Elemental Analysis for $C_{25}H_{23}NO_4$: | | | |
|---|---|---|---|
| Calcd. | C 74.80; | H 5.77; | N 3.49 |
| Found | C 74.52; | H 5.85; | N 4.42 |

Example 13

Trans-1-benzyl-2-oxo-5-phenyl-1,2,3,5-tetrahydro-4,1-benzoxazepine-3-propionic acid

In substantially the same manner as in Example 12, 1.0 g of ethyl ester of trans-1-benzyl-2-oxo-5-phenyl-1,2,3,5-tetrahydro-4,1-benzoxazepine-3-propionic acid obtained in Example 7 was subjected to hydrolysis to give 0.81 g of trans-1-benzyl-2-oxo-5-phenyl-1,2,3,5-tetrahydro-4,1-benzoxazepine-3-propionic acid as plates, m.p. 148-150°C.
IR$\nu_{max}^{KBr}$ $cm^{-1}$ : 1735, 1650(C=O)

| Elemental Analysis for $C_{25}H_{23}NO_4$: | | | |
|---|---|---|---|
| Calcd. | C 74.80; | H 5.77; | N 3.49 |
| Found. | C 74.66; | H 5.78; | N 3.55 |

In substantially the same manner as above, 0.9 g of methyl ester of trans-1-benzyl-2-oxo-5-phenyl-1,2,3,5-tetrahydro-4,1-benzoxazepine-3-propionic acid obtained in Example 11 was subjected to hydrolysis to give 0.86 g of trans-1-benzyl-2-oxo-5-phenyl-1,2,3,5-tetrahydro-4,1-benzoxazepine-3-propionic acid.

Example 14

Cis-1-benzyl-7-chloro-5-(2-chlorophenyl)-2-oxo-1,2,3,5-tetrahydro-4,1-benzoxazepine-3-propionic acid

In substantially the same manner as in Example 12, 3.1 g of ethyl ester of cis-l-benzyl-7-chloro-5-(2-chlorophenyl)-2-oxo-1,2,3,5-tetrahydro-4,1-benzoxazepine-3-propionic acid obtained in Example 8 was subjected to hydrolysis to give 2.9 g of cis-1-benzyl-7-chloro-5-(2-chlorophenyl)-2-oxo-1,2,3,5-tetrahydro-4,1-benzoxazepine-3-propionic acid as prisms, m.p. 135-137°C.
IR$\nu_{max}^{KBr}$ $cm^{-1}$ : 1725, 1640(C=O)

| Elemental Analysis for $C_{25}H_{21}Cl_2NO_4$: | | | |
|---|---|---|---|
| Calcd. | C 63.84; | H 4.50; | N 2.98 |
| Found. | C 64.02; | H 4.76; | N 2.88 |

Example 15

Trans-1-benzyl-7-chloro-5-(2-chlorophenyl)-2-oxo-1,2,3,5-tetrahydro-4,1-benzoxazepine-3-propionic acid

In substantially the same manner as in Example 12, 2.7 g of ethyl ester of trans-1-benzyl-7-chloro-5-(2-chlorophenyl)-2-oxo-1,2,3,5-tetrahydro-4,1-benzoxazepine-3-propionic acid obtained in Example 8 was subjected to hydrolysis to give 2.5 g trans-1-benzyl-7-chloro-5-(2-chlorophenyl)-2-oxo-1,2,3,5-tetrahydro-4,1-benzoxazepine-3-propionic acid as prisms, m.p. 192-194°C.
$IR\nu_{max}^{KBr} cm^{-1}$ : 1730, 1640(C=O)

| Elemental Analysis for $C_{25}H_{21}Cl_2NO_4$: | | | |
|---|---|---|---|
| Calcd. | C 63.84; | H 4.50; | N 2.98 |
| Found | C 63.99; | H 4.57; | N 2.92 |

Example 16

7-Chloro-5-(2-chlorophenyl)-1-methyl-2-oxo-1,2,3,5-tetrahydro-4,1-benzoxazepine-3-propionic acid

In substantially the same manner as in Example 12 4.1 g of ethyl ester of 7-chloro-5-(2-chlorophenyl)-1-methyl-2-oxo-1,2,3,5-tetrahydro-4,1-benzoxazepine-3-propionic acid obtained in Example 9 was subjected to hydrolysis to give 3.7 g of 7-chloro-5-(2-chlorophenyl)-1-methyl-2-oxo-1,2,3,5-tetrahydro-4,1-benzoxazepine-3-propionic acid as an oily product of the mixture of cis- and trans-compounds (1:1).
$IR\nu_{max}^{Neat} cm^{-1}$ : 1705, 1670(C=O)
$^1H$-NMR spectrum (200MHz,$CDCl_3$) δ : 2.0-2.4(4H,m), 2.45-2.7(4H,m), 3.14(3H,s), 3.50(3H,s), 4.00(1H,dd,J=7.2 x 5.4Hz), 4.35(1H,t,J=6.7Hz), 5.99(1H,s), 6.16(1H,s), 6.50(1H,d,J=2.2Hz), 7.0-7.81(13H,m)

Example 17

Cis-1-benzyl-5-(4-methoxyphenyl)-2-oxo-1,2,3,5-tetrahydro-4,1-benzoxazepine-3-propionic acid

In substantially the same manner as in Example 12 1.7 g of ethyl ester of cis-1-benzyl-5-(4-methoxyphenyl)-2- oxo-1,2,3,5-tetrahydro-4,1-benzoxazepine-3-propionic acid obtained in Example 10 was subjected to hydrolysis to give 1.5 g of cis-1-benzyl-5-(4-methoxyphenyl)-2-oxo-1,2,3,5-tetrahydro-4,1-benzoxazepine-3-propionic acid as prisms, m.p. 107°C-109°C.
$IR\nu_{max}^{KBr} cm^{-1}$ : 1715, 1670(C=O)

| Elemental Analysis for $C_{26}H_{25}NO_5$: | | | |
|---|---|---|---|
| Calcd. | C 72.37; | H 5.84; | N 3.25 |
| Found | C 72.26; | H 5.90; | N 3.10 |

Example 18

Trans-l-benzyl-5-(4-methoxyphenyl)-2-oxo-1,2,3,5-tetrahydro-4,1-benzoxazepine-3-propionic acid

In substantially the same manner as in Example 12 0.85 g of ethyl ester of trans-1-benzyl-5-(4-methoxyphenyl)-2-oxo-1,2,3,5-tetrahydro-4,1-benzoxazepine-3-propionic acid was subjected to hydrolysis to give 0.78 g of trans-1-benzyl-5-(4-methoxyphenyl)-2-oxo-1,2,3,5-tetrahydro-4,1-benzoxazepine-3-propionic acid as an oily product.
$IR\nu_{max}^{Neat} cm^{-1}$ : 1710, 1670(C=O)
$^1H$-NMR spectrum (200MHz,$CDCl_3$) δ : 2.05-2.35(2H,m), 2.52(2H,t,J=7.1Hz), 3.81(1H,s), 4.00(1H,dd,J=7.3 x 5.7Hz), 4.39(1H,d,J=14.4Hz), 5.40(1H,s), 5.49(1H,d,J=14.4Hz), 6.57(1H,d,J=7.2Hz), 6.8-7.5(12H,m)

Example 19

Trans-1-benzyl-3-(3-hydroxypropyl)-5-phenyl-1,5-dihydro-4,1-benzoxazepine-2(3H)-one

The mixture of 0.4 g of methyl ester of trans-1-benzyl-2-oxo-5-phenyl-1,2,3,5-tetrahydro-4,1-benzoxazepine-3-propionic acid obtained in Example 11, 0.16 g of sodium borohydride and 0.16 g of lithium chloride in 15 ml of tetrahydrofuran were stirred for 10 minutes at room temperature. To the resultant was added 30 ml of ethanol, which was stirred for 2 hours at 60°C. To the reaction mixture were added 100 ml of 1N hydrochloric acid and 150 ml of ethyl acetate. The ethyl acetate layer was washed with an aqueous solution of sodium hydrogencarbonate, which was dried over anhydrous magnesium sulfate, then the solvent was distilled off under reduced pressure. The residue was purified by means of a silica gel column chromatography (eluent, hexane : ethyl acetate = 2:1) to give 0.19 g of trans-1-benzyl-3-(3-hydroxypropyl)-5-phenyl-1,5-dihydro-4,1-benzoxazepin-2(3H)-one as needles, m.p. 59-62°C.
$IR\nu_{max}^{KBr} cm^{-1}$ : 1645 (C=O)

| Elemental Analysis for $C_{25}H_{25}NO_3$: | | | |
|---|---|---|---|
| Calcd. | C 77.49; | H 6.50; | N 3.61 |
| Found | C 77.12; | H 6.44; | N 3.87 |

Example 20

Cis-1-benzyl-3-(3-3-hydroxypropyl)-5-phenyl-1,5-dihydro-4,1-benzoxazepin-2(3H)-one

In substantially the same manner as in Example 19, 0.23 g of methyl ester of cis-1-benzyl-2-oxo-5-phenyl-1,2,3,5-tetrahydro-4,1-benzoxazepine-3-propionic acid obtained in Example 12 was subjected to reduction to give 0.09 g of cis-1-benzyl-3-(3-hydroxypropyl)-5-phenyl-1,5-dihydro-4,1-bezoxazepin-2(3H)-one as needles, m.p. 94-95°C.
$IR\nu_{max}^{KBr} cm^{-1}$ : 1675, 1660(C=O)

| Elemental Analysis for $C_{25}H_{25}NO_3$: | | | |
|---|---|---|---|
| Calcd. | C 77.49; | H 6.50; | N 3.61 |
| Found | C 77.44; | H 6.49; | N 3.76 |

Example 21

N-(cis-1-benzyl-2-oxo-5-phenyl-1,2,3,5-tetrahydro-4,1-benzoxazepine-3-propionyl)aminoethanol

In 5 ml of N,N-dimethylformamide were dissolved 0.3 g of cis-1-benzyl-2-oxo-5-phenyl-1,2,3,5-tetrahydro-4,1-benzoxazepine-3-propionic acid obtained in Example 12 and 0.054 ml of ethanolamine. To the solution were added, under ice-cooling, 0.17 g of diethyl phosphorocyanidate and 0.14 ml of triethylamine. The mixture was stirred for 30 minutes at room temperature, to which was added 100 ml of water. The mixture was subjected to extraction with ethyl acetate (100 ml x 2). The ethyl acetate layer was washed with 1N hydrochloric acid and an aqueous solution of sodium hydrogencarbonate, successively, which was then dried over anhydrous magnesium sulfate.
The solvent was distilled off under reduced pressure.
The residue was purified by means of a silica gel column chromatography (eluent, hexane: methylene chloride: ethanol=5:5:1) to give 0.28 g of N-(cis-1-benzyl-2-oxo-5-phenyl-1,2,3,5-tetrahydro-4,1-benzoxazepine-3-propionyl) aminoethanol as needles, m.p. 117-119°C.
$IR\nu_{max}^{KBr} cm^{-1}$ : 1670, 1640 (C=O)

| Elemental Analysis for $C_{27}H_{28}N_2O_4.0.5H_2O$: | | | |
|---|---|---|---|
| Calcd. | C 71.50; | H 6.44; | N 6.18 |
| Found | C 71.29; | H 6.47; | N 5.98 |

Example 22

N-(trans-1-benzyl-2-oxo-5-phenyl-1,2,3,5-tetrahydro-4,1-benzoxazepine-3-propionyl)aminoehanol

In substantially the same manner as in Example 21 0.25 g of trans-1-benzyl-2-oxo-5-phenyl-1,2,3,5-tetrahydro-4,1-benzoxazepine-3-propionic acid obtained in Example 14 was subjected to condensation with ethanolamine to give 0.20 g of N-(trans-1-benzyl-2-oxo-5-phenyl-1,2,3,5-tetrahydro-4,1-benzoxazepine-3-propionyl)aminoethanol as plates, m.p. 125°C-127°C.
$IR\nu_{max}^{KBr} cm^{-1}$ : 1675, 1645(C=O)

| Elemental Analysis for $C_{27}H_{28}N_2O_4$: | | | |
|---|---|---|---|
| Calcd. | C 72.95; | H 6.35; | N 6.30 |
| Found | C 72.70; | H 6.36; | N 6.19 |

Example 23

Methyl ester of N-(cis-l-benzyl-2-oxo-5-phenyl-1,2,3,5-tetrahydro-4,1-benzoxazepine-3-propionyl)-L-tryptophane

In 10 ml of N,N-dimethylformamide were dissolved 0.35 g of cis-1-benzyl-2-oxo-5-phenyl-1,2,3,5-tetrahydro-4,1-benzoxazepine-3-propionic acid obtained in Example 12 and 0.23 g of L-tryptophane methyl ester hydrochloride. To the solution were added, under ice-cooling, 0.15 g of diethyl phosphorocyanidate and 0.24 ml of triethylamine. The mixture was stirred for 30 minutes at room temperature, which was subjected to extraction with the addition of 100 ml of water and 100 ml of ethyl acetate. The ethyl acetate layer was washed with 1N hydrochloric acid and an aqueous solution of sodium hydrogencarbonate, successively, which was then dried over anhydrous magnesium sulfate.

The solvent was distilled off, and the residue was purified by means of a silica gel column chromatography (eluent, hexane:ethyl acetate = 1:1) to give 0.50 g of methyl ester of N-(cis-1-benzyl-2-oxo-5-phenyl-1,2,3,5-tetrahydro-4,1-benzoxazepine-3-propionyl)-L-tryptophane as an oily product.

IR$\nu_{max}^{Neat}$ $cm^{-1}$ : 1740, 1665(C=O)

$^1$H-NMR spectrum (200MHz, $CDCl_3$) $\delta$ : 2.15-2.5(4H,m), 3.27(2H,t,J=4.7Hz), 3.62+3.63(3H,each s), 3.71(1H,d, J=16.0Hz), 4.15-4.3(1H,m), 4.68(1H,d,J=16.0Hz), 4.8-5.0(1H,m), 5.85(1H,s), 6.15-6.35(1H,m), 6.9-7.6(19H,m), 8.13 (1H,br)

Example 24

N-(cis-l-benzyl-2-oxo-5-phenyl-1,2,3,5-tetrahydro-4,1-benzoxazepine-3-propionyl)-L-tryptophane

In 10 ml of methanol was dissolved 0.5 g of methyl ester of N-(cis-1-benzyl-2-oxo-5-phenyl-1,2,3,5-tetrahydro-4,1-benzoxazepine-3-propionyl)-L-tryptophane obtained in Example 23. To the solution was added 5 ml of 1N sodium hydroxide, and the mixture was stirred for one hour at room temperature. The reaction mixture was made acid by the addition of 100 ml of 1N hydrochloric acid, which was subjected to extraction with 100 ml of ethyl acetate. The ethyl acetate layer was washed with water and dried over anhydrous magnesium sulfate. The solvent was then distilled off under reduced pressure to give 0.24 g of N-(cis-1-benzyl-2-oxo-5-phenyl-1,2,3,5-tetrahydro-4,1- benzoxazepine-3-propionyl)-L-tryptophane as a powdery product.

IR$\nu_{max}^{KBr}$ $cm^{-1}$ : 1730, 1660(C=O)

| Elemental Analysis for $C_{36}H_{33}N_3O_5$ | | | |
|---|---|---|---|
| Calcd. | C 73.58; | H 5.66; | N 7.15 |
| Found | C 73.56; | H 6.07; | N 6.79 |

Example 25

Methyl ester of N-(trans-1-benzyl-2-oxo-5-phenyl-1,2,3,5-tetrahydro-4,1-benzoxazepine-3-propionyl)-L-tryptophane

In substantially the same manner as in Example 23, 0.2 g of trans-1-benzyl-2-oxo-5-phenyl-1,2,3,5-tetrahydro-4,1-benzoxazepine-3-propionic acid obtained in Example 14 was subjected to condensation with L-tryptophane methyl ester hydrochloride to give 0.28 g of methyl ester of N-(trans-1-benzyl-2-oxo-5-phenyl-1,2,3,5-tetrahydro-4,1-benzoxazepine-3-propionyl)-L-tryptophane as an oily product.

IR$\nu_{max}^{Neat}$ $cm^{-1}$ : 1740,1670(C=0)

$^1$H-NMR spectrum (200MHz,$CDCl_3$) $\delta$ : 2.1-2.4(4H,m), 3.15-3.3(2H,m), 3.6-3.7(3H,m), 3.95-4.15(1H,m), 4.8-5.0(2H, m), 5.45(1H,s), 5.49(1H,d,J=14.2Hz), 6.05-6.2(1H,m), 6.45-6.6(1H,m), 6.9-7.6(18H,m), 8.03(1H,br)

Example 26

N-(trans-1-benzyl-2-oxo-5-phenyl-1,2,3,5-tetrahydro-4,1-benzoxazepine-3-propionyl)-L-tryptophane

In substantially the same manner as in Example 24, 0.28 g of methyl ester of N-(trans-1-benzyl-2-oxo-5-phenyl-1,2,3,5-tetrahydro-4,1-benzoxazepine-3-propionyl)-L-tryptophane obtained in Example 25 was subjected to hydrolysis to give 0.24 g of N-(trans-1-benzyl-2-oxo-5-phenyl-1,2,3,5-tetrahydro-4,1-benzoxazepine-3-propionyl)-L-tryptophane as a powdery product.
$IR\nu_{max}^{KBr} cm^{-1}$ : 1730, 1660(C=O)

| Elemental Analysis for $C_{36}H_{33}N_3O_5.0.4H_2O$: | | | |
|---|---|---|---|
| Calcd. | C 72.69; | H 5.73; | N 7.06 |
| Found | C 72.82; | H 5.84; | N 6.79 |

Example 27

Methyl ester of N-[trans-1-benzyl-7-chloro-5-(2-chlorophenyl)-2-oxo-1,2,3,5-tetrahydro-4,1-benzoxazepine-3-propionyl]-L-tryptophane

In substantially the same manner as in Example 23, 0.3 g of trans-1-benzyl-7-chloro-5-(2-chlorophenyl)-2-oxo-1,2,3,5-tetrahydro-4,1-benzoxazepine-2-propionic acid obtained in Example 15 was subjected to condensation with L-tryptophane methyl ester hydrochloride to give 0.41 g of methyl ester of N-[trans-1-benzyl-7-chloro-5-(2-chlorophenyl)-2-oxo-1,2,3,5-tetrahydro-4,1-benzoxazepine-3-propionyl]-L-tryptophane as an oily product.
$IR\nu_{max}^{Neat} cm^{-1}$ : 1735, 1660(C=O)
$^1H$-NMR spectrum (200MHz,$CDCl_3$) δ : 2.1-2.45(4H,m), 3.2-3.35(2H,m), 3.64+3.66(3H,each s), 3.95-4.15(1H,m), 4.65-4.8(1H,m), 4.8-5.0(1H,m), 5.5-5.65(1H,m), 5.76(1H,s), 6.0-6.2(1H,m), 6.35-6.45(1H,m), 6.9-7.7(16H,m), 8.16 (1H,br)

Example 28

N-[trans-1-benzyl-7-chloro-5-(2-chlorophenyl)-2-oxo-1,2,3,5-tetrahydro-4,1-benzoxazepine-3-propionyl]-L-tryptophane

In substantially the same manner as in Example 24, methyl ester of N-[trans-1-benzyl-7-chloro-5-(2-chlorophenyl)-2-oxo-1,2,3,5-tetrahydro-4,1-benzoxazepine-3-propionyl]-L-tryptophane (0.41 g) obtained in Example 27 was subjected to hydrolysis to give 0.31 g of N-[trans-1-benzyl-7-chloro-5-(2-chlorophenyl)-2-oxo-1,2,3,5-tetrahydro-4,1-benzoxazepine-3-propionyl]-L-tryptophane as a powdery product.
$IR\nu_{max}^{KBr} cm^{-1}$ : 1730, 1660(C=O)

| Elemental Analysis for $C_{36}H_{31}Cl_2N_3O_5$: | | | |
|---|---|---|---|
| Calcd. | C 65.86; | H 4.76; | N 6.40 |
| Found | C 66.13; | H 5.02; | N 6.24 |

Example 29

Ethyl ester of N-[trans-1-benzyl-7-chloro-5-(2-chlorophenyl)-2-oxo-1,2,3,5-tetrahydro-4,1-benzoxazepine-3-propionyl] -D-tryptophane

In substantially the same manner as in Example 23, 0.3 g of trans-1-benzyl-7-chloro-5-(2-chlorophenyl)-2-oxo-1,2,3,5-tetrahydro-4,1-benzoxazepine-3-propionic acid obtained in Example 15 was subjected to condensation with D-tryptophane ethyl ester hydrochloride to give 0.43 g of ethyl ester of N-[trans-1-benzyl-7-chloro-5-(2-chlorophenyl)-2-oxo-1,2,3,5-tetrahydro-4,1-benzoxazepine-3-propionyl]-D-tryptophane as an oily product.
$IR\nu_{max}^{Neat} cm^{-1}$ : 1730, 1665(C=O)
$^1H$-NMR spectrum (200MHz,$CDCl_3$) δ : 1.1-1.3(3H,m), 2.1-2.45(4H,m), 3.2-3.35(2H,m), 3.95-4.2(3H,m), 4.65-4,8(1H, m), 4.8-5.0(1H,m), 5.5-5.65(1H,m), 5.76(1H,s), 6.0-6.2(1H,m), 6.35-6.45(1H,m), 6.9-7.7(16H,m), 8.13(1H,br)

Example 30

N-[trans-1-benzyl-7-chloro-5-(2-chlorophenyl)-2-oxo-1,2,3,5-tetrahydro-4,1-benzoxazepine-3-propionyl]-D-tryptophane

In substantially the same manner as in Example 24, 0.43 g of ethyl ester of N-[trans-1-benzyl-7-chloro-5-(2-chlorophenyl)-2-oxo-1,2,3,5-tetrahydro-4,1-benzoxazepine-3-propionyl]-D-tryptophane obtained in Example 30 was subjected to hydrolysis to give 0.38 g of N-[trans-1-benzyl-7-chloro-5-(2-chlorophenyl)-2-oxo-1,2,3,5-tetrahydro-4,1-benzoxazepine-3-propionyl]-D-tryptophane as a powdery product.
$IR\nu_{max}^{KBr}\ cm^{-1}$ : 1725, 1660(C=O)

| Elemental Analysis for $C_{36}H_{31}Cl_2N_3O_5$: | | | |
|---|---|---|---|
| Calcd. | C 65.86; | H 4.76; | N 6.40 |
| Found | C 65.90; | H 5.15; | N 6.03 |

Example 31

Methyl ester of N-[trans-7-chloro-5-(2-chlorophenyl)-1-methyl-2-oxo-1,2,3,5-tetrahydro-4,1-benzoxazepine-3-ylacetyl]-L-tryptophane

To a mixture of 0.6 g of trans-7-chloro-5-(2-chlorophenyl)-1-methyl-2-oxo-1,2,3,5-tetrahydro-4,1-benzoxazepine-3-acetic acid obtained in Reference Example 4, 0.42 g of tryptophane methyl ester hydrochloride and 10 ml of dimethylformamide was added, while stirring under ice-cooling,. 0.3 g of diethyl phosphorocyanidate. To the resultant was then added 0.55 ml of triethylamine. The reaction mixture was stirred for 40 minutes at room temperature, which was poured into ice-water, followed by subjecting the mixture to extraction with ethyl acetate. The organic layer was washed with a dilute aqueous solution of potassium hydrogensulfate, an aqueous solution of sodium hydrogencarbonate and water, successively, dried and concentrated under reduced pressure. The concentrate was purified by means of a silica gel column chromatography (eluent, hexane : ethyl acetate = 1:1 to 1:3). From the first fraction of the eluate, 0.33 g of methyl ester of N-[trans-7-chloro-5-(2-chlorophenyl)-1-methyl-2-oxo-1,2,3,5-tetrahydro-4,1-benzoxazepin-3-yl acetyl]tryptophane of a crystalline form was obtained, m.p. 236°C-237°C.

| Elemental Analysis for $C_{30}H_{27}Cl_2N_3O_5$: | | | |
|---|---|---|---|
| Calcd. | C 62.08; | H 4.69; | N 7.24 |
| Found | C 61.82; | H 4.71; | N 6.95 |

From the subsequent fraction of the eluate, 0.28 g of the steric isomer of the above compound was obtained, m.p. 159°C-160°C.

| Elemental Analysis for $C_{30}H_{27}Cl_2N_3O_5$: | | | |
|---|---|---|---|
| Calcd. | C 62.08; | H 4.69; | N 7.24 |
| Found | C 61.94; | H 4.50; | N 6.96 |

Example 32

In substantially the same manner as in Example 31, compounds shown in Table 24 through Table 28 were obtained by using the compounds in Reference Example 5 and Example 2.
The compounds concerned have the following formula

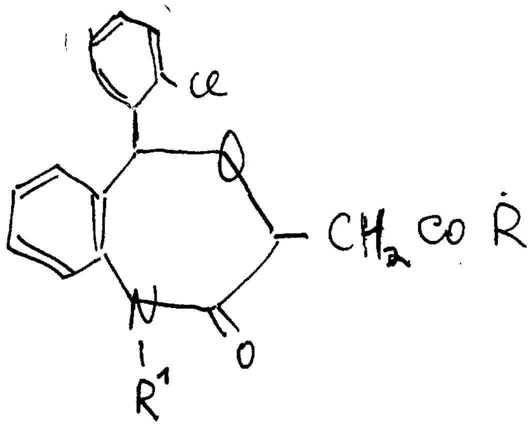

Table 24
Example 32

| compd. No. | $R_1$ | R | m.p. (℃) | Formula | Elemental Analysis (Found) C | H | N |
|---|---|---|---|---|---|---|---|
| 1 | 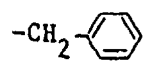 | $-NHCH_2COOC_2H_5$ | 153-155 | $C_{28}H_{28}Cl_2N_2O_5$ | 62.11 (62.05) | 4.84 (4.65) | 5.17 (4.98) |
| 2 | 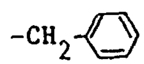 | $-NH(CH_2)_2COOC_2H_5$ | 130-131 | $C_{29}H_{28}Cl_2N_2O_5$ | 62.71 (63.00) | 5.08 (5.03) | 5.04 (4.99) |
| 3 | 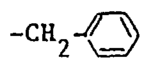 | Trp·OMe = 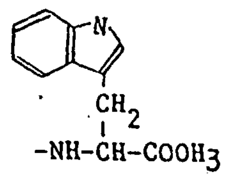 | amorphous solid | $C_{38}H_{31}Cl_2N_3O_5$ $\cdot H_2O$ | 64.10 (64.32) | 4.93 (4.78) | 6.23 (6.07) |
| 4 | 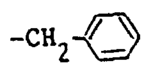 | Trp·OMe | amorphous solid | $C_{38}H_{31}Cl_2N_3O_5$ $\cdot 5/4H_2O$ | 63.67 (63.94) | 4.97 (4.84) | 6.19 (5.90) |
| 5 | 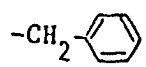 | D-Trp·OMe | amorphous solid | $C_{38}H_{31}Cl_2N_3O_5$ $\cdot 1/2C_4H_8O_2 \cdot 1/2H_2O$ | 60.10 (60.11) | 4.88 (4.89) | 6.78 (6.69) |

Table 25

Example 32

| compd. No. | $R_1$ | R | m. p. (℃) | Formula | Elemental Analysis (Found) C | H | N |
|---|---|---|---|---|---|---|---|
| 6 | 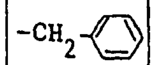 | D-Trp·OMe | amorphous solid | $C_{36}H_{31}Cl_2N_3O_5 \cdot 1/2C_4H_8O_2 \cdot 1/4H_2O$ | 60.53 (60.44) | 4.84 (4.85) | 6.83 (6.83) |
| 7 | 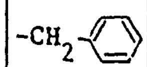 | Phe·OEt = 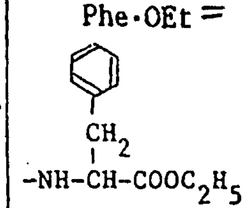 | amorphous solid | $C_{35}H_{32}Cl_2N_2O_5 \cdot 1/4H_2O$ | 66.09 (66.10) | 5.15 (5.15) | 4.41 (4.17) |
| 8 | 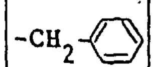 | Phe·OEt | amorphous solid | $C_{35}H_{32}Cl_3N_2O_5 \cdot 1/4H_2O$ | 66.09 (66.20) | 5.15 (5.15) | 4.41 (4.17) |
| 9 | 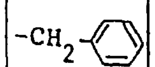 | D-Phe·OMe | amorphous solid | $C_{34}H_{30}Cl_2N_2O_5 \cdot 1/4H_2O$ | 65.65 (65.62) | 4.94 (5.17) | 4.50 (4.51) |
| 10 | 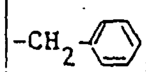 | D-Phe·OMe | amorphous solid | $C_{34}H_{30}Cl_2N_2O_5 \cdot 1/4H_2O$ | 65.65 (65.48) | 4.94 (5.22) | 4.50 (4.31) |

Table 26
Example 32

| compd. No. | $R_1$ | R | m.p. (℃) | Formula | Elemental Analysis (Found) C | H | N |
|---|---|---|---|---|---|---|---|
| 11 | 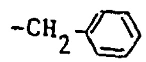 | 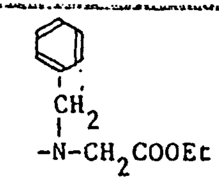 | 164-165 | $C_{35}H_{32}Cl_2N_2O_5 \cdot 1/4H_2O$ | 66.08 (66.18) | 5.17 (5.44) | 4.40 (4.13) |
| 12 | 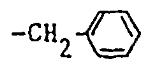 | 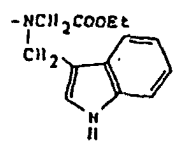 | amorphous solid | $C_{37}H_{33}Cl_2N_3O_5 \cdot 2.5H_2O$ | 62.10 (62.32) | 5.35 (5.18) | 5.87 (5.63) |
| 13 | $-C_2H_5$ | $-NHCH_2COOC_2H_5$ | 175-176 | $C_{23}H_{24}Cl_2N_2O_5$ | 57.63 (57.29) | 5.05 (4.94) | 5.84 (5.90) |
| 14 | $-(CH_2)_6CH_3$ | $-NHCH_2COOC_2H_5$ | 160-161 | $C_{28}H_{34}Cl_2N_2O_5$ | 61.20 (61.47) | 6.24 (6.23) | 5.10 (5.39) |
| 15 | $-CH_2CH(CH_3)_2$ | $-NHCH_2COOC_2H_5$ | 168-170 | $C_{25}H_{28}Cl_2N_2O_5$ | 59.18 (58.97) | 5.56 (5.65) | 5.52 (5.40) |
| 16 | 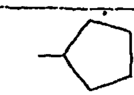 | $-NHCH_2COOC_2H_5$ | 206-207 | $C_{26}H_{28}Cl_2N_2O_5$ | 60.12 (60.02) | 5.43 (5.51) | 5.39 (5.03) |

Table 27

Example 32

| Compd. No. | $R_1$ | $R_2$ | m.p. (℃) | Formula | Elemental Analysis(Found) C | H | N |
|---|---|---|---|---|---|---|---|
| 17 | $-CH_2CH(C_2H_5)_2$ | $-NHCH_2COOC_2H_5$ | 142-143 | $C_{27}H_{32}Cl_2N_2O_5$ | 60.56 (60.80) | 6.02 (6.06) | 5.23 (5.24) |
| 18 | $-CH_2C(CH_3)_3$ | $-NHCH_2COOC_2H_5$ | 174-175 | $C_{26}H_{30}Cl_2N_2O_5$ | 59.89 (59.97) | 5.80 (5.91) | 5.37 (5.64) |
| 19 | $-(CH_2)_2CH(CH_3)_2$ | $-NHCH_2COOC_2H_5$ | 174-175 | $C_{26}H_{30}Cl_2N_2O_5$ | 59.89 (59.69) | 5.80 (5.64) | 5.37 (5.34) |
| 20 | $-CH(C_2H_5)_2$ | $-NHCH_2COOC_2H_5$ | 128-129 | $C_{26}H_{30}Cl_2N_2O_5 \cdot 3/4H_2O$ | 58.37 (58.37) | 5.93 (5.85) | 5.24 (5.36) |
| 21 | $-CH_2C(CH_3)_3$ | $-NHCH_2CH_2COOC_2H_5$ | 172-173 | $C_{27}H_{32}Cl_2N_2O_5$ | 60.56 (60.49 | 6.02 6.15 | 5.23 4.99) |

Table 28

Example 32

| compd. No. | $R_1$ | R[illegible] | m. p. (℃) | Formula | Elemental Analysis(Found) C | H | N |
|---|---|---|---|---|---|---|---|
| 22 | $-CH_2C(CH_3)_3$ | $CH(CH_3)_2$<br>\|<br>NHCHCOOMe | 190-192 | $C_{28}H_{34}Cl_2N_2O_5$ | 61.20<br>(61.25 | 6.24<br>6.28 | 5.10<br>5.18) |

Example 33

N-[trans-7-chloro-5-(2-chlorophenyl)-1-methyl-1,2,3,5-tetrahydro-2-oxo-4,1-benzoxazepine-3-ylacetyl]-L-tryptophane

0.2 g of methyl ester of N-[trans-7-chloro-5-(2-chlorophenyl)-1-methyl-1,2,3,5-tetrahydro-2-oxo-4,1-benzoxazepin-3-ylacetyl]tryptophane obtained from the first fraction in Example 31 was dissolved in a mixture of 8 ml of methanol and 4 ml of tetrahydrofuran. To the solution were added 200 mg of potassium carbonate and 5 ml of water, and the mixture was stirred for 3 hours at 60°C. The reaction mixture was concentrated under reduced pressure. With 1N hydrochloric acid, the pH of the concentrate was adjusted to 3, followed by extraction with ethyl acetate. The organic layer was washed with water and dried, and the solvent was distilled off under reduced pressure. The residue was purified by means of a silica gel column chromatography (eluent, methylene chloride : methanol : water = 100:15:1) to give 0.13 g of N-[trans-7-chloro-5-(2-chlorophenyl)-1-methyl-1,2,3,5-tetrahydro-2-oxo-4,1-benzoxazepin-3-ylacetyl]-L-tryptophane as a colorless powdery product.

$IR\nu^{KBr}_{max}$ $cm^{-1}$ : 3700-2200(COOH), 1660(CO), 1485, 1250, 1110, 740

$^1$H-NMR spectrum (200MHz, $CDCl_3$) δ : 2.2-3.2(4H,m), 3.24(3H,s,N-$\underline{CH_3}$), 4.2(1H,m,$C_3$-H), 4.63(1H,m), 5.73(1H,s,$C_5$-H), 6.34(1H,d,$C_6$-H), 6.6-7.7(10H,m)

| Elemental Analysis for $C_{29}H_{25}Cl_2N_3O_5$.1/2 $C_4H_8O_2$.1/2 $H_2O$: | | | |
|---|---|---|---|
| Calcd. | C 60.10; | H 4.88; | N 6.78 |
| Found | C 60.11; | H 4.89; | N 6.69 |

0.2 g of the compound subsequently eluted in the silica gel column chromatography was subjected to hydrolysis in substantially the same manner as described above to give 0.11 g of crystals of a steric isomer, m.p. 165°C -167°C.

| Elemental Analysis for $C_{29}H_{25}Cl_2N_3O_5$.1/2$C_4H_8O_2$.1/2$H_2O$: | | | |
|---|---|---|---|
| Calcd. | C 60.53; | H 4.84; | N 6.83 |
| Found | C 60.44; | H 4.88; | N 6.83 |

Example 34

In substantially the same synthetic procedure as in Example 33, compounds shown in Table 29 through Table 33 were obtained.

Table 29

Example 34

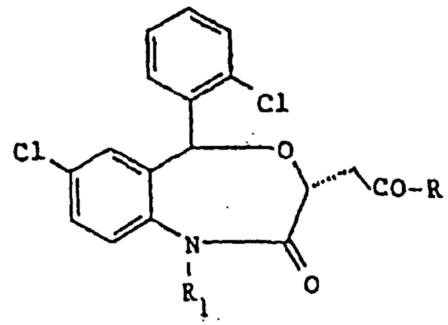

| compd. No. | $R_1$ | R | m.p. (℃) | Formula | Elemental Analysis (Found) C | H | N |
|---|---|---|---|---|---|---|---|
| 1 | 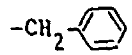 | $-NHCH_2COOH$ | 130-133 | $C_{26}H_{22}Cl_2N_2O_5 \cdot 1/2H_2O$ | 59.78 (59.81) | 4.44 (4.78) | 5.36 (4.90) |
| 2 | 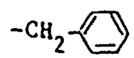 | $-NH(CH_2)_2COOH$ | 195-197 | $C_{27}H_{24}Cl_2N_2O_5$ | 61.49 (61.24) | 4.59 (4.35) | 5.31 (5.27) |
| 3 | 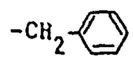 | Trp-OH = 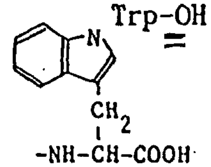 | 158-160 $[\alpha]_D^{25°}$ =-100.3° | $C_{35}H_{29}Cl_2N_3O_5 \cdot 1/4H_2O$ | 64.97 (64.99) | 4.60 (4.82) | 6.49 (6.14) |
| 4 | 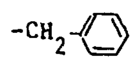 | Trp-OH | amorphous solid $[\alpha]_D^{25°}$ =+120.7° | $C_{35}H_{29}Cl_2N_3O_5 \cdot 1/4C_4H_8O_2$ | 65.06 (65.09) | 4.70 (5.08) | 6.32 (6.00) |
| 5 | 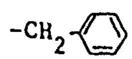 | D-Trp-OH | 157-158 $[\alpha]_D^{25°}$ =+97.0° | $C_{35}H_{29}Cl_2N_3O_5 \cdot 3/2H_2O$ | 62.78 (62.77) | 4.81 (4.58) | 6.28 (6.04) |

Table 30
Example 34

| compd. No. | $R_1$ | R | m. p. (℃) | Formula | Elemental Analysis (Found) C | H | N |
|---|---|---|---|---|---|---|---|
| 6 | 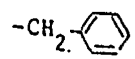 | D-Trp-OH | amorphous solid $[\alpha]_D^{25°}$ =-122.9° | $C_{35}H_{29}Cl_2N_3O_5$ ·3/2$H_2O$ | 62.78 (62.51) | 4.81 (4.98) | 6.28 (6.02) |
| 7 | 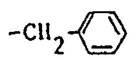 | Phe-OH = 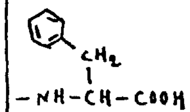 | 120-125 $[\alpha]_D^{25°}$ =-109.2° | $C_{33}H_{28}Cl_2N_2O_5$ ·1/2$H_2O$ | 64.71 (64.91) | 4.77 (4.70) | 4.57 (4.42) |
| 8 | 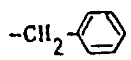 | Phe-OH | 119-122 $[\alpha]_D^{25°}$ =+142.0° | $C_{33}H_{28}Cl_2N_2O_5$ ·1/2$H_2O$ | 64.71 (64.62) | 4.77 (4.69) | 4.57 (4.41) |
| 9 | 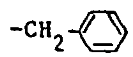 | D-Phe-OH | amorphous solid | $C_{33}H_{28}Cl_2N_2O_5$ ·3/2$H_2O$ | 62.86 (62.71) | 4.96 (4.66) | 4.44 (4.30) |
| 10 | 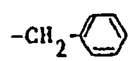 | D-Phe-OH | amorphous solid | $C_{33}H_{28}Cl_2N_2O_5$ ·3/2$H_2O$ | 62.86 (62.89) | 4.96 (4.72) | 4.44 (4.38) |
| 11 | 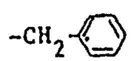 | 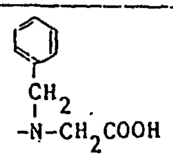 | 193-194 | $C_{33}H_{28}Cl_2N_2O_5$ | 65.68 (65.81) | 4.68 (4.91) | 4.64 (4.58) |
| 12 | 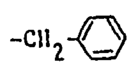 | 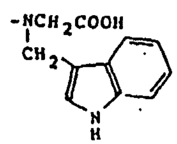 | amorphous solid | $C_{37}H_{33}Cl_2N_3O_5$ ·2.5$H_2O$ | 62.10 (62.32) | 5.35 (5.18) | 5.87 (5.63) |

Table 31

Example 34

| compd. No. | $R_1$ | R | m.p. (℃) | Formula | Elemental Analysis (Found) C | H | N |
|---|---|---|---|---|---|---|---|
| 13 | $-C_2H_5$ | $-NHCH_2COOH$ | 167-168 | $C_{21}H_{20}Cl_2N_2O_5 \cdot 1/2H_2O$ | 54.79 (54.56) | 4.60 (4.57) | 6.09 (6.11) |
| 14 | $-(CH_2)_6CH_3$ | $-NHCH_2COOH$ | 185-186 | $C_{26}H_{30}Cl_2N_2O_5$ | 59.89 (59.84) | 5.80 (5.73) | 5.37 (5.53) |
| 15 | $-CH_2CH(CH_3)_2$ | $-NHCH_2COOH$ | 233-234 | $C_{23}H_{24}Cl_2N_2O_5$ | 57.63 (57.63) | 5.05 (5.23) | 5.84 (5.66) |
| 16 | 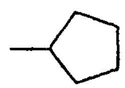 | $-NHCH_2COOH$ | 215-216 | $C_{24}H_{24}Cl_2N_2O_5$ | 58.67 (58.79) | 4.92 (5.00) | 5.70 (5.96) |
| 17 | $-CH_2CH(C_2H_5)_2$ | $-NHCH_2COOH$ | 110-115 | $C_{25}H_{28}Cl_2N_2O_5$ | 59.18 (58.32) | 5.56 (5.83) | 5.52 (5.47) |
| 18 | $-CH_2C(CH_3)_3$ | $-NHCH_2COOH$ | 235-236 | $C_{24}H_{26}Cl_2N_2O_5$ | 58.43 (58.57) | 5.31 (5.58) | 5.68 (5.60) |
| 19 | $-(CH_2)_2CH(CH_3)_2$ | $-NHCH_2COOH$ | 114-116 | $C_{24}H_{26}Cl_2N_2O_5 \cdot 1/2H_2O$ | 57.59 (57.24) | 5.42 (5.44) | 5.58 (5.50) |

Table 32

Example 34

| compd. No. | $R_1$ | R | m.p. (℃) | Formula | Elemental Analysis (Found) C | H | N |
|---|---|---|---|---|---|---|---|
| 20 | $-CH(C_2H_5)_2$ | $-NHCH_2COOH$ | 214-215 | $C_{24}H_{28}Cl_2N_2O_5$ | 58.43 (58.56) | 5.31 (5.39) | 5.68 (5.75) |
| 21 | $-CH_2C(CH_3)_3$ | $-NHCH_2CH_2COOH$ | 213- 214 | $C_{25}H_{28}Cl_2N_2O_5$ | 59.18 (59.12) | 5.56 (5.73) | 5.52 (5.27) |

Table 33

Example 34

| compd. No. | $R_1$ | R | p.m. (℃) | Formula | Elemental Analysis (Found) C | H | N |
|---|---|---|---|---|---|---|---|
| 22 | $-CH_2C(CH_3)_3$ | $NHCH(CH_3)COOH$ | 139-141 | $C_{25}H_{28}Cl_2N_2O_5$ 1/2$H_2O$ | 58.14 (58.36) | 5.66 (5.89) | 5.42 (5.55) |

Example 35

[Trans-1-benzyl-7-chloro-5-(2-chlorophenyl)-2-oxo-1,2,3,5-tetrahydro-4,1-benzoxazepine-3-yl]methylamine

In 5 ml of dimethylformamide was dissolved 1.0 g of trans-1-benzyl-7-chloro-5-(2-chlorophenyl)-2-oxo-1,2,3,5-tetrahydro-4,1-benzoxazepine-3-acetic acid obtained obtained in Reference Example 5. To the solution was added 0.3 ml of triethylamine, to which was added dropwise, while stirring under ice-cooling, 0.6 g of diphenylphosphoryl

azide. The reaction mixture was stirred for one hour at room temperature, which was then poured into ice-water. The mixture was subjected to extraction with ether. The organic layer was washed with water and dried, which was then concentrated under reduced pressure. The concentrate was dissolved in 100 ml of benzene, which was heated for 30 minutes under reflux. The reaction mixture was concentrated under reduced pressure. To the concentrate was added 6 ml of conc. hydrochloric acid, and the mixture was heated for one hour under reflux. The reaction mixture was concentrated under reduced pressure. The concentrate was made alkaline by the addition of a 5% aqueous solution of potassium carbonate, followed by extraction with ethyl acetate. The organic layer was washed with water and dried, then the solvent was distilled off under reduced pressure. To the residue was added an ethanol solution of 4N hydrochloric acid to lead it to hydrochloride to give 0.85 g of [trans-1-benzyl-7-chloro-5-(2-chlorophenyl)-2-oxo-1,2,3,5-tetrahydro-4,1-benzoxazepine-3-yl]methylamine hydrochloride as crystals, m.p. 245-250°C.

| Elemental Analysis for $C_{23}H_{20}Cl_2N_2O_2.HCl.3/2H_2O$: | | | |
|---|---|---|---|
| Calcd. | C 56.34; | H 4.82; | N 5.71 |
| Found | C 56.65; | H 4.44; | N 6.09 |

Example 36

By substantially the same procedure as in Example 36, compounds shown in Table 34 were obtained.

Table 34

Example 36

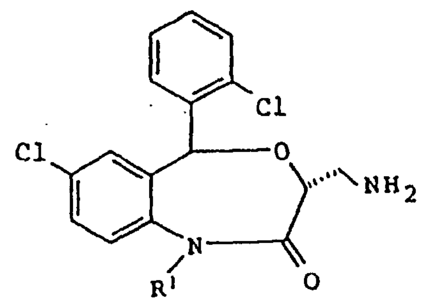

| Compd. No. | $R^1$ | m.p. (℃) | Formula | Elemental Analysis (Found) C | H | N |
|---|---|---|---|---|---|---|
| 1 | $-CH_3$ | 114-115 | $C_{17}H_{16}Cl_2N_2O_2$ | 58.13 (58.16) | 4.59 (4.79) | 7.98 (7.93) |
| 2 | 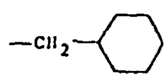 | 250-253 | $C_{23}H_{26}Cl_2N_2O_2 \cdot HCl \cdot 1/4H_2O$ | 58.23 (58.08) | 5.52 (5.66) | 5.91 (5.68) |
| 3 | $-CH(CH_3)_2$ | 136-137 | $C_{19}H_{20}Cl_2N_2O_2$ | 60.17 (59.93) | 5.31 (5.38) | 7.39 (7.06) |

Example 37

Ethyl ester of N-[trans-1-benzyl-7-chloro-5-(2-chlorophenyl)-2-oxo-1,2,3,5-tetrahydro-4,1-benzoxazepin-3-yl-methylaminocarbonyl]glycine

In 4 ml of dimethylformamide was dissolved 0.3 g of trans-1-benzyl-7-chloro-5-(2-chlorophenyl)-2-oxo-1,2,3,5-tetrahydro-4,1-benzoxazepine-3-acetic acid obtained in Reference Example 5. To the solution was added 0.15 ml of triethylamine. To the mixture was added, while stirring under ice-cooling, 0.18 g of diphenylphosphoryl azide. The reaction mixture was stirred for one hour at room temperature, to which was added ice-water, followed by extraction with ethyl acetate. The organic layer was washed with water, dried and concentrated under reduced pressure. To the concentrate was added 10 ml of benzene, and the mixture was heated for one hour under reflux while stirring.

To the reaction mixture were added 0.14 g of glycine ethyl ester hydrochloric acid and 0.15 ml of triethylamine. The mixture was then heated for 3 hours under reflux. The reaction mixture was concentrated under reduced pressure. The concentrate was subjected to extraction with ethyl acetate. The organic layer was washed with water and dried, then the solvent was distilled off under reduced pressure. Crystals obtained from the residue were recrystallized from a mixture of ethyl acetate and hexane to give 0.33 g of the title compound as white crystals, m.p. 200-201°C.

| Elemental Analysis for $C_{28}H_{27}Cl_2N_3O_5$: | | | |
|---|---|---|---|
| Calcd. | C 60.44; | H 4.89; | N 7.55 |
| Found. | C 60.29; | H 4.82; | N 7.68 |

Example 38

In 8 ml of methanol was dissolved 0.2 g of ethyl ester of N-[trans-1-benzyl-7-chloro-5-(2-chlorophenyl)-2-oxo-1,2,3,5-tetrahydro-4,1-benzoxazepin-3-yl-methylaminocarbonyl]glycine. To the solution were added 0.2 g of potassium carbonate and 2 ml of water, and the mixture was stirred for 3 hours at 60°C. The reaction mixture was concentrated under reduced pressure, to which was added water, followed by extraction with ether. The aqueous layer was acidified to pH 3 with dilute hydrochloric acid, which was subjected to extraction with ethyl acetate. The organic layer was washed with water and dried, then the solvent was distilled off under reduced pressure to leave crystalline residue. Recrystallization from ethyl acetate afforded 0.18 g of N-[trans-1-benzyl-7-chloro-5-(2-chlorophenyl)-2-oxo-1,2,3,5-tetrahydro-4,1-benzoxazepin-3-yl-methylaminocarbonyl]glycine as colorless needles, m.p. 153-155°C.

| Elemental Analysis for $C_{26}H_{23}Cl_2N_3O_5.1/4H_2O$: | | | |
|---|---|---|---|
| Calcd. | C 58.60; | H 4.45; | N 7.88 |
| Found | C 58.63; | H 4.42; | N 7.58 |

Example 39

Ethyl ester of N-[trans-1-benzyl-7-chloro-5-(2-chlorophenyl)-2-oxo-1,2,3,5-tetrahydro-4,1-benzoxazepin-3-ylmethyl] glycine

In 10 ml of acetonitrile was dissolved 0.4 g of trans-1-benzyl-7-chloro-5-(2-chlorophenyl)-2-oxo-1,2,3,5-tetrahydro-4,1-benzoxazepine-3-methylamine obtained in Example 35. To the solution were added 0.15 g of ethyl chloroacetate and 0.5 g of potassium carbonate. The mixture was heated under reflux for 15 hours while stirring. The reaction mixture was concentrated under reduced pressure, and the concentrate was subjected to extraction with ethyl acetate. The organic layer was washed with water and dried, then the solvent was distilled under reduced pressure. The residue was purified by means of a silica gel column chromatography (eluent, hexane : ethyl acetate = 3:2 to 1:2) to give 0.28 g of ethyl ester of N-[trans-1-benzyl-7-chloro-5-(2-chlorophenyl)-2-oxo-1,2,3,5-tetrahydro-4,1-benzoxazepin-3-ylmethyl]glycine as an oily product.

This product was made into hydrochloride salt to give white crystals, m.p. 182-184°C.

| Elemental Analysis for $C_{27}H_{26}Cl_2N_2O_4.HCl$: | | | |
|---|---|---|---|
| Calcd. | C 58.98; | H 4.95; | N 5.09 |
| Found | C 58.70; | H 4.98; | N 5.07 |

Example 40

N-[trans-1-benzyl-7-chloro-5-(2-chlorophenyl)-2-oxo-1,2,3,5-tetrahydro-4,1-benzoxazepin-3-ylmethyl]glycine

In 6 ml of methanol was dissolved 0.15 g of ethyl ester of N-[trans-1-benzyl-7-chloro-5-(2-chlorophenyl)-2-oxo-1,2,3,5-tetrahydro-4,1-benzoxazepin-3-ylmethyl]glycine obtained in Example 39. To the solution were added 0.4 g of potassium carbonate and 2 ml of water. The mixture was stirred for 2 hours at 50°C. The reaction mixture was concentrated under reduced pressure, whose pH was adjusted to 3 with dilute hydrochloric acid, followed by extraction with methylene chloride. The organic layer was washed with water and dried, then the solvent was distilled off under reduced pressure. From the residue was obtained 0.13 g of N-[trans-1-benzyl-7-chloro-5-(2-chlorophenyl)-2-oxo-1,2,3,5-tetrahydro-4,1-benzoxazepin-3-ylmethyl]glycine as crystals, m.p. 224-226°C.

| Elemental Analysis for $C_{25}H_{22}Cl_2N_2O_4.1/2H_2O$: | | | |
|---|---|---|---|
| Calcd. | C 60.74; | H 4.69; | N 5.67 |
| Found | C 60.74; | H 4.48; | N 5.70 |

Example 41

N-[trans-1-benzyl-7-chloro-5-(2-chlorophenyl)-2-oxo-1,2,3,5-tetrahydro-4,1-benzoxazepin-3-ylmethyl]-N-methylglycine

To 0.13 g of ethyl ester of N-[traps-1-benzyl-7-chloro-5-(2-chlorophenyl)-2-oxo-1,2,3,5-tetrahydro-4,1-benzoxazepin-3-ylmethyl]glycine obtained in Example 39 were added 2 ml of formalin and 2 ml of oxalic acid. The mixture was heated at 80°C for 2 hours. The reaction mixture was concentrated under reduced pressure, and the concentrate was subjected to extraction with ethyl acetate. The organic layer was washed with an aqueous solution of sodium hydrogencarbonate, then with water, followed by drying. The solvent was distilled off under reduced pressure. The residue was dissolved in 6 ml of methanol. To the solution were added 0.2 g of potassium carbonate and 2 ml of water. The mixture was stirred for 2 hours at 60°C. The reaction mixture was concentrated under reduced pressure, to which was added water, followed by extraction with ether. The aqueous layer was acidified to pH 4 with dilute hydrochloric acid, followed by extraction with methylene chloride. The organic layer was washed with water and dried, then the solvent was distilled off under reduced pressure. From the residue was obtained 0.1 g of N-[trans-1-benzyl-7-chloro-5-(2-chlorophenyl)-2-oxo-1,2,3,5-tetrahydro-4,1-benzoxazepin-3-ylmethyl]-N-methylglycine as crystals, m.p. 195-197°C.

| Elemental Analysis for $C_{26}H_{24}Cl_2N_2O_4.1/4H_2O$: | | | |
|---|---|---|---|
| Calcd. | C 61.97; | H 4.90; | N 5.56 |
| Found | C 61.90; | H 4.73; | N 5.67 |

Example 42

N-[trans-1-benzyl-7-chloro-5-(2-chlorophenyl)-2-oxo-1,2,3,5-tetrahydro-4,1-benzoxazepin-3-ylmethyl]-N-acetylglycine

In 4 ml of methanol was dissolved 60 mg of ethyl ester of N-[trans-1-benzyl-7-chloro-5-(2-chlorophenyl)-2-oxo-1,2,3,5-tetrahydro-4,1-benzoxazepin-3-ylmethyl]glycine obtained in Example 39. To the solution were added 0.2 ml of acetic anhydride and 0.2 ml of triethylamine. The mixture was heated for one hour under reflux while stirring. The reaction mixture was concentrated under reduced pressure, which was subjected to extraction with ethyl acetate. The organic layer was washed with an aqueous solution of potassium hydrogensulfate, an aqueous solution of sodium hydrogencarbonate and water, which was then dried, followed by distilling off the solvent. The residue was dissolved in 4 ml of methanol, to which was added 2 ml of a 5% aqueous solution of potassium carbonate. The mixture was stirred for 30 minutes at 60°C. The reaction mixture was concentrated under reduced pressure, to which was added 1N hydrochloric acid to acidify the solution, followed by extraction with ethyl acetate. The organic layer was washed with water and dried, then the solvent was distilled off under reduced pressure. From the residue, 45 mg of N-[trans-1-benzyl-7-chloro-5-(2-chlorophenyl)-2-oxo-1,2,3,5-tetrahydro-4,1-benzoxazepin-3-ylmethyl]-N-acetyl glycine was obtained as crystals, m.p. 143-145°C.

| Elemental Analysis for $C_{27}H_{24}Cl_2N_2O_5.1/4H_2O$: | | | |
|---|---|---|---|
| Calcd. | C 60.96; | H 4.64; | N 5.27 |
| Found | C 60.90; | H 4.39; | N 5.32 |

Example 43

Trans-1-benzyl-7-chloro-5-(2-chlorophenyl)-3-(3-methoxycarbonylmethylcarbamoyl)methyl-2-oxo-1,2,3,5-tetrahydro-4,1-benzoxazepine

To a mixture of 0.25 g of [trans-1-benzyl-7-chloro-5-(2-chlorophenyl)-2-oxo-1,2,3,5-tetrahydro-4,1-benzoxazepine-3-yl]methylamine obtained in Example 35, 0.1 g of malonic acid half ester potassium salt and 4 ml of dimethylformamide were added, while stirring under ice-cooling, 0.13 g of diethyl phosphorocyanidate and 0.23 ml of triethylamine. The reaction mixture was stirred for 40 minutes at room temperature, to which was added ice-water, followed by extraction with ethyl acetate. The organic layer was washed with an aqueous solution of sodium hydrogensulfate, an aqueous solution of sodium hydrogen carbonate and water, successively, which was then dried. The solvent was distilled off, and the residue was purified by means of a silica gel column chromatography (eluent, hexane : ethyl acetate : ethanol = 10:10:1) to give 0.22 g of trans-1-benzyl-7-chloro-5-(2-chlorophenyl)-3-(3-methoxycarbonylmethylcarbamoyl)methyl-2-oxo-1,2,3,5-tetrahydro-4,1-benzoxazepine as a white powdery product.
$IR\nu^{KBr}_{max}$ $cm^{-1}$ : 3370(NH), 2950, 1745(CO), 1670(CO), 1480, 1420, 1250
$^1$H-NMR spectrum (200MHz,$CDCl_3$) δ : 3.31(2H,s,CO$\underline{CH_2}$ $CO_2$), 3.74(3H,s,$OCH_3$), 3.7-4.2(3H,m), 4.8(1H,d), 5.6(1H, d), 5.8(1H,s,$C_5$-H), 6.42(1H,d,$C_6$-H), 7.2-7.8(1H,m)

| Elemental Analysis for $C_{27}H_{24}Cl_2N_2O_5.1/2H_2O$: | | | |
|---|---|---|---|
| Calcd. | C 60.46; | H 4.70; | N 5.22 |
| Found | C 60.27; | H 4.85; | N 4.95 |

In 5 ml of methanol was dissolved 0.15 g of the white powdery product obtained above. To the solution were added 0.2 g of potassium carbonate and 1.5 ml of water, then the mixture was stirred for 2 hours at 50°C. The reaction mixture concentrated under reduced pressure. The concentrate was neutralized with dilute hydrochloric acid, followed by extraction with ethyl acetate. The organic layer was washed with water and dried, then the solvent was distilled off under reduced pressure. From the residue was obtained 0.14 g of Trans-1-benzyl-7-chloro-5-(2-chlorophenyl)-3-(3-hydroxycarbonylmethylcarbamoyl)methyl-2-oxo-1,2,3,5-tetrahydro-4,1-benzoxazepine as a white powdery product.
$IR\nu^{KBr}_{max}$ $cm^{-1}$ : 3700-2200(COOH), 1730(CO), 1670(CO), 1480, 1240
$^1$H-NMR spectrum (200MHz,$CDCl_3$) δ : 3.31(2H,s,-CO $\underline{CH_2}$COOH), 3.7-4.2(3H,m), 4.75(1H,d), 5.62(1H,d), 5.77(1H, s,$C_5$-H), 6.44(1H,d,$C_6$-H), 7.04(1H,t,NH), 7.2-7.7(11H,m)

| Elemental Analysis for $C_{26}H_{22}Cl_2N_2O_5.1/2H_2O$: | | | |
|---|---|---|---|
| Calcd. | C 59.78; | H 4.44; | N 5.36 |
| Found | C 59.47; | H 4.69; | N 5.13 |

Example 44

Trans-1-benzyl-7-chloro-5-(2-chlorophenyl)-2-oxo-1,2,3,5-tetrahydro-4,1-benzoxazepine-3-methanol and 3,5-trans-7-chloro-3-chloromethyl-5-(2-chlorophenyl)-2-oxo-1,2,3,5-tetrahydro-4,1-benzoxazepine

In mixture of 12 ml of glacial acetic acid and 10 ml of water was suspended 2.0 g of [trans-1-benzyl-7-chloro-5-(2-chlorophenyl)-2-oxo-1,2,3,5-tetrahydro-4,1-benzoxazepine-3-yl]methylamine hydrochloride obtained in Example 35. To the suspension was added dropwise, while stirring under ice-cooling, 1 ml of an aqueous solution of 1.0 g of sodium nitrite, in the course of 10 minutes. The reaction mixture was stirred for one hour at room temperature, which was added to ice-water, followed by extraction with ethyl acetate. The organic layer was washed with an aqueous solution of sodium hydrogencarbonate and water, successively, which was then dried, followed by distilling off the solvent under reduced pressure. The residue was dissolved in 50 ml of methanol, to which was added 5 ml of a 10% aqueous solution of potassium carbonate. The mixture was stirred for 15 minutes at 60°C. The reaction mixture was concentrated under reduced pressure. The concentrate was subjected to extraction with ethyl acetate. The organic

layer was washed with water and dried, then the solvent was distilled off under reduced pressure. The residue was purified by means of a silica gel column chromatography (eluent, hexane : ethyl acetate = 4:1 to 1:1). From the preceding portion of the eluate, 0.2 g of trans-7-chloro-3-chloromethyl-5-(2-chlorophenyl)-2-oxo-1,2,3,5-tetrahydro-4,1-benzoxazepine as colorless prisms, m.p. 177-179°C.
Mass spectrum (m/e) : 445 ($M^+$)

| Elemental Analysis for $C_{23}H_{18}Cl_3NO_2$: | | | |
|---|---|---|---|
| Calcd. | C 61.83; | H 4.06; | N 3.14 |
| Found | C 62.11; | H 4.01; | N 3.38 |

From the subsequent portion of the eluate, 1.05 g of trans-1-benzyl-7-chloro-5-(2-chlorophenyl)-2-oxo-1,2,3,5-tetrahydro-4,1-benzoxazepine-3-methanol as colorless needles, m.p. 158-159°C Mass spectrum (m/e) : 427, 429 ($M^+$)

| Elemental Analysis for $C_{23}H_{19}Cl_2NO_3$: | | | |
|---|---|---|---|
| Calcd. | C 64.49; | H 4.47; | N 3.27 |
| Found | C 64.36; | H 4.39; | N 3.34 |

Example 45

By substantially the same procedure as in Example 44, compounds shown in Table 35 were obtained.

**Table 35**

Example 45

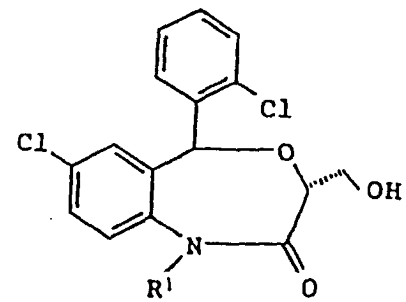

| compd. No. | $R^1$ | m. p. (℃) | Formula | Elemental Analysis (Found) C | H | N |
|---|---|---|---|---|---|---|
| 1 | $-CH_3$ | 208-209 | $C_{17}H_{15}Cl_2NO_3$ | 57.97 (58.18) | 4.29 (4.46) | 3.98 (3.79) |
| 2 | $-CH_2-$ 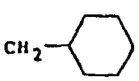 | 171-172 | $C_{23}H_{25}Cl_2NO_3$ | 63.60 (63.60) | 5.80 (5.89) | 3.22 (3.02) |
| 3 | $-CH(CH_3)_2$ | 154-155 | $C_{19}H_{19}Cl_2NO_3$ | 60.01 (59.83) | 5.04 (4.91) | 3.68 (3.79) |

Example 46

Trans-1-benzyl-7-chloro-5-(2-chlorophenyl)-2-oxo-1,2,3,5-tetrahydro-4,1-benzoxazepine-3-carboxylic acid

In 20 ml of acetone was dissolved 0.5 g of trans-1-benzyl-7-chloro-5-(2-chlorophenyl)-2-oxo-1,2,3,5-tetrahydro-4,1-benzoxazepine-3-methanol obtained in Example 44. To the solution was added dropwise, while stirring at room temperature, 0.5 ml of a Jones' reagent. The reaction mixture was stirred for one hour at room temperature, which was then concentrated under reduced pressure The concentrate was subjected to extraction with ethyl acetate. The organic layer was washed with water and dried, then the solvent was distilled off under reduced pressure. From the residue was obtained 0.23 g of trans-1-benzyl-7-chloro-5-(2-chlorophenyl)-2-oxo-1,2,3,5-tetrahydro-4,1-benzoxazepine-3-carboxylic acid as white crystals, m.p.177-178°C.

| Elemental Analysis for $C_{23}H_{17}Cl_2NO_4$: | | | |
|---|---|---|---|
| Calcd. | C 62.46; | H 3.87; | N 3.17 |
| Found | C 62.24; | H 3.93; | N 3.30 |

Example 47

By substantially the same synthetic procedure as in Example 46, compounds listed in Table 36 were obtained.

Table 36

Example 47

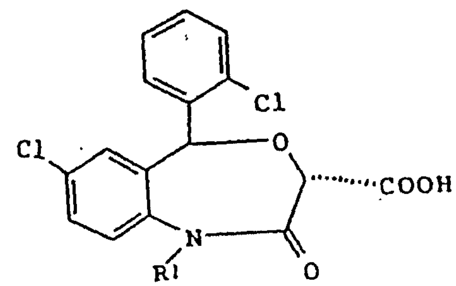

| compd. No. | $R^1$ | m.p. (℃) | Formula | Elemental Analysis (Found) C | H | N |
|---|---|---|---|---|---|---|
| 1 | $-CH_3$ | 167-168 | $C_{17}H_{13}Cl_2NO_4 \cdot 1/4C_4H_{10}O \cdot 1/4H_2O$ | 55.54 (55.72) | 4.14 (4.34) | 3.60 (3.34) |
| 2 | 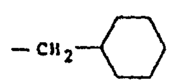 | 189-190 | $C_{23}H_{23}Cl_2NO_4$ | 57.88 (57.76) | 4.35 (4.55) | 3.55 (3.43) |
| 3 | $-CH(CH_3)_2$ | 181-182 | $C_{19}H_{17}Cl_2NO_4$ | 61.62 (61.69) | 5.17 (5.39) | 3.12 (3.39) |

Example 48

Ethyl ester of 3-[trans-1-benzyl-7-chloro-5-(2-chlorophenyl)-2-oxo-1,2,3,5-tetrahydro-4,1-benzoxazepin-3-ylmethyl] thioglycolic acid

In 6 ml of acetonitrile was dissolved 0.2 g of trans-1-benzyl-7-chloro-5-(2-chlorophenyl)-3-chloromethyl-2-oxo-1,2,3,5-tetrahydro-4,1-benzoxazepine. To the solution were added 0.08 g of ethyl thioglycolate and 0.1 g of cesium fluoride. The mixture was heated under reflux for 40 minutes while stirring. The reaction mixture was concentrated under reduced pressure. The concentrate was subjected to extraction with ethyl acetate. The organic layer was washed with water and dried, then the solvent was distilled off under reduced pressure. The residue was purified by means of a silica gel column chromatography (eluent, hexane : ethyl acetate = 4:1) to afford 0.16 g of ethyl 3-(trans-1-benzyl-7-chloro-5-(2-chlorophenyl)-2-oxo-1,2,3,5-tetrahydro-4,1-benzoxazepin-3-ylmethyl] thioglycolate as colorless needles, m.p.153-154°C.

| Elemental Analysis for $C_{27}H_{25}Cl_2NO_4S$: | | | |
|---|---|---|---|
| Calcd. | C 61.13; | H 4.75; | N 2.64 |
| Found | C 61.04; | H 4.72; | N 2.54 |

Example 49

3-[Trans-1-benzyl-7-chloro-5-(2-chlorophenyl)-2-oxo-1,2,3,5-tetrahydro-4,1-benzoxazepin-3-ylmethyl]glycolic acid

In a mixture of 4 ml of methanol and 2 ml of tetrahydrofuran was dissolved 0.11 g of 3-[trans-1-benzyl-7-chloro-5-(2-chlorophenyl)-2-oxo-1,2,3,5-tetrahydro-4,1-benzoxazepin-3-ylmethyl]thioglycolic acid ester. To the solution were added 0.2 g of potassium carbonate and 2 ml of water, then the mixture was stirred for one hour at 60°C. The reaction mixture was concentrated under reduced pressure. The concentrate was rendered acid with dilute hydrochloric acid, which was then subjected to extraction with ethyl acetate. The organic layer ws washed with water and dried, then the solvent was distilled off under reduced pressure. From the residue was obtained 90 mg of 3-[trans-1-benzyl-7-chloro-5-(2-chlorophenyl)-2-oxo-1,2,3,5-tetrahydro-4,1-benzoxazepin-3-ylmethyl]thioglycolic acid as white crystals, m.p. 148-149°C.

| Elemental Analysis for $C_{25}H_{21}Cl_2NO_4S$: | | | |
|---|---|---|---|
| Calcd. | C 59.77; | H 4.21; | N 2.79 |
| Found | C 59.89; | H 4.31; | N 2.77 |

Example 50

3-[Trans-1-benzyl-7-chloro-5-(2-chlorophenyl)-2-oxo-1,2,3,5-tetrahydro-4,1-benzoxazepin-3-ylmethyl]glycolic acid

To a suspension of 50 mg of sodium hydride in 4 ml of dimethylformamide was added 0.2 g of trans-1-benzyl-7-chloro-5-(2-chlorophenyl)-2-oxo-1,2,3,5-tetrahydro-4,1-benzoxazepine3-methanol, while stirring under ice-cooling. The mixture was stirred for 10 minutes at the same temperature, to which was then added 0.1 g of ethyl chloroacetate, followed by stirring for 30 minutes at room temperature. The reaction mixture was poured into ice-water, which was subjected to extraction with ethyl acetate. The organic layer was washed with an aqueous solution of potassium hydrogensulfate, an aqueous solution of sodium hydrogencarbonate and water, successively and dried, then the solvent was distilled off under reduced pressure. The residue was purified by means of a silica gel column chromatography (eluent, hexane : ethyl acetate = 5:1) to give crystals. The crystals were dissolved in 5 ml of methanol. To the solution were added 0.2 g of potassium carbonate and 2 ml of water. The mixture was stirred for 5 hours at 60°C. The reaction mixture was concentrated udner reduced pressure. The concentrate was rendered acid with dilute hydrochloric acid, followed by extraciton with ethyl acetate. The organic layer was washed with water and dried, then the solvent was distilled off under reduced pressure. From the residue was obtained 50 mg of 3-[trans-1-benzyl-7-chloro-5-(2-chlorophenyl)-2-oxo-1,2,3,5-tetrahydro-4,1-benzoxazepin-3-ylmethyl]glycolic acid as white crystals, m.p.92-94°C.

| Elemental Analysis for $C_{25}H_{21}Cl_2NO_5.1/2C_4H_{10}O.1/2H_2O$: | | | |
|---|---|---|---|
| Calcd. | C 60.91; | H 5.11; | N 2.63 |

(continued)

| Elemental Analysis for $C_{25}H_{21}Cl_2NO_5.1/2C_4H_{10}O.1/2H_2O$: | | | |
|---|---|---|---|
| Found | C 60.95; | H 4.99; | N 2.77 |

Example 51

Methyl ester of N-[trans-1-benzyl-7-chloro-5-(2-chlorophenyl)-2-oxo-1,2,3,5-tetrahydro-4,1-benzoxazepin-3-ylcarbonyl]tryptophane

By substantially the same procedure as in Example 31, 0.4 g of 3,5-trans-1-benzyl-7-chloro-5-(2-chlorophenyl)-2-oxo-1,2,3,5-tetrahydro-4,1-benzoxazepine-3-carboxylic acid obtained in Example 46 was subjected to the reaction to give a crude product, which was purified by means of a silica gel column chromatography (eluent, hexane : ethyl acetate : methylene chloride = 9:3:1). From the preceding portion of the eluate, 0.25 g of the product was obtained as a white powdery product.
$IR\nu^{KBr}_{max}$ $cm^{-1}$ : 3400(NH), 1740(CO), 1690(CO), 1655(CO)
$^1H$-NMR spectrum (200MHz,$CDCl_3$) δ: 3.44(2H,d), 3.64(3H,s,$OCH_3$), 4.55(1H,s,$C_3$-H), 4.70(1H,d,$PhCH_2$), 5.03(1H, m), 5.69(1H,d,$PhCH_2$), 5.79(1H,s,$C_5$-H), 6.37(1H,d,$C_6$-H), 6.9-8.2(15H,m)

From the subsequent poriton of the eluate, 0.22 g of the title compound as an amorphous solid product.
$IR\nu^{KBr}_{max}$ $cm^{-1}$ : 3400(NH), 1740(CO), 1695(CO), 1660(CO), 1520, 1480, 1240
$^1H$-NMR spectrum (200MHz,$CDCl_3$) δ: 3.2-3.55(2H,m), 3.70(3H,s,$OCH_3$), 4.53(1H,s,$C_3$-H), 4.62(1H,d), 5.06(1H,m), 5.63(1H,d), 5.76(1H,s,$C_5$-H), 6.33(1H,d,$C_6$-H), 6.8-8.4(15H,m)

Example 52

N-[trans-1-benzyl-7-chloro-5-(2-chlorophenyl)-2-oxo-1,2,3,5-tetrahydro-4,1-benzoxazepin-3-ylcarbonyl]tryptophane

N-[trans-1-benzyl-7-chloro-5-(2-chlorophenyl)-2-oxo-1,2,3,5-tetrahydro-4,1-benzoxazepin-3-ylcarbonyl]tryptophane ethyl ester obtained in Example 53 was subjected to substantially the same procedure as in Example 34. From 0.25 g of the compound obtained as the preceding eluate, 70 mg of a colorless crystalline product was obtained, m.p.250-155°C (decomp.).

| Elemental Analysis for $C_{34}H_{27}Cl_2N_3O_5.H_2O$: | | | |
|---|---|---|---|
| Calcd. | C 63.16; | H 4.52; | N 6.50 |
| Found | C 63.34; | H 4.63; | N 6.42 |

From 0.22 g of the compound eluted subsequently, 0.11 g of a colorless amorphous solid product was obtained
$IR\nu^{KBr}_{max}$ $cm^{-1}$ : 3700-2200(NH,COOH), 1740(CO), 1680(CO), 1660(CO), 1525, 1240
$^1H$-NMR spectrum (200MHz,$CDCl_3$) δ: 3.40(2H,m), 4.54(1H,s), 4.63(1H,d), 5.05(1H,m), 5.63(1H,d), 5.70(1H,s,$C_5$-H), 6.31(1H,d,$C_6$-H), 6.8-8.4(15H,m)

| Elemental Analysis for $C_{34}H_{27}Cl_2N_3O_5$: | | | |
|---|---|---|---|
| Calcd. | C 64.97; | H, 4.33; | N 6.67 |
| Found | C 64.70; | H, 4.58; | N 6.49 |

Example 53

N-(cis-7-chloro-1-isopropyl-5-phenyl-2-oxo-1,2,3,5-tetrahydro-4,1-benzoxazepin-3-ylacetyl)glycine ethyl ester

In 5 ml of methanol was dissolved 0.3 g of ethyl ester of 3,5-cis-7-chloro-1-isopropyl-5-phenyl-1,2,3,5-tetrahydro-2-oxo-4,1-benzoxazepine-3-acetic acid ethyl ester disclosed in JPA S57(1982)-35576. To the solution were added 0.3 g of potassium carbonate and 2 ml of water. The mixture was stirred for 8 hours at room temperature. The reaction mixture was concentrated under reduced pressure. To the concentrate was added water, which was subjected to extraction with ether. The aqueous layer was separated, to which was added dilute hydrochloric acid to adjust its pH to 3. The organic layer was washed with water and dried, then the solvent was distilled off under reduced pressure. From

the residue was obtained 0.15 g of a powdery product. The product and 0.07 g of glycine ethyl ester hydrochloride were dissolved in 4 ml of dimethylformamide. To the solution were added, while stirring under ice-cooling, 0.1 g of diethyl phosphorocyanidate and, then, 0.2 ml of triethylamine. The reaction mixture was stirred for one hour at room temperature, which was poured into ice-water, followed by extraction with ethyl acetate. The organic layer was washed with an aqueous solution of potassium hydrogensulfate, an aqueous solution.of sodium hydrogencarbonate and water, successively, which was then dried. The solvent was distilled off under reduced pressure. The residue was purified by means of a silica gel column chromatography (eluent, hexane : ethyl acetate = 2:1 to 3:2) to afford 0.11 g of N-[cis-7-chloro-1-isopropyl-5-phenyl-2-oxo-1,2,3,5-tetrahydro-4,1-benzoxazepin-3-ylacetyl]glycine ethyl ester as a crystalline product, m.p.188°C-190°C.

| Elemental Analysis for $C_{24}H_{27}ClN_2O_5$: | | | |
|---|---|---|---|
| Calcd. | C 62.81; | H 5.93; | N 6.10 |
| Found | C 62.52; | H 6.10; | N 6.04 |

Example 54

N-[cis-7-chloro-1-isopropyl-5-phenyl)-1,2,3,5-tetra-hydro-2-oxo-4,1-benzoxazepin-3-ylacetyl]glycine

In a mixture of 2 ml of methanol and 1 ml of water was suspended 48 mg of N-(trans-7-chloro-1-isopropyl-5-phenyl-1,2,3,5-tetrahydro-2-oxo-4,1-benzoxazepin-3-ylacetyl)glycine ethyl ester obtained in Example 53. To the suspension was added 0.1 g of potassium carbonate. The mixture was stirred for 6 hours at room temperature. The reaction mixture was adjusted to pH 4 with dilute hydrochloric acid, which was subjected to extraction with ethyl acetate. The organic layer was washed with water and dried, then the solvent was distilled off under reduced pressure. Crystals obtained form the residue were recrystallized from ether and hexane to afford 29 mg of N-(cis-7-chloro-1-isopropyl-5-phenyl-1,2,3,5-tetrahydro-2-oxo-4,1-benzoxazepin-3-ylacetyl)glycine as white needles.
$IR\nu_{max}^{KBr}$ $cm^{-1}$ : 3700-2200(COOH), 1710(CO), 1680(CO), 1480, 1250, 700
$^1$H-NMR spectrum (200MHz,$CDCl_3$) δ: 2.7-3.4(2H,m), 4.4(1H,dd,$C_3$-H), 4.75(1H,d), 5.34(1H,s,$C_5$-H), 5.5(1H,d), 6.48 (1H,s,$C_6$-H), 6.9-7.6(12H,m)

| Elemental Analysis for $C_{24}H_{20}ClNO_4.3/4H_2O$: | | | |
|---|---|---|---|
| Calcd. | C 66.20; | H 4.98; | N 3.22 |
| Found | C 66.12; | H 5.19; | N 2.97 |

Example 55

Trans-7-chloro-5-(2-chlorophenyl)-1-isopropyl-2-oxo-1,2,3,5-tetrahydro-4,1-benzoxazepine-3-butyric acid ethyl ester

(1) A solution of oxalyl chloride (0.72 ml) in methylene chloride (10 ml) was cooled to -65°C, to which was added dropwise a solution of dimethyl sulfoxide (0.63 ml) in methylene chloride (2 ml) taking 5 minutes, followed by stirring for 5 minutes. To the mixture was added dropwise, taking 15 minutes, a solution of trans-7-chloro-5-(2-chlorophenyl)-1-isopropyl-2-oxo-1,2,3,5-tetrahydro-4,1-benzoxazepine-3-ethanol (2.5 g) obtained in Example 45 in methylene chloride (15 ml), which was stirred for 10 minutes. To the resultant mixture was added triethylamine (3.45 ml) taking 5 minutes, then the cooling bath was removed. The mixture was stirred for 10 minutes at room temperature, to which was then added 1N hydrochloric acid (50 ml), followed by stirring. The methylene chloride layer was dried over anhydrous magnesium sulfate, then the solvent was distilled off under reduced pressure to leave trans-7-chloro-5-(2-chlorophenyl)-1-isopropyl-2-oxo-1,2,3,5-tetrahydro-4,1-benzoxazepine-3-acetaldehyde (2.3 g) as an oily product.
$IR\nu_{max}^{Neat}$ $cm^{-1}$ : 1720, 1670(C=O)
$^1$H-NMR spectrum (200MHz, $CDCl_3$) δ :1.29(3H,t,J=7.1Hz), 1.29(3H,d,J=7.0Hz), 1.56(3H,d,J=6.8Hz), 2.7-2.85 (2H,m), 3.91(1H,t,J=6.5Hz), 4.19(2H,q,J=7.1Hz), 4.75-5.0(1H,m), 5.90(1H,dt,J=15.6, 1.5Hz), 6.01(1H,s), 6.51(1H, d,J=2.4Hz), 6.97(1H,dt,J=15.6, 7.2Hz), 7.2-7.8(6H,m), 9.83(1H,s)
(2) In toluene (20 ml) was dissolved trans-7-chloro-5-(2-chlorophenyl)-1-isopropyl-2-oxo-1,2,3,5-tetrahydro-4,1-benzoxazepine-3-acetaldehyde (1.5 g). To the solution was added (ethoxycarbonylmethylene)-triphenylphosphorane (2.0 g), and the mixture was stirred for 3 hours at 90°C. The solvent was distilled off, and the residue was

purified by means of a silica gel column chromatography (hexane : ethyl acetate = 10:1) to give ethyl ester of trans-7-chloro-5-(2-chlorophenyl)-1-isopropyl-2-oxo-1,2,3,5-tetrahydro-4,1-benzoxazepine-3-crotonic acid (1.28 g) as prisms, m.p.126-127°C.
$IR\nu_{max}^{KBr}cm^{-1}$ : 1715(C=O), 1670(C=C)

| Elemental Analysis for $C_{24}H_{25}Cl_2NO_4$: | | | |
|---|---|---|---|
| Calcd. | C 62.34; | H 5.45; | N 3.03 |
| Found | C 62.47; | H 5.28; | N 3.08 |

(3) In ethyl acetate (20 ml) was dissolved ethyl ester of trans-7-chloro-5-(2-chlorophenyl)-1-isopropyl-2-oxo-1,2,3,5-tetrahydro-4,1-benzoxazepine-3-crotonic acid (0.45 g). To the solution was added 10% palladium carbon (0.1 g), and the mixture was subjected to catalytic reduction at room temperatures under atmospheric pressure. The theoretical amount of hydrogen was allowed to be absorbed, then the catalyst was removed, followed by distilling off ethyl acetate under reduced pressure. The residue was crystallized from a small volume of hexane to give ethyl ester of trans-7-chloro-5-(2-chlorophenyl)-1-isopropyl-2-oxo-1,2,3,5-tetrahydro-4,1-benzoxazepine-3-butyric acid (0.37 g) as prisms, m.p.100-101°C.
$IR\nu_{max}^{KBr}cm^{-1}$ : 1730, 1670(C=O)

| Elemental Analysis for $C_{24}H_{27}Cl_2NO_4$: | | | |
|---|---|---|---|
| Calcd. | C 62.07; | H 5.86; | N 3.02 |
| Found | C 62.35; | H 5.93; | N 2.95 |

Example 56

Trans-7-chloro-5-(2-chlorophenyl)-1-isopropyl-2-oxo-1,2,3,5-tetrahydro-4,1-benzoxazepine-3-butyric acid

In ethanol (5 ml) was dissolved ethyl ester of trans-7-chloro-5-(2-chlorophenyl)-1-isopropyl-2-oxo-1,2,3,5-tetrahydro-4,1-benzoxazepine-3-butyric acid (0.25 g). To the solution was added 1N sodium hydroxide (4 ml), then the mixture was stirred for 30 minutes at room temperatures. The mixture was acidified with 1N aqueous solution of hydrochloric acid (50 ml), followed by extraction with ethyl acetate (100 ml). The ethyl acetate layer was washed with water and dried over anhydrous magnesium sulfate. The solvent was distilled off under reduced pressure to leave trans-7-chloro-5-(2-chlorophenyl)-1-isopropyl-2-oxo-1,2,3,5-tetrahydro-4,1-benzoxazepine-3-butyric acid (0.20 g) as prisms, m.p.158-160°C.
$IR\nu_{max}^{KBr}cm^{-1}$ : 1710, 1670(C=O)

| Elemental Analysis for $C_{22}H_{23}Cl_2NO_4$: | | | |
|---|---|---|---|
| Calcd. | C 60.56; | H 5.31; | N 3.21 |
| Found | C 60.62; | H 5.18; | N 3.23 |

Example 57

Ethyl ester of N-[trans-1-isobutyl-2-oxo-5-(o-tolyl)-1,2,3,5-tetrahydro-4;1-benzoxazepine-3-acetyl]aminoacetic acid

Trans-1-isobutyl-2-oxo-5-(o-tolyl)-1,2,3,5-tetrahydro-4,1-benzoxazepine-3-acetic acid (0.4 g) obtained in Example 5 and glycine ethyl ester hydrochloride were subjected to substantially the same procedure as in Example 23 to give ethyl ester of N-[trans-1-isobutyl-2-oxo-5-(o-tolyl)-1,2,3,5-tetrahydro-4,1-benzoxazepine-3-acetyl]aminoacetic acid (0.47 g) as needles, m.p. 137-138°C.
$IR\nu_{max}^{KBr}cm^{-1}$ : 3360(NH), 1745, 1655(C=O)

| Elemental Analysis for $C_{26}H_{32}N_2O_5$: | | | |
|---|---|---|---|
| Calcd. | C 69.01; | H 7.13; | N 6.19 |
| Found | C 69.01; | H 7.18; | N 6.26 |

Example 58

N-[trans-1-isobutyl-2-oxo-5-(o-tolyl)-1,2,3,5-tetrahydro-4,1-benzoxazepine-3-acetyl]aminoacetic acid

Ethyl ester of N-[trans-1-isobutyl-2-oxo-5-(o-tolyl)-1,2,3,5-tetrahydro-4,1-benzoxazepine-3-acetyl]aminoacetic acid (0.3 g) obtained in Example 57 was subjected to hydrolysis in substantially the same manner as in Example 34 to afford N-[trans-1-isobutyl-2-oxo-5-(o-tolyl)-1,2,3,5-tetrahydro-4,1-benzoxazepine-3-acetyl]aminoacetic acid (0.26 g) as prisms, m.p.220-223°C.
$IR\nu_{max}^{KBr}$ $cm^{-1}$ : 1755, 1670, 1630(C=O)

| Elemental Analysis for $C_{24}H_{26}N_2O_5$: | | | |
|---|---|---|---|
| Calcd. | C 67.91; | H 6.65; | N 6.60 |
| Found | C 67.96; | H 6.86; | N 6.69 |

Example 59

Ethyl ester of N-[trans-5-(2-fluorophenyl)-1-isobutyl-2-oxo-1,2,3,5-tetrahydro-4,1-benzoxazepine-3-acetyl] aminoacetic acid

Trans-5-(2-fluorophenyl)-1-isobutyl-2-oxo-1,2,3,5-tetrahydro-4,1-benzoxazepine-3-acetic acid (0.4 g) obtained in Example 6 was allowed to react with glycine ethyl ester hydrochloride in substantially the same manner as in Example 23 to afford ethyl ester of N-[trans-5-(2-fluorophenyl)-1-isobutyl-2-oxo-1,2,3,5-tetrahydro-4,1-benzoxazepine-3-acetyl]aminoacetic acid (0.45 g) as needles, m.p.166-168°C.
$IR\nu_{max}^{KBr}$ $cm^{-1}$ : 3370(NH), 1750, 1660(C=O)

| Elemental Analysis for $C_{25}H_{29}FN_2O_5$: | | | |
|---|---|---|---|
| Calcd. | C 65.78; | H 6.40; | N 6.14 |
| Found | C 65.89; | H 6.34; | N 6.15 |

Example 60

N-[trans-5-(2-fluorophenyl)-1-isobutyl-2-oxo-1,2,3,5-tetrahydro-4,1-benzoxazepine-3-acetyl]aminoacetic acid

Ethyl ester of N-[trans-5-(2-fluorophenyl)-1-isobutyl-2-oxo-1,2,3,5-tetrahydro-4,1-benzoxazepine-3-acetyl] aminoacetic acid (0.3 g) obtained in Example 59 was subjected to hydrolysis in substantially the same manner as in Example 24 to afford N-[trans-5-(2-fluorophenyl)-1-isobutyl-2-oxo-1,2,3,5-tetrahydro-4,1-benzoxazepine-3-acetyl) aminoacetic acid (0.27 g) as prisms, m.p.201-203°C.
$IR\nu_{max}^{KBr}$ $cm^{-1}$ : 1755, 1670, 1630(C=O)

| Elemental Analysis for $C_{23}H_{25}FN_2O_5$: | | | |
|---|---|---|---|
| Calcd. | C 64.48; | H 5.88; | N 6.54 |
| Found | C 64.54; | H 5.95; | N 6.51 |

Example 61

Ethyl ester of N-[trans-1-isobutyl-5-(2-methoxyphenyl)-2-oxo-1,2,3,5-tetrahydro-4,1-benzoxazepine-3-acetyl] aminoacetic acid

In N,N-dimethylformamide (10 ml) were dissolved trans-1-isobutyl-5-(2-methoxyphenyl)-2-oxo-1,2,3,5-tetrahydro-4,1-benzoxazepine-3-acetic acid (0.4 g) obtained in Example 6 and glycine ethyl ester hydrochloride (0.18 g). To the solution were added, under ice-cooling, diethyl phosphorocyanidate (0.22 g) and triethylamine (0.35 ml). The mixture was stirred for 30 minutes at room temperature, to which were then added water (100 ml) and ethyl acetate (100 ml), followed by extraction. The ethyl acetate layer was washed with 1N hydrochloric acid and an aqueous solution of sodium hydrogencarbonate, which was then dried on anhydrous magnesium sulfate, followed by distilling off the solvent under reduced pressure. The residue was purified by means of a silica gel column chromatography (hexane :

ethyl acetate = 1:1) to give ethyl ester of N-[trans-1-isobutyl-5-(2-methoxyphenyl)-2-oxo-1,2,3,5-tetrahydro-4,1-benzoxazepine-3-acetyl) aminoacetic acid (0.45 g) as needles, m.p.135-137°C.
IR$\nu_{max}^{KBr}$ $cm^{-1}$ : 1750, 1670(C=O)

| Elemental Analysis for $C_{26}H_{32}N_2O_6$: | | | |
|---|---|---|---|
| Calcd. | C 66.65; | H 6.88; | N 5.98 |
| Found | C 66.72; | H 6.91; | N 5.98 |

Example 62

N-[trans-1-isobutyl-5-(2-methoxyphenyl)-2-oxo-1,2,3,5-tetrahydro-4,1-benzoxazepine-3-acetyl]aminoacetic acid

In ethanol (5 ml) was dissolved ethyl ester of N-[trans-1-isobutyl-5-(2-methoxyphenyl)-2-oxo-1,2,3,5-tetrahydro-4,1- benzoxazepine-3-acetyl]aminoacetic acid (0.3 g) obtained in Example 61. To the solution was added 1N sodium hydroxide, and the mixture was stirred for 15 minutes, which was acidified by the addition of 1N aqueous solution of hydrochloric acid (50 ml), followed by extraction with ethyl acetate (100 ml). The ethyl acetate layer was washed with water, which was then dried over anhydrous magnesium sulfate. The solvent was distilled off under reduced pressure to leave N-[trans-1-isobutyl-5-(2-methoxyphenyl)-2-oxo-1,2,3,5-tetrahydro-4,1-benzoxazepine-3-acetyl]aminoacetic acid (0.27 g) as prisms, m.p.210-211°C.
IR$\nu_{max}^{KBr}$ $cm^{-1}$ : 1760, 1670, 1630(C=O)

| Elemental Analysis for $C_{24}H_{28}N_2O_6$: | | | |
|---|---|---|---|
| Calcd. | C 65.44; | H 6.41; | N 6.36 |
| Found | C 65.32; | H 6.38; | N 6.33 |

Example 63

Trans-7-chloro-5-(2-chlorophenyl)-1-isopropyl-2-oxo-1,2,3,5-tetrahydro-4,1-benzoxazepine-3-ethanol

In tetrahydrofuran (7 ml) were dissolved trans-7-chloro-5-(2-chlorophenyl)-1-isopropyl-2-oxo-1,2,3,5-tetrahydro-4,1-benzoxazepine-3-acetic acid (0.5 g) obtained in Reference Example 5, and N-methylmorpholine (0.15 ml). To the solution was added ethyl chlorocarbonate (0.13 ml) at -10°C, and the mixture was stirred for 10 minutes. To the mixture was added sodium borohydride (0.15 g), to which was then added dropwise methanol taking 5 minutes, followed by stirring for 30 minutes at 0°C. The reaction mixture was poured into 1N hydrochloric acid (50 ml), followed by extraction with ethyl acetate (100 ml). The ethyl acetate layer was washed with an aqueous solution of sodium hydrogencarbonate, then dried over anhydrous magnesium sulfate, followed by distilling off the solvent under reduced pressure. The residue was purified by means of a silica gel column chromatography to afford trans-7-chloro-5-(2-chlorophenyl)-1-. isopropyl-2-oxo-1,2,3,5-tetrahydro-4,1-benzoxazepine-3-ethanol (0.41 g) as prisms, m.p.188-189°C.
IR$\nu_{max}^{KBr}$ $cm^{-1}$ : 3430(OH), 1650(C=O)

| Elemental Analysis for $C_{20}H_{21}Cl_2NO_3$: | | | |
|---|---|---|---|
| Calcd. | C 60.92; | H 5.37; | N 3.55 |
| Found | C 61.12; | H 5.39; | N 3.72 |

Example 64

Ethyl ester of N-[trans-7-chloro-5-(2-chlorophenyl)-1-isopropyl-2-oxo-1,2,3,5-tetrahydro-4,1-benzoxazepine-3-acetyl] aminoacetic acid

In N,N-dimethylformamide (10 ml) were dissolved trans-7-chloro-5-(2-chlorophenyl)-1-isopropyl-2-oxo-1,2,3,5-tetrahydro-4,1-benzoxazepine-3-acetic acid (0.3 g) and glycine ethyl ester hydrochloride (0.12 g). To the solution were added, under ice-cooling, diethyl phosphorocyanidate (0.15 g) and triethylamine (0.24 ml). The mixture was stirred for 30 minutes at room temperature, to which were added water (100 ml) and ethyl acetate (100 ml), followed by extraction. The ethyl acetate layer was washed with 1N hydrochloric acid and an aqueous solution of sodium hydrogencarbonate, which was then dried over anhydrous magnesium sulfate, followed by distilling off the solvent under

reduced pressure. The residue was purified by means of a silica gel column chromatography (hexane : ethyl acetate = 1:1) to afford ethyl ester of N-[trans-7-chloro-5-(2-chlorophenyl)-1-isopropyl-2-oxo-1,2,3,5-tetrahydro-4,1-benzoxazepine-3-acetyl]aminoacetic acid (0.31 g) as needles, m.p.197-199°C.
$IR\nu_{max}^{KBr}$ $cm^{-1}$ : 1755, 1670, 1655(C=O)

| Elemental Analysis for $C_{24}H_{26}Cl_2N_2O_5$: | | | |
|---|---|---|---|
| Calcd. | C 58.43; | H 5.31; | N 5.68 |
| Found | C 58.73; | H 5.33; | N 5.80 |

Example 65

N-[trans-7-chloro-5-(2-chlorophenyl)-1-isopropyl-2-oxo-1,2,3,5-tetrahydro-4,1-benzoxazepine-3-acetyl]aminoacetic acid

In ethanol (10 ml) was dissolved ethyl ester of N-[trans-7-chloro-5-(2-chlorophenyl)-1-isopropyl-2-oxo-1,2,3,5-tetrahydro-4,1-benzoxazepine-3-acetyl]aminoacetic acid (0.2 g) obtained in Example 64. To the solution was added 1N sodium hydroxide (2 ml), and the mixture was stirred for 15 minutes. To the reaction mixture was added 1N aqueous solution of hydrochloric acid (100 ml) to make the solution acid, followed by extraction with ethyl acetate (100 ml). The ethyl acetate layer was washed with water, which was then dried over anhydrous magnesium sulfate, followed by distilling off the solvent to leave N-[trans-7-chloro-5-(2-chlorophenyl)-1-isopropyl-2-oxo-1,2,3,5- tetrahydro-4,1-benzoxazepine-3-acetyl]aminoacetic acid (0.18 g) as crystals, m.p.134-135°C.
$IR\nu_{max}^{KBr}$ $cm^{-1}$ : 1725, 1650(C=O)

| Elemental Analysis for $C_{22}H_{22}Cl_2N_2O_5.1/2Et_2O$: | | | |
|---|---|---|---|
| Calcd. | C 57.38; | H 5.42; | N 5.58 |
| Found | C 57.48; | H 5.59; | N 5.58 |

Example 66

By substantially the same synthetic method as in Example 64, compounds listed in Table 37 and Table 38 were obtained as crystalline or oily products.

Table 37

Example 66

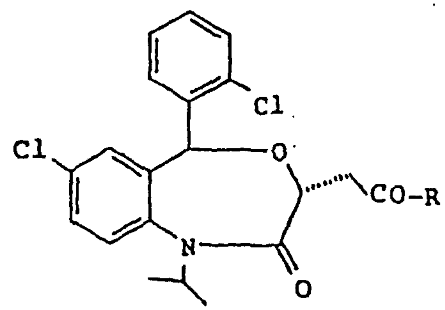

| compd. No. | R | m.p. (℃) | Formula | Elemental Analysis (Found) C | H | N |
|---|---|---|---|---|---|---|
| 1 | $NHCH_2CH_2COOEt$ | 182-183 | $C_{25}H_{28}Cl_2N_2O_5$ | 59.18 (59.13) | 5.56 (5.56) | 5.52 (5.45) |
| 2 | 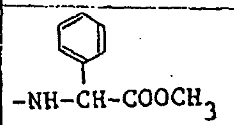 (D, L) | 160-164 | $C_{29}H_{28}Cl_2N_2O_5$ | 62.71 (62.55) | 5.08 (5.11) | 5.04 (4.86) |
| 3 | 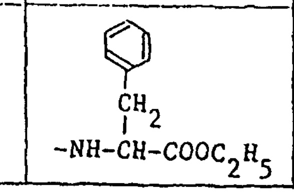 | 187-188 | $C_{31}H_{32}Cl_2N_2O_5$ | 63.81 (63.97) | 5.53 (5.59) | 4.80 (4.98) |

Example 67

By substantially the same synthetic method as in Example 65, compounds listed in Table 38 and Table 39 were obtained.

Table 38

Example 67

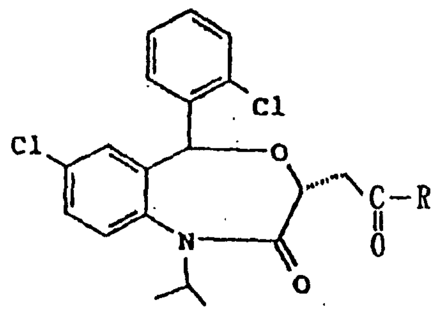

| compd. No. | R | m.p. (℃) | Formula | Elemental Analysis(Found) C | H | N |
|---|---|---|---|---|---|---|
| 1 | $NHCH_2CH_2COOH$ | 182-184 | $C_{23}H_{24}Cl_2N_2O_6$ | 57.63 (57.36) | 5.05 (5.08) | 5.84 (5.67) |
| 2 | 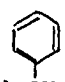 NH-CH-COOH | amorphous solid | $C_{28}H_{26}Cl_2N_2O_5$ | 62.11 (62.09) | 4.84 (5.02) | 5.17 (5.24) |
| 3 | 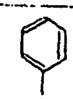 $CH_2$ NH-CH-COOH | 213-215 | $C_{29}H_{28}Cl_2N_2O_5$ | 62.71 (62.61) | 5.08 (4.96) | 5.04 (5.13) |

Example 68

By substantially the same synthetic method as in Reference Example 2, a compound listed in Table 39 was obtained.

Table 39

Example 68

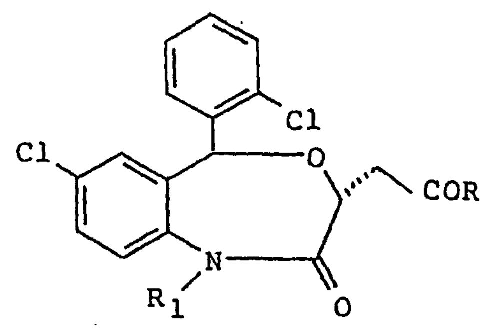

| $R_1$ | R | m. p. (℃) | Formula | Elemental Analysis(Found) C H N |
|---|---|---|---|---|
| 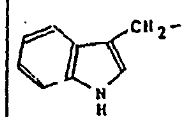 | O-$CH_2$-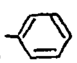 | amorphous solid | $C_{33}H_{26}Cl_2N_2O_4$ | IR $\nu_{max}^{neat}$ $cm^{-1}$: 3400(NH), 1740(CO), 1670(CO) $^1H$-NMR($CDCl_3$) $\delta$: 2.9(1H, dd), 3.2(1H,dd), 4.47(1H,dd,$C_3$-H), 5.54(1H,s,$C_5$-H) |

Also, physicochemical properties of an intermediate are shown in Table 40.

Table 40
Example 68

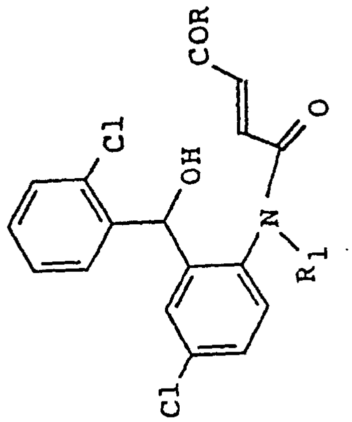

| $R_1$ | R | m.p. (°C) | Formula | Elemental Analysis (Found) C H N |
|---|---|---|---|---|
| 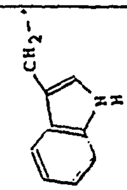 | 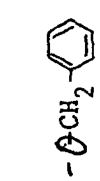 | amorphous solid | $C_{33}H_{28}Cl_2N_2O_4$ | 1H-NMR($CDCl_3$) δ: 4.7(1H, d), 5.03(2H,s,$\underline{CH_2}$Ph), 5.65(1H,d), 6.0-8.3(20H,m) |

Example 69

Ethyl ester of trans-7-chloro-5-(2,4,6-trimethoxyphenyl)-1-neopentyl-2-oxo-1,2,3,5-tetrahydro-4,1-benzoxazepine-3-acetic acid

(1) 2-acetylamino-5-chloro-2',4',6'-trimethoxybenzophenone

A solution of 2.41 g of 1,3,5-trimethoxybenzene in 10 ml of dry tetrahydrofuran was cooled to -78°C, and 9.1 ml of n-butyl lithium (1.58 M solution in hexane) was added dropwise over a period of 10 minutes. This solution was added dropwise to a solution of 2.0 g of 6-chloro-4-methyl-4H-3,1-benzoxazin-4-one in 20 ml of dry tetrahydrofuran. After mixture stirring at 0°C for 1 hour, the solvent was removed, after which the residue was acidified with dilute hydrochloric acid and then extracted with ethyl acetate. After the extract was washed with dilute hydrochloric acid and an aqueous solution of sodium hydrogen carbonate, the solvent was removed, and the residue was subjected to silica gel column chromatography to yield 1.28 g of a crystal. Melting point: 159-160°C.

| Elemental analysis (for $C_{18}H_{18}ClNO_5$) | | | |
|---|---|---|---|
| Calcd. | C 59.43; | H 4.99; | N 3.85 |
| Found | C 59.39; | H 4.94; | N 3.86 |

(2) 2-amino-5-chloro-2',4',-6'-trimethoxybenzophenone

A mixture of 4.7 g of 2-acetylamino. -5-chloro-2',4',6'-trimethoxybenzophenone, 50 ml of 6 N hydrochloric acid and 50 ml of ethanol was refluxed for 1 hour while heating. After the solvent was distilled off, the residue was basefied with an aqueous solution of sodium hydrogen carbonate and then extracted with ethyl acetate. After the extract was washed with water and dried, the solvent was removed, and the residue was subjected to silica gel column chromatography to yield 3.75 g of a crystal.

(3) 2-amino-5-chloro-α-(2,4,6-trimethoxyphenyl)benzyl alcohol

To a solution of 3.0 g of 2-amino-5-chloro-2',4',6'-trimethoxybenzophenone in 50 ml of tetrahydrofuran, 0.43 g of lithium aluminum hydride was added, followed by stirring for 1 hour. After water was added, the solution was extracted with ethyl acetate and dried over anhydrous sodium sulfate, after which the solvent was removed and the residue was subjected to silica gel column chromatography to yield 2.9 g of a crystal.

(4) 5-chloro-α-(2,4,6-trimethoxyphenyl)-2-(neopentylamino)benzyl alcohol

After a solution of 2.5 g of 2-amino-5-chloro-α-(2,4,6-trimethoxyphenyl)benzyl alcohol, 1.01 ml of trimethylacetaldehyde and 0.56 g of acetic acid in 30 ml of ethanol was stirred at room temperature for 1.5 hours, 0.81 g of sodium cyanoborohydride was added, followed by stirring overnight. After concentration and subsequent dilution with water, the solution was extracted with ethyl acetate. After solvent removal, the residue was subjected to silica gel column chromatography to yield 2.2 g of a crystal.

(5) Ethylester of 3-[N-(4-chloro-2-(α-hydroxy-2,4,6-trimethoxybenzyl)phenyl]-N-neopentylcarbamoyl]acrylic acid

2.0 g of 5-chloro-α-(2,4,6-trimethoxyphenyl)-2-(neopentylamino)benzyl alcohol, 0.99 g of monoethyl ester of chlorofumaric acid and 0.85 g of sodium hydrogen carbonate were added to 30 ml of dichloromethane, and this mixture was stirred for 30 minutes. To the reaction mixture was added water, and the organic layer was dried, after which the solvent was removed and the residue was subjected to silica gel column chromatography to yield 2.5 g of an oily compound.

(6) Ethyl ester of trans-7-chloro-5-(2,4,6-trimethoxyphenyl)-1-neopentyl-2-oxo-1,2,3,5-tetrahydro-4,1-benzoxazepine-3-acetic acid.

2.5 g of ethyl ester of 3-[N-(4-chloro-2-(α-hydroxy-2,4,6-trimethoxybenzyl)phenyl]-N-neopentylcarbamoyl] acrylic acid and 1.33 g of potassium carbonate were added to 30 ml of ethanol, and this mixture was stirred at room temperature overnight. After ethyl acetate was added, the mixture was washed with water and dried, after which the solvent was removed and the residue was subjected to silica gel column chromatography to yield 2.0 g of a crystal.
Melting point: 154-155°C.

| Elemental analysis (for $C_{27}H_{34}ClNO_7$) | | | |
|---|---|---|---|
| Calcd. | C 62.36; | H 6.59; | N 2.69 |
| Found | C 62.51; | H 6.32; | N 2.67 |

Example 70

By the same procedure as in Example 69, the compounds listed in Tables 41 and 42 were obtained.

Table 41

Example 70

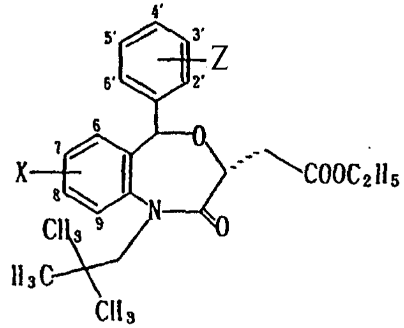

| Compd. No. | X | Z | m.p. (°C) | Formula | Elemental Analysis(Found) C | H | N |
|---|---|---|---|---|---|---|---|
| 1 | 7-Cl | 2',4'-$OCH_3$ | 117-119 | $C_{26}H_{32}ClNO_6$ | 63.73 (63.63 | 6.58 6.81 | 2.86 2.83) |
| 2 | 7-Cl | 2',6'-$OCH_3$ | 175-177 | $C_{26}H_{32}ClNO_6$ | 63.73 (63.54 | 6.58 6.49 | 2.86 2.89) |
| 3 | 7-Cl | 2',5'-$OCH_3$ | 165-166 | $C_{26}H_{32}ClNO_6$ | 63.73 (63.63 | 6.58 6.59 | 2.86 2.82) |
| 4 | 7-Cl | 2',3'-$OCH_2O$- | 164-167 | $C_{25}H_{28}ClNO_6$ | 63.35 (63.31 | 5.95 5.88 | 2.96 2.92) |
| 5 | 7-Cl | 3',4'-$OCH_2O$- | 133-134 | $C_{25}H_{28}ClNO_6$ | 63.36 (63.13 | 5.95 5.96 | 2.96 2.79) |

Table 42

Example 70

| Compd. No. | X | Z | m.p. (℃) | Formula | Elemental Analysis(Found) C | H | N |
|---|---|---|---|---|---|---|---|
| 6 | 8-Cl | 2'-Cl | 142-143 | $C_{24}H_{27}Cl_2NO_4$ | 62.07 (61.87 | 5.86 6.01 | 3.02 2.89) |
| 7 | 7,9-Cl | 2'-OCH$_3$ | 167-169 | $C_{25}H_{29}Cl_2NO_5$ | 60.73 (60.73 | 5.91 5.97 | 2.83 2.74) |
| 8 | 7-Cl | 2'-OCH$^3$ | 173-174 | $C_{25}H_{30}ClNO_5$ | 65.28 (65.32 | 6.57 6.64 | 3.05 3.11) |
| 9 | 7-Cl | 2'-Br | 128-129 | $C_{24}H_{27}BrClNO_4$ | 56.65 (56.77 | 5.35 5.42 | 2.75 2.76) |
| 10 | 7-Br | 2'-Cl | 143-146 | $C_{24}H_{27}BrClNO_4$ | 56.65 (56.50 | 5.35 5.23 | 2.75 2.58) |
| 11 | 7-Cl | 2',3'-OCH$_3$ | 184-185 | $C_{26}H_{32}ClNO_6$ | 63.73 (63.75 | 6.58 6.82 | 2.86 2.67) |

Example 71

By the same procedure as in Example 69 except that isobutyl aldehyde was used in place of trimethylacetaldehyde, the compounds listed in Tables 43 through 45 were obtained.

**Table 43**

Example 71

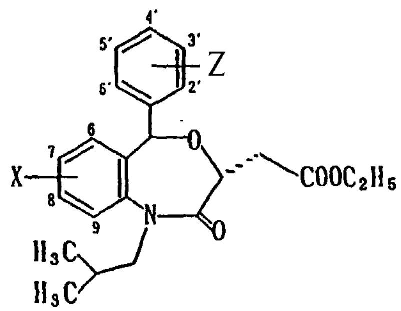

| Compd. No. | X | Z | m.p. (°C) | Formula | Elemental Analysis(Found) C H N |
|---|---|---|---|---|---|
| 1 | 7-F | 2'-Cl | oil | $C_{23}H_{25}ClFNO_4$ | $^1$H-NMR($CDCl_3$)δ:0.93 and 1.03(each 3H, d, J=6.6Hz), 1.25(3H, t, J=7.2Hz), 1.86-2.10 (1H, m), 2.80(1H, dd, J=16.5, 6.2Hz), 3.06(1H, dd, J=16.5, 7.4 Hz), 3.44(1H, dd, J=13.8, 5.4Hz), 4.14(2H, q, J=7.2Hz), 4.32 (1H, dd, J=13.8, 8.4Hz), 4.43(1H, dd, J=7.4, 6.2Hz), 6.15 (1H, s), 6.26(1H, dd, J=9.0, 2.9Hz), 7.12(1H, ddd, J=8.8, 7.6, 2.9Hz), 7.29-7.47(4H, m), 7.66-7.75(1H, m) |

Table 44

Example 71

| Compd. No. | X | Z | m.p. (℃) | Formula | Elemental Analysis(Found) C H N |
|---|---|---|---|---|---|
| 2 | 7-Cl | 2',4'-Cl | amorphous solid | $C_{23}H_{24}Cl_3NO_4$ | 56.98 4.99 2.89<br>(56.92 5.00 2.95) |
| 3 | 7-Cl | 2'-CF$_3$ | 120-122 | $C_{24}H_{25}ClF_3NO_4$ | 59.57 5.21 2.89<br>(59.57 5.18 2.84) |
| 4 | 7-Cl | 2-OCH$_3$ | 124-125 | $C_{24}H_{28}ClNO_5$ | 64.64 6.33 3.14<br>(64.54 6.34 3.07) |
| 5 | 7-Cl | 2'-Br | oil | $C_{23}H_{25}BrClNO_4$ | $^1$H-NMR($CDCl_3$)δ:0.95(3H, d, J=6.8Hz), 1.06 (3H, d, J=6.6Hz), 1.25(3H, t, J=7.1Hz), 1.85-2.09(1H, m), 2.80(1H, dd, J=16.6, 6.2Hz), 3.05(1H, dd, J=16.6, 7.4Hz), 3.48(1H, dd, J=13.8, 5.2Hz), 4.14(2H, q, J=7.1Hz), 4.29 (1H, dd, J=13.8, 8.8Hz), 4.43(1H, dd, J=7.4, 6.2Hz), 6.07(1H, s), 6.50(1H, d, J=2.2Hz), 7.22-7.75(6H, m) |
| 6 | 7-Br | 2'-Cl | oil | $C_{23}H_{25}BrClNO_4$ | $^1$H-NMR($CDCl_3$)δ:0.93 and 1.02(each 3H, d, J=6.6Hz), 1.25(3H, t, J=7.2Hz), 1.86-2.08 (1H, m), 2.79(1H, dd, J=16.6, 6.2Hz), 3.06(1H, dd, J=16.6, 7.4Hz), 3.44(1H, dd, J=13.8, 5.6Hz), 4.13(2H, q, J=7.2Hz), 4.31(1H, dd, J=13.8, 8.4Hz), 4.43(1H, dd, J=7.4, 6.2Hz), 6.13(1H, s), 6.65(1H, d, J=2.4Hz), 7.20-7.75(6H, m) |

Table 45

Example 71

| Compd. No. | X | Z | m. p. (°C) | Formula | Elemental Analysis(Found) C H N |
|---|---|---|---|---|---|
| 7 | 7-Cl | 4'-Cl | oil | $C_{23}H_{25}Cl_2NO_4$ | $^1$H-NMR(CDCl$_3$)δ:0. 91 and 0. 98(each 3H, d, J=6. 6Hz), 1. 24(3H, t, J=7. 1Hz), 1. 93-2. 20(1H, m), 2. 76(1H, dd, J=16. 6, 6. 0Hz), 3. 05(1H, dd, J=16. 6, 7. 6Hz), 3. 44(1H, dd, J=13. 6, 7. 6Hz), 4. 12(2H, q, J=7. 1Hz), 4. 26(1H, dd, J=13. 6, 7. 6Hz), 4. 41(1H, dd, J=7. 6, 6. 0Hz), 5. 84(1H, s), 6. 57(1H, s), 7. 20-7. 50(6H, m) |
| 8 | H | 2'-$CH_3$ | 88- 90 | $C_{24}H_{29}NO_4$ | 72. 89 7. 39 3. 54 (73. 18 7. 25 3. 54) |
| 9 | 7-Cl | 3'-Cl | oil | $C_{23}H_{25}Cl_2NO_4$ | $^1$H-NMR(CDCl$_3$)δ:0. 91(3H, d, J=20Hz), 0. 98(3H, d, J=6. 6Hz), 1. 25(3H, t, J=7. 1Hz), 1. 95-2. 18 (1H, m), 2. 78(1H, dd, J=16. 4, 6. 0Hz), 3. 05(1H, dd, J=16. 4, 7. 6Hz), 3. 44(1H, dd, J=13. 6, 6. 4Hz), 4. 13(2H, q, J=7. 1Hz), 4. 26(1H, dd, J=13. 6, 7. 6Hz), 4. 40(1H, dd, J=7. 6, 6. 0Hz), 5. 84(1H, s), 6. 59(1H, d, J=2. 0Hz), 7. 14-7. 50(6H, m) |
| 10 | 7-Cl | H | oil | $C_{23}H_{26}ClNO_4$ | $^1$H-NMR(CDCl$_3$)δ:0. 91 and 0. 99(each 3H, d, J=6. 6Hz), 1. 24(3H, t, J=7. 2Hz), 1. 95-2. 20(1H, m), 2. 78(1H, dd, J=16. 6, 5. 8Hz), 3. 07(1H, dd, J=16. 6, 7. 8Hz), 3. 44(1H, dd, J=13. 8, 6. 4Hz), 4. 13(2H, q, J=7. 2Hz), 4. 27(1H, dd, J=13. 8, 7. 6Hz), 4. 43(1H, d, J=7. 8, 5. 8Hz), 5. 88(1H, s), 6. 61(1H, d, J=2. 4Hz), 7. 24-7. 49(7H, m) |

Example 72

The compounds obtained in Examples 69 and 70 were hydrolyzed by the same procedure as in Reference Examples 5 and Example 2 to yield the compounds listed in Tables 46 and 47.

Table 46

Example 72

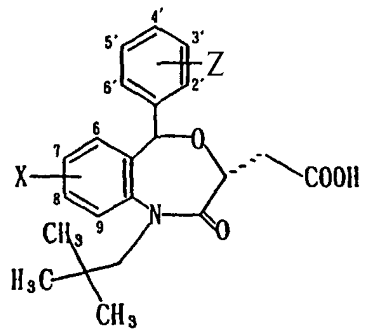

| Compd. No. | X | Z | m.p. (℃) | Formula | Elemental Analysis(Found) C | H | N |
|---|---|---|---|---|---|---|---|
| 1 | 7-Cl | 2',4'-$OCH_3$ | 260-263 | $C_{24}H_{28}ClNO_6$ | 62.40 (62.27 | 6.11 6.26 | 3.03 2.97) |
| 2 | 7-Cl | 2',6'-$OCH_3$ | 263-270 (decomp) | $C_{24}H_{28}ClNO_6$ +0.3$H_2O$ | 61.68 (61.72 | 6.17 6.15 | 3.00 2.90) |
| 3 | 7-Cl | 2',5'-$OCH_3$ | 230-232 | $C_{24}H_{28}ClNO_6$ | 62.40 (62.31 | 6.11 6.13 | 3.03 3.03) |
| 4 | 7-Cl | 2',4',6' -$OCH_3$ | 260-263 (decomp) | $C_{25}H_{30}ClNO_7$ | 61.04 (60.97 | 6.15 6.12 | 2.85 2.71) |
| 5 | 7-Cl | 2',3'-$OCH_2O$- | 215-218 | $C_{23}H_{24}ClNO_6$ | 61.95 (61.78 | 5.42 5.56 | 3.14 3.31) |

**Table 47**

Example 72

| Compd. No. | X | Z | m.p. (°C) | Formula | Elemental Analysis (Found) C | H | N |
|---|---|---|---|---|---|---|---|
| 6 | 7-Cl | 3',4'-$OCH_2O$- | 211-212 | $C_{23}H_{24}ClNO_6$ | 61.95<br>(61.79 | 5.43<br>5.37 | 3.14<br>2.94) |
| 7 | 8-Cl | 2'-Cl | 186-187 | $C_{22}H_{23}Cl_2NO_4$ | 60.56<br>(60.23 | 5.31<br>5.58 | 3.21<br>3.06) |
| 8 | 7,9-Cl | 2'-$OCH_3$ | 265-267 | $C_{23}H_{25}Cl_2NO_5$<br>+0.5$H_2O$ | 58.12<br>(58.18 | 5.51<br>5.27 | 2.94<br>2.69) |
| 9 | 7-Cl | 2'-$OCH_3$ | 167-168 | $C_{23}H_{26}ClNO_5$ | 63.96<br>(63.56 | 6.07<br>6.14 | 3.24<br>3.07) |
| 10 | 7-Cl | 2'-Br | 251-253 | $C_{22}H_{23}BrClNO_4$ | 54.96<br>(54.97 | 4.82<br>4.85 | 2.91<br>2.87) |
| 11 | 7-Br | 2'-Cl | 247-250 | $C_{22}H_{23}BrClNO_4$ | 54.96<br>(54.73 | 4.82<br>5.12 | 2.91<br>2.82) |
| 12 | 7-Cl | 2',3'-$OCH_3$ | 244-247 | $C_{24}H_{28}ClNO_6$<br>+0.3$H_2O$ | 61.68<br>(61.65 | 6.17<br>6.16 | 3.00<br>2.89) |

Example 73

The compounds obtained in Example 71 were hydrolyzed by the same procedure as in Reference Example 5 and Example 2 to yield the compounds listed in Tables 48 and 49.

Table 48

Example 73

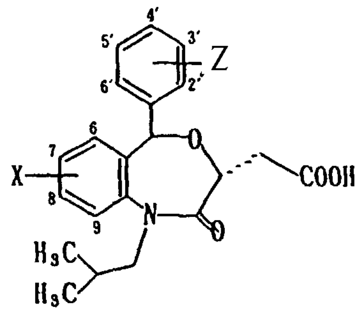

| Compd. No. | X | Z | m.p. (℃) | Formula | Elemental Analysis(Found) C | H | N |
|---|---|---|---|---|---|---|---|
| 1 | 7-F | 2'-Cl | 204-206 | $C_{21}H_{21}ClFNO_4$ | 62.15 (61.96 | 5.22 5.18 | 3.45 3.66) |
| 2 | 7-Cl | 2',4'-Cl | 236-238 | $C_{21}H_{20}Cl_3NO_4$ | 55.22 (55.12 | 4.41 4.45 | 3.07 3.00) |
| 3 | 7-Cl | 2'-CF₃ | 212-225 | $C_{22}H_{21}ClF_3NO_4$ | 57.97 (57.72 | 4.64 4.67 | 3.07 3.05) |

Table 49
Example 73

| Compd. No. | X | Z | m.p. (℃) | Formula | Elemental Analysis(Found) C | H | N |
|---|---|---|---|---|---|---|---|
| 4 | 7-Cl | 2'-$OCH_3$ | 219-222 | $C_{22}H_{24}ClNO_5$ | 63.23 (62.88 | 5.79 6.09 | 3.35 3.16) |
| 5 | 7-Cl | 2'-Br | 212-216 | $C_{21}H_{21}BrClNO_4$ | 54.04 (53.68 | 4.53 4.56 | 3.00 3.10) |
| 6 | 7-Br | 2'-Cl | 194-196 | $C_{21}H_{21}BrClNO_4$ +0.2$H_2O$ | 53.61 (53.44 | 4.59 4.49 | 2.98 2.91) |
| 7 | 7-Cl | 4'-Cl | 223-225 | $C_{21}H_{21}Cl_2NO_4$ | 59.73 (59.58 | 5.01 5.01 | 3.32 3.23) |
| 8 | H | 2'-$CH_3$ | 192-195 | $C_{22}H_{25}NO_4$ | 71.91 (71.86 | 6.86 6.78 | 3.81 3.80) |
| 9 | 7-Cl | 3'-Cl | 158-160 | $C_{21}H_{21}Cl_2NO_4$ +0.2$H_2O$ | 59.22 (59.00 | 5.06 5.00 | 3.29 3.23) |
| 10 | 7-Cl | H | 190-192 | $C_{21}H_{22}ClNO_4$ | 65.03 (64.94 | 5.72 5.61 | 3.61 3.48) |

Example 74

n-butyl ester of trans-7-chloro-5-(2-chlorophenyl)-1-neopentyl-2-oxo-1,2,3,5-tetrahydro-4,1-benzoxazepine-3-acetic acid

A solution of 0.4 g of trans-7-chloro-5-(2-chlorophenyl)-1-neopentyl-2-oxo-1,2,3,5-tetrahydro-4,1-benzoxazepine-3-acetic acid as obtained in Example 2, 0.21 ml of n-butyl iodide and 0.21 ml of triethylamine in 5 ml of dimethylformamide was stirred overnight at room temperature. After ethyl acetate was added, the mixture was washed with dilute hydrochloric acid, an aqueous solution of sodium hydrogen carbonate and water. After the solution was dried, the solvent was removed and the residue was subjected to silica gel column chromatography to yield 0.25 g of a crystal. Melting point: 106-109°C.

| Elemental analysis (for $C_{26}H_{31}Cl_2NO_4$) | | | |
|---|---|---|---|
| Calcd. | C 63.42; | H 6.35; | N 2.84 |
| Found | C 63.42; | H 6.39; | N 2.71 |

Example 75

By the same procedure as in Example 74, the compounds listed in Table 50 were synthesized.

Table 50

Example 75

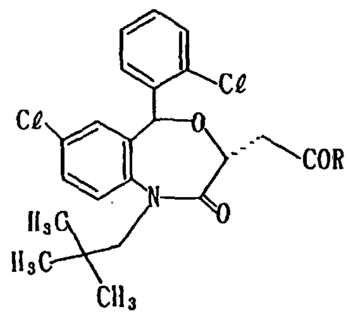

| Compd. No. | R | m. p. (℃) | Formula | Elemental Analysis(Found) C | H | N |
|---|---|---|---|---|---|---|
| 1 | $-OCH_3$ | 158-159 | $C_{23}H_{25}Cl_2NO_4$ | 61.34<br>(61.48 | 5.60<br>5.78 | 3.11<br>2.84) |

Example 76

The compounds obtained in Example 2 were subjected to the same procedure as before to yield the compounds listed in Table 51.

Table 51

Example 76

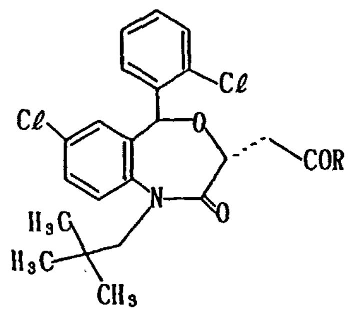

| Compd. No. | R | m. p. (℃) | Formula | Elemental Analysis(Found) C | H | N |
|---|---|---|---|---|---|---|
| 1 | $-O-CH(CH_3)_2$ | 100-101 | $C_{25}H_{29}Cl_2NO_4$ | 62.76 (62.91 | 6.11 6.32 | 2.93 2.72) |

Example 77

t-butyl ester of trans-7-chloro-5-(2-chlorophenyl)-1-neopentyl-2-oxo-1,2,3,5-tetrahydro-4,1-benzoxazepine-3-acetic acid.

To a solution of 1.2 g of trans-7-chloro-5-(2-chlorophenyl)-1-neopentyl-2-oxo-1,2,3,5-tetrahydro-4,1-benzoxazepine-3-acetic acid as obtained in Example 2 in 40 ml of dichloromethane, 10 ml of isobutene and 0.1 ml of concentrate sulfuric acid were added, and the mixture was kept standing at room temperature for 3 days. After reaction mixture concentration, ethyl acetate and an aqueous solution of sodium hydrogen carbonate were added, and the organic layer was washed with water and dried, afte which the solvent was removed and the residue was subjected to silica gel column chromatography to yield 1.07 g of a crystal.
Melting point: 141-142°C.

| Elemental analysis (for $C_{26}H_{31}ClNO_4$) | | | |
|---|---|---|---|
| Calcd. | C 63.42; | H 6.35; | N 2.84 |
| Found | C 63.52; | H 6.37; | N 2.80 |

Example 78

Carboxymethyl ester of trans-7-chloro-5-(2-chlorophenyl)-1-neopentyl-2-oxo-1,2,3,5-tetrahydro-4,1-benzoxazepine-3-acetic acid

0.3 g of t-butoxycarbonylmethyl ester of trans-7-chloro-5-(2-chlorophenyl)-1-neopentyl-2-oxo-1,2,3,5-tetrahydro-4,1-benzoxazepine-3-acetic acid as obtained in Example 75 was dissolved in 3 ml of trifluoroacetic acid, and this solution was kept standing at room temperature for 30 minutes. After the solution was concentrated, ethyl acetate was added, followed by washing with water and subsequent drying, after which the solvent was removed and the residue was recrystallized from petroleum ether to yield 0.24 g of a crystal.
Melting point: 186-190°C.

| Elemental analysis (for $C_{24}H_{25}Cl_2NO_6\cdot 1/2H_2.O$) | | | |
|---|---|---|---|
| Calcd. | C 57.27; | H 5.21; | N 2.78 |
| Found | C 57.38; | H 5.05; | N 2.78 |

Example 79 (Reference)

Methyl ester of 2-[trans-7-chloro-5-(2-chlorophenyl)-1-isopropyl-2-oxo-1,2,3,5-tetrahydro-4,1-benzoxazepine-3-acetyl]aminobenzoic acid

0.35 g of trans-7-chloro-5-(2-chlorophenyl)-1-isopropyl-2-oxo-1,2,3,5-tetrahydro-4,1-benzoxazepine-3-acetic acid as obtained in Reference Example 5 was dissolved in 10 ml of toluene, and 0.63 ml of thionyl chloride was added, followed by stirring for 30 minutes while heating at 90°C. The solution was concentrated and the residue was dissolved in 10 ml of dichloromethane, and 0.13 ml of methyl ester of 2-aminobenzoic acid and 0.14 ml of triethylamine were added, followed by stirring at room temperature for 30 minutes. After the solution was concentrated, ethyl acetate was added, and the mixture was washed with dilute hydrochloric acid, an aqueous solution of sodium hydrogen carbonate and water. The solvent was removed and the residue was subjected to silica gel column chromatography to yield 0.25 g of a crystal.
Melting point: 188-190°C.

| Elemental analysis (for $C_{28}H_{26}Cl_2N_2O_5$) | | | |
|---|---|---|---|
| Calcd. | C 62.11; | H 4.91; | N 5.24 |
| Found | C 61.94; | H 4.91; | N 5.24 |

Example 80 (Reference)

By the same procedure as in Example 79 (Reference), the compounds listed in Tables 52 and 53 were ob-

tained.

Table 52

Example 80 (Reference)

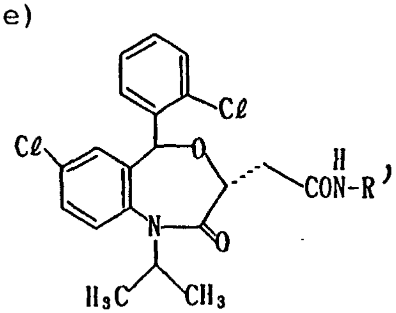

| Compd. No. | R′ | m. p. (℃) | Formula | Elemental Analysis(Found) C | H | N |
|---|---|---|---|---|---|---|
| 1 | 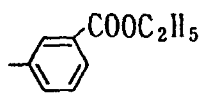 | 161-163 | $C_{29}H_{28}Cl_2N_2O_5$ | 62.71 (62.62 | 5.08 4.96 | 5.04 5.15) |
| 2 | 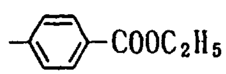 | 236-238 | $C_{29}H_{28}Cl_2N_2O_5$ | 62.71 (62.67 | 5.08 5.26 | 5.04 5.09) |
| 3 | 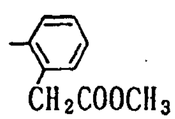 | 172-174 | $C_{29}H_{28}Cl_2N_2O_5$ | 62.71 (62.63 | 5.08 5.00 | 5.04 5.28) |
| 4 | 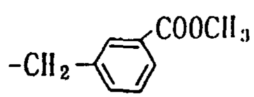 | 92- 94 | $C_{20}H_{28}Cl_2N_2O_5$ $\cdot\frac{1}{2}H_2O$ | 61.71 (61.85 | 5.18 5.33 | 4.96 5.10) |

Table 53

Example 80 (Reference)

| Compd. No. | R' | m.p. (℃) | Formula | Elemental Analysis(Found) C | H | N |
|---|---|---|---|---|---|---|
| 5 | 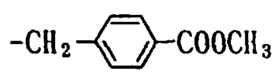 | 139-142 | $C_{29}H_{28}Cl_2N_2O_5$ | 62.71 (62.62 | 5.08 5.12 | 5.04 4.96) |
| 6 | 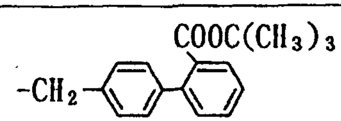 | oil | $C_{38}H_{38}Cl_2N_2O_5$ | $^1$H-NMR($CDCl_3$)δ: 1.27(9H, s, Bu$^t$), 1.29(3H, d, J=6.8Hz), 1.54(3H, d, J=6.8Hz), 2.71(1H, dd, J=14.2, 6.0Hz), 2.95(1H, dd, J=14.2, 7.4Hz), 4.38(1H, t, J=6.6Hz), 4.45-4.6(1H, m), 4.7-4.9(2H, m), 6.00(1H, s), 6.3-6.45 (1H, m, NH), 6.50(1H, d, J=2.4Hz), 7.2-7.85(14H, m) | | |

Example 81

2-[trans-7-chloro-5-(2-chlorophenyl)-1-isopropyl-2-oxo-1,2,3,5-tetrahydro-4,1-benzoxazepine-3-acetyl]aminobenzoic acid

0.15 g of methyl ester of 2-[trans-7-chloro-5-(2-chlorophenyl)-1-isopropyl-2-oxo-1,2,3,5-tetrahydro-4,1-benzoxazepine-3-acetyl]aminobenzoic acid as obtained in Example 79 (Reference) was subjected to the same procedure of ester hydrolysis as in Example 33 to yield 0.11 g of a crystal.
Melting point: 208-211°C.

| Elemental analysis (for $C_{27}H_{24}Cl_2N_2O_5$) | | | |
|---|---|---|---|
| Calcd. | C 61.49; | H 4.59; | N 5.31 |
| Found | C 61.22; | H 4.68; | N 5.35 |

Example 82

The compounds obtained in Example 80 (Reference) were subjected to the same procedure of ester hydrolysis as in Example 33 to yield the compounds listed in Table 54.

Table 54
Example 82

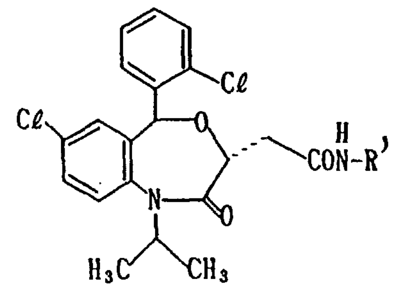

| Compd. No. | R' | m.p. (℃) | Formula | Elemental Analysis(Found) C | H | N |
|---|---|---|---|---|---|---|
| 1 | 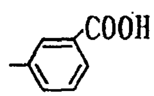 | 149-152 | $C_{27}H_{24}Cl_2N_2O_5$ | 61.49 (61.81 | 4.59 4.79 | 5.31 5.45) |
| 2 | 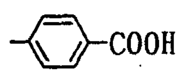 | >300 | $C_{27}H_{24}Cl_2N_2O_5 \cdot \frac{1}{4}H_2O$ | 60.97 (60.97 | 4.64 4.76 | 5.27 5.39) |
| 3 | 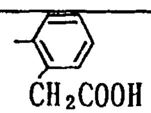 | 223-225 | $C_{28}H_{26}Cl_2N_2O_5$ | 62.11 (61.96 | 4.84 4.82 | 5.17 5.35) |
| 4 | 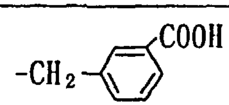 | 240-244 | $C_{28}H_{26}Cl_2N_2O_5$ | 62.11 (62.13 | 4.84 5.00 | 5.17 5.17) |
| 5 | 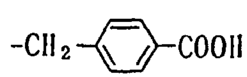 | 242-244 (decomp) | $C_{28}H_{26}Cl_2N_2O_5$ | 62.11 (61.96 | 4.84 4.85 | 5.17 5.25) |

Example 83 (Reference)

Ethyl ester of 4-[trans-7-chloro-5-(2-chlorophenyl)-1-neopentyl-2-oxo-1,2,3,5-tetrahydro-4,1-benzoxazepine-3-acetyl] aminobenzoic acid

0.6 g of trans-7-chloro-5-(2-chlorophenyl)-1-neopentyl-2-oxo-1,2,3,5-tetrahydro-4,1-benzoxazepine-3-acetic acid as obtained in Example 2 and 0.27 g of ethyl ester of 4-aminobenzoic acid were subjected to the same procedure as in Example 79 to yield 0.43 g of a crystal.
Melting point: 210-218 °C.

| Elemental analysis (for $C_{31}H_{32}Cl_2N_2O_5$) | | | |
|---|---|---|---|
| Calcd. | C 63.81; | H 5.53; | N 4.80 |
| Found | C 63.79; | H 5.38; | N 4.70 |

Example 84 (Reference)

By the same procedure as in Example 79, the compounds listed in Tables 55 and 56 were synthesized.

Table 55

Example 84 (Reference)

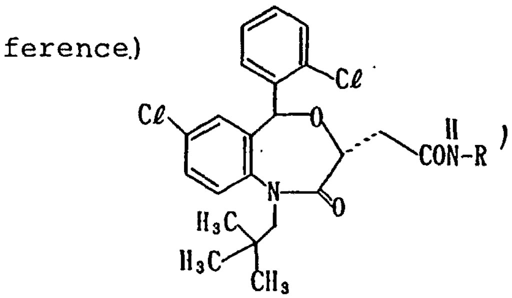

| Compd. No. | R' | m.p. (°C) | Formula | Elemental Analysis (Found) C | H | N |
|---|---|---|---|---|---|---|
| 1 | 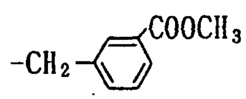 | 181-182 | $C_{31}H_{32}Cl_2N_2O_5$ | 63.81 (63.85 | 5.53 5.41 | 4.80 4.66) |
| 2 | 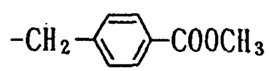 | 208-209 | $C_{31}H_{32}Cl_2N_2O_5$ | 63.81 (63.62 | 5.53 5.53 | 4.80 4.79) |
| 3 | 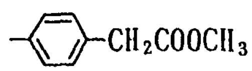 | 224-225 | $C_{31}H_{32}Cl_2N_2O_5$ | 63.81 (63.69 | 5.53 5.60 | 4.80 4.54) |
| 4 | 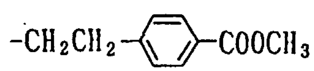 | 114-115 | $C_{32}H_{34}Cl_2N_2O_5$ | 64.32 (64.22 | 5.74 5.97 | 4.69 4.43) |

Table 56

Example 84 (Reference)

| Compd. No. | R′ | m.p. (°C) | Formula | Elemental Analysis(Found) C H N |
|---|---|---|---|---|
| 5 | 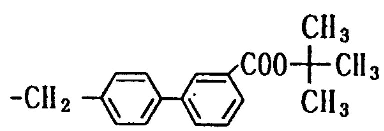 | oil | 1H-NMR($CDCl_3$)δ:0.94(9H, s, Bu$^t$), 1.27(9H, s, Bu$^t$), 2.72(1H, dd, J=14.2, 6.0Hz), 2.92(1H, dd, J=14.2, 7.2Hz), 3.40(1H, d, J=14.0 Hz), 4.30-4.7(4H, m), 6.27(1H, s), 5.52(1H, d, J=1.6Hz), 7.1 -7.9(14H, m) | |

Example 85

The compounds synthesized in Examples 83 and 84 were subjected to the same procedure as in Examples 23 and 78 to yield the compounds listed in Tables 57 and 58.

Table 57

Example 85

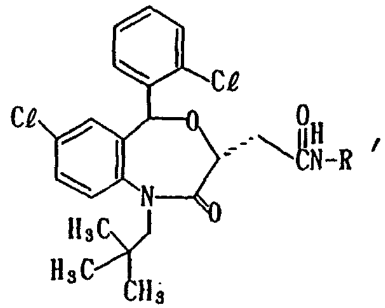

| Compd. No. | R′ | m. p. (℃) | Formula | Elemental Analysis(Found) C | H | N |
|---|---|---|---|---|---|---|
| 1 | 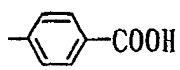 | 283-286 | $C_{29}H_{28}Cl_2N_2O_5$ | 62.71 (62.95 | 5.08 5.39 | 5.04 5.01) |
| 2 | 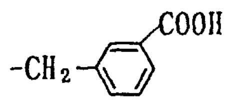 | 220-223 | $C_{30}H_{30}Cl_2N_2O_5$ | 63.27 (63.24 | 5.31 5.23 | 4.92 4.78) |
| 3 | 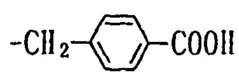 | 251-253 | $C_{30}H_{30}Cl_2N_2O_5$ | 63.27 (62.98 | 5.31 5.43 | 4.92 4.61) |
| 4 | 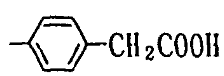 | 156-158 | $C_{30}H_{30}Cl_2N_2O_5$ | 63.27 (63.39 | 5.31 5.21 | 4.92 4.76) |

Table 58

Example 85

| Compd. No. | R' | m. p. (℃) | Formula | Elemental Analysis(Found) C | H | N |
|---|---|---|---|---|---|---|
| 5 | 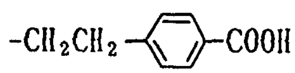 | 143-145 | $C_{31}H_{32}Cl_2N_2O_5$ ½$H_2O$ | 62.28 (62.32 | 5.61 5.78 | 4.73 4.49) |
| 6 | 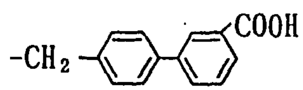 | 158-161 | $C_{36}H_{36}Cl_2N_2O_5$ | 66.77 (66.76 | 5.60 5.80 | 4.33 4.39) |

Example 86

Trans-7-chloro-5-(2-chlorophenyl)-1-isobutyl-2-oxo-1,2,3,5-tetrahydro-4,1-benzoxazepine-3-acetamide

1.0 g of trans-7-chloro-5-(2-chlorophenyl)-1-isobutyl-2-oxo-1,2,3,5-tetrahydro-4,1-benzoxazepine-3-acetic acid and 0.5 g of ammonium chloride were dissolved in 8 ml of dimethylformamide, and 0.46 g of 1-ethyl-3-(3-dimethylaminopropyl)carbodiimide hydrochloride and 0.7 ml of triethylamine were added, followed by stirring at room temperature for 30 minutes. After water was added, the solution was extracted with ethyl acetate, followed by washing with water, after which the solvent was removed and the residue was recrustallized to yield 0.75 g of a crystal.
Melting point: 271-272°C.

| Elemental analysis (for $C_{21}H_{22}Cl_2N_2O_3$) | | | |
|---|---|---|---|
| Calcd. | C 59.87; | H 5.26; | N 6.65 |
| Found | C 59.91; | H 5.33; | N 6.91 |

Example 87

The compound obtained in Example 2 and Reference Example 5 and various amines were subjected to the same procedure as in Example 86 to yield the compounds listed in Tables 59 through 63.

**Table 59**

Example 87

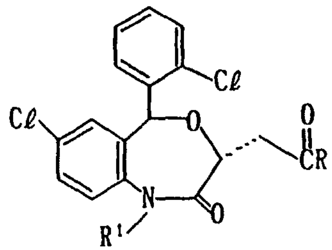

| Compd. No. | $R^1$ | R | m.p. (℃) | Formula | Elemental Analysis (Found) C | H | N |
|---|---|---|---|---|---|---|---|
| 1 | $CH(CH_3)_2$ | $NH_2$ | 226-227 | $C_{20}H_{20}Cl_2N_2O_3$ | 58.98 (58.93 | 4.95 4.85 | 6.88 6.75) |
| 2 | $CH_3$ | $NH_2$ | 187-189 | $C_{18}H_{16}Cl_2N_2O_3$ | 56.34 (56.53 | 4.33 4.55 | 7.30 6.94) |
| 3 | 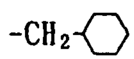 | $NH_2$ | 246-248 | $C_{24}H_{26}Cl_2N_2O_3$ | 62.48 (62.28 | 5.68 5.87 | 6.07 5.86) |
| 4 | 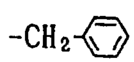 | $NH_2$ | 185-187 | $C_{24}H_{20}Cl_2N_2O_3$ | 63.31 (63.23 | 4.43 4.49 | 6.15 6.10) |

Table 60

Example 87

| Compd. No. | $R^1$ | R | m.p. (℃) | Formula | Elemental Analysis(Found) C | H | N |
|---|---|---|---|---|---|---|---|
| 5 | $-CH_2-$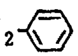 | $NHCH_3$ | 189-190 | $C_{25}H_{22}Cl_2N_2O_3$ | 63.97 (64.01 | 4.72 4.53 | 5.97 5.90) |
| 6 | $-CH_2-$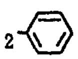 | $N(CH_3)_2$ | 183-184 | $C_{26}H_{24}Cl_2N_2O_3 \cdot ¼H_2O$ | 64.00 (63.97 | 5.06 5.03 | 5.74 5.81) |
| 7 | $-CH_2CH_2-$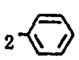 | $NHCH_3$ | 193-195 | $C_{26}H_{24}Cl_2N_2O_3$ | 64.60 (64.42 | 5.00 4.99 | 5.80 5.73) |
| 8 | $CH_2CH(CH_3)_2$ | $N(CH_3)_2$ | 134-135 | $C_{23}H_{26}Cl_2N_2O_3$ | 61.47 (61.33 | 5.83 5.76 | 6.23 6.18) |

**Table** 61

Example 87

| Compd. No. | $R^1$ | R | m. p. (℃) | Formula | Elemental Analysis(Found) C | H | N |
|---|---|---|---|---|---|---|---|
| 9 | $CH_2CH(CH_3)_2$ | H<br>$N(CH_2)_3CH_3$ | 135-136 | $C_{25}H_{30}Cl_2N_2O_3$ | 62.89<br>(63.15 | 6.33<br>6.38 | 5.87<br>5.87) |
| 10 | $CH_2CH(CH_3)_2$ | H<br>$NCH_2$- 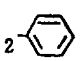 | 168-169 | $C_{28}H_{28}Cl_2N_2O_3$ | 65.76<br>(65.89 | 5.52<br>5.67 | 5.48<br>5.57) |
| 11 | $CH_2C(CH_3)_3$ | $NHN(CH_3)_2$ | 232-235 | $C_{24}H_{29}Cl_2N_3O_3$ | 60.25<br>(60.14 | 6.11<br>6.20 | 8.78<br>8.51) |

Table 62

Example 87

| Compd. No. | $R^1$ | $R^2$ | m. p. (℃) | Formula | Elemental Analysis(Found) C | H | N |
|---|---|---|---|---|---|---|---|
| 12 | $CH_2CH(CH_3)_2$ | 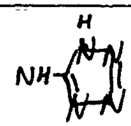 | 270-271 | $C_{22}H_{22}Cl_2N_6O_3$ $\cdot \frac{1}{4}H_2O$ | 53.50 (53.63 | 4.59 4.43 | 17.02 16.88) |
| 13 | $CH(CH_3)_2$ | $\overset{H}{N}(CH_2)_2\overset{O}{\overset{\|}{C}}NH_2$ | 247-248 | $C_{22}H_{23}Cl_2N_3O_4$ | 56.91 (56.69 | 4.99 4.90 | 9.05 8.96) |
| 14 | $CH_2CH(CH_3)_2$ | $NH_2$ | 271-272 | $C_{21}H_{22}Cl_2N_2O_3$ | 59.87 (59.91 | 5.26 5.33 | 6.65 6.91) |

Table 63

Example 87

| Compd. No. | $R^1$ | $R^2$ | m. p. (℃) | Formula | Elemental Analysis(Found) C | H | N |
|---|---|---|---|---|---|---|---|
| 15 | $CH_2C(CH_3)_3$ | 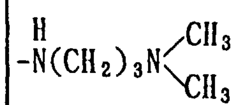 | 118-121 | $C_{27}H_{35}Cl_2N_3O_3$ $(COOH)_2 \cdot 1/2H_2O$ | 56.22 (56.37 | 6.18 6.35 | 6.78 6.56) |
| 16 | $CH_2C(CH_3)_3$ | 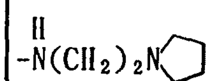 | 179-180 | $C_{28}H_{35}Cl_2N_3O_3$ $\cdot 1/4H_2O$ | 62.62 (62.66 | 6.66 6.54 | 7.83 7.85) |

Reference Example 8

Ethyl ester of trans-7-chloro-5-(2-chlorophenyl)-1-(2,2,2-trifluoroethyl)-1,2,3,5-tetrahydro-2-oxo-4,1-benzoxazepine-3-acetic acid (1) 5-chloxo-α-(2-chlorophenyl)-2-(trifluoroacetylamino)benzyl alcohol

To a solution of 1.0 g of 2-amino-5-chloro-α-(2-chlorophenyl)benzyl alcohol in 12 ml of dichloromethane, a solution of 0.8 g of anhydrous trifuoroacetic acid in 2 ml of dichloromethane was added. After reaction, an aqueous solution of sodium hydrogen carbonate was added, and the organic layer was dried, after which the solvent was removed and the residue was recrystallized from hexane to yield 1.3 g of a crystal.

(2) 5-chloro-α-(2-chlorophenyl)-2-(2,2,2-trifluoroethylamino)benzyl alcohol

To a suspension of 0.25 g of lithium aluminum hydride in 20 ml of absolute ethyl ether, a solution of 1.2 g of 5-chloro-α-(2-chlorophenyl)-2-(trifluoroacetylamino)benzyl alcohol in 5 ml of tetrahydrofuran was added dropwise. After mixture stirring at room temperature for 1 hour, water was added, and the organic layer was dried, after which the solvent was removed and the residue was subjected to silica gel column chromatography to yield 1.0 g of an oily compound.

(3) Ethyl ester of 3-[N-[4-chloro-2-(α-hydroxy-2-chlorophenylmethyl)phenyl]-N-(2,2,2-trifluoroethyl]carbamoyl]acrylic acid

5-chloro-α-(2-chlorophenyl)-2-(2,2,2-trifluoroethylamino)benzyl alcohol and monomethyl ester of chlorofumaric acid were subjected to the same procedure as in Reference Example 1 to yield an oily compound.

(4) Ethyl ester of trans-7-chloro-5-(2-chlorophenyl)-1-(2,2,2-trifluoroethyl)-1,2,3,5-tetrahydro-2-oxo-4,1-benzoxazepine-3-acetic acid

Ethyl ester of 3-[N-[4-chloro-2-(α-hydroxy-2-chlorophenylmethyl)phenyl]-N-(2,2,2-trifluoroethyl)carbamoyl] acrylic acid was subjected to the same procedure as in Reference Example 2 to yield a crystal.
Melting point: 163-164°C.

| Elemental analysis (for $C_{21}H_{18}Cl_2F_3NO_4$) | | | |
|---|---|---|---|
| Calcd. | C 52.96; | H 3.81; | N 2.94 |
| Found | C 52.93; | H 3.88; | N 2.91 |

Reference Example 9

By the same procedure as in Reference Examples 2 and 8, the compounds listed in Table 64 were synthesized.

**Table** 64

Reference Example 9

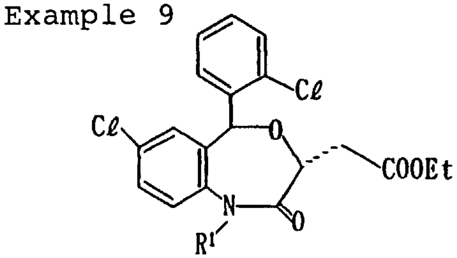

| Compd. No. | $R^1$ | m. p. (℃) | Formula | Elemental Analysis(Found) C | H | N |
|---|---|---|---|---|---|---|
| 1 | $CH_2CF_2CF_3$ | oil | $^1$H-NMR($CDCl_3$)δ:1.25(3H, t), 2.8, 3.1(2H, 各dd), 4.0-4.3(3H, m), 4.48(1H, dd), 5.1-5.35(1H, m), 6.18(1H, s), 6.56(1H, d), 7.2-7.8 (6H, m) | | | |
| 2 | $CH_2$ 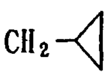 | 106-107 | $C_{23}H_{23}Cl_2NO_4$ | 61.62<br>(61.66 | 5.17<br>5.31 | 3.12<br>3.16) |

Example 88

By the same procedure as in Reference Examples 2 and 8, the compounds listed in Table 65 were synthesized.

**Table** 65

Example 88

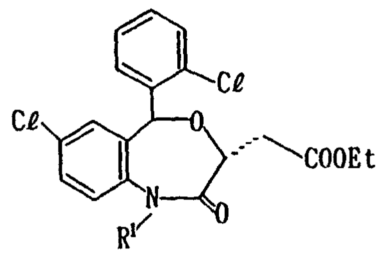

| Compd. No. | $R^1$ | m.p. (℃) | Formula Elemental Analysis(Found) C H N |
|---|---|---|---|
| 1 | $CH_2CH_2C(CH_3)_3$ | oil | $^1$H-NMR($CDCl_3$)δ:0.94(9H, s), 1.25(3H, t), 1.3-1.8(2H, m), 2.8, 3.08(2H, 各dd), 3.54(1H, dt), 4.13(2H, dq), 4.35-4.6(2H, m), 6.0(1H, s), 6.52(1H, d), 7.1-7.8(6H, m) |

Reference Example 10

The compounds obtained in Reference Examples 8 and 9 were subjected to the same procedure as in Reference Example 4 to yield the compounds listed in Table 66.

Table 66

Reference Example 10

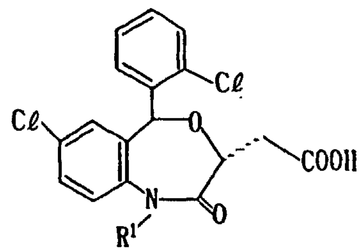

| Compd. No. | $R^1$ | m.p. (℃) | Formula | Elemental Analysis(Found) C | H | N |
|---|---|---|---|---|---|---|
| 1 | $CH_2CF_2CF_3$ | 186-187 | $C_{20}H_{14}Cl_2F_5NO_4$ | 48.21<br>(47.91 | 2.83<br>2.82 | 2.81<br>2.95) |
| 2 | $CH_2$ 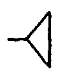 | 230-232 | $C_{21}H_{19}Cl_2NO_4$ | 60.01<br>(59.74 | 4.56<br>4.55 | 3.33<br>3.29) |
| 3 | $CH_2CF_3$ | 213-215 | $C_{19}H_{14}Cl_2F_3NO_4$ | 50.91<br>(50.97 | 3.15<br>3.26 | 3.12<br>3.05) |

Example 89

The compounds obtained in Example 88 were subjected to the same procedure as in Reference Example 4 to yield the compounds listed in Table 67.

Table 67
Example 89

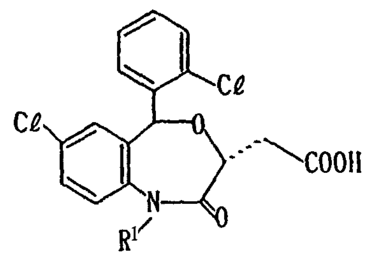

| Compd. No. | $R^1$ | m. p. (℃) | Formula | Elemental Analysis(Found) C | H | N |
|---|---|---|---|---|---|---|
| 1 | $CH_2CH_2C(CH_3)_3$ | 176-177 | $C_{23}H_{25}Cl_2NO_4$ | 61.34 | 5.60 | 3.11 |
| | | | | (61.45 | 5.68 | 3.25) |

Also, physicochemical properties of intermidiates of compounds obtained in Reference Examples 8 and 9

and Example 88 are shown in Tables 68 and 69.

Table 68

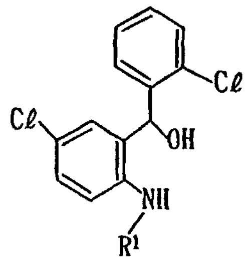

| $R^1$ | m. p. (℃) | Formula | Elemental Analysis(Found) C H N |
|---|---|---|---|
| $CH_2CF_2CF_3$ | oil | $C_{16}H_{12}Cl_2F_5NO$ | $^1$H-NMR($CDCl_3$) δ :3. 82(2H, dt), 6. 17(1H, s), 6. 69(1H, d), 6. 84(1H, d), 7. 1-7. 5(5H, m) |
| $CH_2$– 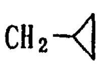 | oil | $C_{17}H_{17}Cl_2NO$ | $^1$H-NMR($CDCl_3$) δ :0. 1-0. 6(4H, m), 1. 0-1. 2 (1H, m), 2. 92(2H, d), 6. 16(1H, s), 6. 57(1H, d), 6. 91(1H, d), 7. 1-7. 6(5H, m) |
| $CH_2CH_2C(CH_3)_3$ | oil | $C_{19}H_{23}Cl_2NO$ | $^1$H-NMR($CDCl_3$) δ :0. 94(9H, s), 1. 4-1. 6(2H, m), 3. 1(2H, m), 6. 10(1H, s), 6. 62(1H, d), 6. 83(1H, d), 7. 1-7. 5(5H, m) |
| $CH_2CF_3$ | oil | $C_{15}H_{12}Cl_2F_3NO$ | $^1$H-NMR($CDCl_3$) δ :2. 68(2H, d), 5. 23(1H, m), 6. 15(1H, d), 6. 7(1H, d), 6. 83(1H, d), 7. 1-7. 6 (5H, m) |

Table 69

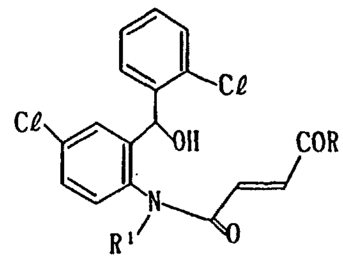

| $R^4$ | R | m.p. (℃) | Formula | Elemental Analysis(Found) C H N |
|---|---|---|---|---|
| $CH_2CF_2CF_3$ | $-OC_2H_5$ | oil | $C_{22}H_{18}Cl_2F_5NO_4$ | $^1$H-NMR($CDCl_3$) δ :1. 15-1. 4(3H, m), 3. 2-5. 45 (4H, m), 6. 0-7. 6(10H, m) |
| $CH_2$- 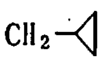 | $-OC_2H_5$ | oil | $C_{23}H_{23}Cl_2NO_4$ | $^1$H-NMR($CDCl_3$) δ :0. 1-0. 55(4H, m), 0. 9-1. 4 (4H, m), 2. 4-4. 4(4H, m), 6. 0-7. 7(10H, m) |
| $CH_2CH_2CH(CH_3)_3$ | $-OC_2H_5$ | oil | $C_{25}H_{30}Cl_2NO_4$ | $^1$H-NMR($CDCl_3$) δ :0. 76, 0. 91(9H, both s), 1. 1-1. 8(2H, m), 2. 6-4. 5(4H, m), 5. 95-7. 7 (10H, m) |
| $CH_2CF_3$ | $-OC_2H_5$ | oil | $C_{21}H_{18}Cl_2F_3NO_4$ | $^1$H-NMR($CDCl_3$) δ :1. 17-1. 4(3H, m), 3. 2-5. 3 (4H, m), 6. 0-7. 6(10H, m) |

Example 90

Ethyl ester of trans-7-chloro-5-(2-chlorophenyl)-1-neopentyl-1,2,3,5-tetrahydro-2-oxo-4,1-benzoxazepine-3-propionic acid.

(1) Trans-7-chloro-5-(2-chlorophenyl)-3-(2-cyanoethyl)-1-neopentyl-1,2,3,5-tetrahydro-2-oxo-4,1-benzoxazepine

0.3 g of 7-chloro-3-(2-chloroethyl)-5-(2-chlorophenyl)-1-neopentyl-1,2,3,5-tetrahydro-2-oxo-4,1-benzoxazepine and 0.1 g of sodium cyanide were suspended in 6 ml of dimethylsulfoxide, followed by stirring at 100°C for 1 hour. Water was added and the suspension was extracted with ethyl acetate, after which the solvent was removal and the residue was subjected to silica gel column chromatography to yield 0.25 g of a crystal.
Melting point: 194-195°C.

| Elemental analysis (for $C_{23}H_{24}Cl_2N_2O_2$) | | | |
|---|---|---|---|
| Calcd. | C 64.04; | H 5.61; | N 6.49 |
| Found | C 63.96; | H 5.56; | N 6.66 |

(2) Ethyl ester of trans-7-chloro-5-(2-chlorophenyl)-1-neopentyl-1,2,3,5-tetrahydro-2-oxo-4,1-benzoxazepine-3-propionic acid

To 0.2 g of traps-7-chloro-5-(2-chlorophenyl)-3-(2-cyanoethyl)-1-neopentyl-1,2,3,5-tetrahydro-2-oxo-4,1-benzoxazepine, 6 ml of an ethanol solution of 6 N hydrogen chloride was added, followed by refluxing with heating for 6 hours. After solvent removal, ethyl acetate and water were added, the organic layer was dried, the solvent was removed, and the residue was subjected to silica gel column chromatography to yield 0.18 g of a crystal.
Melting point: 130-131°C.

| Elemental analysis (for $C_{25}H_{29}Cl_2NO_4$) | | | |
|---|---|---|---|
| Calcd. | C 62.76; | H 6.11; | N 2.93 |
| Found | C 62.78; | H 6.12; | N 2.68 |

Example 91

Trans-7-chloro-5-(2-chlorophenyl)-1-neopentyl-1,2,3,5-tetrahydro-2-oxo-4,1-benzoxazepine-3-propionic acid

90 mg of ethyl ester of trans-7-chloro-5-(2-chlorophenyl)-1-neopentyl-1,2,3,5-tetrahydro-2-oxo-4,1-benzoxazepine-3-propionic acid as obtained in Example 90 was subjected to the same procedure as in Reference Example 4 to yield 80 mg of a crystal.
Melting point: 225-227°C.

| Elemental analysis (for $C_{23}H_{25}Cl_2NO_4$) | | | |
|---|---|---|---|
| Calcd. | C 61.34; | H 5.60; | N 3.11 |
| Found | C 61.34; | H 5.65; | N 2.89 |

Example 92

Trans-7-chloro-5-(2-chlorophenyl)-1-isobutyl-1,2,3,5-tetrahydro-2-oxo-4,1-benzoxazepine-3-ethanol

Trans-7-chloro-5-(2-chlorophenyl)-1-isobutyl-1,2,3,5-tetrahydro-2-oxo-4,1-benzoxazepine-3-acetic acid as obtained in Example 2 was subjected to the same procedure as in Example 63 to yield 1.5 g of a crystal.

Example 93

Ethyl ester of 3-[trans-7-chloro-5-(2-chlorophenyl)-1-isobutyl-2-oxo-1,2,3,5-tetrahydro-4,1-benzoxazepine-3-ylethyl] thioglycolic acid

(1) Trans-7-chloro-3-(2-chloroethyl)-5-(2-chlorophenyl)-1-isobutyl-1,2,3,5-tetrahydro-2-oxo-4,1-benzoxazepine

1.0 g of trans-7-chloro-5-(2-chlorophenyl)-1-isobutyl-1,2,3,5-tetrahydro-2-oxo-4,1-benzoxazepine-3-ethanol as obtained in Example 98 was dissolved in 25 ml of toluene, and 0.1 ml of pyridine and 0.4 ml of thionyl chloride were added, followed by stirring for 30 minutes with heating at 100°C. After solvent removal under reduced pressure, the residue was dissolved in ethyl acetate and washed with a saturated aqueous solution of sodium hydrogen carbonate and dried, after which the solvent was removed and the residue was subjected to silica gel column chormatography to yield 0.8 g of a crystal.
Melting point: 138-140°C.

| Elemental analysis (for $C_{21}H_{22}Cl_3NO_2$) | | | |
|---|---|---|---|
| Calcd. | C 59.10; | H 5.20; | N 3.28 |
| Found | C 59.17; | H 5.22; | N 2.25 |

(2) Ethyl ester of 3-[trans-7-chloro-5-(2-chlorophenyl)-1-isobutyl-2-oxo-1,2,3,5-tetrahydro-4,1-benzoxazepine-3-ylethyl]thioglycolic acid

0.15 g of trans-7-chloro-3-(2-chloroethyl)-5-(2-chlorophenyl)-1-isobutyl-1,2,3,5-tetrahydro-2-oxo-4,1-benzoxazepine, 0.08 g of ethyl ester of thioglycolic acid and 0.1 g of cesium fluoride were added to 5 ml of acetonitrile, followed by refluxing with heating for 40 minutes. After ice water was added and the mixture was extracted with ethyl acetate, the organic layer was dried, after which the solvent was removed and the residue was subjected to silica gel column chromatography to yield 0.17 g of an oily compounds.
$^1$H-NMR ($CDCl_3$) δ: 0.93, 1.03 (6H, each, d), 1.26 (3H, t), 1.8-2.4 (3H, m), 2.8 (2H, m), 3.19 (2H, s), 3.42 (1H, dd), 4.0-4.4 (4H, m), 6.12 (1H, s), 6.51 (1H, d), 7.2-7.8 (6H, m)

Example 94

3-[trans-7-chloro-5-(2-chlorophenyl)-1-isobutyl-2-oxo-1,2,3,5-tetrahydro-4,1-benzoxazepine-3-. ylethyl]thioglycolic acid

0.17 g of ethyl ester of 3-[trans-7-chloro-5-(2-chlorophenyl)-1-isobutyl-2-oxo-1,2,3,5-tetrahydro-4,1-benzoxazepine-3-ylethyl]thioglycolic acid as obtained in Example 93 was subjected to the same procedure as in Example 49 to yield 0.18 g of a crystal.
Melting point: 194-195°C.

| Elemental analysis (for $C_{23}H_{25}Cl_2NO_4S$) | | | |
|---|---|---|---|
| Calcd. | C 57.26; | H 5.22; | N 2.90 |
| Found | C 57.27; | H 5.20; | N 2.96 |

Example 95

3-[trans-7-chloro-5-(2-chlorophenyl)-1-isobutyl-2-oxo-1.2.3.5-tetrahydro-4,1-benzoxazepine-3-ylethyl]sulfonylacetic acid

0.18 g of ethyl ester of 3-[trans-7-chloro-5-(2-chlorophenyl)-1-isobutyl-2-oxo-1,2,3,5-tetrahydro-4,1-benzoxazepine-3-ylethyl]thioglycolic acid as obtained in Example 93 was dissolved in 8 ml of dichloromethane, and 0.25 g of m-chloroperbenzoic acid was added, followed by stirring for 1 hour. To the reaction mixture was added an aqueous solution of sodium hydrogen carbonate, followed by washing and the organic layer was dried and the solvent was removed, after which the residue was subjected to silica gel column chromatography to yield 0.18 g of an oily compound, which was then subjected to the same procedure of ethyl ester hydrolysis as in Example 49 to yield 0.13 g of a crystal.
Melting point: 171-172°C.

| Elemental analysis (for $C_{23}H_{25}Cl_2NO_6S$) | | | |
|---|---|---|---|
| Calcd. | C 53.70; | H 4.90; | N 2.72 |
| Found | C 53.62; | H 4.95; | N 2.68 |

Example 96

Trans-7-chloro-5-(2-chlorophenyl)-1-neopentyl-3-[3-(tetrazol-5-yl)phenylaminocarbonylmethyl]-2-oxo-1,2,3,5-tetrahydro-4,1-benzoxazepine

(1) Trans-7-chloro-5-(2-chlorophenyl)-3-(3-cyanophenylaminocarbonylmethyl)-1-neopentyl-2-oxo-1,2,3,5-tetrahydro-4,1-benzoxazepine

To a solution of 0.5 g of trans-7-chloro-5-(2-chlorophenyl)-1-isobutyl-2-oxo-1,2,3,5-tetrahydro-4,1-benzoxazepine-3-acetic acid as obtained in Example 2 and 0.15 g of m-cyanoaniline in 10 ml of dichloromethane, 0.35 g of 1-ethyl-3-(dimethylaminopropyl)carbodiimide hydrochloride and 20 mg of dimethylaminopyridine were added, followed by stirring at room temperature for 4 hours. The solution was concentrated, and the residue was dissolved in ethyl acetate, followed by washing with water, after which the solvent was removed and the residue was subjected to silica gel column chromatography to yield 0.48 g of a crystal.
Melting point: 213-214°C.

| Elemental analysis (for $C_{29}H_{27}Cl_2N_3O_3$) | | | |
|---|---|---|---|
| Calcd. | C 64.93; | H 5.07; | N 7.83 |
| Found | C 64.68; | H 4.96; | N 7.90 |

(2) Trans-7-chloro-5-(2-chlorophenyl)-1-neopentyl-3-[3-(tetrazol-5-yl)phenylaminocarbonylmethyl]-2-oxo-1,2,3,5-tetrahydro-4,1-benzoxazepine

A solution of 0.2 g of trans-7-chloro-5-(2-chlorophenyl)-3-(3-cyanophenylaminocarbonylmethyl)-1-neopentyl-2-oxo-1,2,3,5-tetrahydro-4,1-benzoxazepine and 80 mg of sodium azide in 5 ml of dimethylformamide was stirred at 90°C for 60 hours. After water was added, the mixture was washed with water, after which the solvent was removed and the residue was subjected to silica gel column chromatography to yield 0.11 g of a crystal.
Melting point: 218-220°C.

| Elemental analysis (for $C_{29}H_{28}Cl_2N_6O_3$) | | | |
|---|---|---|---|
| Calcd. | C 59.64; | H 4.92; | N 14.39 |
| Found | C 59.44; | H 4.87; | N 14.30 |

Example 97

Trans-7-chloro-5-(2-chlorophenyl)-1-neopentyl-3-[4-(tetrazol-5-yl)phenylaminocarbonylmethyl)-2-oxo-1,2,3,5-tetrahydro-4,1-benzoxazepine. The title compound was synthesized by the same procedure as in

Example 96.

(1) Trans-7-chloro-5-(2-chlorophenyl)-3-(4-cyanophenylaminocarbonylmethyl)-1-neopentyl-2-oxo-1,2,3,5-tetrahydro-4,1-benzoxazepine

Melting point: 257-259°C.

| Elemental analysis (for $C_{29}H_{27}Cl_2N_3O_3.1/4H_2O$) | | | |
|---|---|---|---|
| Calcd. | C 64.39; | H 5.12; | N 7.77 |
| Found | C 64.33; | H 5.13; | N 7.64 |

(2) Trans-7-chloro-5-(2-chlorophenyl)-1-neopentyl-3-[4-(tetrazol-5-yl)phenylaminocarbonylmethyl]-2-oxo-1,2,3,5-tetrahydro-4,1-benzoxazepine

Melting point: 206-210°C

| Elemental analysis (for $C_{29}H_{28}Cl_2N_6O_3 \cdot 1/4H_2O$) | | | |
|---|---|---|---|
| Calcd. | C 59.64; | H 4.92; | N 14.39 |
| Found | C 59.71; | H 4.96; | N 14.34 |

Example 98

Trans-7-chloro-5-(2-chlorophenyl)-1-isopropyl-3-[(tetrazol-5-yl)methylaminocarbonylmethyl]-2-oxo-1,2,3,5-tetrahydro-4,1-benzoxazepine

(1) Trans-7-chloro-5-(2-chlorophenyl)-3-(cyanomethylaminocarbonylmethyl)-1-isopropyl-2-oxo-1,2,3,5-tetrahydro-4,1-benzoxazepine

0.3 g of trans-7-chloro-5-(2-chlorophenyl)-1-isopropyl-2-oxo-1,2,3,5-tetrahydro-4,1-benzoxazepine-3-acetic acid as obtained in Reference Example 5 and 0.1 g of aminoacetonitrile sulfuric acid salt were dissolved in 5 ml of dimethylformaldehyde, and 0.18 g of diethyl phosphorocyanidate and 0.17 ml of triethylamine were added, followed by stirring at room temperature for 1 hour. After water was added, the mixture was extracted with ethyl acetate, washed with water and then dried. After solvent removal, the residue was subjected to silica gel column chromatography to yield 0.31 g of a crystal.
Melting point: 216-217°C.

| Elemental analysis ( for $C_{22}H_{21}Cl_2N_3O_3$) | | | |
|---|---|---|---|
| Calcd. | C 59.20; | H 4.74; | N 9.41 |
| Found | C 58.92; | H 4.68; | N 9.12 |

(2) Trans-7-chloro-5-(2-chlorophenyl)-1-isopropyl-3-[(tetrazol-5-yl)methylaminocarbonylmethyl]-2-oxo-1,2,3,5-tetrahydro-4,1-benzoxazepine

Trans-7-chloro-5-(2-chlorophenyl)-3-(cyanomethylaminocarbonylmethyl)-1-isopropyl-2-oxo-1,2,3,5-tetrahydro-4,1-benzoxazepine and sodium azide were subjected to the same procedure as in Example 96 to yield a crystal. Melting point: 253-254°C.

| Elemental analysis (for $C_{22}H_{22}Cl_2N_6O_3$) | | | |
|---|---|---|---|
| Calcd. | C 54.00; | H 4.53; | N 17.17 |
| Found | C 53.85; | H 4.68; | N 17.02 |

Example 99

Trans-7-chloro-5-(2-chlorophenyl)-1-isobutyl-3-[(tetrazol-5-yl)methylaminocarbonylmethyl]-2-oxo-1,2,3,5-tetrahydro-4,1-benzoxazepine

Trans-7-chloro-5-(2-chlorophenyl)-1-isobutyl-2-oxo-1,2,3,5-tetrahydro-4,1-benzoxazepine-3-acetic acid as obtained in Example 2 was subjected to the same procedure as in Examples 96 and 98 to yield a crystalline compound.

(1) Trans-7-chloro-5-(2-chlorophenyl)-3-(cyanomethylaminocarbonylmethyl)-1-isobutyl-2-oxo-1,2,3,5-tetrahydro-4,1-benzoxazepine

Melting point: 168-169°C.

| Elemental analysis (for $C_{23}H_{23}Cl_2N_3O_3$) | | | |
|---|---|---|---|
| Calcd. | C 60.01; | H 5.04; | N 9.13 |
| Found | C 60.11; | H 5.28; | N 9.15 |

(2) Trans-7-chloro-5-(2-chlorophenyl)-1-isobutyl-3-[(tetrazol-5-yl)methylaminocarbonylmethyl]-2-oxo-1,2,3,5-tetrahydro-4,1-benzoxazepine

Melting point: 234-235°C.

| Elemental analysis (for $C_{23}H_{24}Cl_2N_6O_3$) | | | |
|---|---|---|---|
| Calcd. | C 54.88; | H 4.81; | N 16.69 |
| Found | C 55.07; | H 4.84; | N 16.92 |

Example 100

Trans-7-chloro-5-(2-chlorophenyl)-1-isobutyl-3-[(tetrazol-5-yl)methyl]-2-oxo-1,2,3,5-tetrahydro-4,1-benzoxazepine

(1) Trans-7-chloro-5-(2-chlorophenyl)-3-cyanomethyl-1-isobutyl-2-oxo-1,2,3,5-tetrahydro-4,1-benzoxazepine

0.5 g of Trans-7-chloro-5-(2-chlorophenyl)-1-isobutyl-2-oxo-1,2,3,5-tetrahydro-4,1-benzoxazepine-3-acetamide as obtained in Example 87, 0.38 g of carbonyldiimidazole and 1.14 g of allyl bromide were dissolved in 10 ml of acetonitrile, followed by refluxing with heating for 4 hours. The solution was concentrated and ethyl acetate was added, followed by washing with water, after which the solvent was removed and the residue was subjected to silica gel column chromatography to yield 0.44 g of a crystal.
Melting point: 158-159°C.

| Elemental analysis (for $C_{21}H_{20}Cl_2N_2O_2$) | | | |
|---|---|---|---|
| Calcd. | C 62.54; | H 5.00; | N 6.95 |
| Found | C 62.68; | H 5.13; | N 7.22 |

(2) Trans-7-chloro-5-(2-chlorophenyl)-1-isobutyl-3-[tetrazol-5-yl)methyl)-2-oxo-1,2,3,5-tetrahydro-4,1-benzoxazepine

0.2 g of trans-7-chloro-5-(2-chlorophenyl)-3-cyanomethyl-1-isobutyl-2-oxo-1,2,3,5-tetrahydro-4,1-benzoxazepine was subjected to the same procedure as in Example 102 and to yield 90 mg of a crystal.
Melting point: 202-203°C.

| Elemental analysis (for $C_{21}H_{21}Cl_2N_5O_2$) | | | |
|---|---|---|---|
| Calcd. | C 56.51; | H 4.74; | N 15.69 |
| Found | C 56.29; | H 4.70; | N 15.67 |

Example 101

Trans-7-chloro-5-(2-chlorophenyl)-1-isobutyl-3-[2-(tetrazol-5-yl)ethyl]-2-oxo-1,2,3,5-tetrahydro-4,1-benzoxazepine

(1) Trans-7-chloro-5-(2-chlorophenyl)-1-isobutyl-3-(2-cyanoethyl)-1-isobutyl-2-oxo-1,2,3,5-tetrahydro-4,1-benzoxazepine

0.5 g of trans-7-chloro-3-(2-chloroethyl)-5-(2-chlorophenyl)-1-isobutyl-2-oxo-1,2,3,5-tetrahydro-4,1-benzoxazepine as obtained in Example 93 and 0.4 g of sodium cyanide were added to 8 ml of dimethylsulfoxide, followed by stirring at 100°C for 40 minutes. After water was added, the reaction mixture was extracted with ethyl acetate, and the organic layer was washed with water and dried, after which the solvent was removed and the residue was subjected to silica gel column chromatography to yield 0.45 g of an oily substance.
$^1$H-NMR ($CDCl_3$) δ: 0.93, 1.03 (6H, each, d), 1.85-2.4 (3H, m), 2.59 (2H, t), 3.43 (1H, dd), 4.05 (1H, dd), 4.33 (1H, dd),

6.14 (1H, s), 6.53 (1H, d), 7.3-7.8 (6H, m)

(2) Trans-7-chloro-5-(2-chlorophenyl)-1-isobutyl-3-[2-(tetrazol-5-yl)ethyl]-2-oxo-1,2,3,5-tetrahydro-4,1-benzoxazepine

Trans-7-chloro-5-(2-chlorophenyl)-3-(2-cyanoethyl)-1-isobutyl-2-oxo-1,2,3,5-tetrahydro-4,1-benzoxazepine was subjected to the same procedure as in Example 96 to yield 0.22 g of a crystal.
Melting point: 125-127°C

| Elemental analysis (for $C_{22}H_{23}Cl_2N_5O_2$) | | | |
|---|---|---|---|
| Calcd. | C 57.40; | H 5.04; | N 15.21 |
| Found | C 57.15; | H 5.26; | N 14.97 |

Example 102

Trans-7-chloro-5-(2-chlorophenyl)-3-[2-(4,5-dihydro-5-oxo-1,2,4-oxadiazol-3-yl)ethyl]-1-isobutyl-2-oxo-1,2,3,5-tetrahydro-4,1-benzoxazepine

0.25 g of trans-7-chloro-5-(2-chlorophenyl)-3-(2-cyanoethyl)-1-isobutyl-1,2,3,5-tetrahydro-2-oxo-4,1-benzoxazepine as obtained in Example 101, 120 mg of hydroxylamine hydrochloride and 0.23 g of sodium carbonate were added to 5 ml of ethanol, followed by refluxing with heating for 18 hours. After solvent removal, ethyl acetate was added, and the mixture was washed with water an dried, after which the solvent was removed and the residue was recrystallized from ethyl acetate.
Melting point: 212-214°C.

| Elemental analysis (for $C_{22}H_{25}Cl_2N_3O_3$) | | | |
|---|---|---|---|
| Calcd. | C 58.67; | H 5.60; | N 9.33 |
| Found | C 58.84; | H 5.68; | N 9.24 |

0.1 g of this amidoxime, 0.1 g of carbonyldiimidazole and 0.05 ml of triethylamine were added to 5 ml of ethyl acetate, followed by refluxing with heating for 24 hours. The solution was concentrated and the residue was dissolved in ethyl acetate, washed with water and dried, after which the solvent was removed and the residue was recrystallized from ethyl acetate to yield 85 mg of a crystal.
Melting point: 241-242°C.

| Elemental analysis (for $C_{22}H_{23}Cl_2N_3O_4$) | | | |
|---|---|---|---|
| Calcd. | C 57.99; | H 4.87; | N 8.82 |
| Found | C 57.79; | H 5.07; | N 9.00 |

Example 103

Tert-butyl ester of N-[(3S,5R)-7-chloro-5-(2-chlorophenyl)-1-isobutyl-2-oxo-1,2,3,5-tetrahydro-4,1-benzoxazepine-3-acetyl]-L-alanine; and tert-butyl ester of N-[(3R,5S)-7-chloro-5-(2-chlorophenyl)-1-isobutyl-2-oxo-1,2,3,5-tetrahydro-4,1-benzoxazepine-3-acetyl]-L-alanine

In 20 ml of N,N-dimethylformamide were dissolved 0.5 g of trans-7-chloro-5-(2-chlorophenyl)-1-isobutyl-2-oxo-1,2,3,5-tetrahydro-4,1-benzoxazepine-3-acetic acid, as obtained in Example 2, and 0.26 g of L-alanine tert-butyl ester hydrochloride; under ice-cooling, to this solution were added 0.26 g of diethyl phosphorocyanidate and 0.41 ml of triethylamine. After stirring for 30 minutes at room temperature, extraction was conducted with the addition of 100 ml of water and 150 ml of ethyl acetate. The ethyl acetate layer was washed with 5% aqueous solutions of potassium hydrogen sulfate and sodium bicarbonate, and after drying over anhydrous magnesium sulfate, it was distilled off under reduced pressure. The residue was then purified by silica gel column chromatography (eluent, hexane : ethyl acetate = 2 : 1) to obtain, as the first fraction, 0.17 g of tert-butyl ester of N-[(3S,5R)-7-chloro-5-(2-chlorophenyl)-1-isobutyl-2-oxo-1,2,3,5-tetrahydro-4,1-benzoxazepine-3-acetyl]-L-alanine as plate crystals, m.p. 146 to 147°C.

| Elemental Analysis for $C_{28}H_{34}Cl_2N_2O_5$: | | | |
|---|---|---|---|
| Calcd. | C 61.20; | H 6.24; | N 5.10 |
| Found | C 61.20; | H 6.33; | N 5.15 |

Also, as the second fraction, 0.22 g of tert-butyl ester of N-[(3R,5S)-7-chloro-5-(2-chlorophenyl)-1-isobutyl-2-oxo-1,2,3,5-tetrahydro-4,1-benzoxazepine-3-acetyl]-L-alanine was obtained as an oily product.

IR$\nu_{max}^{neat}$ $cm^{-1}$: 3330 (NH); 1730, 1670 (C = O).

$^1$H-NMR (200 MHz, $CDCl_3$) δ: 0.93 (3H, d, J = 6.8 Hz), 1.03 (3H, d, J = 6.8 Hz), 1.34 (3H, t, J = 7.0 Hz), 1.46 (9H, s, $Bu^t$), 1.85-2.1 (1H, m), 2.71 (1H, dd, J = 14.6, 6.2 Hz), 2.89 (1H dd, J = 14.6, 7.2 Hz), 3.43 (1H, dd, J = 14.0, 5.4 Hz), 4.25-4.55 (3H, m), 6.13 (1H, s), 6.31 (1H, brd, NH), 6.51 (1H, d, J = 2.2 Hz), 7.2-7.85 (6H, m)

Example 104

N-[(3S,5R)-7-chloro-5-(2-chlorophenyl)-1-isobutyl-2-oxo-1,2,3,5-tetrahydro-4,1-benzoxazepine-3-acetyl]-L-alanine

In 10 ml of a 4N solution of hydrochloric acid in dioxane was dissolved 0.16 g of tert-butyl ester of N-[(3S, 5R)-7-chloro-5-(2-chlorophenyl)-1-isobutyl-2-oxo-1,2,3,5-tetrahydro-4,1-benzoxazepine-3-acetyl]-L-alanine, as obtained in Example 109, and the solution was stirred overnight at room temperature. After distillation under reduced pressure, it was crystallized with hexanediethylether to form 0.12 g of N-[(3S,5R)-7-chloro-5-(2-chlorophenyl)-1-isobutyl-2-oxo-1,2,3,5-tetrahydro-4,1-benzoxazepine-3-acetyl]-L-alanine as a powdery product.

| Elemental Analysis for $C_{24}H_{26}Cl_2N_2O_5$: | | | |
|---|---|---|---|
| Calcd. | C 58.43; | H 5.31; | N 5.68 |
| Found | C 58.43; | H 5.65; | N 5.54 |

$^1$H-NMR spectrum (200 MHz, $d_6$-DMSO)δ: 0.89 (3H, d, J = 6.8 Hz), 0.94 (3H, d, J = 6.6 Hz), 1.25 (3H, d, J = 7.2 Hz), 1.7-1.9 (1H, m), 2.4-2.55 (2H, m), 3.58 (1H, dd, J = 14.0, 5.0 Hz), 4.1-4.4 (3H, m), 5.99 (1H, s), 6.30 (1H, d, J = 2.4 Hz), 7.45-7.8 (4H, m), 8.33 (1H, d, J = 7.6 Hz), 12.53 (1H, br, NH)

Example 105

N-[(3R,5S)-7-chloro-5-(2-chlorophenyl)-1-isobutyl-2-oxo-1,2,3,5-tetrahydro-4,1-benzoxazepine-3-acetyl]-L-alanine

In 10 ml of a 4N solution of hydrochloric acid in dioxane was dissolved 0.2 g of tert-butyl ester of N-[(3R,5S)-7-chloro-5-(2-chlorophenyl)-1-isobutyl-2-oxo-1,2,3,5-tetrahydro-4,1-benzoxazepine-3-acetyl]-L-alanine, as obtained in Example 109, and the mixture was stirred for 4 hours at room temperature. After distillation under reduced pressure, it was crystallized from hexanediethylether to form 0.17 g of N-[(3R,5S)-7-chloro-5-(2-chlorophenyl)-1-isobutyl-2-oxo-1,2,3,5-tetrahydro-4,1-benzoxazepine-3-acetyl]-L-alanine as plate crystals, m.p. 120 to 124°C.

| Elemental Analysis for $C_{24}H_{26}Cl_2N_2O_5 \cdot 1/2H_2O$: | | | |
|---|---|---|---|
| Calcd. | C 57.37; | H 5.42; | N 5.57 |
| Found | C 57.37; | H 5.69; | N 5.48 |

Example 106 (Reference)

Methyl ester of (R)-O-[(3R,5S)-7-chloro-5-(2-chlorophenyl)-1-isobutyl-2-oxo-1,2,3,5-tetrahydro-4,1-benzoxazepine-3-acetyl]lactic acid; and methyl ester of (R)-O-[(3S,5R)-7-chloro-5-(2-chlorophenyl)-1-isobutyl-2-oxo-1,2,3,5-tetrahydro-4,1-benzoxazepine-3-acetyl]lactic acid.

In 50 ml of methylene chloride were dissolved 2.0 g of trans-7-chloro-5-(2-chlorophenyl)-1-isobutyl-2-oxo-1,2,3,5-tetrahydro-4,1-benzoxazepine-3-acetic acid, as obtained in Example 2, and 0.59 g of methyl ester of (R)-lactic acid; to this solution were added 1.09 g of 1-ethyl-3-(3-dimethylaminopropyl) carbodiimide hydrochloride and 0.2 g of 4-dimethylaminopyridine; the mixture was stirred for 1.5 hours at room temperature. The reaction mixture was washed with 1N aqueous solutions of hydrochloric acid and sodium bicarbonate, and after drying over anhydrous magnesium sulfate, the solvent was distilled off under reduced pressure. The residue was separated and purified by means of silica

gel column chromatography (eluent, hexane : diethyl ether = 3 : 1) to yield, as the first fraction, 0.46 g of methyl ester of (R)-O-[(3S,5R)-7-chloro-5-(2-chlorophenyl)-1-isobutyl-2-oxo-1,2,3,5-tetrahydro-4,1-benzoxazepine-3-acetyl]lactic acid as an oily product.

IR$\nu_{max}^{neat}$cm$^{-1}$: 1740, 1675 (C = O)

$^1$H-NMR (200 MHz, $CDCl_3$)δ: 0.93 (3H, d, J = 6.8 Hz), 1.02 (3H, d, J = 6.6 Hz), 1.49 (3H, d, J = 7.2 Hz), 1.85-2.1 (1H, m), 2.91 (1H, dd, J = 17.0, 5.4 Hz), 3.19 (1H, dd, J = 17.0, 8.0 Hz), 3.44 (1H, dd, J = 13.8, 5.4 Hz), 3.72 (3H, s), 4.31 (1H, dd, J = 13.8, 8.4 Hz), 4.44 (1H, dd, J = 8.0, 5.4 Hz), 5.07 (1H, q, J = 7.2 Hz), 6.14 (1H, s), 6.52 (1H, d, J = 2.2 Hz), 7.2-7.8 (6H, m)

As the second fraction, 0.63 g of methyl ester of (R)-O-[(3R,5S)-7-chloro-5-(2-chlorophenyl)-1-isobutyl-2-oxo-1,2,3,5-tetrahydro-4,1-benzoxazepine-3-acetyl]lactic acid was obtained as an oily product.

IR$\nu_{max}^{neat}$ cm$^{-1}$: 1740,1675 (C = O)

$^1$H-NMR (200 MHz, $CDCl_3$)δ: 0.93 (3H, d, J = 6.8 Hz), 1.03 (3H, d, J = 6.6 Hz), 1.48 (3H, d, J = 7.0 Hz), 1.85-2.1 (1H, m), 2.93 (1H, dd, J = 16.8, 7.2 Hz), 3.10 (1H, dd, J = 16.8, 6.2 Hz), 3.43 (1H, dd, J = 13.8, 5.4 Hz), 3.28 (3H, s), 4.34 (1H, dd, J = 13.8, 5.4 Hz), 4.45 (1H, t, J = 6.6 Hz), 5.12 (1H, q, J = 7.0 Hz), 6.16 (1H, s), 6.52 (1H, d, J = 2.2 Hz), 7.2-7.8 (6H, m)

Example 107

(3S,5R)-7-chloro-5-(2-chlorophenyl)-1-isobutyl-2-oxo-1,2,3,5-tetrahydro-4,1-benzoxazepine-3-acetic acid

In 20 ml of methanol was dissolved 0.45 g of methyl ester of (R)-O-[(3S,5R)-7-chloro-5-(2-chlorophenyl)-1-isobutyl-2-oxo-1,2,3,5-tetrahydro-4,1-benzoxazepine-3-acetyl]lactic acid, as obtained in Example 112, to which was added 10 ml of an aqueous solution containing 0.37 g of potassium carbonate; this mixture was stirred for 40 minutes at 60°C. The mixture was concentrated under reduced pressure, and the concentrate subjected to extraction with the addition of 50 ml of 1N hydrochloric acid and 100 ml of ethyl acetate. The ethyl acetate layer was washed with water, dried over anhydrous magnesium sulfate, and distilled off under reduced pressure. The residue was purified by means of silica gel column chromatography (eluent, hexane : ethyl acetate = 3 : 1; eluent, hexane : methylene chloride : ethanol = 5 : 5 : 1) to yield 53 mg of (3S, 5R)-7-chloro-5-(2-chlorophenyl)-1-isobutyl-2-oxo-1,2,3,5-tetrahydro-4,1-benzoxazepine-3-acetic acid as prism crystals, m.p. 183 to 186°C.

$[\alpha]_D^{20}$ + 223.7° (c = 0.47, methanol)

| Elemental Analysis for $C_{21}H_{21}Cl_2NO_4$: | | | |
|---|---|---|---|
| Calcd. | C 59.73; | H 5.01; | N 3.32 |
| Found | C 59.36; | H 5.09; | N 3.19 |

Example 108

(3R,5S)-7-chloro-5-(2-chlorophenyl)-1-isobutyl-2-oxo-1,2,3,5-tetrahydro-4,1-benzoxazepine-3-acetic acid

In 20 ml of methanol was dissolved 0.6 g of methyl ester of (R)-O-[(3R,5S)-7-chloro-5-(2-chlorophenyl)-1-isobutyl-2-oxo-1,2,3,5-tetrahydro-4,1-benzoxazepine-3-acetyl]lactic acid, as obtained in Example 112, to which was added 10 ml of an aqueous solution containing 0.49 g of potassium carbonate; this mixture was stirred for 40 minutes at 60°C. The mixture was concentrated under reduced pressure, and the concentrate subjected to extraction with the addition of 50 ml of 1N hydrochloric acid and 100 ml of ethyl acetate. The ethyl acetate layer was washed with water, dried over anhydrous magnesium sulfate, and distilled off under reduced pressure. The residue was purified by silica gel column chromatography (eluent, hexane : ethyl acetate = 3 : 1; eluent, hexane : methylene chloride : ethanol = 5 : 5 : 1) to yield 54 mg of (3R,5S)-7-chloro-5-(2-chlorophenyl)-1-isobutyl-2-oxo-1,2,3,5-tetrahydro-4,1-benzoxazepine-3-acetic acid as prism crystals, m.p. 182 to 185°C.

$[\alpha]_D^{20}$ - 215.3° (c = 0.43, methanol)

| Elemental Analysis for $C_{21}H_{21}Cl_2NO_4 \cdot 1/4H_2O$: | | | |
|---|---|---|---|
| Calcd. | C 59.10; | H 5.08; | N 3.28 |
| Found | C 59.05; | H 5.07; | N 3.53 |

Example 109 (Reference)

Methyl ester of(R)-O-[(3R,5S)-7-chloro-5-(2-chlorophenyl)-1-neopentyl-2-oxo-1,2,3,5-tetrahydro-4,1-benzoxazepine-3-acetyl]lactic acid; and methyl ester of (R)-O-[(3S,5R)-7-chloro-5-(2-chlorophenyl)-1-neopentyl-2-oxo-1,2,3,5-tetrahydro-4,1-benzoxazepine-3-acetyl]lacticacid

In 50 ml of methylene chloride were dissolved 2.0 g of trans-7-chloro-5-(2-chlorophenyl)-1-neopentyl-2-oxo-1,2,3,5-tetrahydro-4,1-benzoxazepine-3-acetic acid, as obtained in Example 2, and 0.57 g of methyl ester of (R)-lactic acid. To the solution were added 1.05 g of 1-ethyl-3-(3-dimethylaminopropyl) carbodiimide hydrochloride and 0.2 g of 4-dimethylaminopyridine; the mixture was stirred for 3 hours at room temperature. The reaction mixture was washed with 1N solutions of hydrochloric acid and sodium bicarbonate and dried over anhydrous magnesium sulfate; then the solvent was distilled off under reduced pressure. The residue was separated and purified by silica gel column chromatography (eluent, hexane : diethylether = 3 : 1) to yield, as the first fraction, 0.48 g of methyl ester of (R)-O-[(3S, 5R)-7-chloro-5-(2-chlorophenyl)-1-neopentyl-2-oxo-1,2,3,5-tetrahydro-4,1-benzoxazepine-3-acetyl]lactic acid as prism crystals, m.p. 155 to 157°C.

$[\alpha]_D^{20}$ + 222.6° (c = 1.0, methanol)

| Elemental Analysis for $C_{26}H_{29}Cl_2NO_6$: | | | |
|---|---|---|---|
| Calcd. | C 59.78; | H 5.60; | N 2.68 |
| Found | C 59.87; | H 5.75; | N 2.41 |

As the second fraction, 0.6 g of methyl ester of (R)-O-[(3R,5S)-7-chloro-5-(2-chlorophenyl)-1-neopentyl-2-oxo-1,2,3,5-tetrahydro-4,1-benzoxazepine-3-acetyl]lactic acid was recovered as an oily product.

IR$\nu_{max}^{neat}$cm$^{-1}$: 1745,1675 (C = O)

$^1$H-NMR (200 MHz, $CDCl_3$)δ: 0.94 (9H, s, Bu$^t$), 1.47 (3H, d, J = 7.0 Hz), 2.92 (1H, dd, J = 16.8, 7.2 Hz), 3.08 (1H, dd, J = 16.8, 6.0 Hz), 3.39 (1H, d, J = 14.0 Hz), 3.72 (3H, s), 4.45 (1H, dd, J = 7.2, 6.0 Hz), 4.52 (1H, d, J = 14.0 Hz), 5.11 (1H, q, J = 7.0 Hz), 6.28 (1H, s), 6.52 (1H, s), 7.3- 7.8 (6H, m)

Example 110

Methyl ester of (S)-O-[(3R,5S)-7-chloro-5-(2-chlorophenyl)-1-neopentyl-2-oxo-1,2,3,5-tetrahydro-4,1-benzoxazepine-3-acetyl]lactic acid; and methyl ester of (S)-O-[(3S,5R)-7-chloro-5-(2-chlorophenyl)-1-neopentyl-2-oxo-1,2,3,5-tetrahydro-4,1-benzoxazepine-3-acetyl]lactic acid.

In 150 ml of methylene chloride were dissolved 10 g of trans-7-chloro-5-(2-chlorophenyl)-1-neopentyl-2-oxo-1,2,3,5-tetrahydro-4,1-benzoxazepine-3-acetic acid, as obtained in Example 2, and 4.77 g of methyl ester of (S)-lactic acid. To the solution were added, under ice-cooling, 5.27 g of 1-ethyl-3-(3-dimethylaminopropyl) carbodiimide hydrochloride and 0.3 g of 4-dimethylaminopyridine. The mixture was stirred for 2 hours at room temperature, followed by washing with 1N aqueous solutions of hydrochloric acid and sodium bicarbonate, drying over magnesium sulfate. The solvent was then distilled off under reduced pressure. The residue was separated and purified by means of silica gel column chromatography (eluent, hexane : diethylether = 3 : 1) to yield, as the first fraction, 2.7 g of methyl ester of (S) -O-[(3R,5S)-7-chloro-5-(2-chlorophenyl)-1-neopentyl-2-oxo-1,2,3,5-tetrahydro-4,1-benzoxazepine-3-acetyl]lactic acid as prism crystals, m.p. 155 to 157°C.

$[\alpha]_D^{20}$ - 227.1° (c = 0.94, methanol)

| Elemental Analysis for $C_{26}H_{29}Cl_2NO_6$: | | | |
|---|---|---|---|
| Calcd. | C 59.78; | H 5.60; | N 2.68 |
| Found | C 59.82; | H 5.69; | N 2.51 |

As the second fraction, 2.8 g of methyl ester of (S)-O-[(3S,5R)-7-chloro-5-(2-chlorophenyl)-1-neopentyl-2-oxo-1,2,3,5-tetrahydro-4,1-benzoxazepine-3-acetyl]lactic acid was recovered as prism crystals, m.p. 119 to 120°C.

$[\alpha]_D^{20}$ + 160.1° (c = 0.7, methanol)

| Elemental Analysis for $C_{26}H_{29}Cl_2NO_6$: | | | |
|---|---|---|---|
| Calcd. | C 59.78; | H 5.60; | N 2.68 |

(continued)

| Elemental Analysis for $C_{26}H_{29}Cl_2NO_6$: | | | |
|---|---|---|---|
| Found | C 59.69; | H 5.63; | N 2.67 |

Example 111

(3S,5R)-7-chloro-5-(2-chlorophenyl)-1-neopentyl-2-oxo-1,2,3,5-tetrahydro-4,1-benzoxazepine-3-acetic acid

In 20 ml of methanol was dissolved 0.4 g of methyl ester of (R)-O-[(3S,5R)-7-chloro-5-(2-chlorophenyl)-1-neopentyl-2-oxo-1,2,3,5-tetrahydro-4,1-benzoxazepine-3-acetyl]lactic acid, as obtained in Example 109. To the solution was added 10 ml of an aqueous solution containing 0.32 g of potassium carbonate; the reaction mixture was then. stirred for 2 hours at 60°C and concentrated under reduced pressure, followed by extraction by the addition of 50 ml of 1N hydrochloric acid and 50 ml of ethyl acetate. The ethyl acetate layer was washed with water, dried over anhydrous magnesium sulfate, then distilled off under reduced pressure. The residue was purified by means of silica gel column chromatography (eluent, hexane : ethyl acetate = 2 : 1; methylene chloride : methanol = 3 : 1) to yield 0.15 g of (3S, 5R)-7-chloro-5-(2-chlorophenyl)-1-neopentyl-2-oxo-1,2,3,5-tetrahydro-4,1-benzoxazepine-3-acetic acid as needles, m.p. 241 to 245°C.

$[\alpha]_D^{20}$ + 244.8° (c = 0.52, methanol)

| Elemental Analysis for $C_{22}H_{23}Cl_2NO_4 \cdot 1/4H_2O$: | | | |
|---|---|---|---|
| Calcd. | C 59.94; | H 5.37; | N 3.18 |
| Found | C 60.19; | H 5.47; | N 2.97 |

Example 112

(3R,5S)-7-chloro-5-(2-chlorophenyl)-1-neopentyl-2-oxo-1,2,3,5-tetrahydro-4,1-benzoxazepine-3-acetic acid (1); and ethyl ester of (3R,5S)-7-chloro-5-(2-chlorophenyl)-1-neopentyl-2-oxo-1,2,3,5-tetrahydro-4,1-benzoxazepine-3-acetic acid (2)

In 45 ml of methanol was dissolved 2.2 g of methyl ester of (S)-O-[(3R,5S)-7-chloro-5-(2-chlorophenyl)-1-neopentyl-2-oxo-1,2,3,5-tetrahydro-4,1-benzoxazepine-3-acetyl]lactic acid, as obtained in Example 116, to which was added 15 ml of an aqueous solution containing 1.75 g of potassium carbonate; the mixture was then stirred for 2 hours at 60°C, concentrated under reduced pressure and subjected to extraction with the addition of 100 ml of 1N hydrochloric acid and 100 ml of ethyl acetate. The ethyl acetate layer was washed with water, dried over anhydrous magnesium sulfate, then distilled off under reduced pressure. The residue was purified by silica gel column chromatography (eluent, hexane : ethyl acetate = 2 : 1; methylene chloride : methanol = 3 : 1) to yield crystals. Recrystallization from hexane-ethanol yielded 0.85 g of (3R,5S)-7-chloro-5-(2-chlorophenyl)-1-neopentyl-2-oxo-1,2,3,5-tetrahydro-4,1-benzoxazepine-3-acetic acid as needles, m.p. 250 to 252°C.

$[\alpha]_D^{20}$ - 252.0° (c = 0.5, methanol)

| Elemental Analysis for $C_{22}H_{23}Cl_2NO_4$: | | | |
|---|---|---|---|
| Calcd. | C 59.94; | H 5.37; | N 3.28 |
| Found | C 59.84; | H 5.28; | N 3.31 |

The filtrate was distilled off under reduced pressure; the residue was then dissolved in 30 ml of ethanol, to which 0.3 ml of concentrated sulfuric acid was added; the mixture was then stirred for 2 days. Distillation under reduced pressure was followed by extraction with the addition of 100 ml of water and 100 ml of ethyl acetate. The ethyl acetate layer was washed with aqueous sodium bicarbonate and with water, dried over anhydrous magnesium sulfate, and distilled off under reduced pressure. The residue was purified by means of silica gel column chromatography (eluent, hexane : ethyl acetate = 5 : 1) to yield 0.63 g of ethyl ester of (3R,5S)-7-chloro-5-(2-chlorophenyl)-1-neopentyl-2-oxo-1,2,3,5-tetrahydro-4,1-benzoxazepine-3-acetic acid as prism crystals, m.p. 120 to 121°C.

$[\alpha]_D^{20}$ - 235.5° (c = 0.51, methanol)

| Elemental Analysis for $C_{24}H_{27}Cl_2NO_4$: | | | |
|---|---|---|---|
| Calcd. | C 62.07; | H 5.86; | N 3.02 |
| Found | C 62.10; | H 5.95; | N 2.93 |

Also, in substantially the same manner as above, the hydrolysis of 0.6 g of methyl ester of (R)-O-[(3R,5S)-7-chloro-5-(2-chlorophenyl)-1-neopentyl-2-oxo-1,2,3,5-tetrahydro-4,1-benzoxazepine-3-acetyl]lactic acid yielded 0.37 g of (3R,5S)-7-chloro-5-(2-chlorophenyl)-1-neopentyl-2-oxo-1,2,3,5-tetrahydro-4,1-benzoxazepine-3-acetic acid.

Example 113

Ethyl ester of N-[(3R,5S)-7-chloro-5-(2-chlorophenyl)-1-neopentyl-2-oxo-1,2,3,5-tetrahydro-4,1-benzoxazepine-3-acetyl]aminoacetic acid

In 5 ml of N,N-dimethylformamide were dissolved 0.15 g of (3R,5S)-7-chloro-5-(2-chlorophenyl)-1-neopentyl-2-oxo-1,2,3,5-tetrahydro-4,1-benzoxazepine-3-acetic acid, as obtained in Example 112, and 58 mg of glycine ethylester hydrochloride. To the solution were added, under ice-cooling, 71 mg of diethyl phosphorocyanidate and 0.12 ml of triethylamine. After the reaction mixture was stirred for 30 minutes at room temperature, it was subjected to extraction with the addition of 50 ml of water and 50 ml of ethyl acetate. The ethyl acetate layer was washed with 1N hydrochloric acid and an aqueous solution of sodium bicarbonate, dried over anhydrous magnesium sulfate, and distilled off under reduced pressure. The residue was purified by means of silica gel column chromatography (eluent, hexane : ethyl acetate = 1 : 1) to yield 0.16 g of ethyl ester of N-[(3R,5S)-7-chloro-5-(2-chlorophenyl)-1-neopentyl-2-oxo-1,2,3,5-tetrahydro-4,1-benzoxazepine-3-acetyl]aminoacetic acid as plate crystals, m.p.138 to 139°C.

$[\alpha]_D^{20}$ - 220.8° (c = 0.45, methanol)

| Elemental Analysis for $C_{26}H_{30}Cl_2N_2O_5$: | | | |
|---|---|---|---|
| Calcd. | C 59.89; | H 5.80; | N 5.37 |
| Found | C 59.86; | H 5.94; | N 5.12 |

Example 114

N-[(3R,5S)-7-chloro-5-(2-chlorophenyl)-1-neopentyl-2-oxo-1,2,3,5-tetrahydro-4,1-benzoxazepine-3-acetyl] aminoacetic acid

In 5 ml of ethanol was dissolved 0.12 g of ethyl ester of N-[(3R,5S)-7-chloro-5-(2-chlorophenyl)-1-neopentyl-2-oxo-1,2,3,5-tetrahydro-4,1-benzoxazepine-3-acetyl]aminoacetic acid, as obtained in Example 113, to which was added 2 ml of 1N sodium hydroxide. The reaction mixture was stirred for 15 minutes at room temperature, after which was subjected to extraction with the addition of 50 ml of 1N hydrochloric acid and 50 ml of ethyl acetate. The ethyl acetate layer was washed with water, dried over anhydrous magnesium sulfate, and distilled off under reduced pressure to leave 0.10 g of N-[(3R,5S)-7-chloro-5-(2-chlorophenyl)-1-neopentyl-2-oxo-1,2,3,5-tetrahydro-4,1-benzoxazepine-3-acetyl]aminoacetic acid as a powdery product.

$[\alpha]_D^{20}$ - 229.8° (c = 0.46, methanol)

| Elemental Analysis for $C_{24}H_{26}Cl_2N_2O_5$: | | | |
|---|---|---|---|
| Calcd. | C 58.43; | H 5.31; | N 5.68 |
| Found | C 58.56; | H 5.63; | N 5.57 |

Example 115

(3S,5R)-7-chloro-5-(2-chlorophenyl)-1-neopentyl-2-oxo-1,2,3,5-tetrahydro-4,1-benzoxazepine-3-acetic acid; and (3R, 5S)-7-chloro-5-(2-chlorophenyl)-1-neopentyl-2-oxo-1,2,3,5-tetrahydro-4,1-benzoxazepine-3-acetic acid

In ethanol were dissolved 10 g of 7-chloro-5-(2-chlorophenyl)-1-neopentyl-2-oxo-1,2,3,5-tetrahydro-4,1-benzoxazepine-3-acetic acid, as obtained in Example 2, and quinine of equal molarity. After this mixture was concentrated, 150 ml of ethyl ether was added, and crystallization was allowed to occur under ice-cooling. The crystals thus obtained

were dissolved in a mixture of ethanol and ethyl ether and allowed to recrystallize. The mother liquor was removed from the crystals thus formed, and was concentrated, leaving a residue. This residue underwent recrystallization using hexane, and the crystals thus formed were dissolved in ethyl acetate. The quinine in this solution was removed with dilute hydrochloric acid. The residue after removal of the solvent underwent recrystallization using hexane, resulting in (3S,5R)-7-chloro-5-(2-chlorophenyl)-1-neopentyl-2-oxo-1,2,3,5-tetrahydro-4,1-benzoxazepine-3-acetic acid as crystals. The solvent was then removed from the mother liquor remaining from the very first crystallization. The residue was dissolved in ethyl acetate, and the quinine was removed with dilute hydrochloric acid. The solvent was removed, leaving a residue which was recrystallized using a mixture solvent of ethanol and ethyl ether. After removal of the crystals thus formed, the mother liquor was concentrated, and the residue recrystallized using hexane, resulting in (3R,5S)-7-chloro-5-(2-chlorophenyl)-1-neopentyl-2-oxo-1,2,3,5-tetrahydro-4,1-benzoxazepine-3-acetic acid as crystals.

Example 116

Ethyl ester of (3S,5R)-7-chloro-5-(2-chlorophenyl)-1-neopentyl-2-oxo-1,2,3,5-tetrahydro-4,1-benzoxazepine-3-acetic acid; and ethyl ester of (3R,5S)-7-chloro-5-(2-chlorophenyl)-1-neopentyl-2-oxo-1,2,3,5-tetrahydro-4,1-benzoxazepine-3-acetic acid

To ethyl iodide in dimethyl formamide as a solvent, and under the presence of potassium carbonate, were added, as separate reactions, (3S,5R)-7-chloro-5-(2-chlorophenyl)-1-neopentyl-2-oxo-1,2,3,5-tetrahydro-4,1-benzoxazepine-3-acetic acid and (3R,5S)-7-chloro-5-(2-chlorophenyl)-1-neopentyl-2-oxo-1,2,3,5-tetrahydro-4,1-benzoxazepine-3-acetic acid, as obtained in Example 121, resulting in the above compounds. Ethyl ester of (3S,5R)-7-chloro-5-(2-chlorophenyl)-1-neopentyl-2-oxo-1,2,3,5-tetrahydro-4,1-benzoxazepine-3-acetic acid, m.p.117 to 118°C.

$[\alpha]_D^{20}$ + 224.6° (c = 0.5, MeOH)

| Elemental Analysis for $C_{24}H_{27}Cl_2NO_4$: | | | |
|---|---|---|---|
| Calcd. | C 62.07; | H 5.86; | N 3.02 |
| Found | C 62.01; | H 5.88; | N 2.97 |

Example 117

Ethyl ester of trans-7-chloro-5-(2-ethoxyphenyl)-1-isobutyl-2-oxo-1,2,3,5-tetrahydro-4,1-benzoxazepine-3-acetic acid

(1) 2-amino-5-chloro-2'-hydroxybenzophenone

A mixture of 2.15 g of 2-amino-5-chloro-2'-methoxybenzophenone and 20 ml of 47% hydrogen bromide was heated for 1 hour under reflux. The solution was rendered neutral with sodium bicarbonate, and subjected to extraction with 100 ml of ethyl acetate. The extract solution was washed with water and dried over anhydrous magnesium sulfate; the solvent was then distilled off under reduced pressure. The residue was purified by means of silica gel column chromatography (eluent, hexane : ethyl acetate = 10: 1) to yield 1.9 g of 2-amino-5-chloro-2'-hydroxybenzophenone as needles, m.p. 51 to 52°C.

| Elemental Analysis for $C_{13}H_{10}ClNO_2$: | | | |
|---|---|---|---|
| Calcd. | C 63.04; | H 4.07; | N 5.66 |
| Found | C 62.90; | H 4.04; | N 5.61 |

A mixture of 10 g of 2-acetylamino-5-chloro-2'-methoxybenzophenone and 100 ml of 47% hydrogen bromide was heated for 2 hours under reflux. The solution was rendered neutral with sodium hydroxide, and was subjected to extraction with 200 ml of ethyl acetate. The extract solution was washed with water and dried over anhydrous magnesium sulfate; the solvent was then distilled off under reduced pressure. The residue was purified by means of silica gel column chromatography (eluent, hexane : ethyl acetate = 5 : 1) to yield 8.0 g of 2-amino-5-chloro-2'-hydroxybenzophenone as needles.

(2) 2-amino-5-chloro-2'-ethoxybenzophenone

In 10 ml of N,N-dimethylformamide was dissolved 1.5 g of 2-amino-5-chloro-2'-hydroxybenzophenone, as

obtained in (1), to which were added 1.26 g of potassium carbonate and 0.63 ml of ethyl iodide. The reaction mixture was stirred for 3 hours at room temperature, after which it was subjected to extraction with 100 ml of water and 100 ml of ethyl acetate. The ethyl acetate layer was washed with water, dried over anhydrous magnesium sulfate, and distilled off under reduced pressure. The residue was purified by means of silica gel column chromatography (eluent, hexane : ethyl acetate = 5 : 1) to yield 1.6 g of 2-amino-5-chloro-2'-ethoxybenzophenone as an oily product.

IR$\nu_{max}^{neat}$ $cm^{-1}$: 3460, 3340 (NH); 1610 (C = O)

$^1$H-NMR spectrum (200 MHz, $CACl_3$) δ: 1.21 (3H, t, J = 7.0 Hz), 4.04 (2H, q, J = 7.0 Hz), 6.1-6.5 (2H, br, $NH_2$), 6.65 (1H, d, J = 8.8 Hz), 6.9-7.1 (2H, m), 7.15-7.35 (3H, m), 7.35-7.5 (1H, m)

(3) 2-amino-5-chloro-α-(2-ethoxyphenyl)benzyl alcohol

In 30 ml of ethanol was dissolved 1.5 g of 2-amino-5-chloro-2'-ethoxybenzophenone, as obtained in (2), to which was added 0.34 g of sodium borohydride. The reaction mixture was stirred overnight at room temperature. After the ethanol was distilled off under reduced pressure and after dissociation by the addition of an aqueous solution of hydrochloric acid, 50 ml of 1N sodium hydroxide was added to render the solution neutral, and extraction was carried out with 100 ml of ethyl acetate. The ethyl acetate layer was washed with water, dried over anhydrous magnesium sulfate, and distilled off under reduced pressure. The residue was purified by means of silica gel column chromatography (eluent, hexane : ethyl acetate = 3 : 1) to yield 1.35 g of 2-amino-5-chloro-α-(2-ethoxyphenyl)benzyl alcohol as prism crystals, m.p. 80 to 81°C.

| Elemental Analysis of $C_{15}H_{16}ClNO_2$: | | | |
|---|---|---|---|
| Calcd. | C 64.87; | H 5.81; | N 5.04 |
| Found | C 64.77; | H 5.81; | N 4.85 |

(4) 5-chloro-α-(2-ethoxyphenyl)-2-isobutylaminobenzyl alcohol

In 10 ml of acetic acid were dissolved 1.3 g of 2-amino-5-chloro-α-(2-ethoxyphenyl)benzyl alcohol, as obtained in (3), and 0.47 ml of isobutylaldehyde, to which was added, under ice-cooling, 0.24 g of sodium borohydride. The reaction mixture was stirred for 30 minutes at room temperature, after which it was subjected to extraction with the addition of 100 ml of water and 100 ml of ethyl acetate. The ethyl acetate layer was washed with 1N sodium hydroxide, dried over anhydrous magnesium sulfate, and then distilled off under reduced pressure. The residue was purified by means of silica gel column chromatography (eluent, hexane : ethyl acetate = 20 : 1 to 5 : 1) to yield 1.5 g of 5-chloro-α-(2-ethoxyphenyl)-2-isobutylaminobenzyl alcohol as an oily product.

IR $\nu_{max}^{neat}$ $cm^{-1}$: 3520, 3410 (NH, OH)

$^1$H-NMR spectrum (200 MHz, $CDCl_3$) δ: 0.90 (3H, d, J = 6.8 Hz), 0.91 (3H, d, J = 6.6 Hz), 1.41 (3H, t, J = 7.0 Hz), 1.7-2.0 (1H, m), 2.89 (2H, d, J = 6.8 Hz), 3.2-3.6 (1H, br), 4.13 (2H, q, J = 7.0 Hz), 4.7-5.1 (1H, br), 5.97 (1H, s), 6.55 (1H, d, J = 8.6 Hz), 6.85-7.4 (6H, m)

(5) Ethyl ester of 3-[N-[4-chloro-2-[2-ethoxy-α-hydroxybenzyl]phenyl]-N-isobutylcarbamoyl]acrylic acid

A solution containing 0.84 g of monoethyl ester of fumaric acid and 1.28 ml of thionyl chloride in 10 ml of toluene was stirred for 30 minutes at 90°C. The solvent was distilled off under reduced pressure, leaving monoethyl ester acid chloride of fumaric acid. This product and 1.5 g of 5-chloro-α-(2-methoxyphenyl)-2-isobutylaminobenzyl alcohol, as obtained in (4), were then dissolved in 30 ml of methylene chloride, to which 0.75 g of sodium bicarbonate was added; the mixture was then stirred for 30 minutes at room temperature. The reaction mixture was washed with water and dried over anhydrous magnesium sulfate; the solvent was then distilled off under reduced pressure. The residue was purified by means of silica gel column chromatography (eluent, hexane : ethyl acetate = 4 : 1) to yield 1.95 g of ethyl ester of 3-[N-[4-chloro-2-[2-ethoxy-α-hydroxybenzyl]phenyl]-N-isobutylcarbamoyl]acrylic acid as an oily product.

IR$\nu_{max}^{neat}$ $cm^{-1}$: 3400 (OH); 1720, 1660, 1630 (C = C, C = O)

$^1$H-NMR spectrum (200 MHz, $CDCl_3$) δ: 0.65-1.0 (6H, m), 1.15-1.5 (6H, m), 1.6-2.0 (1H, m), 2.3-3.0 (2H, m), 3.9-4.4 (5H, m), 5.8-6.3 (3H, m), 6.6-8.05 (7H, m)

(6) Ethyl ester of trans-7-chloro-5-(2-ethoxyphenyl)-1-isobutyl-2-oxo-1,2,3,5-tetrahydro-4,1-benzoxazepine-3-acetic acid

In 30 ml of ethanol was dissolved 1.95 g of ethyl ester of 3-[N-[4-chloro-2-[2-ethoxy-α-hydroxybenzyl]phenyl]-N-isobutylcarbamoyl]acrylic acid, as obtained in (5), to which was added 1.17 g of potassium carbonate, and the mixture was stirred overnight. The reaction mixture was subjected to extraction with 100 ml of water and 100 ml of ethyl acetate. The ethyl acetate layer was washed with water, dried over anhydrous magnesium sulfate, and distilled off under reduced pressure. The residue was purified by means of silica gel column chromatography (eluent, hexane : ethyl acetate = 5 : 1) to yield 1.55 g of ethyl ester of trans-7-chloro-5-(2-ethoxyphenyl)-1-isobutyl-2-oxo-1,2,3,5-tetrahydro-4,1-benzoxazepine-3-acetic acid as prism crystals, m.p. 129 to 130°C.

| Elemental Analysis for $C_{25}H_{30}ClNO_5$: | | | |
|---|---|---|---|
| Calcd. | C 65.28; | H 6.57; | N 3.05 |
| Found | C 65.38; | H 6.44; | N 2.91 |

Example 118

Trans-7-chloro-5-(2-ethoxyphenyl)-1-isobutyl-2-oxo-1,2,3,5-tetrahydro-4,1-benzoxazepine-3-acetic acid

In 30 ml of methanol was dissolved 1.25 g of ethyl ester of trans-7-chloro-5-(2-ethoxyphenyl)-1-isobutyl-2-oxo-1,2,3,5-tetrahydro-4,1-benzoxazepine-3-acetic acid, as obtained in Example 117, to which was added 10 ml of an aqueous solution containing 0.75 g of potassium carbonate; the mixture was then stirred for 3 hours at 60°C. After the reaction mixture was concentrated under reduced pressure, it was rendered acid by the addition of 50 ml of 1N hydrochloric acid, and subjected to extraction with 100 ml of ethyl acetate. The extract solution was washed with water and dried over anhydrous magnesium sulfate; the solvent was then distilled off under reduced pressure. The residue was purified by means of silica gel column chromatography (eluent, hexane : ethyl acetate = 2 : 1; eluent, hexane : dichloromethane : ethanol = 5 : 5 : 1) to yield 0.66 g of traps-7-chloro-5-(2-ethoxyphenyl)-1-isobutyl-2-oxo-1,2,3,5-tetrahydro-4,1-benzoxazepine-3-acetic acid as prism crystals, m.p.190 to 192°C.

| Elemental Analysis for $C_{23}H_{26}ClNO_5$: | | | |
|---|---|---|---|
| Calcd. | C 63.96; | H 6.07; | N 3.24 |
| Found | C 63.64; | H 5.96; | N 3.58 |

Example 119

Ethyl ester of trans-5-(2-benzyloxyphenyl)-7-chloro-1-isobutyl-2-oxo-1,2,3,5-tetrahydro-4,1-benzoxazepine-3-acetic acid

(1) 2-amino-2'-benzyloxy-5-chlorobenzophenone

In 30 ml of N,N-dimethylformamide was dissolved 5.0 g of 2-amino-5-chloro-2'-hydroxybenzophenone, as obtained in Example 117 (1), to which were added 4.2 g of potassium carbonate and 2.9 ml of benzyl bromide; the mixture was then stirred for 2 hours at room temperature. The mixture was subjected to extraction with 150 ml of water and 200 ml of ethyl acetate, and the ethyl acetate layer was washed with water, dried over anhydrous magnesium sulfate, and distilled off under reduced pressure. The residue was purified by means of silica gel column chromatography (eluent, hexane : ethyl acetate = 10 : 1 to 5 : 1) to yield 6.4 g of 2-amino-2'-benzyloxy-5-chlorobenzophenone as prism crystals, m.p.110 to 111°C.

| Elemental Analysis for $C_{20}H_{16}ClNO_2$: | | | |
|---|---|---|---|
| Calcd. | C 71.11; | H 4.77; | N 4.15 |
| Found | C 71.34; | H 4.80; | N 4.23 |

(2) 2-amino-α-(2-benzyloxyphenyl)-5-chlorobenzyl alcohol

In 50 ml of methanol was dissolved 6.0 g of 2-amino-2'-benzyloxy-5-chlorobenzophenone, as obtained in (1), to which was added 1.12 g of sodium borohydride; the mixture was then stirred for 4 hours at room temperature. After ethanol was distilled off under reduced pressure, with dissociation by the addition of an aqueous solution of hydrochloric acid, the reaction mixture was rendered neutral by the addition of 50 ml of 1N sodium hydroxide and subjected to extraction with 200 ml of ethyl acetate. The ethyl acetate layer was washed with water, dried over anhydrous magnesium

sulfate, then distilled off under reduced pressure to leave 5.9 g of 2-amino-α-(2-benzyloxyphenyl)-5-chlorobenzyl alcohol as plate crystals, m.p. 120 to 121 °C.

| Elemental Analysis for $C_{20}H_{18}ClNO_2$: | | | |
|---|---|---|---|
| Calcd. | C 70.69; | H 5.34; | N 4.12 |
| Found | C 70.85; | H 5.16; | N 4.24 |

(3) α-(2-benzyloxyphenyl)-5-chloro-2-isobutylaminobenzylalcohol

In 50 ml of acetic acid were dissolved 5.0 g of 2-amino-α-(2-benzyloxyphenyl)-5-chlorobenzyl alcohol, as obtained in (2), and 1.47 ml of isobutylaldehyde. To the solution was added, under ice-cooling, 0.74 g of sodium borohydride. The reaction mixture was stirred for 30 minutes at room temperature, and, after addition of 150 ml of water, was subjected to extraction with 200 ml of ethyl acetate. The extract solution was washed with 1N sodium hydroxide and dried over anhydrous magnesium sulfate; then the solvent was distilled off under reduced pressure. The residue was purified by means of silica gel column chromatography (eluent, hexane : ethyl acetate = 20 : 1 to 5 : 1) to yield 5.6 g of α-(2-benzyloxyphenyl)-5-chloro-2-isobutylaminobenzyl alcohol as prism crystals, m.p. 79 to 80 °C.

| Elemental Analysis for $C_{24}H_{26}ClNO_2$: | | | |
|---|---|---|---|
| Calcd. | C 72.81; | H 6.62; | N 3.54 |
| Found | C 72.95; | H 6.62; | N 3.62 |

(4) Ethyl ester of 3-[N-[4-chloro-2-[2-benzyloxy-α-hydroxybenzyl] phenyl]-N-isobutylcarbamoyl]acrylic acid

A solution comprised of 2.65 g of monoethyl ester of fumaric acid and 4.0 ml of thionyl chloride in 30 ml of toluene was stirred for 1 hour at 90°C. It was then distilled under reduced pressure, leaving monoethyl ester acid chloride of fumaric acid. This product and 5.6 g of α-(2-benzyloxyphenyl)-5-chloro-2-isobutylaminobenzyl alcohol, as obtained in (3), were dissolved in 100 ml of methylene chloride; after the addition of 2.38 g of sodium bicarbonate, the mixture was stirred for 30 minutes at room temperature. The reaction mixture was washed with water and dried over anhydrous magnesium sulfate; then the solvent was distilled off under reduced pressure to yield 6.4 g ethyl ester of 3-[N-[4-chloro-2-[2-benzyloxy-α-hydroxybenzyl]phenyl]-N-isobutylcarbamoyl]acrylic acid as prism crystals, m.p.169 to 171°C.

| Elemental Analysis for $C_{30}H_{32}ClNO_5$: | | | |
|---|---|---|---|
| Calcd. | C 69.02; | H 6.18; | N 2.68 |
| Found | C 69.24; | H 6.04; | N 2.71 |

(5) Ethyl ester of trans-5-(2-benzyloxyphenyl)-7-chloro-1-isobutyl-2-oxo-1,2,3,5-tetrahydro-4,1-benzoxazepine-3-acetic acid

In 150 ml of ethanol was dissolved 6.0 g of ethyl ester of 3-[N-[4-chloro-2-[2-benzyloxy-a-hydroxybenzyl] phenyl]-N-isobutylcarbamoyl]acrylic acid, as obtained in (4), to which was added 3.18 g of potassium carbonate; the mixture was then stirred overnight. The solvent was distilled off under reduced pressure, and the residue was subjected to extraction with 150 ml of water and 200 ml of ethyl acetate. The ethyl acetate layer was washed with water and dried over anhydrous magnesium sulfate, then distilled off under reduced pressure. The residue was purified by means of silica gel column chromatography (eluent, hexane : ethyl acetate = 5 : 1) to yield 5.8 g of ethyl ester of trans-5-(2-benzyloxyphenyl)-7-chloro-1-isobutyl-2-oxo-1,2,3,5-tetrahydro-4,1-benzoxazepine-3-acetic acid as prism crystals, m.p. 146 to 147°C.

| Elemental Analysis for $C_{30}H_{32}ClNO_5$: | | | |
|---|---|---|---|
| Calcd. | C 69.02; | H 6.18; | N 2.68 |
| Found | C 69.20; | H 6.21; | N 2.95 |

Example 120

Trans-5-(2-benzyloxyphenyl)-7-chloro-1-isobutyl-2-oxo-1,2,3,5-tetrahydro-4,1-benzoxazepine-3-acetic acid

In a mixture of 20 ml of methanol and 10 ml of tetrahydrofuran was dissolved 0.5 g of ethyl ester of trans-5-(2-benzyloxyphenyl)-7-chloro-1-isobutyl-2-oxo-1,2,3,5-tetrahydro-4,1-benzoxazepine-3-acetic acid, as obtained in Example 119. To this solution was added 10 ml of an aqueous solution containing 0.66 g of potassium carbonate, and the mixture was stirred for 6 hours at 60°C. The reaction mixture was concentrated under reduced pressure, rendered acid by the addition of 50 ml of 1N hydrochloric acid, and subjected to extraction with 100 ml of ethyl acetate. The extract solution was washed with water and dried over anhydrous magnesium sulfate; then the solvent was distilled off under reduced pressure. The residue was purified by means of silica gel column chromatography (eluent, hexane : ethyl acetate = 3 1; eluent, hexane : dichloromethane : ethanol = 5 : 5 : 1) to yield 0.21 g of trans-5-(2-benzyloxyphenyl)-7-chloro-1-isobutyl-2-oxo-1,2,3,5-tetrahydro-4,1-benzoxazepine-3-acetic acid as prism crystals, m.p. 194 to 197°C.

| Elemental Analysis for $C_{28}H_{28}ClNO_5$: | | | |
|---|---|---|---|
| Calcd. | C 68.08; | H 5.71; | N 2.84 |
| Found | C 68.07; | H 5.75; | N 2.85 |

Example 121

Ethyl ester of trans-7-chloro-5-(2-hydroxyphenyl)-1-isobutyl-2-oxo-1,2,3,5-tetrahydro-4,1-benzoxazepine-3-aceticacid

In 50 ml of ethyl acetate was dissolved 3.5 g of ethyl ester of trans-5-(2-benzyloxyphenyl)-7-chloro-1-isobutyl-2-oxo-1,2,3,5-tetrahydro-4,1-benzoxazepine-3-acetic acid, as obtained in Example 119. To this solution was added 1.0 g of 10% palladium carbon and hydrogenolysis was then allowed to occur at normal temperature and pressure. After the calculated amount of hydrogen was absorbed, the catalyst was removed, and the ethyl acetate was distilled off under reduced pressure. The residue was purified by means of silica gel column chromatography (eluent, hexane : ethyl acetate = 3 : 1) to yield 2.8 g of ethyl ester of trans-7-chloro-5-(2-hydroxyphenyl)-1-isobutyl-2-oxo-1,2,3,5-tetrahydro-4,1-benzoxazepine-3-acetic acid as an oily product.

$IR\nu_{max}^{neat}$ $cm^{-1}$: 3380 (OH); 1730, 1670, 1650 (C = O)

$^1$H-NMR spectrum (200 NHz, $CDCl_3$) δ: 0.90 (3H, d, J = 6.6 Hz), 0.99 (3H, d, J = 6.6 Hz), 1.24 (3H, t, J = 7.2 Hz), 1.9-2.2 (1H, m), 2.85 (1H, dd, J = 17.8, 5.0 Hz), 2.97 (1H, d, J = 17.8, 8.2 Hz), 3.41 (1H, dd, J = 13.8, 6.4 Hz), 4.16 (2H, q, J = 7.2 Hz), 4.30 (1H, dd, J = 13.8, 7.8 Hz), 4.49 (1H, dd, J = 8.2, 4.8 Hz), 5.97 (1H, s), 6.85 (1H, d, J = 2.4 Hz), 6.85-7.5 (6H, m), 7.54 (1H, br, OH)

Example 122

Trans-7-chloro-5-(2-hydroxyphenyl)-1-isobutyl-2-oxo-1,2,3,5-tetrahydro-4,1-benzoxazepine-3-acetic acid

In 10 ml of methanol was dissolved 0.4 g of ethyl ester of trans-7-chloro-5-(2-hydroxyphenyl)-1-isobutyl-2-oxo-1,2,3,5-tetrahydro-4,1-benzoxazepine-3-acetic acid, as obtained in Example 121, to which was added 5 ml of an aqueous solution containing 0.51 g of potassium carbonate; the mixture was then stirred for 1 hour at 60°C. The reaction mixture was condensed under reduced pressure, rendered acid by the addition of 50 ml of 1N hydrochloric acid, and subjected to extraction with 100 ml of ethyl acetate. The extract solution was washed with water and dried over anhydrous magnesium sulfate; the solvent was then distilled off under reduced pressure. The residue was purified by means of silica gel column chromatography (eluent, hexane : ethyl acetate = 2 : 1; eluent, hexane : dichloromethane : ethanol = 5 : 5 : 2) to yield 0.11 g of trans-7-chloro-5-(2-hydroxyphenyl)-1-isobutyl-2-oxo-1,2,3,5-tetrahydro-4,1-benzoxazepine-3-acetic acid as prism crystals, m.p. 238 to 242°C (decomposition).

| Elemental Analysis for $C_{21}H_{22}ClNO_5$: | | | |
|---|---|---|---|
| Calcd. | C 62.45; | H 5.49; | N 3.47 |
| Found | C 62.55; | H 5.68; | N 3.41 |

Example 123

Ethyl ester of trans-7-chloro-1-isobutyl-5-(2-isopropyloxyphenyl)-2-oxo-1,2,3,5-tetrahydro-4,1-benzoxazepine=3-acetic acid

In 10 ml of N,N-dimethylformamide was dissolved 0.7 g of ethyl ester of trans-7-chloro-5-(2-hydroxyphenyl)-1-isobutyl-2-oxo-1,2,3,5-tetrahydro-4,1-benzoxazepine-3-acetic acid, as obtained in Example 121, to which was added 0.34 g of potassium carbonate and 0.24 ml of isopropyl iodide; the mixture was then stirred overnight at room temperature. The mixture was subjected to extraction with 100 ml of water and 150 ml of ethyl acetate, after which the ethyl acetate layer was washed with 1N hydrochloric acid and sodium bicarbonate, dried over anhydrous magnesium sulfate, and distilled off under reduced pressure. The residue was purified by means of silica gel column chromatography (eluent, hexane : ethyl acetate = 3 : 1) to yield 0.55 g of ethyl ester of trans-7-chloro-1-isobutyl-5-(2-isopropyloxyphenyl)-2-oxo-1,2,3,5-tetrahydro-4,1-benzoxazepine-3-acetic acid as needles, m.p. 135 to 137°C.

| Elemental Analysis for $C_{26}H_{32}ClNO_5$: | | | |
|---|---|---|---|
| Calcd. | C 65.88; | H 6.80; | N 2.96 |
| Found | C 66.09; | H 6.83; | N 3.24 |

Example 124

Trans-7-chloro-1-isobutyl-5-(2-isopropyloxyphenyl)-2-oxo-1,2,3,5-tetrahydro-4,1-benzoxazepine-3-acetic acid

In 20 ml of methanol was dissolved 0.4 g of ethyl ester of trans-7-chloro-5-(2-isopropyloxyphenyl)-1-isobutyl-2-oxo-1,2,3,5-tetrahydro-4,1-benzoxazepine-3-acetic acid, as obtained in Example 123. To this solution was added 10 ml of an aqueous solution containing 0.23 g of potassium carbonate; the mixture was then stirred for 3 hours at 80°C. The reaction mixture was concentrated under reduced pressure, rendered acid by the addition of 50 ml of 1N hydrochloric acid, and subjected to extraction with 100 ml of ethyl acetate. The extract solution was washed with water and dried over anhydrous magnesium sulfate, the solvent was then distilled off under reduced pressure. The residue was purified by means of silica gel column chromatography (eluent, hexane : ethyl acetate = 3 : 1; eluent, hexane : dichloromethane : ethanol = 5 : 5 : 1) to yield 0.24 g of trans-7-chloro-1-isobutyl-5-(2-isopropyloxyphenyl)-2-oxo-1,2,3,5-tetrahydro-4,1-benzoxazepine-3-acetic acid as prism crystals, m.p. 146 to 149°C.

| Elemental Analysis for $C_{24}H_{28}ClNO_5 \cdot 0.2H_2O$: | | | |
|---|---|---|---|
| Calcd. | C 64.12; | H 6.37; | N 3.11 |
| Found | C 64.18; | H 6.37; | N 3.28 |

Example 125

Ethyl trans-7-bromo-1-neopentyl-2-oxo-5-(2-pyridyl)- 1,2,3,5-tetrahydro-4,1-benzoxazepine-3-acetate

(1) 2-Amino-5-bromo-α-(2-pyridyl)benzyl alcohol

In 100 ml of methanol was dissolved 10 g of 2-amino-5- bromophenyl-2-pyridylketone. To the solution was added 1.7 g of sodium borohydride and stirred for 30 minutes. The solvent methanol was evaporated off under reduced pressure. The residue was treated with an aqueous solution of hyrochloric acid. The decomposed residue was then neutralized with 200 ml of a sodium bicarbonate aqeous solution, and extracted with ethyl acetate. The ethyl acetate layer was washed with water, dried over anhydrous magnesium sulfate and concentrated under reduced pressure. The residue was purified by silica gel column chromatography (eluent, hexane : ethyl acetate = 2:1) to give 2-amino-5- bromo-α-(2-pyridyl)benzyl alcohol (9.0 g) as prisms, m.p. 104 - 105°C

| Elementary analysis for $C_{12}H_{11}BrN_2O$ | | | |
|---|---|---|---|
| Calcd. | C 51.64; | H 3.97; | N 10.04 |
| Found | C 51.61; | H 3.93; | N 10.04 |

(2) 5-Bromo-2-neopentylamino-α-(2-pyridyl)benzyl alcohol

In 20 ml of acetic acid was dissolved 2.0 g of 2-amino-5-bromo-α-(2-pyridyl)benzyl alcohol obtained in (1) together with 0.86 ml of trimethylacetoaldehyde. To the solution was added 0.36 g of sodium borohydride under ice-cooling. The reaction mixture was stirred for 30 minutes at room temperature and then subjected to extraction with a mixture of 100 ml of water and 150 ml of ethyl acetate. The ethyl acetate layer was washed with 1N sodium hyroxide, dried over anhydrous magnesium sulfate, and concentrated to dryness under reduced pressure. The residue was purified by silica gel column chromatography (eluent, hexane : ethyl acetate = 5:1) to afford 5-bromo-2- neopentylamino-α-(2-pyridyl)benzyl alcohol (2.4 g) as an oily product.
$IR\nu^{Neat}_{Max}$ $cm^{-1}$: 3390, 3280 (NH,OH)
$^1$H-NMR spectrum (200MHz, CDCl3) δ: 0.80 (9H, s, But), 2.68 (2H, s), 5.69 (1H, s), 6.48 (1H, d, J=8.4Hz), 7.1-7.35 (4H, m), 7.55-7.7 (1H, m), 8.5-8.6 (1H, m).

(3) Ethyl 3-[N-[4-bromo-2-[α-(2-pyridyl) hydroxymethyl]phenyl]-N-neopentylcarbamoyl]acrylate

In 50 ml of dichloromethane was dissolved 2.4 g of 5-bromo-2-neopentylamino-α-(2-pyridyl)benzyl alcohol obtained in (2). To the solution were added 1.15 g of sodium borohydride and 1.23 g of monomethyl fumarate acid chloride. After stirring for 2 hours at room temperature, the reaction mixture was washed with water and dried over anhydrous magnesium sulfate. The solvent was evaporated off under reduced pressure. The residue was purified by column chromatography using silica gel (eluent, hexane : ethyl acetate = 2:1) to give ethyl 3-[N-[4-bromo- 2-[α-(2-pyridyl) hydroxymethyl]phenyl]-N- neopentylcarbamoyl]acrylate as prisms, m.p.165 -166°C.

| Elementary analysis for $C_{23}H_{27}BrN_2O_4$: | | | |
|---|---|---|---|
| Calcd. | C 58.11; | H 5.72; | N 5.89 |
| Found | C 58.21; | H 5.65; | N 6.14 |

(4) Ethyl trans-7-bromo-1-neopentyl-5-(2-pyridyl)-2-oxo-1,2, 3, 5-tetrahydro-4,1-benzoxazepine-3-acetate

In 30 ml of ethanol was dissolved 2.0 g of ethyl 3-[N-[4- bromo-2-[α-(2-pyridyl) hydroxymethyl]phenyl]-N-neopentylcarbamoyl]acrylate obtained in (3). To the solution was added 1.16 g of potassium carbonate and the mixture was stirred overnight at room temperature. The solvent was evaporated off under reduced pressure. The residue was then extracted with 100 ml of water and 100 ml of ethyl acetate. The organic layer was washed with water, dried over anhydrous magnesium sulfate, and concentrated under reduced pressure. The redudue was purified on a silica gel column (eluent, hexane : ethyl acetate = 3:1) to give 1.9 g of ethyl trans-7-bromo-1-neopentyl- 5-(2-pyridyl)-2-oxo-1,2,3,5-tetrahydro-4,1-benzoxazepine-3- acetate as an oily product.
$IR\nu^{Neat}_{Max}$ $cm^{-1}$: 1730, 1675 (C=O)
$^1$H-NMR ($CDCl_3$) δ: 0.94(9H,s,$Bu^t$), 1.25(3H,t,J = 7.2Hz), 2.80(1H,dd,J = 16.6,5.8Hz), 3.07(1H,dd,J = 16.6,7.8Hz), 3.31(1H,d,J = 13.8Hz), 4.13(2H,q, J = 7.2Hz), 4.44(1H,dd,J=7.8,5.8Hz), 4.49(1H,d,J=13.8Hz), 6.07 (1H, s), 6.59(1H,d,J=2.2Hz), 7.2-7.9 (5H,m), 8.6-8.7(1H,m)

Example 126

Trans-7-bromo-1-neopentyl-5-(2-pyridyl)-2-oxo-1,2,3,5-tetrahydro-4,1-benzoxazepine-3-acetic acid

In 20 ml of methanol was dissolved 1.9 g of ethyl trans-7-bromo-1-neopentyl-5-(2-pyridyl)-2-oxo-1,2,3,5-tetrahydro-4,1-benzoxazepine-3-acetate obtained in Example 125. To the solution was added 10 ml of an aqueous solution containing 1.1 g of potassium carbonate. The mixture was heated and refluxed for 30 minutes, and then concentrated under reduced pressure. The concentrate was crystallized by the addition of 30 ml of a hydrochloric acid aqueous solution. The product was collected by filteration and recrystallized from hexane and ethanol to give 1.25 g of trans-7-bromo-1-neopentyl-5-(2-pyridyl)-2-oxo-1,2,3,5-tetrahydro-4,1-benzoxazepine-3-acetic acid as prisms, m.p. > 263°C (decomp.)

| Elementary analysis for $C_{21}H_{23}BrN_2O_4$ | | | |
|---|---|---|---|
| Calcd. | C 56.39; | H, 5.18; | N 6.26 |
| Found | C 56.39; | H, 5.18; | N 6.10 |

Example 127

Ethyl N-benzyl-N-[trans-7-chloro-5-(2-chlorophenyl)-1-neopentyl-2-oxo-1,2,3,5-tetrahydro-4,1-benzoxazepine-3-acetyl]aminoacetate

In 10 ml of dimethylformamide were dissolved 0.3 g of trans-7-chloro-5-(2-chlorophenyl)-1-neopentyl-2-oxo-1,2,3,5-tetrahydro-4,1-benzoxazepine-3-acetic acid obtained in Example 2 and 0.27 g of N-benzylglycine ethyl ester.

To the solution was added 0.24 g of diethyl phosphorocyanidate together with 0.19 ml of triethylamine under ice-cooling. The mixture was stirred for 30 minutes at room temperature and then subjected to extraction with a mixture of 100 ml of water and 100 ml of ethyl acetate. The ethyl acetate layer was washed with 1N hydrochloric acid and with a sodium bicarbonate aqueous solution, dried over anhydrous magnesium sulfate, and concentrated under reduced pressure. The residue was purified by silica gel column chromatography (eluent, hexane : ethyl acetate = 3:1) to afford ethyl N-benzyl-N-[trans-7-chloro-5-(2-chlorophenyl)-1-neopentyl-2-oxo-1,2,3,5-tetrahydro-4,1-benzoxazepine-3-acetyl]amino acetate (0.56 g) as prisms, m.p. 195-197°C

| Elementary Analysis for $C_{33}H_{36}Cl_2N_2O_5$ | | | |
|---|---|---|---|
| Calcd. | C 64.81; | H 5.93; | N,4.58 |
| Found | C 64.84; | H 5.97; | N 4.45 |

Example 128

N-Benzyl-N-[trans-7-chloro-5-(2-chlorophenyl)-1-neopentyl-2-oxo-1,2,3,5-tetrahydro-4,1-benzoxazepine-3-acetyl] amino acetic acid

In 15 ml of methanol was dissolved 0.3 g of ethyl N-benzyl-N-[trans-7-chloro-5-(2-chlorophenyl)-1- neopentyl-2-oxo-1,2,3,5-tetrahydro-4,1-benzoxazepine-3-acetyl]amino acetate obtained in Example 127. To the solution was added 4 ml of sodium hydroxide and the mixture was stirred for 20 minutes. The reaction mixture, after acidified with 50 ml of 1N HCl, was subjected to extraction with 100 ml of ethyl acetate. The ethyl acetate layer was washed with water and dried over anhydrous magnesium sulfate. The solvent was ' evaporated under reduced pressure to yield N-benzyl-N- [trans-7-chloro-5-(2-chlorophenyl)-1-neopentyl-2-oxo-1,2,3,5-tetrahydro-4,1-benzoxazepine-3-acetyl]amino acetic acid as prisms, m.p. 190 - 192°C.

| Elementary Analysis for $C_{31}H_{32}Cl_2N_2O_5$: | | | |
|---|---|---|---|
| Calcd. | C 63.81; | H 5.53; | N 4.80 |
| Found | C 64.02; | H 5.85; | N 4.81 |

Example 129

Methyl N-[trans-7-chloro-5-(2-chlorophenyl)-1-neopentyl-2- oxo-1,2,3,5-tetrahydro-4,1-benzoxazepine-3-acetyl]-N-phenyl aminoacetate

A mixture of 0.6 g of trans-7-chloro-5-(2- chlorophenyl)-1-neopentyl-2-oxo-1,2,3,5-tetrahydro-4,1-benzoxazepine-3-acetic acid obtained in Example 2,10 ml of thionyl chloride and 10 ml of toluene was stirred for 30 minutes at 90°C, and then concentrated under reduced pressure. The concentrate trans-7-chloro-5-(2- chlorophenyl)-1- neopentyl-2-oxo-1,2,3,5-tetrahydro-4,1-benzoxazepine-3-acetyl chloride was dissolved in 10 ml-of methylene chloride. To the solution were added 0.27 g of N-phenylglycine methyl ester and 0.23 ml of triethylamine. The mixture was stirred for one hour at room temperature, and then subjected to extraction with 100 ml of ethyl acetate. The organic layer was washed with 1N HCl and with a sodium bicarbonate aqueous solution, dried over anhydrous magnesium sulfate and concentrated under reduced pressure.

The residue was purified on a silica gel column (eluent, hexane : ethyl acetate = 3:1) to give methyl N-[trans-7- chloro-5-(2-chlorophenyl)-1-neopentyl-2-oxo-1,2,3,5- tetrahydro-4,1-benzoxazepine-3-acetyl]-N-phenyl amino acetate (0.35 g) as prisms, m.p. 226 - 228°C

| Elementary Analysis for $C_{31}H_{32}Cl_2N_2O_5$: | | | |
|---|---|---|---|
| Calcd. | C 63.81; | H 5.53; | N 4.80 |

(continued)

| Elementary Analysis for $C_{31}H_{32}Cl_2N_2O_5$: | | | |
|---|---|---|---|
| Found | C 63.78; | H 5.59; | N 4.60 |

Example 130

N-[trans-7-chloro-5-(2-chlorophenyl)-1-neopentyl-2-oxo-1,2,3,5-tetrahydro-4,1-benzoxazepine-3-acetyl]-N-phenylamino acetic acid

In 3 ml of methanol was dissolved 0.25 g of methyl N-[trans-7-chloro-5-(2-chlorophenyl)-1-neopentyl-2-oxo-1,2,3,5-tetrahydro-4,1-benzoxazepine-3-acetyl]-N-phenyl amino acetate obtained in Example 129. To the solution was added 2 ml of 1N sodium hydroxide. The mixture was stirred for 1.5 hours at 60°C and then acidified with 50 ml of 1N HCl for extraction with ethyl acetate. The ethyl acetate layer was washed with water, dried over anhydorus magnesium sulfate, and concentrated under reduced pressure to give 0.23 g of N-[trans-7- chloro-5-(2-chlorophenyl)-1- neopentyl-2-oxo-1,2,3,5-tetrahydro-4,1-benzoxazepine-3-acetyl]-N-phenylaminoacetic acid as prisms, m.p. 238 - 240°C.

| Elementary Analysis for $C_{30}H_{30}Cl_2N_2O_5$: | | | |
|---|---|---|---|
| Calcd. | C 63.27; | H 5.31; | N 4.92 |
| Found | C 63.46; | H 5.54; | N 4.70 |

Example 131

Ethyl N-[trans-7-chloro-5-(2-methoxyphenyl)-1-neopentyl-2- oxo-1,2,3,5-tetrahydro-4,1-benzoxazepine-3-acetyl] amino acetate

In 10 ml of dimethylformamide were dissolved 0.3 g of trans-7-chloro-5-(2-methoxyphenyl)-1-neopentyl-2-oxo-1,2,3,5-tetrahydro-4,1-benzoxazepine-3-acetic acid obtained in Example 72 and 0.12 g of glycine ethyl ester hydrochloride. To the solution were added 0.14 g of diethyl phosphorocyanidate and 0.24 ml of triethylamine under ice-cooling. The mixture was stirred for 30 minutes at room temperature and then subjected to extraction with 100 ml of water and 100 ml of ethyl acetate. The ethyl acetate layer was washed with 1N HCl and with a sodium bicarbonate aqueous solution and dried over anhydrous magnesium sulfate. Ethyl acetate was evaporated off under reduced pressure. The residue was purified by silica gel column chromatography (eluent, hexane : ethyl acetate = 1:1) to yield ethyl N-[trans-7-chloro-5-(2-methoxyphenyl)-1-neopentyl-2-oxo- 1,2,3,5-tetrahydro-4,1- benzoxazepine-3-acetyl]amino acetate (0.33 g) as needles, m.p. 233 - 236°C.

| Elementary Analysis for $C_{27}H_{33}ClN_2O_6$ | | | |
|---|---|---|---|
| Calcd. | C 62.72; | H 6.43; | N 5.42 |
| Found | C 62.54; | H 6.47; | N 5.28 |

Example 132

N-[Trans-7-chloro-5-(2-methoxyphenyl)-1-neopentyl-2-oxo-1,2,3,5-tetrahydro-4,1-benzoxazepine-3-acetyl] aminoacetic acid

In 5 ml of ethanol was dissolved 0.25 g of ethyl N-[trans-7-chloro-5-(2-methoxyphenyl)-1-neopentyl-2-oxo-1,2,3,5-tetrahydro-4,1-benzoxazepine-3-acetyl]aminoacetate obtained in Example 131. To the solution was added 2 ml of 1N NaOH. After stirring for 15 minutes, the reaction mixture was acidified with 100 ml of 1N HCl and subjected to extraction with ethyl acetate. The organic layer was washed with water and dried over anhydrous magnesium sulfate. Ethyl acetate was evaporated off under reduced pressure to afford N-[trans-7-chloro-5-(2-methoxyphenyl)-1-neopentyl-2- oxo-1,2,3,5-tetrahydro-4,1-benzoxazepine-3- acetyl]amino acetic acid as prisms, m.p. 239 - 242°C

| Elementary Analysis for $C_{25}H_{29}ClN_2O_5$ | | | |
|---|---|---|---|
| Calcd. | C 61.41; | H 5.98; | N 5.73 |
| Found | C 61.38; | H 5.91; | N 5.83 |

Example 133

Trans-7-chloro-5-(2-chlorophenyl)-1-neopentyl-2-oxo-1,2,3,5-tetrahydro-4,1-benzoxazepine-3-methylamine hydrochloride

In the same manner as done in Example 35 was treated 1.0 g of trans-7-chloro-5-(2-chlorophenyl)-1-neopentyl- 2-oxo-1,2,3,5-tetrahydro-4,1-benzoxazepine-3-acetic acid obtained in Example 2. The product was 0.90 g of trans-7-chloro-5-(2-chlorophenyl)-1-neopentyl-2-oxo- 1,2,3,5-tetrahydro-4,1-benzoxazepine-3-methylamine hydrochloride as plate crystals, m.p.173 - 175°C

| Elementary Analysis for $C_{21}H_{24}Cl_2N_2O_2 \cdot HCl \cdot H_2O$ | | | |
|---|---|---|---|
| Calcd. | C 54.62; | H 5.89; | N 6.06 |
| Found | C 54.77; | H 5.95; | N 5.83 |

Example 134

Methyl N-[trans-7-chloro-5-(2-chlorophenyl)-1-neopentyl-2- oxo-1,2,3,5-tetrahydro-4,1-benzoxazepine-3-methyl] carbamoyl acetate

In 10 ml of N,N-dimethyformamide were dissolved 0.3 g of trans-7-chloro-5-(2-chlorophenyl)-1-neopentyl-2-oxo- 1,2,3,5-tetrahydro-4,1-benzoxazepine-3-methylamine hydrochloride obtained in Example 133 and 0.12 g of monomethyl malonate potassium salt. To the solution was added 0.13 g of diethyl phosphorocyanate together with 0.11 ml of triethylamine under ice-cooling. After stirring for 30 minutes at room temperature, the reaction mixture was subjected to extraction with 100 ml of water and 100 ml of ethyl acetate. The ethyl acetate layer was washed with 1N HCl and with a sodium bicarbonate aqueous solution, and dried over anhydrous magnesium sulfate. The solvent ethyl acetate was then evaporated off under reduced pressure. The residue was purified by silica-gel column chromatogrphy (eluent, hexane : ethyl acetate = 1:1) to give 0.28 g of methyl N-[trans-7-chloro-5-(2-chlorophenyl)-1-neopentyl-2- oxo-1,2,3,5-tetrahydro-4,1-benzoxazepine-3-methyl]carbamoyl acetate as an oily product. IR$\nu_{Max}^{Neat}$ $cm^{-1}$: 3330(NH); 1740, 1670 (C=O)

$^1$H-NMR (200MHz, $CDCl_3$) $\delta$: 0.94 (9H,s,$Bu^t$), 3.28 (2H,s), 3.38 (1H,d,J=13.9Hz), 3.74(3H,s), 3.7-3.85(2H,m), 3.99(1H,t,J = 6.1Hz), 4.54(1H,d,J = 13.9Hz), 6.25(1H,s), 6.52(1H,d,J = 1.9Hz), 7.2-7.9(7H,m,NH).

Example 135

N-[Trans-7-chloro-5-(2-chlorophenyl)-1-neopentyl-2-oxo-1,2,3,5-tetrahydro-4,1-benzoxazepine-3-methyl]carbamoyl acetic acid

In 5 ml of ethanol was dissolved 0.28 g of methyl N-[trans-7-chloro-5-(2-chlorophenyl)-1-neopentyl-2-oxo-1,2,3,5-tetrahydro-4,1-benzoxazepine-3-methyl]carbamoyl acetate obtained in Example 134. To the solution was added 2 ml of 1N sodium hydroxide and the mixture was stirred for 10 minutes at room temperature. The reaction mixture was acidified with 50 ml of 1N hydrochloric acid and then subjected to extraction with 50 ml of ethyl acetate. The ethyl acetate layer was washed with water, dried over anhydrous magnesium sulfate, and concentrated under reduced pressure to afford 0.23 g of N-[trans-7-chloro-5-(2- chlorophenyl)-1-neopenyl-2-oxo-1,2,3,5-tetrahydro-4,1-benzoxazepine-3-methyl]carbamoyl acetic acid as plate crystals, m.p. 135 - 138°C

| Elementary analysis for $C_{24}H_{26}Cl_2N_2O_5$: | | | |
|---|---|---|---|
| Calcd. | C 58.43; | H 5.31; | N 5.68 |
| Found | C 58.48; | H 5.42; | N 5.52 |

Example 130

Tert-butyl N-[trans-7-chloro-5-(2-chlorophenyl)-1-neopentyl-2-oxo-1,2,3,5-tetrahydro-4,1-benzoxazepine 3-methyl) aminoacetate

A mixture of 2.5 g of trans-7-chloro-5-(2-chlorophenyl)-1-neopentyl-2-oxo-1,2,3,5-tetrahydro-4,1-benzoxazepine-3-methylamine hydrochloride obtained in Example 133, 0.93 ml of ethyl chloroacetate, 1.87 g of potassium

carbonate, and 50 ml of acetonitrile was heated and refluxed overnight. The reaction mixture was concentrated under reduced prressure and the concentrate was extracted with 100 ml of water and 150 ml of ethyl acetate. The organic layer was washed with water and dried over anhydrous magnesium sulfate. Ethyl acetate was evaporated under reduced pressure to give 1.95 g of tert-butyl N-[trans-7-chloro-5- (2-chlorophenyl)-1-neopentyl-2-oxo-1,2,3,5-tetrahydro-4,1-benzoxazepine-3-methyl]aminoacetate as an oily product.

IR$\nu_{Max}^{Neat}$ $cm^{-1}$:3330 (NH); 1735,1675 (C = O)

1H-NMR (200MHz, CDCl3) δ: 0.94 (9H,s,But), 1.44 (9H,s, But), 3.00 (1H,dd,J=12.2,6.4Hz), 3.11 (1H,dd, J=12.2,6.2Hz), 3.33(2H,s), 3.36(1H,d,J=14.0Hz), 4.04(1H,t,J=6.3Hz), 4.52(1H,d,J=14.0Hz), 6.26(1H,s), 6.52(1H,d, J=1.8Hz), 7.2-7.85( 6H,m)

Example 137

N-[Trans-7-chloro-5-(2-chlorophenyl)-1-neopentyl-2-oxo-1,2,3,5-tetrahydro-4,1-benzoxazepine-3-methyl] aminoacetic acid hydrochloride

In 15 ml of 4N HCl-dioxane solution was dissolved 0.3g of tert-butyl N-[trans-7-chloro-5-(2-chlorophenyl)-1-neopentyl-2-oxo-1,2,3,5-tetrahydro-4,1-benzoxazepine-3- methyl]aminoacetate obtained in Example 136. After stirring for 8 hours at room temperature, the reaction mixture was concentrated under reduced pressure. The addition of hexane and ethyl acetate to the residue yielded 0.23 g of powdered 'N-[trans-7-chloro-5-(2-chlorophenyl)-1-neopentyl-2-oxo-1,2,3,5-tetrahydro-4;1-benzoxazepine-3-methyl]aminoacetic acid hydrochloride.

| Elementary analysis for $C_{23}H_{26}Cl_2N_2O_4 \cdot HCl$ | | | |
|---|---|---|---|
| Calcd. | C 55.05; | H 5.42; | N 5.58 |
| Found | C 55.38; | H 5.77; | N 5.39 |

$^1$H-NMR spectrum (200MHz, $d_6$-DMSO) δ: 0.89(9H,s,But), 3.2-3.55(2H,m), 3.68(1H,d,J = 14.0Hz), 3.88(2H,s), 4.32(1H,d,J = 14.0Hz), 4.3-4.5(1H,m), 6.19(1H, s), 6.39(1H,d,J = 2.4Hz), 7.5-8.1(6H,m).

Example 138

Tert-butyl N-[trans-7-chloro-5-(2-chlorophenyl)-1-neopentyl-2-oxo-1,2,3,5-tetrahydro-4,1-benzoxazepine-3-methyl] -N-methansulfonylaminoacetate

In 5 ml of N,N-dimethylformamide was dissolved 0.44 g of tert-butyl N-[trans-7-chloro-5-(2-chlorophenyl)-1-neopentyl-2-oxo-1,2,3,5-tetrahydro-4,1-benzoxazepine-3-methyl]aminoacetate obtained in Example 136. To the solution was added 0.072 ml of methanesulfonyl chloride and 0.14 ml of triethylamine under ice-cooling. After stirring for 30 minutes at room temperature, the reaction mixture was subjected to extraction with 100 ml of water and 100 ml of ethyl acetate. The ethyl acetate layer was washed with 5% potassium bisulfate solution and with a sodium bicarbonate aqueous solution and dried over anhydrous magnesium sulfate. Ethyl acetate was evaporated off under reduced pressure. The residue was purified by silica gel column chromatography (hexane : ethyl acetate = 3:1) to afford 0.37 g of tert-butyl N-[trans-7-chloro-5-(2-chlorophenyl)-1-neopentyl-2-oxo-1,2,3,5-tetrahydro-4,1-benzoxazepine-3- methyl] -N-methanesulfonylaminoacetate as prisms, m.p. 178 - 180°C.

| Elementary analysis for $C_{28}H_{36}Cl_2N_2O_6S$ | | | |
|---|---|---|---|
| Calcd. | C 56.09; | H 6.05; | N 4.67 |
| Found | C 55.92: | H 6.06; | N 4.47 |

Example 139

N-[Trans-7-chloro-5-(2-chlorophenyl)-1-neopentyl-2-oxo- 1,2,3,5-tetrahydro-4,1-benzoxazepine-3-methyl]-N-methanesulfonylaminoacetic acid

In 20 ml of 4N HCl-dioxane solution was dissolved 0.25 g of tert-butyl N-[trans-7-chloro-5-(2-chlorophenyl)-1- neopentyl-2-oxo-1,2,3,5-tetrahydro-4,1-benzoxazepine-3-methyl]-N-methanesulfonylaminoacetate obtained in Example 138. After stirring for 8 hours at room temperature, the reaction mixture was concentrated under reduced pressure. The residue was treated with hexane and ethyl acetate to give 0.21 g of N-[trans-7-chloro-5-(2-chlorophenyl)-1-

neopentyl-2-oxo-1,2,3,5-tetrahydro-4,1-benzoxazepine-3-methyl] N-methanesulfonylaminoacetic acid as prisms, m.p. 236 - 238°C.

| Elementary analysis for $C_{24}H_{28}Cl_2N_2O_6S$ | | | |
|---|---|---|---|
| Calcd. | C 53.04; | H 5.19; | N 5.15 |
| Found | C 53.32; | H 5.42; | N 5.02 |

Example 140

Tert-butyl N-[trans-7-chloro-5-(2-chlorophenyl)-1-neopentyl-2-oxo-1,2,3,5-tetrahydro-4,1-benzoxazepine-3- methyl] -N-(p-toluensulfonyl)aminoacetate

In 10 ml of N,N-dimethylformamide was dissolved 0.5 g of tent-butyl N-[trans-7-chloro-5-(2-chlorophenyl)-1-neopentyl-2-oxo-1,2,3,5-tetrahydro-4,1-benzoxazepine-3- methyl] aminoacetate obtained in Example 136. To the solution was added 0.22 g of p-toluenesulfonyl chloride together with 0.16 ml of triethylamine under ice-cooling.

The mixture was stirred for one hour at room temperature and then subjected to extraction with 100 ml of water and 100 ml of ethyl acetate. The ethyl acetate layer was washed with 5% potassium bisulfate solution and with sodium bicarbonate aqueous solution, dried over anhydrous magnesium sulfate. The solvent was evaporated off under reduced pressure. The residue was purified by column chromatography using silica gel (eluent, hexane : ethyl acetate = 5:1) to afford tert-butyl N-[trans-7-chloro-5-(2-chlorophenyl)-1- neopentyl-2-oxo-1,2,3,5-tetrahydro-4,1-benzoxazepine-3- methyl]-N-(p-toluenesulfonyl)aminoacetate as an oily product.

IR$\nu_{Max}^{Neat}$ cm$^{-1}$: 1740, 1670 (C = O)

$^{1}$H-NMR (200MHz, $CDC_{13}$) δ: 0.92 (9H,s,But), 2.39(3H,s), 3.36(1H,d,J=14.0Hz), 3.66(1H,dd,J=15.6,6.4Hz), 3.84 (1H,dd, J=15.6,5.9Hz), 3.98(1H,d,J=18.4Hz), 4.2-4.3(1H,m), 4.29(1H, d,J= 18.4Hz), 4.45(1H,d,J=14.0Hz), 6.20(1H, s), 6.46(1H,s, J=2.1Hz), 7.15-7.7(10H,m).

Example 141

N-[Trans-7-chloro-5-(2-chlorophenyl)-1-neopentyl-2-oxo-1,2,3,5-tetrahydro-4,1-benzoxazepine-3- methyl]-N-(p-toluensulfonyl)aminoacetic acid

In 15 ml of 4N HCl-dioxane solution was dissolved 0.5 g of tert-butyl N-[trans-7-chloro-5-(2-chlorophenyl)-1-neopentyl-2-oxo-1,2,3,5-tetrahydro-4,1-benzoxazepine-3-methyl]-N-(p-toluensulfonyl)aminoacetate obtained in Example 140. After stirring for 4 hours at room temperature, the reaction solution was concentrated under reduced pressure. The residue was treated with hexane and diethyl ether to afford 0.41 g of N-[trans-7-chloro-5-(2-chlorophenyl)-1-neopentyl-2-oxo-1,2,3,5-tetrahydro-4,1-benzoxazepine-3-methyl]-N-(p-toluensulfonyl)aminoacetic acid as needles, m.p.132 - 134°C.

| Elementary analysis for $C_{30}R_{32}Cl_2N_2O_6S$: | | | |
|---|---|---|---|
| Calcd. | C 58.16; | H 5.21; | N 4.52 |
| Found | C 58.01; | H 5.32; | N 4.55 |

Example 142

Tert-butyl N-acetyl-N-[trans-7-chloro-5-(2-chlorophenyl)-1-neopentyl-2-oxo-1,2,3,5-tetrahydro-4,1-benzoxazepine-3-methyl]aminoacetate.

In 10 ml of N, N-dimethylformamide was dissolved 0.5 g of tert-butyl N-[trans-7-chloro-5-(2-chlorophenyl)-1-neopentyl-2-oxo-1,2,3,5-tetrahydro-4,1-benzoxazepine-3- methyl]aminoacetate obtained in Example 136. To the solution was added 0.082 ml of acetyl chloride and 0.16 ml of triethylamine under ice-cooling.

The mixture was stirred for 30 minutes at room temperature and then subjected to extraction with 100 ml of water and 100 ml of ethyl acetate. The ethyl acetate layer was washed with 5% potassium bisulfate solution and with a sodium bicarbonate aqueous solution, dried over anhydrous magnesium sulfate, and concentrated under reduced pressure. The residue was purified on a silica gel column (eluent, hexane : ethyl acetate = 1:1) to give 0.5 g of tert-butyl N-acetyl-N-[trans-7-chloro-5-(2-chlorophenyl)-1-neopentyl-2-oxo- 1,2,3,5-tetrahydro-4,1-benzoxazepine-3-methyl]aminoacetate as an oily product.

$IR\nu_{Max}^{Neat}$ cm$^{-1}$: 1740, 1670 (C = O)

$^1$H-NMR (200MHz, $CDCl_3$) δ: 0.92 and 0.94 (9H,each s,Bu$^t$), 1.95 and 2.15 (3H,each s,Bu$^t$), 3.35 and 3.38 (1H, each d,J= 14.0Hz), 3.7-4.3 (5H,m), 4.45 and 4.48 (1H,each d,J=14.0Hz), 6:23 and 6.28 (1H, each s), 6.45-6.55 (1H, m), 7.3-7.8 (6H, m).

Example 143

N-Acetyl-N-[trans-7-chloro-5-(2-chlorophenyl)-1-neopentyl-2-oxo-1,2,3,5-tetrahydro-4,1-benzoxazepine-3-methyl] aminoacetic acid

In 20 ml of 4N HCl-dioxane solution was dissolved 0.5 g of tert-butyl N-acetyl-N-[trans-7-chloro-5-(2-chlorophenyl)-1-neopentyl-2-oxo-1,2,3,5-tetrahydro-4,1-benzoxazepine-3-methyl]aminoacetate obtained in Example 142. After stirring overnight at room temperature, the mixture was concentrated under reduced pressure. The concentrate was treated with hexane and diethylether to afford 0.37 g of powdery N-acetyl-N-[trans-7-chloro-5-(2-chlorophenyl)-1-neopentyl-2-oxo-1,2,3,5-tetrahydro-4,1-benzoxazepine-3-methyl]aminoacetic acid.

| Elementary analysis for $C_{25}H_{28}Cl_2N_2O_5$: | | | |
|---|---|---|---|
| Calcd. | C 59.18; | H 5.56; | N 5.52 |
| Found | C 59.36; | H 5.70; | N 5.50 |

$^1$H-NMR spectrum (200 MHz, $CDCl_3$) δ: 0.92 and 0.94 (9H, each s, Bu$^t$), 2.00 and 2.20 (3H, each s), 3.3-3.5 (1H, m), 3.7-4.5 (6H, m), 6.22 and 6.28 (1H, each s), 6.45-6.55 (1H, m), 7.3-7.8 (6H, m)

Examplel 144 (Reference)

Methyl ester of 4-N-[trans-7-chloro-5-(2-chlorophenyl)-1-neopentyl-2-oxo-1,2,3,5-tetrahydro-4,1-benzoxazepine-3-methyl]aminomethylbenzoic acid

(A) In 30 ml of acetonitrile was mixed 1.0 g of trans-7-chloro-5-(2-chlorophenyl)-1-neopentyl-2-oxo-1,2,3,5-tetrahydro-4,1-benzoxazepine-3-methylamine hydrochloride obtained in Example 133 with 0.6 g of methyl 4-bromomethylbenzoate and 0.75 g of potassium carbonate. The mixture was refluxed by heating for 2 hours. After the addition of 0.2 g of methyl 4-bromomethylbenzoate, reflux was confined by heating for 5 hours, followed by concentration under reduced pressure. The concentrate was extracted with 100 ml of water and 150 ml of ethyl acetate. The ethyl acetate layer was washed with water and dried over anhydrous magnesium sulfate. The solvent was evaporated off under reduced pressure and the residue was purified by silica gel column chromatography (eluent, hexane : ethyl acetate = 10 : 1 - 1 : 2) to yield 0.15 g of methyl ester of 4-N-[trans-7-chloro-5-(2-chlorophenyl)-1-neopentyl-2-oxo-1,2,3,5-tetrahydro-4,1-benzoxazepine-3-methyl]aminomethylbenzoic acid as prisms, m.p. 171 to 173°C.

| Elementary analysis for $C_{30}H_{32}Cl_2N_2O_4$: | | | |
|---|---|---|---|
| Calcd. | C 64.87; | H 5.81; | N 5.04 |
| Found | C 64.88; | H 5.97; | N 4.76 |

(B) In 20 ml of ethanol was dissolved 0.45 g of trans-7-chloro-5-(2-chlorophenyl)-1-neopentyl-2-oxo-1,2,3,5-tetrahydro-4,1-benzoxazepine-3-methylamine hydrochloride obtained in Example 133. To the solution was added 0.19 g of terephthalaldehydic acid methyl ester together with 0.5 ml of acetic acid. After stirring for 30 minutes at room temperature, 82 mg of sodium cyanoborohydride was added to the mixture. After further stirring for 2 hours at room temperature, the mixture was concentrated under reduced pressure and the concentrate was extracted with 100 ml of water and 100 ml of ethyl acetate. The organic layer was washed with water and dried over anhydrous magnesium sulfate. The organic solvent was evaporated off under reduced pressure and the residue was purified by column chromatography using silica gel (eluent, hexane : ethyl acetate = 1 : 1) to yield 0.31 g of methyl ester of 4-N-[trans-7-chloro-5-(2-chlorophenyl)-1-neopentyl-2-oxo-1,2,3,5-tetrahydro-4,1-benzoxazepine-3-methyl] aminomethylbenzoic acid.

Example 145 (Reference)

Methyl ester of 4-[N-[trans-7-chloro-5-(2-chlorophenyl)-1-neopentyl-2-oxo-1,2,3,5-tetrahydro-4,1-benzoxazepine-3-methyl)-N-methanesulfonyl] aminomethylbenzoic acid

In 5 ml of N,N-dimethylformamide was dissolved 0.3 g of methyl ester of 4-N-[trans-7-chloro-5-(2-chlorophenyl)-1-neopentyl-2-oxo-1,2,3,5-tetrahydro-4,1-benzoxazepine-3-methyl]aminomethylbenzoic acid obtained in Example 150. To the solution were added 0.05 ml of methanesulfonyl chloride and 0.09 ml of triethylamine. After stirring for one hour at room temperature, the reaction mixture was subjected to extraction with 100 ml of water and 100 ml of ethyl acetate. The ethyl acetate layer was washed with 1N hydrochloric acid and with a sodium bicarbonate aqueous solution, dried over anhydrous magnesium sulfate, and concentrated under reduced pressure. The residue was purified by silica gel column chromatography (eluent, hexane : ethyl acetate = 2 : 1) to afford 0.27 g of methyl ester of 4-[N-[trans-7-chloro-5-(2- chlorophenyl)-1-neopentyl-2-oxo-1,2,3,5-tetrahydro-4,1-benzoxazepine-3-methyl]-N-methanesulfonyl] aminomethylbenzoic acid as needles. m.p. 173 to 174°C.

| Elementary analysis for $C_{31}H_{34}Cl_2N_2O_6S$: | | | |
|---|---|---|---|
| Calcd. | C 58.77; | H 5.41; | N 4.42 |
| Found | C 58.59; | H 5.68; | N 4.19 |

Example 146

4-[N-[trans-7-chloro-5-(2-chlorophenyl)-1-neopentyl-2-oxo-1,2,3,5-tetrahydro-4,1-benzoxazepine-3-methyl]-N-methanesulfonyl] aminomethylbenzoic acid

In 10 ml of methanol was dissolved 0.17 g of methyl ester of 4-[N-[trans-7-chloro-5-(2-chlorophenyl)-1-neopentyl-2-oxo-1,2,3,5-tetrahydro-4,1-benzoxazepine-3-methyl]-N-methanesulfonyl]aminomethylbenzoic acid obtained in Example 145. After the addition of 4 ml of 1N sodium hydroxide the solution was stirred for one hour at 60°C. The reaction mixture was then acidified with 50 ml of 1N hydrochloric acid and subjected to extraction with 50 ml of ethyl acetate. The ethyl acetate layer was washed with water, dried over anhydrous magnesium sulfate and concentrated under reduced pressure to give 0.16 g of 4-[N-[trans-7-chloro-5-(2-chlorophenyl)-1-neopentyl-2-oxo-1,2,3,5-tetrahydro-4,1-benzoxazepine-3-methyl]-N-methanesulfonyl]aminomethylbenzoic acid as prisms, m.p. 235 to 237°C.

| Elementary analysis for $C_{30}H_{32}Cl_2N_2O_6S$: | | | |
|---|---|---|---|
| Calcd. | C 58.16; | H 5.21; | N 4.52 |
| Found | C 58.25; | H 5.49; | N 4.29 |

Example 147

Trans-7-chloro-5-(2-chlorophenyl)-1-neopentyl-2-oxo-1,2,3,5-tetrahydro-4,1-benzoxazepine-3-ethanol

In 200 ml of tetrahydrofuran was dissolved 14.7 g of trans-7-chloro-5-(2-chlorophenyl)-1-neopentyl-2-oxo-1,2,3,5-tetrahydro-4,1-benzoxazepine-3-acetic acid obtained in Example 2 and 4.51 ml of N-methylmorpholine. To the solution was added 3.92 ml of ethyl chlorocarbonate at -10°C; the mixture was then stirred for 15 minutes. After the addition of 3.86 g of sodium borohydride, 200 ml of methanol was added dropwise to the solution. The mixture was stirred for one hour at room temperature and then concentrated. After the addition of 200 ml of 1N hydrochloric acid, the concentrate was extracted with 200 ml of ethyl acetate. The organic layer was washed with a saturated sodium bicarbonate aqueous solution and dried over anhydrous magnesium sulfate. The solvent was evaporated off under reduced pressure. The residue was purified by silica gel column chromatography (eluent, hexane : ethyl acetate = 1 : 1) to yield 14.2 g of trans-7-chloro-5-(2-chlorophenyl)-1-neopentyl-2-oxo-1,2,3,5-tetrahydro-4,1-benzoxazepine-3-ethanol as colorless crystals, m.p. 157 to 159 °C.

| Elementary analysis for $C_{22}H_{25}Cl_2NO_3$: | | | |
|---|---|---|---|
| Calcd. | C 62.56; | H 5.97; | N 3.32 |
| Found | C 62.30; | H 6.02; | N 3.17 |

Example 148

Ethyl ester of N-[trans-7-chloro-5-(2-chlorophenyl)-1-neopentyl-2-oxo-1,2,3,5-tetrahydro-4,1-benzoxazepine-3-ethyl] aminoacetic acid

(1) Trans-7-chloro-5-(2-chlorophenyl)-1-neopentyl-2-oxo-1,2,3,5-tetrahydro-4,1-benzoxazepine-3-acetaldehyde

To 25 ml of dichloromethane solution containing 0.70 ml of oxalyl chloride was added 5 ml of dichloromethane solution containing 0.71 ml of dimethylsulfoxide at -78°C, followed by stirring for 5 minutes. To the solution was slowly added 10 ml of dichloromethane solution containing 1.69 g of trans-7-chloro-5-(2-chlorophenyl)-1-neopentyl-2-oxo-1,2,3,5-tetrahydro-4,1-benzoxazepine-3-ethanol obtained in Example 154, followed by stirring for 15 minutes at-78°C. To the reaction mixture was added 2.79 ml of triethylamine and the mixture was further stirred for one hour at 0°C and for 1.5 hours at room temperature. After the addition of 100 ml of water, extraction was performed with 100 ml of dichloromethane. The dichloromethane layer was washed with saturated saline and dried over anhydrous magnesium sulfate. The solvent was evaporated off under reduced pressure. The residue was purified by column chromatography using silica gel (eluent, hexane : ethyl acetate = 1 : 1) to give 1.08 g of trans-7-chloro-5-(2-chlorophenyl)-1-neopentyl-2-oxo-1,2,3,5-tetrahydro-4,1-benzoxazepine-3-acetaldehyde as light-yellow crystals, m.p.173 to 176°C.

| Elementary analysis for $C_{22}H_{23}Cl_2NO_3 \cdot 0.5H_2O$: | | | |
|---|---|---|---|
| Calcd. | C 61.55; | H 5.63; | N 3.26 |
| Found | C 61.27; | H 5.49; | N 3.17 |

(2) Ethyl ester of N-[trans-7-chloro-5-(2-chlorophenyl)-1-neopentyl-2-oxo-1,2,3,5-tetrahydro-4,1-benzoxazepine-3-ethyl]aminoacetic acid

To 40 ml of ethanol containing 1.12 g of trans-7-chloro-5-(2-chlorophenyl)-1-neopentyl-2-oxo-1,2,3,5-tetrahydro-4,1-benzoxazepine-3-acetaldehyde obtained in Example 155 (1) and 0.56 g of glycine ethyl ester hydrochloride was added 10 ml of ethanol containing 0.13 g of sodium cyanoborohydride dropwise over 1.5 hours. After additional 3-hour stirring, the reaction mixture was subjected to extraction with 200 ml of water and 200 ml of ethyl acetate. The ethyl acetate layer was washed with saturated saline and dried over anhydrous magnesium sulfate. The solvent was evaporated off under reduced pressure and the residue was purified by silica gel column chromatography (eluent, ethyl acetate) to afford 0.75 g of amorphous solid ethyl ester of N-[trans-7-chloro-5-(2-chlorophenyl)-1-neopentyl-2-oxo-1,2,3,5-tetrahydro-4,1-benzoxazepine-3-ethyl]aminoacetic acid.

| Elementary analysis for $C_{26}H_{32}Cl_2N_2O_4$: | | | |
|---|---|---|---|
| Calcd. | C 61.54; | H 6.36; | N 5.52 |
| Found | C 61.16; | H 6.29; | N 5.56 |

$^1$H-NMR ($CDCl_3$) δ: 0.93 (9H, s, $Bu^t$), 1.26 (3H, t, J = 7.2 Hz), 1.90-2.15 (3H, m), 2.76 (2H, dd, J = 7.2, 6.6 Hz), 3.38 (1H, d, J = 13.9 Hz), 3.38 (2H, s), 4.03 (1H, dd, J = 6.6, 6.2 Hz), 4.17 (2H, q, J = 7.2 Hz), 4.51 (1H, d, J = 13.9 Hz), 6.24 (1H, s), 6.51 (1H, d, J = 1.6 Hz), 7.30-7.50 (5H, m), 7.70-7.80 (1H, m)

Example 149

Ethyl ester of N-acetyl-N-[trans-7-chloro-5-(2-chlorophenyl)-1-neopentyl-2-oxo-1,2,3,5-tetrahydro-4,1-benzoxazepine-3-ethyl]aminoacetic acid

In 5 ml of N,N-dimethylformamide was dissolved 0.15 g of ethyl ester of N-[trans-7-chloro-5-(2-chlorophenyl)-1-neopentyl-2-oxo-1,2,3,5-tetrahydro-4,1-benzoxazepine-3-ethyl]aminoacetate obtained in Example 148. To the solution were added 0.031 ml of acetyl chloride and 0.050 ml of triethylamine under ice-cooling. After stirring for 30 minutes under ice-cooling, the reaction mixture was subjected to extraction with 40 ml of water and 40 ml of ethyl acetate. The ethyl acetate layer was washed with saturated saline and dried over anhydrous magnesium sulfate. The solvent was evaporated off under reduced pressure. The residue was purified by column chromatography using silica gel (eluent, hexane : ethyl acetate = 1 : 3) to give 0.15 g of amorphous solid ethyl ester of N-acetyl-N-[trans-7-chloro-5-(2-chlorophenyl)-1-neopentyl-2-oxo-1,2,3,5-tetrahydro-4,1-benzoxazepine-3-ethyl]aminoacetic acid.

| Elementary analysis for $C_{28}H_{34}Cl_2N_2O_5 \cdot 0.4H_2O$: | | | |
|---|---|---|---|
| Calcd. | C 60.41; | H 6.30; | N 5.03 |
| Found | C 60.44; | H 6.43; | N 4.96 |

$^1$H-NMR($CDCl_3$) δ: 0.93 and 0.94 (9H, each s, $Bu^t$), 1.20- 1.32 (3H, m), 1.95-2.20 (2H, m), 1.95 and 2.13 (total 3H, each s), 3.32-3.68 (3H, m), 3.90-4.27 (5H, m), 4.48 and 4.50 (total 1H, each d, J = 13.8 Hz), 6.22 and 6.24 (total 1H, each s), 6.50 and 6.53 (total 1H, each d, J = 2.0 Hz), 7.29-7.50 (5H, m), 7.65-7.80 (1H, m)

Example 150

N-acetyl-N-[trans-7-chloro-5-(2-chlorophenyl)-1-neopentyl-2-oxo-1,2,3,5-tetrahydro-4,1-benzoxazepine-3-ethyl] aminoacetic acid

To 5 ml of methanol containing 0.11 g of ethyl ester of N-acetyl-N-[trans-7-chloro-5-(2-chlorophenyl)-1-neopentyl-2-oxo-1,2,3,5-tetrahydro-4,1-benzoxazepine-3-ethyl]aminoacetate obtained in Example 149 was added 1 ml of 0.5M potassium carbonate aqueous solution. After stirring for 30 minutes at 60°C, the reaction mixture was acidified with 0.5 ml of 1N hydrochloric acid and subjected to extraction with 20 ml of water and 20 ml of ethyl acetate. The ethyl acetate layer was washed with saturated saline and dried over anhydrous magnesium sulfate. The solvent was evaporated off under reduced pressure. The residue was treated with ether to yield 0.07 g of white powdery N-acetyl-N-[trans-7-chloro-5-(2-chlorophenyl)-1-neopentyl-2-oxo-1,2,3,5-tetrahydro-4,1-benzoxazepine-3-ethyl]aminoacetic acid, m.p. 210 to 212°C.

| Elementary analysis for $C_{26}H_{30}Cl_2N_2O_5$: | | | |
|---|---|---|---|
| Calcd. | C 59.89; | H 5.80; | N 5.37 |
| Found | C 59.78; | H 5.85; | N 5.13 |

Example 151

Ethyl ester of N-(trans-7-chloro-5-(2-chlorophenyl)-1-neopentyl-2-oxo-1,2,3,5-tetrahydro-4,1-benzoxazepine-3-ethyl] -N-methanesulfonylaminoacetic acid

In 5 ml of N,N-dimethylformamide was dissolved 0.15 g of ethyl ester of N-[trans-7-chloro-5-(2-chlorophenyl)-1-neopentyl-2-oxo-1,2,3,5-tetrahydro-4,1-benzoxazepine-3-ethyl]aminoacetate obtained in Example 148. To the solution were added 0.028 ml of methanesulfonyl chloride and 0.050 ml of triethylamine under ice-cooling. After stirring for 30 minutes under ice-cooling, extraction was performed with 40 ml of water and 40 ml of ethyl acetate. The ethyl acetate layer was washed with saturated saline and dried over anhydrous magnesium sulfate. The solvent was evaporated off under reduced pressure, and the residue was purified on a silica gel column chromatography (eluent, hexane : ethyl acetate = 2 : 1) to afford 0.12 g of ethyl ester of N-trans-7-chloro-5-(2-chlorophenyl)-1-neopentyl-2-oxo-1,2,3,5-tetrahydro-4,1-benzoxazepine-3-ethyl]-N-methanesulphonylaminoacetic acid as a white powdery product, m.p. 134 to 135°C.

| Elementary analysis for $C_{27}H_{34}Cl_2N_2O_6S$: | | | |
|---|---|---|---|
| Calcd. | C 55.38; | H 5.85; | N 4.78 |
| Found | C 55.66; | H 5.98; | N 4.61 |

Example 152

N-[trans-7-chloro-5-(2-chlorophenyl)-1-neopentyl-2-oxo-1,2,3,5-tetrahydro-4,1-benzoxazepine-3-ethyl]-N-methanesulfonylaminoacetic acid

In the same manner as in Example 150 was hydrolyzed 0.12 g of ethyl ester of N-[trans-7-chloro-5-(2-chlorophenyl)-1-neopentyl-2-oxo-1,2,3,5-tetrahydro-4,1-benzoxazepine-3-ethyl]-N-methanesulfonylaminoacetic acid obtained in Example 157 to give 0.10 g of white powdery N-[trans-7-chloro-5-(2-chlorophenyl)-1-neopentyl-2-oxo-1,2,3,5-tetrahydro-4,1-benzoxazepine-3-ethyl]-N-methanesulfonylaminoacetic acid, m.p. 135 to 137°C.

| Elementary analysis for $C_{25}H_{30}Cl_2N_2O_6S \cdot 0.5H_2O$: | | | |
|---|---|---|---|
| Calcd. | C 53.00; | H 5.52; | N 4.94 |
| Found | C 53.25; | H 5.78; | N 4.71 |

Example 153

Ethyl ester of N-[trans-7-chloro-5-(2-chlorophenyl)-1-neopentyl-2-oxo-1,2,3,5-tetrahydro-4,1-benzozazepine-3-ethyl]-N-(p-toluensulfonyl)aminoacetic acid

In 5 ml of N,N-dimethylformamide was dissolved 0.15 g of ethyl ester of N-[trans-7-chloro-5-(2-chlorophenyl)-1-neopentyl-2-oxo-1,2,3,5-tetrahydro-4,1-benzoxazepine-3-ethyl]aminoacetic acid obtained in Example 148. To the solution was added 0.069 g of p-toluenesulfonyl chloride together with 0.050 ml of triethylamine under ice-cooling. After stirring for 30 minutes under ice-cooling, the reaction mixture was subjected to extraction with 40 ml of water and 40 ml of ethyl acetate. The ethyl acetate layer was washed with saturated saline and dried over anhydrous magnesium sulfate. Ethyl acetate was evaporated off under reduced pressure. The residue was purified by column chromatography using silica gel (eluent, hexane : ethyl acetate = 3 : 1) to yield 0.17 g of amorphous ethyl ester of N-[trans-7-chloro-5-(2-chlorophenyl)-1-neopentyl-2-oxo-1,2,3,5-tetrahydro-4,1-benzoxazepine-3-ethyl]-N-p-toluenesulfonylaminoacetic acid.

| Elementary analysis for $C_{33}H_{38}Cl_2N_2O_6S$: | | | |
|---|---|---|---|
| Calcd. | C 59.91; | H 5.79; | N 4.23 |
| Found | C 60.25; | H 6.01; | N 4.04 |

$^1$H-NMR ($CDCl_3$) δ: 0.94 (9H, s, $Bu^t$), 1.15 (3H, t, J = 7.2 Hz), 1.90-2.30 (2H, m), 2.41 (3H, s), 3.20-3.60 (2H, m), 3.41 (1H, d, J = 140 Hz), 3.97-4.18 (5H, m), 4.50 (1H, d, J = 14.0 Hz), 6.23 (1H, s), 6.52 (1H, s), 7.21-7.50 (7H, m), 7.62-7.80 (3H, m)

Example 154

N-[trans-7-chloro-5-(2-chlorophenyl)-1-neopentyl-2-oxo-1,2,3,5-tetrahydro-4,1-benzoxazepine-3-ethyl]-N-p-toluensulfonylaminoacetic acid

In the same manner as in Example 150, 0.15 g of ethyl ester of N-[trans-7-chloro-5-(2-chlorophenyl)-1-neopentyl-2-oxo-1,2,3,5-tetrahydro-4,1-benzoxazepine-3-ethyl]-N-p-toluensulfonylaminoacetic acid was hydrolyzed to yield 0.11 g of N-[trans-7-chloro-5-(2-chlorophenyl)-1-neopentyl-2-oxo-1,2,3,5-tetrahydro-4,1-benzoxazepine-3-ethyl]-N-p-toluensulfonylaminoacetic acid as white powder, m.p. 274 to 277°C.

| Elementary analysis for $C_{31}H_{34}Cl_2N_2O_6S$: | | | |
|---|---|---|---|
| Calcd. | C 58.77; | H 5.41; | N 4.42 |
| Found | C 58.70; | H 5.59; | N 4.13 |

Example 155 (Reference)

Methyl ester of 4-N-[trans-7-chloro-5-(2-chlorophenyl)-1-neopentyl-2-oxo-1,2,3,5-tetrahydro-4,1-benzoxazepine-3-ethyl]aminomethylbenzoic acid

A mixture of 0.42 g of trans-7-chloro-5-(2-chlorophenyl)-1-neopentyl-2-oxo-1,2,3,5-tetrahydro-4,1-benzoxazepine-3-acetaldehyde obtained in Example 148 (1), 0.30 g of 4-aminomethylbenzoic acid methyl ester hydrochloric acid salt, and 30 ml of ethanol was stirred for 2 hours at room temperature. Subsequently 5 ml of ethanol containing 0.06 g of sodium cyanoborohydride was slowly added dropwise to the mixture. After stirring for 2 hours, the reaction mixture was subjected to extraction with 100 ml of water and 100 ml of ethyl acetate. The ethyl acetate layer was washed with saturated saline and dried over anhydrous magnesium sulfate. The organic solvent was evaporated off under reduced pressure. The residue was purified by column chromatography using silica gel (eluent, ethyl acetate) to give amorphous methyl ester of 4-N-[trans-7-chloro-5-(2-chlorophenyl)-1-neopentyl-2-oxo-1,2,3,5-tetrahydro-

4,1-benzoxazepine-3-ethyl] aminomethylbenzoic acid.

| Elementary analysis for $C_{31}H_{34}Cl_2N_2O_4$: | | | |
|---|---|---|---|
| Calcd. | C 65.38; | H 6.02; | N 4.92 |
| Found | C 64.99; | H 6.11; | N 5.19 |

$^1$H-NMR($CDCl_3$) δ: 0.94 (9H, s, $Bu^t$), 1.98-2.26 (3H, m), 2.65-2.90 (2H, m), 3.38 (1H, d, J = 14.0 Hz), 3.83 (2H, s), 3.91 (3H, s), 4.06 (1H, t, J = 6.2 Hz), 4.49 (1H, d, J = 14.0 Hz), 6.21 (1H, s), 5.51 (1H, d, J = 2.0 Hz), 7.26-7.62 (8H, m), 7.92-8.02 (2H, m)

Example 156 (Reference)

Methyl ester of 4-[N-acetyl-N-[trans-7-chloro-5-(2-chlorophenyl)-1-neopentyl-2-oxo-1,2,3,5-tetrahydro-4,1-benzoxazepine-3-ethyl]] aminomethylbenzoic acid

In the same manner as in Example 149, 0.13 g of methyl ester of 4-N-[trans-7-chloro-5-(2-chlorophenyl)-1-neopentyl-2-oxo-1,2,3,5-tetrahydro-4,1-benzoxazepine-3-ethyl]aminomethylbenzoic acid obtained in Example 155 was treated to yield 0.13 g of amorphous methyl ester of 4-[N-acetyl-N-[trans-7-chloro-5-(2-chlorophenyl)-1-neopentyl-2-oxo-1,2,3,5-tetrahydro-4,1-benzoxazepine-3-ethyl]]aminomethylbenzoic acid.

| Elementary analysis for $C_{33}H_{36}Cl_2N_2O_5$: | | | |
|---|---|---|---|
| Calcd. | C 64.81; | H 5.93; | N 4.58 |
| Found | C 65.14; | H 6.12; | N 4.11 |

$^1$H-NMR ($CDCl_3$) δ: 0.93 (9H, s, $Bu^t$), 2.00-2.20 (2H, m), 2.02 and 2.18 (total 3H, each s), 3.20-3.60 (3H, m), 3.78-4.00 (1H, m), 3.91 and 3.93 (total 3H, each s), 4.42-4.76 (3H, m), 6.21 (1H, s), 6.50 (1H, d, J = 1.8 Hz), 7.17-7.70 (8H, m), 7.95 (1H, d, J = 8.0 Hz), 8.02 (1H, d, J = 8.2 Hz)

Example 157

4-[N-acetyl-N-[trans-7-chloro-5-(2-chlorophenyl)-1-neopentyl-2-oxo-1,2,3,5-tetrahydro-4,1-benzoxazepine-3-ethyl]] aminomethylbenzoic acid

In the same manner as in Example 150, 0.10 g of methyl ester of 4-[N-acetyl-N-[trans-7-chloro-5-(2-chlorophenyl)-1-neopentyl-2-oxo-1,2,3,5-tetrahydro-4,1-benzoxazepine-3-ethyl]]aminomethylbenzoic acid obtained in Example 162 was hydrolyzed to afford 0.08 g of white powdery 4-[N-acetyl-N-[trans-7-chloro-5-(2-chlorophenyl)-1-neopentyl-2-oxo-1,2,3,5-tetrahydro-4,1-benzoxazepine-3-ethyl]]aminomethylbenzoic acid, m.p. 242 to 244°C.

| Elementary analysis for $C_{32}H_{34}Cl_2N_2O_5$: | | | |
|---|---|---|---|
| Calcd. | C 64.32; | H 5.74; | N 4.69 |
| Found | C 64.34; | H 5.87; | N 4.66 |

Example 158 (Reference)

Methyl ester of 4-(N-[trans-7-chloro-5-(2-chlorophenyl)-1-neopentyl-2-oxo-1,2,3,5-tetrahydro-4,1-benzoxazepine-3-ethyl]-N-methanesulfonyl] aminomethylbenzoic acid

In the same manner as in Example 151, 0.13 g of methyl ester of 4-N-[trans-7-chloro-5-(2-chlorophenyl)-1-neopentyl-2-oxo-1,2,3,5-tetrahydro-4,1-benzoxazepine-3-ethyl]aminomethylbenzoic acid obtained in Example 155 was treated to give 0.12 g of methyl ester of 4-[N-[trans-7-chloro-5-(2-chlorophenyl)-1-neopentyl-2-oxo-1,2,3,5-tetrahydro-4,1-benzoxazepine-3-ethyl]-N-methanesulfonyl]aminomethylbenzoic acid as colorless crystals, m.p. 172 to 174°C.

| Elementary analysis for $C_{32}H_{36}Cl_2N_2O_6S$: | | | |
|---|---|---|---|
| Calcd. | C 59.35; | H 5.60; | N 4.33 |

(continued)

| Elementary analysis for $C_{32}H_{36}Cl_2N_2O_6S$: | | | |
|---|---|---|---|
| Found | C 59.15; | H 5.79; | N 4.16 |

Example 159

4-[N-[trans-7-chloro-5-(2-chlorophenyl)-1-neopentyl-2-oxo-1,2,3,5-tetrahydro-4,1-benzoxazepine-3-ethyl]-N-methanesulfonyl]aminomethylbenzoic acid

In the same manner as in Example 150, 0.09 g of methyl ester of 4-[N-[trans-7-chloro-5-(2-chlorophenyl)-1-neopentyl-2-oxo-1,2,3,5-tetrahydro-4,1-benzoxazepine-3-ethyl]-N-methanesulfonyl]aminomethylbenzoic acid obtained in Example 164 was hydrolyzed to afford 0.06 g of 4-[N-[trans-7-chloro-5-(2-chlorophenyl)-1-neopentyl-2-oxo-1,2,3,5-tetrahydro-4,1-benzoxazepine-3-ethyl]-N-methanesulfonyl]aminomethylbenzoic acid as colorless crystals, m.p. 185 to 187°C.

| Elementary analysis for $C_{31}H_{34}Cl_2N_2O_6S \cdot 0.5H_2O$: | | | |
|---|---|---|---|
| Calcd. | C 57.95; | H 5.49; | N 4.36 |
| Found | C 58.30; | H 5.83; | N 4.19 |

Example 160 (Reference)

Methyl ester of 4-[N-[trans-7-chloro-5-(2-chlorophenyl)-1-neopentyl-2-oxo-1,2,3,5-tetrahydro-4,1-benzoxazepine-3-ethyl]-N-p-toluenesulfonyl] aminomethylbenzoic acid

In the same manner as in Example 153, 0.13 g of methyl ester of 4-N-[trans-7-chloro-5-(2-chlorophenyl)-1-neopentyl-2-oxo-1,2,3,5-tetrahydro-4,1-benzoxazepine-3-ethyl]aminomethylbenzoic acid obtained in Example 155 was treated to give 0.14 g of methyl ester of 4-[N-[trans-7-chloro-5-(2-chlorophenyl)-1-neopentyl-2-oxo-1,2,3,5-tetrahydro-4,1-benzoxazepine-3-ethyl]-N-p-toluenesulfonyl]aminomethylbenzoic acid as colorless crystals, m.p. 145 to 147°C.

| Elementary analysis for $C_{38}H_{40}Cl_2N_2O_6S$: | | | |
|---|---|---|---|
| Calcd. | C 63.07; | H 5.57; | N 3.87 |
| Found | C 63.09; | H 5.50; | N 3.93 |

Example 161

4-[N-[trans-7-chloro-5-(2-chlorophenyl)-1-neopentyl-2-oxo-1,2,3,5-tetrahydro-4,1-benzoxazepine-3-ethyl]-N-p-toluenesulfonyl] aminomethylbenzoic acid

In the same manner as in Example 150, 0.12 g of methyl ester of 4-[N-[trans-7-chloro-5-(2-chlorophenyl)-1-neopentyl-2-oxo-1,2,3,5-tetrahydro-4,1-benzoxazepine-3-ethyl]-N-p-toluenesulfonyl]aminomethylbenzoic acid obtained in Example 160 was hydrolyzed to afford 0.09 g of 4-[N-[trans-7-chloro-5-(2-chlorophenyl)-1-neopentyl-2-oxo-1,2,3,5-tetrahydro-4,1-benzoxazepine-3-ethyl]-N-p-toluenesulfonyl]aminomethylbenzoic acid as colorless powder, m.p. 265 to 268°C.

| Elementary analysis for $C_{37}H_{38}Cl_2N_2O_6S$: | | | |
|---|---|---|---|
| Calcd. | C 62.62; | H 5.40; | N 3.95 |
| Found | C 62.67; | H 5.36; | N 3.96 |

Example 162

Trans-7-chloro-5-(2-chlorophenyl)-1-neopentyl-3-[(tetrazol-5-yl)methylaminocarbonylmethyl]-2-oxo-1,2,3,5-tetrahydro-4,1-benzoxazepine

In the same manner as in Examples 98, trans-7-chloro-5-(2-chlorophenyl)-1-neopentyl-2-oxo-1,2,3,5-tetrahydro-4,1-benzoxazepine-3-acetic acid obtained in Example 2 was treated to afford the following crystalline compounds:

(1) Trans-7-chloro-5-(2-chlorophenyl)-3-(cyanomethylaminocarbonylmethyl)-1-neopentyl-2-oxo-1,2,3,5-tetrahydro-4,1-benzoxazepine, m.p. 171 to 172°C.

| Elementary analysis for $C_{24}H_{25}Cl_2N_3O_3$: | | | |
|---|---|---|---|
| Calcd. | C 60.77; | H 5.31; | N 8.86 |
| Found | C 60.68; | H 5.24; | N 8.62 |

(2) Trans-7-chloro-5-(2-chlorophenyl)-1-neopentyl-3-[(tetrazol-5-yl)methylaminocarbonylmethyl]-2-oxo-1,2,3,5-tetrahydro-4,1-benzoxazepine, m.p. 251 to 252°C.

| Elementary analysis for $C_{24}H_{26}Cl_2N_6O_3$: | | | |
|---|---|---|---|
| Calcd. | C 55.71; | H 5.06; | N 16.24 |
| Found | C 55.38; | H 5.22; | N 16.03 |

Materials and intermediate compounds included in Examples are listed in Tables 74 to 88.
2-aminobenzophenones as the starting material can be synthesized by, or in accordance with, the method described in D.A. Walsh, Synthesis, 677 (1980) or the method cited in said reference.

Table 70

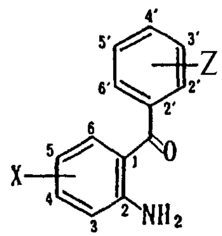

| X | Z | m.p. (℃) | Formula | Elemental Analysis(Found) C | H | N |
|---|---|---|---|---|---|---|
| 5-F | 2'-Cl | | subjected to the next procedure without isolating | | | |
| 5-MeO | 2'-Cl | oil | $^1$H-NMR($CDCl_3$)δ:3.59(3H, s), 5.88(2H, br), 6.51-7.52(7H, m) | | | |
| 5-Cl | 2',4'-Cl | 81- 84 | $C_{13}H_8Cl_3NO$ | 51.95 (51.98 | 2.68 2.51 | 4.66 4.83) |
| 5-Cl | 2',4'-OMe | 102-103 | $C_{15}H_{14}ClNO_3$ | 61.76 (61.79 | 4.84 4.88 | 4.80 4.72) |
| 5-Cl | 2',6'-OMe | 172 | $C_{15}H_{14}ClNO_3$ | 61.76 (61.54 | 4.84 4.72 | 4.80 4.87) |

Table 71

| X | Z | m.p. (℃) | Formula | Elemental Analysis(Found) C | H | N |
|---|---|---|---|---|---|---|
| 5-Cl | 2',5'-OMe | oil | $^1H$-NMR($CDCl_3$)δ:3.72(3H, s), 3.78(3H, s), 6.39(2H, br s), 6.64 (1H, d, J=8.6Hz), 6.80(1H, d, J=2.6Hz), 6.87-7.28(4H, m) | | | |
| 5-Cl | 2',4',6'-OMe | 188-189 | $C_{16}H_{16}ClNO_4$ | 59.73<br>(59.93 | 5.01<br>5.00 | 4.35<br>4.30) |
| 5-Cl | 2',3'-OCH$_2$O- | 119-120 | $C_{14}H_{10}ClNO_3$ | 60.99<br>(60.81 | 3.66<br>3.43 | 5.08<br>5.07) |
| 5-Cl | 3',4'-OCH$_2$O- | oil | $^1H$-NMR($CDCl_3$)δ:6.06(2H, s), 6.67(1H, d), 6.86(1H, d), 7.15-7.5(4H, m) | | | |
| 4-Cl | 2'-Cl | 112-113 | $C_{13}H_9Cl_2NO$ | 58.67<br>(58.47 | 3.41<br>3.45 | 5.26<br>5.16) |
| 3,5-Cl | 2'-OMe | 88- 90 | $C_{14}H_{11}Cl_2NO$ | 60.02<br>(59.96 | 3.96<br>3.85 | 5.00<br>4.96) |
| 5-Cl | 2',3'-OMe | 91- 92 | $C_{15}H_{14}ClNO_3$ | 61.76<br>(61.59 | 4.84<br>4.87 | 4.80<br>4.79) |

Table 72

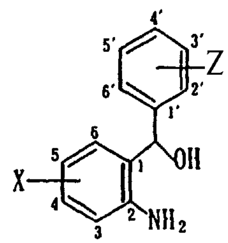

| X | Z | m.p. (℃) | Formula | Elemental Analysis(Found) C | H | N |
|---|---|---|---|---|---|---|
| 5-F | 2'-Cl | 77-79 | $C_{13}H_{11}ClFNO$ | 62.04 (62.10 | 4.41 4.40 | 5.57 5.57) |
| 5-Cl | 2',4'-Cl | 104-106 | $C_{13}H_{10}Cl_3NO$ +0.7$H_2O$ | 49.54 (49.59 | 3.65 3.48 | 4.44 4.34) |
| 5-Cl | 2',4'-OMe | oil | $^1$H-NMR($CDCl_3$)δ:3.81(3H, s), 3.85(3H, s), 5.94(1H, s), 6.4-6.7 (3H, m), 6.95-7.1(3H, m) | | | |

Table 73

| X | Z | m.p. (°C) | Formula | Elemental Analysis(Found) C | H | N |
|---|---|---|---|---|---|---|
| 5-Cl | 2',6'-OMe | 130-135 | $C_{15}H_{16}ClNO_3$ | 61.33 (61.26 | 5.49 5.46 | 4.77 4.71) |
| 5-Cl | 2',5'-OMe | 177-178 | $C_{15}H_{16}ClNO_3$ | 61.33 (61.22 | 5.49 5.54 | 4.77 4.72) |
| 5-Cl | 2',4',6'-OMe | 163-164 | $C_{16}H_{18}ClNO_4$ | 59.35 (59.57 | 5.60 5.63 | 4.33 4.39) |
| 5-Cl | 2',3'-OCH$_2$O- | 119-120 | $C_{14}H_{12}ClNO_3$ | 60.55 (60.49 | 4.36 4.20 | 5.04 5.10) |
| 5-Cl | 3',4'-OCH$_2$O- | 136-137 | $C_{14}H_{12}ClNO_3$ | 60.55 (60.41 | 4.36 4.48 | 5.04 4.96) |
| 4-Cl | 2'-Cl | oil | $^1$H-NMR($CDCl_3$)δ:6.06(1H, s), 6.55-6.75(3H, m), 7.2-7.5(5H, m) | | | |
| 5-Cl | 2',3'-OMe | 124-126 | $C_{15}H_{16}ClNO_3$ | 61.33 (61.25 | 5.49 5.58 | 4.77 4.66) |
| 3,5-Cl | 2'-OMe | 84- 86 | $C_{14}H_{13}Cl_2NO_2$ | 56.40 (56.06 | 4.39 4.35 | 4.70 4.59) |
| H | 2'-Cl | oil | $^1$H-NMR($CDCl_3$)δ:5.30(1H, s), 6.17(1H, s), 6.5-7.6(7H, m) | | | |

Table 74

| X | Z | m.p. (℃) | Formula | Elemental Analysis(Found) C | H | N |
|---|---|---|---|---|---|---|
| 5-Cl | 2'-F | 100-101 | $C_{13}H_{11}ClFNO$ | 62.03<br>(61.86 | 4.41<br>4.62 | 5.57<br>5.51) |
| H | 2'-F | 106-107 | $C_{13}H_{12}FNO$ | 71.87<br>(71.76 | 5.57<br>5.59 | 6.45<br>6.36) |
| H | 2'-OMe | 105-106 | $C_{14}H_{15}NO_2$ | 73.34<br>(73.11 | 6.59<br>6.63 | 6.11<br>6.19) |
| 5-Cl | 2'-CF$_3$ | 127-129 | $C_{14}H_{11}ClF_3NO$ | 55.74<br>(55.66 | 3.68<br>3.64 | 4.64<br>4.70) |
| 5-Cl | 2'-OMe | 81- 82 | $C_{14}H_{14}ClNO_2$ | 63.76<br>(63.83 | 5.35<br>5.56 | 5.31<br>5.32) |
| 5-Cl | 2'-Br | 87- 88 | $C_{13}H_{11}BrClNO$ | 49.95<br>(50.03 | 3.55<br>3.71 | 4.48<br>4.44) |
| 5-Br | 2'-Cl | 97- 99 | $C_{13}H_{11}BrClNO$ | 49.95<br>(49.87 | 3.55<br>3.76 | 4.48<br>4.37) |
| 5-Cl | 4'-Cl | 127-128 | $C_{13}H_{11}Cl_2NO$ | 58.23<br>(58.47 | 4.13<br>4.17 | 5.22<br>5.19) |

Table 75

| X | Z | m.p. (°C) | Formula | Elemental Analysis(Found) C | H | N |
|---|---|---|---|---|---|---|
| H | 2'-Me | 110-111 | $C_{14}H_{15}NO$ | 78.84<br>(78.67 | 7.09<br>6.87 | 6.57<br>6.60) |
| 5-Cl | 3'-Cl | 136-138 | $C_{13}H_{11}Cl_2NO$ | 58.23<br>(58.36 | 4.13<br>4.23 | 5.22<br>5.14) |
| 5-Cl | H | 107-109 | $C_{13}H_{12}ClNO$ | 66.81<br>(66.80 | 5.18<br>5.19 | 5.99<br>5.97) |

Table 76

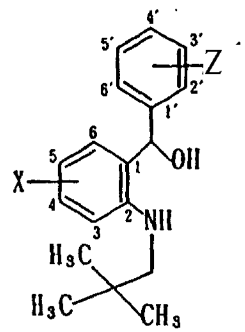

| X | Z | m. p. (℃) | Formula | Elemental Analysis(Found) C H N |
|---|---|---|---|---|
| 5-Cl | 2′, 4′-$OCH_3$ | oil | $C_{20}H_{26}ClNO_3$ | $^1$H-NMR($CDCl_3$)δ:0.92(9H, s, Bu$^t$), 2.82(2H, s), 2.9-3.3(1H, br), 3.81(3H, s), 3.86(3H, s), 4.6-5.0(1H, br), 5.92(1H, s), 6.4-6.6(3H, m), 6.95-7.2(3H, m) |
| 5-Cl | 2′, 6′-$OCH_3$ | oil | $C_{20}H_{26}ClNO_3$ | $^1$H-NMR($CDCl_3$)δ:1.06(9H, s), 2.87(1H, d, J=11.4Hz), 2.96(1H, d, J=11.4Hz), 3.80(6H, s), 4.28, 5.61(each 1H, br), 6.26(1H, br), 6.53(1H, d, J=2.6Hz), 6.60(1H, d, J=8.6Hz), 6.66(2H, d, J=8.4Hz), 7.09(1H, dd, J=2.4, 8.6Hz), 7.31(1H, t, J=8.4Hz) |
| 5-Cl | 2′, 5′-$OCH_3$ | 126-128 | $C_{20}H_{26}ClNO_3$ | 66.02 7.20 3.85<br>(66.18 7.18 3.83) |

Table 77

| X | Z | m. p. (℃) | Formula | Elemental Analysis(Found) C H N |
|---|---|---|---|---|
| 5-Cl | 2',4',6'-$OCH_3$ | 150-151 | $C_{21}H_{28}ClNO_4$ | 64.03 7.16 3.56<br>(64.19 7.00 3.48) |
| 5-Cl | 2',3'-$OCH_2O$- | | subjected to the next procedure without isolating | |
| 5-Cl | 3',4'-$OCH_2O$- | oil | $C_{19}H_{22}ClNO_3$ | $^1$H-NMR($CDCl_3$)δ:0.86(9H, s, Bu$^t$), 2.75 (2H, s), 5.67(1H, s), 5.95(2H, s), 6.5-7.3 (6H, m) |
| 4-Cl | 2'-Cl | oil | $C_{18}H_{21}Cl_2NO$ | $^1$H-NMR($CDCl_3$)δ:0.94(9H, s, Bu$^t$), 2.84 (2H, s), 6.12(1H, s), 6.5-7.5(7H, m) |
| 3,5-Cl | 2'-$OCH_3$ | oil | $C_{19}H_{23}Cl_2NO_2$ | $^1$H-NMR($CDCl_3$)δ:0.98(9H, s, Bu$^t$), 2.75 (2H, s), 3.84(3H, s), 4.05(1H, br s), 6.28 (1H, s), 6.9-7.4(6H, m) |
| 5-Cl | 2'-$OCH_3$ | oil | $C_{19}H_{24}ClNO_2$ | $^1$H-NMR($CDCl_3$)δ:0.92(9H, s, Bu$^t$), 2.83 (2H, s), 3.1-3.5(1H, br), 3.89(3H, s), 4.6-5.0(1H, br), 5.99(1H, s), 6.59(1H, d, J=8.8Hz), 6.9-7.4(6H, m) |
| 5-Cl | 2'-Br | 99-100 | $C_{18}H_{21}BrClNO$ | 56.49 5.53 3.66<br>(56.69 5.50 3.42) |
| 5-Br | 2'-Cl | 105-107 | $C_{18}H_{21}BrClNO$ | 56.49 5.53 3.66<br>(56.48 5.50 3.59) |
| 5-Cl | 2',3'-OMe | 120-121 | $C_{20}H_{26}ClNO_3$ | 66.02 7.20 3.85<br>(65.74 7.01 3.71) |

Table 78

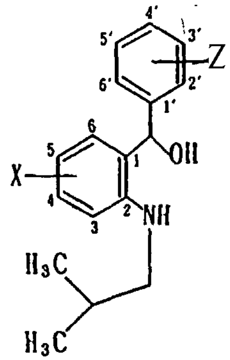

| X | Z | m.p. (°C) | Formula | Elemental Analysis(Found) C | H | N |
|---|---|---|---|---|---|---|
| 5-F | 2'-Cl | 84-86 | $C_{17}H_{19}ClFNO$ | 66.34 (66.21 | 6.22 6.22 | 4.55 4.50) |
| 5-Cl | 2',4'-Cl | 104-106 | $C_{17}H_{18}Cl_3NO$ | 56.93 (56.96 | 5.06 4.85 | 3.90 3.66) |
| 5-Cl | 2'-CF₃ | 72-74 | $C_{18}H_{19}ClF_3NO$ | 60.42 (59.96 | 5.35 5.37 | 3.91 3.83) |
| 5-Cl | 2'-OCH₃ | oil | $C_{18}H_{22}ClNO_2$ | $^1$H-NMR($CDCl_3$)δ:0.92(6H, d, J=6.6Hz), 1.75-2.0(1H, m), 2.89(1H, d, J=6.8Hz), 3.1-3.4(1H, Br), 3.88(3H, s), 4.7-5.1(1H, br), 5.97(1H, s), 6.56(1H, d, J=8.8Hz), 6.9-7.4(6H, m) | | |

Table 79

| X | Z | m.p. (℃) | Formula | Elemental Analysis(Found) C H N |
|---|---|---|---|---|
| 5-Cl | 2'-Br | 96-98 | $C_{17}H_{19}BrClNO$ | 55.38 5.19 3.80 (55.55 5.20 3.74) |
| 5-Br | 2'-Cl | oil | $C_{17}H_{19}BrClNO$ | $^1$H-NMR(CDCl$_3$)δ:0.91(6H, d, J=6.6Hz), 1.7-2.0(1H, m), 2.89(2H, d, J=6.6Hz), 6.11(1H, s), 6.53(1H, d, J=8.6Hz), 7.05(1H, d, J=2.4Hz), 7.14-7.46(5H, m) |
| 5-Cl | 4'-Cl | oil | $C_{17}H_{19}Cl_2NO$ | $^1$H-NMR(CDCl$_3$)δ:0.828(3H, d, J=6.8Hz), 0.834(3H, d, J=6.8Hz), 1.64-1.86(1H, m), 2.2-2.8(1H, br), 2.80(2H, d, J=6.8Hz), 4.2-4.7(1H, br), 5.73(1H, s), 6.54(1H, d, J=8.6Hz), 6.98(1H, d, J=2.4Hz), 7.14(1H, dd, J=8.6, 2.4Hz), 7.31(4H, s) |
| H | 2'-CH$_3$ | oil | $C_{18}H_{23}NO$ | $^1$H-NMR(CDCl$_3$)δ:0.95(6H, d, J=6.6Hz), 1.8-2.0(1H, m), 2.21(3H, s), 2.94(2H, d, J=6.6Hz), 5.98(1H, s), 6.5-6.8(2H, m), 7.1-7.35(3H, m), 7.1-7.35(3H, m), 7.4-7.55(1H, m) |
| 5-Cl | 3'-Cl | 73-74 | $C_{17}H_{19}Cl_2NO$ | 62.97 5.91 4.32 (62.81 5.88 4.19) |
| 5-Cl | H | 56-59 | $C_{17}H_{20}ClNO$ | 70.46 6.96 4.83 (70.45 7.11 4.90) |

**Table** 80

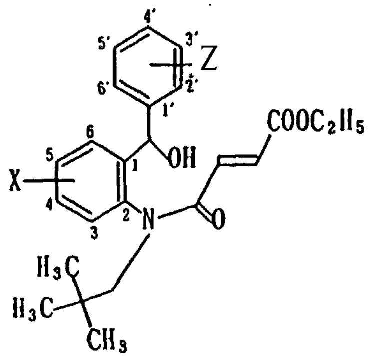

| X | Z | m. p. (℃) | Formula | Elemental Analysis(Found) C H N |
|---|---|---|---|---|
| 5-Cl | 2', 4'-$OCH_3$ | oil | $C_{26}H_{32}ClNO_6$ | $^1$H-NMR($CDCl_3$)δ: 0.82 and 0.93(9H, each s, Bu$^t$), 1.22(3H, t, J=7.2Hz), 2.35-3.1(2H, m), 3.65-3.9(6H, m), 4.0-4.5(3H, m), 5.9-6.55(5H, m), 6.75-7.9 (4H, m) |
| 5-Cl | 2', 6'-$OCH_3$ | oil | $C_{26}H_{32}NO_6Cl$ | $^1$H-NMR($CDCl_3$)δ: 0.90, 0.95(9H, each s, Bu$^t$), 1.19-1.30(3H, m), 2.88-3.10(1H, m), 3.72-3.82(6H, m), 4.04-4.28(2H, m), 4.44-4.57(1H, m), 6.02-7.88 (9H, m) |
| 5-Cl | 2', 5'-$OCH_3$ | 140-142 | $C_{26}H_{32}ClNO_6$ | 63.73 6.58 2.86<br>(63.67 6.50 2.81) |
| 5-Cl | 2', 4', 6'-$OCH_3$ | oil | $C_{27}H_{34}ClNO_7$ | $^1$H-NMR($CDCl_3$)δ: 0.90 and 0.94(9H, each s, Bu$^t$), 1.15-1.35(3H, m), 2.8-3.1(1H, m), 3.4-4.3(12H, m), 4.35-4.6(1H, m), 5.9-6.45(5H, m), 6.8-7.95(3H, m) |

Table 81

| X | Z | m.p. (℃) | Formula | Elemental Analysis(Found) C H N |
|---|---|---|---|---|
| 5-Cl | 2',3'-OCH$_2$O- | oil | $C_{25}H_{28}ClNO_6$ | $^1$H-NMR(CDCl$_3$)δ:0.92 and 1.07(9H, each s, Bu$^t$), 1.18-1.33(3H, m), 2.85-3.04(1H, m), 4.05-4.34(2H, m), 4.46-4.54(1H, m), 5.87(2H, s), 6.05-7.89(9H, m) |
| 5-Cl | 3',4'-OCH$_2$O- | 125-126 | $C_{25}H_{28}ClNO_6$ | 63.36 5.95 2.96<br>(63.26 5.72 2.76) |
| 4-Cl | 2'-Cl | 152-153 | $C_{24}H_{27}Cl_2NO_4$ | 62.07 5.86 3.02<br>(61.78 5.93 2.88) |
| 3,5-Cl | 2'-OCH$_3$ | 162-164 | $C_{25}H_{29}Cl_2NO_5$ | 60.73 5.91 2.83<br>(60.84 5.89 2.70) |
| 5-Cl | 2'-OCH$_3$ | oil | $C_{25}H_{30}ClNO_5$ | $^1$H-NMR(CDCl$_3$)δ:0.84 and 0.93(9H, each s, Bu$^t$), 1.15-1.3(2H, m), 2.5-3.2(2H, m), 3.73 and 3.82(3H, each s), 4.0-4.25(2H, m), 4.25-4.6(1H, m), 6.0-6.5(3H, m), 6.7-7.85(7H, m) |
| 5-Cl | 2'-Br | 168-169 | $C_{24}H_{27}BrClNO_4$ | 56.65 5.35 2.75<br>(56.71 5.21 2.47) |
| 5-Br | 2'-Cl | 171-174 | $C_{24}H_{27}BrClNO_4$ | 56.65 5.35 2.75<br>(56.68 5.39 2.68) |
| 5-Cl | 2',3'-OCH$_3$ | 137-139 | $C_{26}H_{32}ClNO_6$ | 63.73 6.58 2.86<br>(63.66 6.50 2.94) |

Table 82

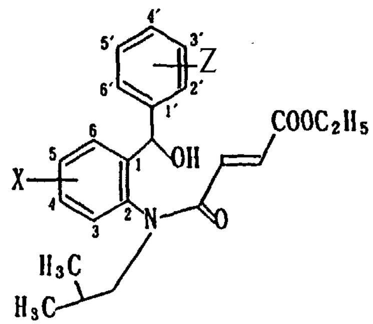

| X | Z | m. p. (℃) | Formula | Elemental Analysis(Found) C | H | N |
|---|---|---|---|---|---|---|
| 5-F | 2′-Cl | 148-151 | $C_{23}H_{25}ClFNO_4$ | 63.67 (63.56 | 5.81 5.73 | 3.23 3.38) |
| 5-Cl | 2′, 4′-Cl | 167-168 | $C_{23}H_{24}Cl_3NO_4$ | 56.98 (56.85 | 4.99 4.84 | 2.89 2.63) |
| 5-Cl | 2′-$CF_3$ | 164-165 | $C_{24}H_{25}ClF_3NO_4$ | 59.57 (59.55 | 5.21 5.22 | 2.89 2.83) |
| 5-Cl | 2′-$OCH_3$ | oil | $C_{24}H_{28}ClNO_5$ | $^1$H-NMR($CDCl_3$)δ: 0.7-1.15(6H, m), 1.15-1.4(3H, m), 1.6-2.1(1H, m), 2.4-3.1(2H, m), 3.76 and 3.80(3H, each s), 4.0-4.45(4H, m), 5.9-6.45(3H, m), 6.7-7.9(7H, m) | | |

Table 83

| X | Z | m.p. (℃) | Formula | Elemental Analysis(Found) C | H | N |
|---|---|---|---|---|---|---|
| 5-Cl | 2'-Br | 158-160 | $C_{23}H_{25}BrClNO_4$ | 55.83<br>(55.89 | 5.09<br>5.10 | 2.83<br>2.89) |
| 5-Br | 2'-Cl | 157-159 | $C_{23}H_{25}BrClNO_4$ | 55.83<br>(55.42 | 5.09<br>5.18 | 2.83<br>2.65) |
| 5-Cl | 4'-Cl | amorphous solid | $C_{23}H_{25}Cl_2NO_4$ +0.2$H_2O$ | 60.85<br>(60.85 | 5.64<br>5.62 | 3.09<br>3.00) |
| H | 2'-$CH_3$ | oil | $C_{24}H_{29}NO_4$ | $^1$H-NMR($CDCl_3$)δ: 0.7-1.1(6H, m), 1.21(3H, t, J=7.0Hz), 1.5-2.0(1H, m), 2.29(3H, s), 2.0-2.3 and 2.9-3.1(1H, each m), 3.9-4.5(3H, m), 5.9-6.4(2H, m), 6.6-6.9(1H, m), 6.95-7.9(8H, m) | | |
| 5-Cl | 3'-Cl | 105-108 | $C_{23}H_{25}Cl_2NO_4$ | 61.34<br>(61.37 | 5.60<br>5.53 | 3.11<br>3.08) |
| 5-Cl | H | 110-113 | $C_{23}H_{26}ClNO_4$ | 66.42<br>(66.05 | 6.30<br>6.29 | 3.37<br>3.35) |

Table 84

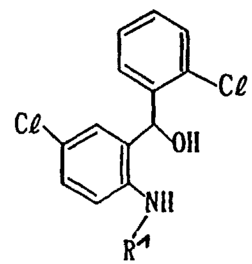

| $R^1$ | m.p. (℃) | Formula | Elemental Analysis(Found) C | H | N |
|---|---|---|---|---|---|
| $\overset{O}{\overset{\|}{C}}CF_2CF_3$ | 101-103 | $C_{16}H_{10}Cl_2F_5NO_2$ | 46.40 (46.28 | 2.43 2.42 | 3.38 3.47) |
| 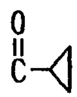 | 115-116 | $C_{17}H_{15}Cl_2NO_2$ | 60.73 (60.61 | 4.50 4.72 | 4.17 4.03) |
| $\overset{O}{\overset{\|}{C}}CH_2C(CH_3)_3$ | 109-110 | $C_{19}H_{21}Cl_2NO_2$ | 62.30 (62.57 | 5.78 5.79 | 3.82 3.73) |
| $\overset{O}{\overset{\|}{C}}CF_3$ | 155-156 | $C_{15}H_{10}Cl_2F_3NO_2$ | 49.47 (49.22 | 2.77 2.86 | 3.85 3.71) |

Experimental Example 1

Assay Method of Squalene Synthetase Inhibitory Activity

The squalene synthetase inhibitory activity was assayed as follows with the enzyme solutions described in the subsequent Experimental Examples 2 and 3.

More specifically, an enzyme solution (protein content 0.8 ug) prepared in Experimental Example 1 or 2 was added to a solution (total volume 50 ul)) containing 5 uM [1-$^3$H] farnesyl pyrophosphate (specific activity 25 uCi/mole), 1 mM NADPH, 5 mM $MgCl_2$, 6 mM glutathione, a 100 mM buffer solution of potassium phosphate (pH 7.4) and a test drug (used as an aqueous solution or a DMSO solution), then the reaction was allowed to proceed at 37°C for 45 minutes. To the reaciton mixture was added 150 ul of a mixture of chloroform and methanol (1:2) to suspend the reaction, followed by adding 50 ul of chloroform and 50 ul of a 3N aqueous solution of sodium hydroxide. 50 ul of the chloroform layer (lower layer) containing the reaction mixture having squalene as the principal component and 3 ml of toluene-based liquid scintillator were mixed, and its radioactivity was determined by means of a liquid scintillation counter.

The squalene synthetase inhibitory activity was expressed in terms of the concentration inhibiting by 50% the radioactivity taken into the chloroform layer ($IC_{50}$, molar concentration (M)).

Experimental Example 2

Preparation of rat-derived enzyme

An SD male rat (6 week old) was killed by bleeding, and its liver was excised. About 10 g of the liver was washed with a physiological saline solution cooled with ice, which was then homogenized in 15 ml of an ice-cooled buffer solution [100 mM potassium phosphate (pH 7.4), 15 mM nicotinamide, 2 mM $MgCl_2$], followed by centrifugation for 20 minutes (4°C) with 10000 Xg. The supernatant layer was separated and subjected to further centrifugation for 90 minutes (4°C) with 105000 Xg. The sediment was then suspended in an ice-cooled 100 mM phosphate buffer solution (pH 7.4), which was again subjected to centrifugation for 90 minutes (4°C) with 105000 Xg. The sediment thus obtained (microsome fraction) was suspended in an ice-cooled 100 mM potassium phosphate buffer (pH 7.4) (about 40 mg/ml protein concentration, determined with BCA protein assay kit of Pias Co., Ltd.). This suspension was used as the enzyme solution.

Experimental Example 3

Preparation of human-derived enzyme

Human hepatic carcinoma cells HepG2 (about 1 x $10^9$ cells) obtained by incubation on a Dulbecco-modified Eagle's medium (37°C in the presence of 5% $CO_2$) were suspended in 10 ml of an ice-cooled buffer solution [100 mM potassium phosphate buffer (pH 7.4), 30 mM nicotinamide and 2.5 mM $MgCl_2$]. The cells were crashed by means of ultrasonication (for 30 seconds, twice). From the sonicate thus obtained, the microsome fraction was obtained by the same procedure as in Experiment Example 1, which was suspended in an ice-cooled 100 mM potassium phosphate buffer (pH 7.4) (about 4 mg/ml protein concentration). This suspension was used as the enzyme solution. The results are shown in Table 74.

Table 85

| Compd. No. | | Rat Enzyme ($10^{-7}$M) | Man Enzyme ($10^{-7}$M) |
|---|---|---|---|
| Ref.Ex. | 5-1 | 83 | 68 |
| | -2 | 47 | 51 |
| | -3 | 40 | 38 |
| | -4 | 63 | 64 |
| | -7 | 5.4 | 7.1 |
| | -8 | 5.3 | - |
| | -12 | 2.3 | - |
| W. Ex. | 2-2 | 0.61 | 0.34 |
| | -4 | 0.72 | 0.24 |

Table 85 (continued)

| Compd. No. | | Rat Enzyme ($10^{-7}$M) | Man Enzyme ($10^{-7}$M) |
|---|---|---|---|
| | 32-2 | 49 | 38 |
| | 34-1 | 72 | 53 |
| | -5 | 43 | 43 |
| | -6 | 34 | 33 |
| | -11 | 26 | 21 |
| | -12 | 40 | 35 |
| | -15 | 0.35 | 0.12 |
| | -18 | 0.28 | 0.30 |
| | -20 | 0.94 | 0.28 |
| | 72-5 | 0.54 | 0.22 |
| | -9 | 0.49 | 0.40 |
| | -11 | 0.79 | 0.14 |
| | -12 | 0.38 | 0.18 |
| | 99 | 0.65 | 0.34 |
| | 108 | 0.43 | 0.34 |
| | 112(1) | 0.42 | 0.16 |
| | 114 | 0.16 | 0.06 |
| | 132 | 0.42 | 0.21 |
| | 162 | 0.67 | 0.18 |
| | 111 | >100 | - |

Experimental Example 4

Effects

The antifungal activities of the Compounds (I") were evaluated by the following method: a sheet of filter paper disc (manufactured by Toyo Seisakusho, 8 mm in diameter) soaked in a 1000µg/ml solution of a compound (I") in methanol was placed on an agar plate, which was incubated at 28°C for 2 days, and the diameter of the growth inhibition zone around the filter paper disc was measured. The following culture media were used:

A: yeast nitrogen base agar medium (pH 7.0)
B: peptone-yeast extract-glucose agar medium (pH 7.0)

The antifungal spectra of the compound (I") are shown in Table 87.
The below mentioned test fungi are deposited at the Institute for Fermentation, Osaka, Japan (IFO). Their accession numbers are shown in Table 90.

| Test fungi | Media | Inhibition zone diameter (mm) | | |
|---|---|---|---|---|
| | | Example 73-7 | Example 73-6 | Example 72-10 |
| Candida albicans IFO 0583 | A | 16 | 14 | 18 |
| Aspergillus niger IFO 4066 | A | 30 | 24 | 0 |
| Cryptococcus neoformans IFO 0410 | A | 19 | 13 | 14 |
| Trichophyton rubrum IFO 6467 | B | 16 | 13 | 20 |
| Trichophyton mentagrophytes IFO 7522 | B | 12 | 12 | 12 |
| Microsporum gypseum IFO 6076 | B | 13 | 12 | 13 |

Experimental Example 5

In vivo cholesterogenesis in the liver

Six-week-old, male Sprague-Dawley rats were given 5%-cholestylamine as a dietary admixture for 4 days. They were administered compound at a dose of 30 mg/kg as a suspension of 0.5%-methylcellulose solution and were intravenously given 2 uCi of [$^{14}$C]acetate 1 hour after the administration. One hour later, rats were sacrificed and livers were removed. Hepatic sterols were extracted with petroleum ether after saponification, and the radioactivity of digitonin-precipitable fraction was measured. Example 1-(4) inhibited the hepatic cholesterogenesis by 38% compared to the control group given only 5%-methylcellulose solution.

Formulation Examples

A squalene synthetase inhibiting agents containing, as its effective component, a 4,1-benzoxazepin-2-one derivative shown by the formula (I) of this invention or a salt thereof, in the case where it is used as a therapeutic agent of hypercholesteremia, can be formulated in accordance with, for example, the following prescriptions.

1. Capsules

| | |
|---|---|
| (1) Compound obtained in Example 27 | 10 mg |
| (2) Lactose | 90 mg |
| (3) Microcrystalline cellulose | 70 mg |
| (4) Magnesium stearate | 10 mg |
| One capsule | 180 mg |

(1), (2) and (3) and one half of (4) were blended and the the mixture was granulated, to which was added the balance of (4). The mixture was filled in a gelatin capsule.

2. Tablets

| | |
|---|---|
| (1) Compound obtained in Example 27 | 10 mg |
| (2) Lactose | 35 mg |
| (3) Corn starch | 150 mg |
| (4) Microcrystalline cellulose | 30 mg |
| (5) Magnesium stearate | 5 mg |
| One tablet | 230 mg |

(1), (2) and (3) and two thirds of (4) and one half of (5) were blended and the mixture was granulated, to which were added the balance of (4) and (5). The mixture was subjected to compression-molding to provide tablets.

3. Injections

| | |
|---|---|
| (1) Sodium salt of the compound obtained in Example 27 | 10 mg |
| (2) Inositol | 100 mg |
| (3) Benzyl alcohol | 20 mg |
| One ampoule | 130 mg |

(1), (2) and (3) were dissolved in distilled water for injection to make the whole volume 2 ml, which was put in an ampoule, and the ampoule was sealed. All the processes were conducted under sterilized conditions.

4. Capsules

| | |
|---|---|
| (1) Compound obtained in Example 1-4 | 10 mg |
| (2) Lactose | 90 mg |
| (3) Microcrystalline cellulose | 70 mg |
| (4) Magnesium stearate | 10 mg |
| One capsule | 180 mg |

(1), (2) and (3) and one half of (4) were blended and the the mixture was granulated, to which was added the balance of (4). The mixture was filled in a gelatin capsule.

5. Tablets

| | |
|---|---|
| (1) Compound obtained in Example 1-4 | 10 mg |
| (2) Lactose | 35 mg |
| (3) Corn starch | 150 mg |
| (4) Microcrystalline cellulose | 30 mg |
| (5) Magnesium stearate | 5 mg |
| One tablet | 230 mg |

(1), (2) and (3) and two thirds of (4) and one half of (5) were blended and the mixture was granulated, to which were added the balance of (4) and (5). The mixture was subjected to compression-molding to provide tablets.

6. Injections

| | |
|---|---|
| (1) Sodium salt of the compound obtained in Example 1-4 | 10 mg |
| (2) Inositol | 100 mg |
| (3) Benzyl alcohol | 20 mg |
| One ampoule | 130 mg |

(1), (2) and (3) were dissolved in distilled water for injection to make the whole volume 2 ml, which was put in an ampoule, and the ampoule was sealed. All the processes were conducted under sterilized conditions.

## Claims

**1.** A 4,1-Benzoxazepin-2-one derivative represented by the formula (I):

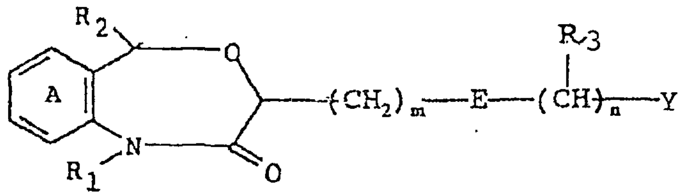

(I)

wherein

$R_1$ is a $C_{1-7}$ alkyl group,

which is unsubstituted or substituted with phenyl, an amino groups a thiol group, a hydroxyl group or halogen;

$R_2$ is

(i) hydrogen;

(ii) a phenyl group unsubstituted or substituted by 1 to 3 substituent(s) selected from among

(1) halogen atoms,

(2) $C_{1-4}$ alkyl groups unsubstituted or substituted with 1 to 3 halogen atoms,

(3) $C_{1-4}$ alkoxy groups unsubstituted or substituted with 1 to 3 halogen atoms,

(4) hydroxyl groups unsubstituted or substituted with benzyl, in which the two adjoining substituents on the phenyl group may cooperate therewith to form a ring of methylenedioxy;

(iii) thienyl, and pyridyl;

m is 0, 1 or 2;

n is 0, 1, 2 or 3;

E stands for a bond or an oxygen atom, sulfur atom, sulfone,

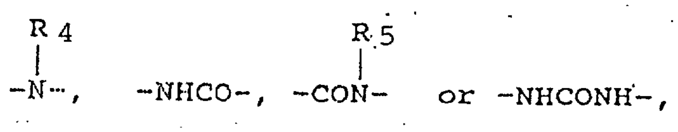

wherein

$R_3$ and $R_5$ independently stand for

(i) a hydrogen atom;

(ii) a $C_{1-6}$ alkyl group unsubstituted or substituted with indolyl;

(iii) benzyl;

(iv) a phenyl group;

$R_4$ stands for a hydrogen atom, a $C_{1-4}$ alkyl, a $C_{1-6}$ alkanoyl group, a $C_{1-3}$ alkanesulfonyl group, or p-toluenesulfonyl;

Y is

(i) an optionally esterified carboxyl group selected from

(a) a carboxyl group, or

(b) $C_{1-5}$ alkoxycarbonyl groups,

wherein the group (b) may have a carboxyl group as substituent(s);

(ii) a hydroxyl group unsubstituted or substituted with a $C_{1-4}$ alkyl group, or benzyl;

(iii) an amino group, unsubstituted or substituted with

(1) $C_{1-4}$ alkyl group(s) or

(2) $C_{3-6}$ cycloalkyl(s),

in which the two substituents on the nitrogen atom may form, taken together with the nitrogen atom, a cyclic amino group of 1-pyrrolidinyl or 1-piperazinyl optionally substituted by 4-halophenyl;

(iv) a phenyl group unsubstituted or substituted with substituents selected from among

(1) $C_{1-4}$ alkyl groups unsubstituted or substituted with a carboxyl group,

(2) a carboxyl group,

(3) phenyl groups unsubstituted or substituted with substituent(s) selected from a carboxyl group, and

(4) tetrazol-5-yl;

(v) a carbamoyl group unsubstituted or substituted with 1 to 2 substituent(s) selected from among (1) $C_{1-4}$ alkyl groups, benzyl or tetrazol-5-yl, or wherein two substituents on the nitrogen atom cooperate therewith to form the cyclic amino group 1-pyrrolidinyl, 1-piperazinyl or 1-piperazinyl having a $C_{1-3}$ alkyl substituent
(vi) tetrazol-5-yl or 2,5-dihydro-5-oxo-1,2,4-oxadiazol-3-yl;

provided that, when m is 1, E stands for a bond, n is 0, and Y is a carboxyl group, or an alkoxycarbonyl group, then $R_1$ is a $C_{5-7}$ alkyl group; and

ring A being optionally substituted by one to three substituents selected from halogen atoms, or

a pharmaceutically acceptable salt thereof.

**2.** A 4,1-Benzoxazepin-2-one derivative according to claim 1, wherein $R_1$ stands for a $C_{1-7}$ alkyl group.

**3.** A 4,1-Benzoxazepin-2-one derivative according to claim 1, wherein $R_1$ stands for a $C_{4-5}$ alkyl group.

**4.** A 4,1-Benzoxazepin-2-one derivative according to claim 1, wherein $R_2$ stands for an optionally substituted phenyl group as defined in claim 1.

**5.** A 4,1-Benzoxazepin-2-one derivative according to claim 1, wherein Y stands for an optionally esterified carboxyl group or tetrazol-5-yl or 2,5-dihydro-5-oxo-1,2,4-oxadiazol-3-yl as defined claim 1.

**6.** A 4,1-Benzoxazepin-2-one derivative as defined in claim 1 and according to any one of claims 1 to 5, with the proviso that when m is 1, E stands for a bond and n is 0, Y is neither a carboxyl group nor an alkoxycarbonyl group.

**7.** A pharmaceutical composition for inhibiting a squalene synthetase which comprises a therapeutically effective amount of a 4,1-Benzoxazepin-2-one derivative according to claims 1-6 or a salt thereof, and a pharmaceutical acceptable carrier.

**8.** A pharmaceutical composition for inhibiting a fungal growth which comprises a therapeutically effective amount of a 4,1-Benzoxazepin-2-one derivative according to claims 1-6 or a salt thereof, and a pharmaceutical acceptable carrier.

## Patentansprüche

**1.** 4,1-Benzoxazepin-2-onderivat dargestellt durch die Formel (I):

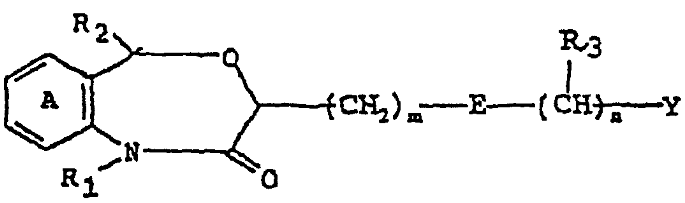

(I)

worin

$R_1$ eine $C_{1-7}$-Alkylgruppe ist, die unsubstituiert oder mit Phenyl, einer Aminogruppe, einer Thiolgruppe, einer Hydroxygruppe oder Halogen substituiert ist;

$R_2$ (i) Wasserstoff,
(ii) eine Phenylgruppe, die unsubstituiert oder durch 1 bis 3 Substituenten substituiert ist, die aus

(1) Halogenatomen,
(2) unsubstituierten oder mit 1 bis 3 Halogenatomen substituierten $C_{1-4}$-Alkylgruppen,

(3) unsubstituierten oder mit 1 bis 3 Halogenatomen substituierten $C_{1-4}$-Alkoxygruppen,
(4) unsubstituierten oder mit Benzyl, bei dem zwei benachbarte Substituenten an der Phenylgruppe unter Bilden eines Methylendioxyringes verbunden sein können, substituierten Hydroxygruppen ausgewählt sind,

(iii) Thienyl und Pyridyl ist;

m 0, 1 oder 2 ist;

n 0, 1, 2 oder 3 ist;

E für eine Bindung oder ein Sauerstoffatom, Schwefelatom, Sulfon,

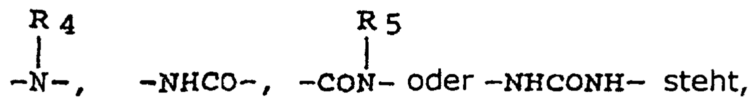
oder steht,

worin

$R_3$ und $R_5$ unabhängig für

(i) ein Wasserstoffatom,
(ii) eine unsubstituierte oder mit Indolyl substituierte $C_{1-6}$-Alkylgruppe,
(iii) Benzyl,
(iv) eine Phenylgruppe stehen;

$R_4$ für ein Wasserstoffatom, eine $C_{1-4}$-Alkyl-, eine $C_{1-6}$-Alkanoylgruppe, eine $C_{1-3}$-Alkansulfonylgruppe oder p-Toluolsulfonyl steht;

Y (i) eine gegebenenfalls veresterte Carboxygruppe ausgewählt aus

(a) einer Carboxygruppe oder
(b) $C_{1-5}$-Alkoxycarbonylgruppen, wobei die Gruppe (b) eine Carboxygruppe als Substituent (en) aufweisen kann,

(ii) eine unsubstituierte oder mit einer $C_{1-4}$-Alkylgruppe oder Benzyl substituierte Hydroxygruppe,
(iii) eine Aminogruppe, die unsubstituiert oder mit

(1) $C_{1-4}$-Alkylgruppe(n) oder
(2) $C_{3-6}$-Cycloalkyl substituiert ist, wobei zwei Substituenten an dem Stickstoffatom mit dem Stickstoffatom zusammengenommen die cyclische Aminogruppe 1-Pyrrolidinyl oder 1-Piperazinyl, das gegebenenfalls durch 4-Halogenphenyl substituiert ist, bilden können;

(iv) eine Phenylgruppe, die unsubstituiert oder mit Substituenten substituiert ist, die aus

(1) unsubstituierten oder mit einer Carboxygruppe substituierten $C_{1-4}$-Alkylgruppen,
(2) einer Carboxygruppe,
(3) Phenylgruppen, die unsubstituiert oder mit (einem) aus Carboxygruppe ausgewählten Substituenten substituiert sind, und
(4) Tetrazol-5-yl ausgewählt sind;

(v) eine Carbamoylgruppe, die unsubstituiert oder mit 1 oder 2 Substituenten substituiert ist, die aus (1) $C_{1-4}$-Alkylgruppen, Benzyl oder Tetrazol-5-yl ausgewählt sind, oder wobei zwei Substituenten an dem Stickstoffatom unter Bilden der cyclischen Aminogruppe 1-Pyrrolidinyl, 1-Piperazinyl oder 1-Piperazinyl mit einem $C_{1-3}$-Alkylsubstituenten verbunden sein können,

(vi) Tetrazol-5-yl oder 2,5-Dihydro-5-oxo-1,2,4-oxadiazol-3-yl ist,

vorausgesetzt, daß wenn m 1 ist, E für eine Bindung steht, n 0 ist und Y eine Carboxygruppe oder eine Alkoxycarbonylgruppe ist, $R_1$ dann eine $C_{5-7}$-Alkylgruppe ist und

Ring A gegebenenfalls durch einen bis drei aus Halogenatomen ausgewählte Substituenten substituiert ist oder

ein pharmazeutisch annehmbares Salz davon.

**2.** 4,1-Benzoxazepin-2-onderivat gemäß Anspruch 1, wobei $R_1$ für eine $C_{1-7}$-Alkylgruppe steht.

**3.** 4,1-Benzoxazepin-2-onderivat gemäß Anspruch 1, wobei $R_1$ für eine $C_{4-5}$-Alkylgruppe steht.

**4.** 4,1-Benzoxazepin-2-onderivat gemäß Anspruch 1, wobei $R_2$ für eine gegebenenfalls substituierte Phenylgruppe wie in Anspruch 1 definiert steht.

**5.** 4,1-Benzoxazepin-2-onderivat gemäß Anspruch 1, wobei Y für eine gegebenenfalls veresterte Carboxygruppe oder Tetrazol-5-yl oder 2,5-Dihydro-5-oxo-1,2,4-oxadiazol-3-yl wie in Anspruch 1 definiert steht.

**6.** 4,1-Benzoxazepin-2-onderivat wie in Anspruch 1 definiert und gemäß einem der Ansprüche 1 bis 5, mit der Maßgabe, daß wenn m 1 ist, E für eine Bindung steht und n 0 ist, Y weder eine Carboxygruppe noch eine Alkoxycarbonylgruppe ist.

**7.** Pharmazeutische Zusammensetzung zum Hemmen einer Squalensynthetase, die eine therapeutisch wirksame Menge eines 4,1-Benzoxazepin-2-onderivats gemäß Anspruch 1-6 oder ein Salz davon und einen pharmazeutisch annehmbaren Träger umfaßt.

**8.** Pharmazeutische Zusammensetzung zum Hemmen des Pilzwachstums, die eine therapeutisch wirksame Menge eines 4,1-Benzoxazepin-2-onderivats gemäß Anspruch 1-6 oder ein Salz davon und einen pharmazeutisch annehmbaren Träger umfaßt.

## Revendications

**1.** Dérivé de 4,1-benzoxazépin-2-one représenté par la formule :

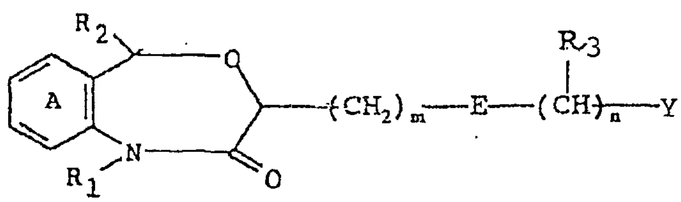

(I)

dans laquelle

$R_1$ représente un groupe alkyle en $C_{1-7}$, sans substituant ou portant un ou des substituants phényle, amino, mercapto, hydroxy ou halogéno ;

$R_2$ représente

a) un atome d'hydrogène,

b) un groupe phényle sans substituant ou portant 1 à 3 substituants choisis parmi

1) les atomes d'halogène,

2) les groupes alkyle en $C_{1-4}$ sans substituant ou portant 1 à 3 substituants halogéno,

3) les groupes alcoxy en $C_{1-4}$ sans substituant ou portant 1 à 3 substituants halogéno,

4) et le groupe hydroxyle, portant ou non un substituant benzyle, auquel cas deux substituants

portés par le groupe phényle en positions adjacentes peuvent constituer ensemble un groupe cyclique méthylènedioxy,

c) ou un groupe thiényle ou pyridyle ;

m vaut 0, 1 ou 2 ;
n vaut 0, 1, 2 ou 3 ;
E représente une liaison, un atome d'oxygène ou de soufre, un groupe sulfonyle ou un groupe de formule $-N(R_4)-$, -NHCO-, $-CON(R_5)-$ ou -NHCONH- ;
$R_3$ et $R_5$ représentent chacun, indépendamment,

a) un atome d'hydrogène,
b) un groupe alkyle en $C_{1-6}$ portant ou non un substituant indolyle,
c) un groupe benzyle,
d) ou un groupe phényle ;

$R_4$ représente un atome d'hydrogène ou un groupe alkyle en $C_{1-4}$, alcanoyle en $C_{1-6}$, alcanesulfonyle en $C_{1-3}$ ou p-toluènesulfonyle ;
Y représente

a) un groupe carboxyle éventuellement estérifié, choisi parmi

1) un groupe carboxyle
2) et un groupe (alcoxy en $C_{1-5}$)carbonyle, lequel groupe (b) peut porter un ou des substituants carboxy,

b) un groupe hydroxyle, portant ou non un substituant alkyle en $C_{1-4}$ ou benzyle,
c) un groupe amino, portant ou non un ou des substituants

1) alkyle en $C_{1-4}$
2) ou cycloalkyle en $C_{3-6}$,
les deux substituants placés sur l'atome d'azote pouvant constituer, conjointement avec cet atome d'azote, un groupe amino cyclique 1-pyrrolidinyle ou 1-pipérazinyle, qui peut éventuellement porter un substituant 4-halogénophényle,

d) un groupe phényle, portant ou non un ou des substituants choisis parmi

1) les groupes alkyle en $C_{1-4}$, sans substituant ou portant un substituant carboxy,
2) le groupe carboxyle,
3) le groupe phényle, sans substituant ou portant un ou des substituants carboxy,
4) et le groupe tétrazol-5-yle,

e) un groupe carbamyle, qui porte ou non un ou deux substituants choisis parmi les groupes alkyle en $C_{1-4}$, benzyle et tétrazol-5-yle, ou dont l'atome d'azote porte deux substituants qui constituent, conjointement avec celui-là, un groupe amino cyclique 1-pyrrolidinyle, 1-pipérazinyle, ou 1-pipérazinyle portant un substituant alkyle en $C_{1-3}$,
f) ou un groupe tétrazol-5-yle ou 2,5-dihydro-5-oxo-1,2,4-oxadiazol-3-yle ; sous réserve que, si m vaut 1, E représente une liaison, n vaut 0 et Y représente un groupe carboxyle ou alcoxycarbonyle, alors $R_1$ représente un groupe alkyle en $C_{5-7}$ ;

et le cycle A porte éventuellement 1 à 3 substituants choisis parmi les atomes d'halogène ;

ou sel d'un tel dérivé, admissible en pharmacie.

**2.** Dérivé de 4,1-benzoxazépin-2-one, conforme à la revendication 1, dans lequel $R_1$ représente un groupe alkyle en $C_{1-7}$.

**3.** Dérivé de 4,1-benzoxazépin-2-one, conforme à la revendication 1, dans lequel $R_1$ représente un groupe alkyle en

$C_{4-5}$.

**4.** Dérivé de 4,1-benzoxazépin-2-one, conforme à la revendication 1, dans lequel $R_2$ représente un groupe phényle éventuellement substitué, tel que défini dans la revendication 1.

**5.** Dérivé de 4,1-benzoxazépin-2-one, conforme à la revendication 1, dans lequel Y représente un groupe carboxyle éventuellement estérifié, tel que défini dans la revendication 1, ou un groupe tétrazol-5-yle ou 2,5-dihydro-5-oxo-1,2,4-oxadiazol-3-yle.

**6.** Dérivé de 4,1-benzoxazépin-2-one, tel que défini dans la revendication 1 et conforme à l'une des revendications 1 à 5, dans lequel, si m vaut 1, E représente une liaison et n vaut 0, alors Y ne représente ni un groupe carboxyle, ni un groupe alcoxycarbonyle.

**7.** Composition pharmaceutique destinée à inhiber une squalène synthétase, laquelle composition contient, en une quantité thérapeutiquement active, un dérivé de 4,1-benzoxazépin-2-one conforme à l'une des revendications 1 à 6 ou un sel d'un tel dérivé, ainsi qu'un véhicule admissible en pharmacie.

**8.** Composition pharmaceutique destinée à inhiber la croissance de champignons, laquelle composition contient, en une quantité thérapeutiquement active, un dérivé de 4,1-benzoxazépin-2-one conforme à l'une des revendications 1 à 6 ou un sel d'un tel dérivé, ainsi qu'un véhicule admissible en pharmacie.